# EUROPEAN PATENT SPECIFICATION

(11) **EP 4 245 314 B1**
(45) Date of publication and mention of the grant of the patent: **15.07.2026**
(21) Application number: 23159088.6
(22) Date of filing: 16.03.2017
(51) Int. Cl.: A61K 38/00, A61K 38/17, A61K 38/39

(54) **COMPOSITIONS FOR USE IN TREATMENT OF TYPE VII COLLAGEN DEFICIENCIES**
ZUSAMMENSETZUNGEN ZUR VERWENDUNG IN DER BEHANDLUNG VON MÄNGELN VON KOLLAGEN TYP VII
COMPOSITIONS POUR L'UTILISATION DANS LE TRAITEMENT DE DÉFICITS EN COLLAGÈNE DE TYPE VII

(30) Priority: 18.03.2016 US 201662310623 P
(43) Date of publication of application: 20.09.2023
(62) Divisional of application: 17767569.1
(73) Proprietor: Innovator 21, LLC, Chicago, IL 60611 (US)
(72) Inventor: DAILEY, Vernon, Walkersville, 21793 (US); CHAKIATH, Marion, Darnestown, 20874 (US); ZHANG, Shyuan, Boyds, 20841 (US); MASLOWSKI, John, Exton, 19341 (US); MALYALA, Anna, Exton, 19341 (US)
(74) Representative: Pohlman, Sandra M.

(56) References cited:
- M P MARINKOVICH ET AL: "Pre-Clinical Development of a Genetically-Modified Human Dermal Fibroblast (FCX-007) for the Treatmentof Recessive Dystrophic Epidermolysis Bullosa", POSTER PRESENTED AT THE AMERICAN SOCIETY OF HUMAN GENETICS ANNUAL MEETING 2015 BALTIMORE, 1 January 2015 (2015-01-01), XP055617051, Retrieved from the Internet <URL:http://fibrocell.com/wp-content/uploads/2015/12/ASHG-2015-poster_RDEB.pdf> [retrieved on 20190830]
- CHRISTOS GEORGIADIS ET AL: "Lentiviral Engineered Fibroblasts Expressing Codon-Optimized COL7A1 Restore Anchoring Fibrils in RDEB", JOURNAL OF INVESTIGATIVE DERMATOLOGY, vol. 136, no. 1, 1 January 2016 (2016-01-01), NL, pages 284 - 292, XP055351934, ISSN: 0022-202X, DOI: 10.1038/JID.2015.364

### SEQUENCE LISTING

<110> Precigen, Inc.
<120> COMPOSITIONS AND METHODS FOR TREATMENT OF TYPE VII COLLAGEN DEFICIENCIES
<130> INX19799PCTEPD1
<140> Not yet assigned
   <141> 2017-03-16
<150> 17 767 569.1
   <151> 2017-03-16
<150> PCT/US2017/022800
   <151> 2017-03-16
<150> 62/310,623
   <151> 2016-03-18
<160> 34
<170> PatentIn version 3.5
<210> 1
   <211> 9169
   <212> DNA
   <213> Homo sapiens
<400> 1
   gatgacgctg cggcttctgg tggccgcgct ctgcgccggg atcctggcag aggcgccccg 60
   agtgcgagcc cagcacaggg agagagtgac ctgcacgcgc ctttacgccg ctgacattgt 120
   gttcttactg gatggctcct catccattgg ccgcagcaat ttccgcgagg tccgcagctt 180
   tctcgaaggg ctggtgctgc ctttctctgg agcagccagt gcacagggtg tgcgctttgc 240
   cacagtgcag tacagcgatg acccacggac agagttcggc ctggatgcac ttggctctgg 300
   gggtgatgtg atccgcgcca tccgtgagct tagctacaag gggggcaaca ctcgcacagg 360
   ggctgcaatt ctccatgtgg ctgaccatgt cttcctgccc cagctggccc gacctggtgt 420
   ccccaaggtc tgcatcctga tcacagacgg gaagtcccag gacctggtgg acacagctgc 480
   ccaaaggctg aaggggcagg gggtcaagct atttgctgtg gggatcaaga atgctgaccc 540
   tgaggagctg aagcgagttg cctcacagcc caccagtgac ttcttcttct tcgtcaatga 600
   cttcagcatc ttgaggacac tactgcccct cgtttcccgg agagtgtgca cgactgctgg 660
   tggcgtgcct gtgacccgac ctccggatga ctcgacctct gctccacgag acctggtgct 720
   gtctgagcca agcagccaat ccttgagagt acagtggaca gcggccagtg gccctgtgac 780
   tggctacaag gtccagtaca ctcctctgac ggggctggga cagccactgc cgagtgagcg      840
   gcaggaggtg aacgtcccag ctggtgagac cagtgtgcgg ctgcggggtc tccggccact      900
   gaccgagtac caagtgactg tgattgccct ctacgccaac agcatcgggg aggctgtgag      960
   cgggacagct cggaccactg ccctagaagg gccggaactg accatccaga ataccacagc     1020
   ccacagcctc ctggtggcct ggcggagtgt gccaggtgcc actggctacc gtgtgacatg     1080
   gcgggtcctc agtggtgggc ccacacagca gcaggagctg ggccctgggc agggttcagt     1140
   gttgctgcgt gacttggagc ctggcacgga ctatgaggtg accgtgagca ccctatttgg     1200
   ccgcagtgtg gggcccgcca cttccctgat ggctcgcact gacgcttctg ttgagcagac     1260
   cctgcgcccg gtcatcctgg gccccacatc catcctcctt tcctggaact tggtgcctga     1320
   ggcccgtggc taccggttgg aatggcggcg tgagactggc ttggagccac cgcagaaggt     1380
   ggtactgccc tctgatgtga cccgctacca gttggatggg ctgcagccgg gcactgagta     1440
   ccgcctcaca ctctacactc tgctggaggg ccacgaggtg gccacccctg caaccgtggt     1500
   tcccactgga ccagagctgc ctgtgagccc tgtaacagac ctgcaagcca ccgagctgcc     1560
   cgggcagcgg gtgcgagtgt cctggagccc agtccctggt gccacccagt accgcatcat     1620
   tgtgcgcagc acccaggggg ttgagcggac cctggtgctt cctgggagtc agacagcatt     1680
   cgacttggat gacgttcagg ctgggcttag ctacactgtg cgggtgtctg ctcgagtggg     1740
   tccccgtgag ggcagtgcca gtgtcctcac tgtccgccgg gagccggaaa ctccacttgc     1800
   tgttccaggg ctgcgggttg tggtgtcaga tgcaacgcga gtgagggtgg cctggggacc     1860
   cgtccctgga gccagtggat ttcggattag ctggagcaca ggcagtggtc cggagtccag     1920
   ccagacactg cccccagact ctactgccac agacatcaca gggctgcagc ctggaaccac     1980
   ctaccaggtg gctgtgtcgg tactgcgagg cagagaggag ggccctgctg cagtcatcgt     2040
   ggctcgaacg gacccactgg gcccagtgag gacggtccat gtgactcagg ccagcagctc     2100
   atctgtcacc attacctgga ccagggttcc tggcgccaca ggatacaggg tttcctggca     2160
   ctcagcccac ggcccagaga aatcccagtt ggtttctggg gaggccacgg tggctgagct     2220
   ggatggactg gagccagata ctgagtatac ggtgcatgtg agggcccatg tggctggcgt     2280
   ggatgggccc cctgcctctg tggttgtgag gactgcccct gagcctgtgg gtcgtgtgtc     2340
   gaggctgcag atcctcaatg cttccagcga cgttctacgg atcacctggg taggggtcac     2400
   tggagccaca gcttacagac tggcctgggg ccggagtgaa ggcggcccca tgaggcacca     2460
   gatactccca ggaaacacag actctgcaga gatccggggt ctcgaaggtg gagtcagcta 2520
   ctcagtgcga gtgactgcac ttgtcgggga ccgcgagggc acacctgtct ccattgttgt 2580
   cactacgccg cctgaggctc cgccagccct ggggacgctt cacgtggtgc agcgcgggga 2640
   gcactcgctg aggctgcgct gggagccggt gcccagagcg cagggcttcc ttctgcactg 2700
   gcaacctgag ggtggccagg aacagtcccg ggtcctgggg cccgagctca gcagctatca 2760
   cctggacggg ctggagccag cgacacagta ccgcgtgagg ctgagtgtcc tagggccagc 2820
   tggagaaggg ccctctgcag aggtgactgc gcgcactgag tcacctcgtg ttccaagcat 2880
   tgaactacgt gtggtggaca cctcgatcga ctcggtgact ttggcctgga ctccagtgtc 2940
   cagggcatcc agctacatcc tatcctggcg gccactcaga ggccctggcc aggaagtgcc 3000
   tgggtccccg cagacacttc cagggatctc aagctcccag cgggtgacag ggctagagcc 3060
   tggcgtctct tacatcttct ccctgacgcc tgtcctggat ggtgtgcggg gtcctgaggc 3120
   atctgtcaca cagacgccag tgtgcccccg tggcctggcg gatgtggtgt tcctaccaca 3180
   tgccactcaa gacaatgctc accgtgcgga ggctacgagg agggtcctgg agcgtctggt 3240
   gttggcactt gggcctcttg ggccacaggc agttcaggtt ggcctgctgt cttacagtca 3300
   tcggccctcc ccactgttcc cactgaatgg ctcccatgac cttggcatta tcttgcaaag 3360
   gatccgtgac atgccctaca tggacccaag tgggaacaac ctgggcacag ccgtggtcac 3420
   agctcacaga tacatgttgg caccagatgc tcctgggcgc cgccagcacg taccaggggt 3480
   gatggttctg ctagtggatg aacccttgag aggtgacata ttcagcccca tccgtgaggc 3540
   ccaggcttct gggcttaatg tggtgatgtt gggaatggct ggagcggacc cagagcagct 3600
   gcgtcgcttg gcgccgggta tggactctgt ccagaccttc ttcgccgtgg atgatgggcc 3660
   aagcctggac caggcagtca gtggtctggc cacagccctg tgtcaggcat ccttcactac 3720
   tcagccccgg ccagagccct gcccagtgta ttgtccaaag ggccagaagg gggaacctgg 3780
   agagatgggc ctgagaggac aagttgggcc tcctggcgac cctggcctcc cgggcaggac 3840
   cggtgctccc ggcccccagg ggccccctgg aagtgccact gccaagggcg agaggggctt 3900
   ccctggagca gatgggcgtc caggcagccc tggccgcgcc gggaatcctg ggacccctgg 3960
   agcccctggc ctaaagggct ctccagggtt gcctggccct cgtggggacc cgggagagcg 4020
   aggacctcga ggcccaaagg gggagccggg ggctcccgga caagtcatcg gaggtgaagg 4080
   acctgggctt cctgggcgga aaggggaccc tggaccatcg ggcccccctg gacctcgtgg 4140
   accactgggg gacccaggac cccgtggccc cccagggctt cctggaacag ccatgaaggg 4200
   tgacaaaggc gatcgtgggg agcggggtcc ccctggacca ggtgaaggtg gcattgctcc 4260
   tggggagcct gggctgccgg gtcttcccgg aagccctgga ccccaaggcc ccgttggccc 4320
   ccctggaaag aaaggagaaa aaggtgactc tgaggatgga gctccaggcc tcccaggaca 4380
   acctgggtct ccgggtgagc agggcccacg gggacctcct ggagctattg gccccaaagg 4440
   tgaccggggc tttccagggc ccctgggtga ggctggagag aagggcgaac gtggaccccc 4500
   aggcccagcg ggatcccggg ggctgccagg ggttgctgga cgtcctggag ccaagggtcc 4560
   tgaagggcca ccaggaccca ctggccgcca aggagagaag ggggagcctg gtcgccctgg 4620
   ggaccctgca gtggtgggac ctgctgttgc tggacccaaa ggagaaaagg gagatgtggg 4680
   gcccgctggg cccagaggag ctaccggagt ccaaggggaa cggggcccac ccggcttggt 4740
   tcttcctgga gaccctggcc ccaagggaga ccctggagac cggggtccca ttggccttac 4800
   tggcagagca ggacccccag gtgactcagg gcctcctgga gagaagggag accctgggcg 4860
   gcctggcccc ccaggacctg ttggcccccg aggacgagat ggtgaagttg gagagaaagg 4920
   tgacgagggt cctccgggtg acccgggttt gcctggaaaa gcaggcgagc gtggccttcg 4980
   gggggcacct ggagttcggg ggcctgtggg tgaaaaggga gaccagggag atcctggaga 5040
   ggatggacga aatggcagcc ctggatcatc tggacccaag ggtgaccgtg ggagccgggg 5100
   tcccccagga cccccgggac ggctggtaga cacaggacct ggagccagag agaagggaga 5160
   gcctggggac cgcggacaag agggtcctcg agggcccaag ggtgatcctg gcctccctgg 5220
   agcccctggg gaaaggggca ttgaagggtt tcggggaccc ccaggcccac agggggaccc 5280
   aggtgtccga ggcccagcag gagaaaaggg tgaccggggt ccccctgggc tggatggccg 5340
   gagcggactg gatgggaaac caggagccgc tgggccctct gggccgaatg gtgctgcagg 5400
   caaagctggg gacccaggga gagacgggct tccaggcctc cgtggagaac agggcctccc 5460
   tggcccctct ggtccccctg gattaccggg aaagccaggc gaggatggca aacctggcct 5520
   gaatggaaaa aacggagaac ctggggaccc tggagaagac gggaggaagg gagagaaagg 5580
   agattcaggc gcctctggga gagaaggtcg tgatggcccc aagggtgagc gtggagctcc 5640
   tggtatcctt ggaccccagg ggcctccagg cctcccaggg ccagtgggcc ctcctggcca 5700
   gggttttcct ggtgtcccag gaggcacggg ccccaagggt gaccgtgggg agactggatc 5760
   caaaggggag cagggcctcc ctggagagcg tggcctgcga ggagagcctg gaagtgtgcc 5820
   gaatgtggat cggttgctgg aaactgctgg catcaaggca tctgccctgc gggagatcgt 5880
   ggagacctgg gatgagagct ctggtagctt cctgcctgtg cccgaacggc gtcgaggccc 5940
   caagggggac tcaggcgaac agggcccccc aggcaaggag ggccccatcg gctttcctgg 6000
   agaacgcggg ctgaagggcg accgtggaga ccctggccct caggggccac ctggtctggc 6060
   ccttggggag aggggccccc ccgggccttc cggccttgcc ggggagcctg gaaagcctgg 6120
   tattcccggg ctcccaggca gggctggggg tgtgggagag gcaggaaggc caggagagag 6180
   gggagaacgg ggagagaaag gagaacgtgg agaacagggc agagatggcc ctcctggact 6240
   ccctggaacc cctgggcccc ccggaccccc tggccccaag gtgtctgtgg atgagccagg 6300
   tcctggactc tctggagaac agggaccccc tggactcaag ggtgctaagg gggagccggg 6360
   cagcaatggt gaccaaggtc ccaaaggaga caggggtgtg ccaggcatca aaggagaccg 6420
   gggagagcct ggaccgaggg gtcaggacgg caacccgggt ctaccaggag agcgtggtat 6480
   ggctgggcct gaagggaagc cgggtctgca gggtccaaga ggcccccctg gcccagtggg 6540
   tggtcatgga gaccctggac cacctggtgc cccgggtctt gctggccctg caggacccca 6600
   aggaccttct ggcctgaagg gggagcctgg agagacagga cctccaggac ggggcctgac 6660
   tggacctact ggagctgtgg gacttcctgg accccccggc ccttcaggcc ttgtgggtcc 6720
   acaggggtct ccaggtttgc ctggacaagt gggggagaca gggaagccgg gagccccagg 6780
   tcgagatggt gccagtggaa aagatggaga cagagggagc cctggtgtgc cagggtcacc 6840
   aggtctgcct ggccctgtcg gacctaaagg agaacctggc cccacggggg cccctggaca 6900
   ggctgtggtc gggctccctg gagcaaaggg agagaaggga gcccctggag gccttgctgg 6960
   agacctggtg ggtgagccgg gagccaaagg tgaccgagga ctgccagggc cgcgaggcga 7020
   gaagggtgaa gctggccgtg caggggagcc cggagaccct ggggaagatg gtcagaaagg 7080
   ggctccagga cccaaaggtt tcaagggtga cccaggagtc ggggtcccgg gctcccctgg 7140
   gcctcctggc cctccaggtg tgaagggaga tctgggcctc cctggcctgc ccggtgctcc 7200
   tggtgttgtt gggttcccgg gtcagacagg ccctcgagga gagatgggtc agccaggccc 7260
   tagtggagag cggggtctgg caggcccccc agggagagaa ggaatcccag gacccctggg 7320
   gccacctgga ccaccggggt cagtgggacc acctggggcc tctggactca aaggagacaa 7380
   gggagaccct ggagtagggc tgcctgggcc ccgaggcgag cgtggggagc caggcatccg 7440
   gggtgaagat ggccgccccg gccaggaggg accccgagga ctcacggggc cccctggcag 7500
   caggggagag cgtggggaga agggtgatgt tgggagtgca ggactaaagg gtgacaaggg 7560
   agactcagct gtgatcctgg ggcctccagg cccacggggt gccaaggggg acatgggtga 7620
   acgagggcct cggggcttgg atggtgacaa aggacctcgg ggagacaatg gggaccctgg 7680
   tgacaagggc agcaagggag agcctggtga caagggctca gccgggttgc caggactgcg 7740
   tggactcctg ggaccccagg gtcaacctgg tgcagcaggg atccctggtg acccgggatc 7800
   cccaggaaag gatggagtgc ctggtatccg aggagaaaaa ggagatgttg gcttcatggg 7860
   tccccggggc ctcaagggtg aacggggagt gaagggagcc tgtggccttg atggagagaa 7920
   gggagacaag ggagaagctg gtcccccagg ccgccccggg ctggcaggac acaaaggaga 7980
   gatgggggag cctggtgtgc cgggccagtc gggggcccct ggcaaggagg gcctgatcgg 8040
   tcccaagggt gaccgaggct ttgacgggca gccaggcccc aagggtgacc agggcgagaa 8100
   aggggagcgg ggaaccccag gaattggggg cttcccaggc cccagtggaa atgatggctc 8160
   tgctggtccc ccagggccac ctggcagtgt tggtcccaga ggccccgaag gacttcaggg 8220
   ccagaagggt gagcgaggtc cccccggaga gagagtggtg ggggctcctg ggtccctggg 8280
   agctcctggc gagagagggg agcaggggcg gccagggcct gccggtcctc gaggcgagaa 8340
   gggagaagct gcactgacgg aggatgacat ccggggcttt gtgcgccaag agatgagtca 8400
   gcactgtgcc tgccagggcc agttcatcgc atctggatca cgacccctcc ctagttatgc 8460
   tgcagacact gccggctccc agctccatgc tgtgcctgtg ctccgcgtct ctcatgcaga 8520
   ggaggaagag cgggtacccc ctgaggatga tgagtactct gaatactccg agtattctgt 8580
   ggaggagtac caggaccctg aagctccttg ggatagtgat gacccctgtt ccctgccact 8640
   ggatgagggc tcctgcactg cctacaccct gcgctggtac catcgggctg tgacaggcag 8700
   cacagaggcc tgtcaccctt ttgtctatgg tggctgtgga gggaatgcca accgttttgg 8760
   gacccgtgag gcctgcgagc gccgctgccc accccgggtg gtccagagcc aggggacagg 8820
   tactgcccag gactgaggcc cagataatga gctgagattc agcatcccct ggaggagtcg 8880
   gggtctcagc agaaccccac tgtccctccc cttggtgcta gaggcttgtg tgcacgtgag 8940
   cgtgcgtgtg cacgtccgtt atttcagtga cttggtcccg tgggtctagc cttcccccct 9000
   gtggacaaac ccccattgtg gctcctgcca ccctggcaga tgactcactg tgggggggtg 9060
   gctgtgggca gtgagcggat gtgactggcg tctgacccgc cccttgaccc aagcctgtga 9120
   tgacatggtg ctgattctgg ggggcattaa agctgctgtt ttaaaaggc 9169
<210> 2
   <211> 2944
   <212> PRT
   <213> Homo sapiens
<400> 2
   Met Thr Leu Arg Leu Leu Val Ala Ala Leu Cys Ala Gly Ile Leu Ala
   1               5                   10                  15
   Glu Ala Pro Arg Val Arg Ala Gln His Arg Glu Arg Val Thr Cys Thr
               20                  25                  30
   Arg Leu Tyr Ala Ala Asp Ile Val Phe Leu Leu Asp Gly Ser Ser Ser
           35                  40                  45
   Ile Gly Arg Ser Asn Phe Arg Glu Val Arg Ser Phe Leu Glu Gly Leu
       50                  55                  60
   Val Leu Pro Phe Ser Gly Ala Ala Ser Ala Gln Gly Val Arg Phe Ala
   65                  70                  75                  80
   Thr Val Gln Tyr Ser Asp Asp Pro Arg Thr Glu Phe Gly Leu Asp Ala
                   85                  90                  95
   Leu Gly Ser Gly Gly Asp Val Ile Arg Ala Ile Arg Glu Leu Ser Tyr
               100                 105                 110
   Lys Gly Gly Asn Thr Arg Thr Gly Ala Ala Ile Leu His Val Ala Asp
           115                 120                 125
   His Val Phe Leu Pro Gln Leu Ala Arg Pro Gly Val Pro Lys Val Cys
       130                 135                 140
   Ile Leu Ile Thr Asp Gly Lys Ser Gln Asp Leu Val Asp Thr Ala Ala
   145                 150                 155                 160
   Gln Arg Leu Lys Gly Gln Gly Val Lys Leu Phe Ala Val Gly Ile Lys
                   165                 170                 175
   Asn Ala Asp Pro Glu Glu Leu Lys Arg Val Ala Ser Gln Pro Thr Ser
               180                 185                 190
   Asp Phe Phe Phe Phe Val Asn Asp Phe Ser Ile Leu Arg Thr Leu Leu
           195                 200                 205
   Pro Leu Val Ser Arg Arg Val Cys Thr Thr Ala Gly Gly Val Pro Val
       210                 215                 220
   Thr Arg Pro Pro Asp Asp Ser Thr Ser Ala Pro Arg Asp Leu Val Leu
   225                 230                 235                 240
   Ser Glu Pro Ser Ser Gln Ser Leu Arg Val Gln Trp Thr Ala Ala Ser
                   245                 250                 255
   Gly Pro Val Thr Gly Tyr Lys Val Gln Tyr Thr Pro Leu Thr Gly Leu
               260                 265                 270
   Gly Gln Pro Leu Pro Ser Glu Arg Gln Glu Val Asn Val Pro Ala Gly
           275                 280                 285
   Glu Thr Ser Val Arg Leu Arg Gly Leu Arg Pro Leu Thr Glu Tyr Gln
       290                 295                 300
   Val Thr Val Ile Ala Leu Tyr Ala Asn Ser Ile Gly Glu Ala Val Ser
   305                 310                 315                 320
   Gly Thr Ala Arg Thr Thr Ala Leu Glu Gly Pro Glu Leu Thr Ile Gln
                   325                 330                 335
   Asn Thr Thr Ala His Ser Leu Leu Val Ala Trp Arg Ser Val Pro Gly
               340                 345                 350
   Ala Thr Gly Tyr Arg Val Thr Trp Arg Val Leu Ser Gly Gly Pro Thr
           355                 360                 365
   Gln Gln Gln Glu Leu Gly Pro Gly Gln Gly Ser Val Leu Leu Arg Asp
       370                 375                 380
   Leu Glu Pro Gly Thr Asp Tyr Glu Val Thr Val Ser Thr Leu Phe Gly
   385                 390                 395                 400
   Arg Ser Val Gly Pro Ala Thr Ser Leu Met Ala Arg Thr Asp Ala Ser
                   405                 410                 415
   Val Glu Gln Thr Leu Arg Pro Val Ile Leu Gly Pro Thr Ser Ile Leu
               420                 425                 430
   Leu Ser Trp Asn Leu Val Pro Glu Ala Arg Gly Tyr Arg Leu Glu Trp
           435                 440                 445
   Arg Arg Glu Thr Gly Leu Glu Pro Pro Gln Lys Val Val Leu Pro Ser
       450                 455                 460
   Asp Val Thr Arg Tyr Gln Leu Asp Gly Leu Gln Pro Gly Thr Glu Tyr
   465                 470                 475                 480
   Arg Leu Thr Leu Tyr Thr Leu Leu Glu Gly His Glu Val Ala Thr Pro
                   485                 490                 495
   Ala Thr Val Val Pro Thr Gly Pro Glu Leu Pro Val Ser Pro Val Thr
               500                 505                 510
   Asp Leu Gln Ala Thr Glu Leu Pro Gly Gln Arg Val Arg Val Ser Trp
           515                 520                 525
   Ser Pro Val Pro Gly Ala Thr Gln Tyr Arg Ile Ile Val Arg Ser Thr
       530                 535                 540
   Gln Gly Val Glu Arg Thr Leu Val Leu Pro Gly Ser Gln Thr Ala Phe
   545                 550                 555                 560
   Asp Leu Asp Asp Val Gln Ala Gly Leu Ser Tyr Thr Val Arg Val Ser
                   565                 570                 575
   Ala Arg Val Gly Pro Arg Glu Gly Ser Ala Ser Val Leu Thr Val Arg
               580                 585                 590
   Arg Glu Pro Glu Thr Pro Leu Ala Val Pro Gly Leu Arg Val Val Val
           595                 600                 605
   Ser Asp Ala Thr Arg Val Arg Val Ala Trp Gly Pro Val Pro Gly Ala
       610                 615                 620
   Ser Gly Phe Arg Ile Ser Trp Ser Thr Gly Ser Gly Pro Glu Ser Ser
   625                 630                 635                 640
   Gln Thr Leu Pro Pro Asp Ser Thr Ala Thr Asp Ile Thr Gly Leu Gln
                   645                 650                 655
   Pro Gly Thr Thr Tyr Gln Val Ala Val Ser Val Leu Arg Gly Arg Glu
               660                 665                 670
   Glu Gly Pro Ala Ala Val Ile Val Ala Arg Thr Asp Pro Leu Gly Pro
           675                 680                 685
   Val Arg Thr Val His Val Thr Gln Ala Ser Ser Ser Ser Val Thr Ile
       690                 695                 700
   Thr Trp Thr Arg Val Pro Gly Ala Thr Gly Tyr Arg Val Ser Trp His
   705                 710                 715                 720
   Ser Ala His Gly Pro Glu Lys Ser Gln Leu Val Ser Gly Glu Ala Thr
                   725                 730                 735
   Val Ala Glu Leu Asp Gly Leu Glu Pro Asp Thr Glu Tyr Thr Val His
               740                 745                 750
   Val Arg Ala His Val Ala Gly Val Asp Gly Pro Pro Ala Ser Val Val
           755                 760                 765
   Val Arg Thr Ala Pro Glu Pro Val Gly Arg Val Ser Arg Leu Gln Ile
       770                 775                 780
   Leu Asn Ala Ser Ser Asp Val Leu Arg Ile Thr Trp Val Gly Val Thr
   785                 790                 795                 800
   Gly Ala Thr Ala Tyr Arg Leu Ala Trp Gly Arg Ser Glu Gly Gly Pro
                   805                 810                 815
   Met Arg His Gln Ile Leu Pro Gly Asn Thr Asp Ser Ala Glu Ile Arg
               820                 825                 830
   Gly Leu Glu Gly Gly Val Ser Tyr Ser Val Arg Val Thr Ala Leu Val
           835                 840                 845
   Gly Asp Arg Glu Gly Thr Pro Val Ser Ile Val Val Thr Thr Pro Pro
       850                 855                 860
   Glu Ala Pro Pro Ala Leu Gly Thr Leu His Val Val Gln Arg Gly Glu
   865                 870                 875                 880
   His Ser Leu Arg Leu Arg Trp Glu Pro Val Pro Arg Ala Gln Gly Phe
                   885                 890                 895
   Leu Leu His Trp Gln Pro Glu Gly Gly Gln Glu Gln Ser Arg Val Leu
               900                 905                 910
   Gly Pro Glu Leu Ser Ser Tyr His Leu Asp Gly Leu Glu Pro Ala Thr
           915                 920                 925
   Gln Tyr Arg Val Arg Leu Ser Val Leu Gly Pro Ala Gly Glu Gly Pro
       930                 935                 940
   Ser Ala Glu Val Thr Ala Arg Thr Glu Ser Pro Arg Val Pro Ser Ile
   945                 950                 955                 960
   Glu Leu Arg Val Val Asp Thr Ser Ile Asp Ser Val Thr Leu Ala Trp
                   965                 970                 975
   Thr Pro Val Ser Arg Ala Ser Ser Tyr Ile Leu Ser Trp Arg Pro Leu
               980                 985                 990
   Arg Gly Pro Gly Gln Glu Val Pro  Gly Ser Pro Gln Thr  Leu Pro Gly
           995                 1000                1005
   Ile Ser  Ser Ser Gln Arg Val  Thr Gly Leu Glu Pro  Gly Val Ser
       1010                1015                1020
   Tyr Ile  Phe Ser Leu Thr Pro  Val Leu Asp Gly Val  Arg Gly Pro
       1025                1030                1035
   Glu Ala  Ser Val Thr Gln Thr  Pro Val Cys Pro Arg  Gly Leu Ala
       1040                1045                1050
   Asp Val  Val Phe Leu Pro His  Ala Thr Gln Asp Asn  Ala His Arg
       1055                1060                1065
   Ala Glu  Ala Thr Arg Arg Val  Leu Glu Arg Leu Val  Leu Ala Leu
       1070                1075                1080
   Gly Pro  Leu Gly Pro Gln Ala  Val Gln Val Gly Leu  Leu Ser Tyr
       1085                 1090                 1095
   Ser His  Arg Pro Ser Pro Leu  Phe Pro Leu Asn Gly  Ser His Asp
       1100                 1105                 1110
   Leu Gly  Ile Ile Leu Gln Arg  Ile Arg Asp Met Pro  Tyr Met Asp
       1115                 1120                 1125
   Pro Ser  Gly Asn Asn Leu Gly  Thr Ala Val Val Thr  Ala His Arg
       1130                 1135                 1140
   Tyr Met  Leu Ala Pro Asp Ala  Pro Gly Arg Arg Gln  His Val Pro
       1145                 1150                 1155
   Gly Val  Met Val Leu Leu Val  Asp Glu Pro Leu Arg  Gly Asp Ile
       1160                 1165                 1170
   Phe Ser  Pro Ile Arg Glu Ala  Gln Ala Ser Gly Leu  Asn Val Val
       1175                 1180                 1185
   Met Leu  Gly Met Ala Gly Ala  Asp Pro Glu Gln Leu  Arg Arg Leu
       1190                 1195                 1200
   Ala Pro  Gly Met Asp Ser Val  Gln Thr Phe Phe Ala  Val Asp Asp
       1205                 1210                 1215
   Gly Pro  Ser Leu Asp Gln Ala  Val Ser Gly Leu Ala  Thr Ala Leu
       1220                 1225                 1230
   Cys Gln  Ala Ser Phe Thr Thr  Gln Pro Arg Pro Glu  Pro Cys Pro
       1235                 1240                 1245
   Val Tyr  Cys Pro Lys Gly Gln  Lys Gly Glu Pro Gly  Glu Met Gly
       1250                 1255                 1260
   Leu Arg  Gly Gln Val Gly Pro  Pro Gly Asp Pro Gly  Leu Pro Gly
       1265                 1270                 1275
   Arg Thr  Gly Ala Pro Gly Pro  Gln Gly Pro Pro Gly  Ser Ala Thr
       1280                 1285                 1290
   Ala Lys Gly Glu Arg Gly Phe Pro Gly Ala Asp Gly Arg Pro Gly
   1295 1300 1305
   Ser Pro Gly Arg Ala Gly Asn Pro Gly Thr Pro Gly Ala Pro Gly
   1310 1315 1320
   Leu Lys Gly Ser Pro Gly Leu Pro Gly Pro Arg Gly Asp Pro Gly
   1325 1330 1335
   Glu Arg Gly Pro Arg Gly Pro Lys Gly Glu Pro Gly Ala Pro Gly
   1340 1345 1350
   Gln Val Ile Gly Gly Glu Gly Pro Gly Leu Pro Gly Arg Lys Gly
   1355 1360 1365
   Asp Pro Gly Pro Ser Gly Pro Pro Gly Pro Arg Gly Pro Leu Gly
   1370 1375 1380
   Asp Pro Gly Pro Arg Gly Pro Pro Gly Leu Pro Gly Thr Ala Met
   1385 1390 1395
   Lys Gly Asp Lys Gly Asp Arg Gly Glu Arg Gly Pro Pro Gly Pro
   1400 1405 1410
   Gly Glu Gly Gly Ile Ala Pro Gly Glu Pro Gly Leu Pro Gly Leu
   1415 1420 1425
   Pro Gly Ser Pro Gly Pro Gln Gly Pro Val Gly Pro Pro Gly Lys
   1430 1435 1440
   Lys Gly Glu Lys Gly Asp Ser Glu Asp Gly Ala Pro Gly Leu Pro
   1445 1450 1455
   Gly Gln Pro Gly Ser Pro Gly Glu Gln Gly Pro Arg Gly Pro Pro
   1460 1465 1470
   Gly Ala Ile Gly Pro Lys Gly Asp Arg Gly Phe Pro Gly Pro Leu
   1475 1480 1485
   Gly Glu Ala Gly Glu Lys Gly Glu Arg Gly Pro Pro Gly Pro Ala
   1490 1495 1500
   Gly Ser Arg Gly Leu Pro Gly Val Ala Gly Arg Pro Gly Ala Lys
   1505 1510 1515
   Gly Pro Glu Gly Pro Pro Gly Pro Thr Gly Arg Gln Gly Glu Lys
   1520 1525 1530
   Gly Glu Pro Gly Arg Pro Gly Asp Pro Ala Val Val Gly Pro Ala
   1535 1540 1545
   Val Ala Gly Pro Lys Gly Glu Lys Gly Asp Val Gly Pro Ala Gly
   1550 1555 1560
   Pro Arg Gly Ala Thr Gly Val Gln Gly Glu Arg Gly Pro Pro Gly
   1565 1570 1575
   Leu Val Leu Pro Gly Asp Pro Gly Pro Lys Gly Asp Pro Gly Asp
   1580 1585 1590
   Arg Gly Pro Ile Gly Leu Thr Gly Arg Ala Gly Pro Pro Gly Asp
   1595 1600 1605
   Ser Gly Pro Pro Gly Glu Lys Gly Asp Pro Gly Arg Pro Gly Pro
   1610 1615 1620
   Pro Gly Pro Val Gly Pro Arg Gly Arg Asp Gly Glu Val Gly Glu
   1625 1630 1635
   Lys Gly Asp Glu Gly Pro Pro Gly Asp Pro Gly Leu Pro Gly Lys
   1640 1645 1650
   Ala Gly Glu Arg Gly Leu Arg Gly Ala Pro Gly Val Arg Gly Pro
   1655 1660 1665
   Val Gly Glu Lys Gly Asp Gln Gly Asp Pro Gly Glu Asp Gly Arg
   1670 1675 1680
   Asn Gly Ser Pro Gly Ser Ser Gly Pro Lys Gly Asp Arg Gly Glu
   1685 1690 1695
   Pro Gly Pro Pro Gly Pro Pro Gly Arg Leu Val Asp Thr Gly Pro
   1700 1705 1710
   Gly Ala  Arg Glu Lys Gly Glu  Pro Gly Asp Arg Gly  Gln Glu Gly
       1715                 1720                 1725
   Pro Arg  Gly Pro Lys Gly Asp  Pro Gly Leu Pro Gly  Ala Pro Gly
       1730                 1735                 1740
   Glu Arg  Gly Ile Glu Gly Phe  Arg Gly Pro Pro Gly  Pro Gln Gly
       1745                 1750                 1755
   Asp Pro  Gly Val Arg Gly Pro  Ala Gly Glu Lys Gly  Asp Arg Gly
       1760                 1765                 1770
   Pro Pro  Gly Leu Asp Gly Arg  Ser Gly Leu Asp Gly  Lys Pro Gly
       1775                 1780                 1785
   Ala Ala  Gly Pro Ser Gly Pro  Asn Gly Ala Ala Gly  Lys Ala Gly
       1790                 1795                 1800
   Asp Pro  Gly Arg Asp Gly Leu  Pro Gly Leu Arg Gly  Glu Gln Gly
       1805                 1810                 1815
   Leu Pro  Gly Pro Ser Gly Pro  Pro Gly Leu Pro Gly  Lys Pro Gly
       1820                 1825                 1830
   Glu Asp  Gly Lys Pro Gly Leu  Asn Gly Lys Asn Gly  Glu Pro Gly
       1835                 1840                 1845
   Asp Pro  Gly Glu Asp Gly Arg  Lys Gly Glu Lys Gly  Asp Ser Gly
       1850                 1855                 1860
   Ala Ser  Gly Arg Glu Gly Arg  Asp Gly Pro Lys Gly  Glu Arg Gly
       1865                 1870                 1875
   Ala Pro  Gly Ile Leu Gly Pro  Gln Gly Pro Pro Gly  Leu Pro Gly
       1880                 1885                 1890
   Pro Val  Gly Pro Pro Gly Gln  Gly Phe Pro Gly Val  Pro Gly Gly
       1895                 1900                 1905
   Thr Gly  Pro Lys Gly Asp Arg  Gly Glu Thr Gly Ser  Lys Gly Glu
       1910                 1915                 1920
   Gln Gly Leu Pro Gly Glu Arg Gly Leu Arg Gly Glu Pro Gly Ser
       1925                1930                1935
   Val Pro Asn Val Asp Arg Leu Leu Glu Thr Ala Gly Ile Lys Ala
       1940                1945                1950
   Ser Ala Leu Arg Glu Ile Val Glu Thr Trp Asp Glu Ser Ser Gly
       1955                1960                1965
   Ser Phe Leu Pro Val Pro Glu Arg Arg Arg Gly Pro Lys Gly Asp
       1970                1975                1980
   Ser Gly Glu Gln Gly Pro Pro Gly Lys Glu Gly Pro Ile Gly Phe
       1985                1990                1995
   Pro Gly Glu Arg Gly Leu Lys Gly Asp Arg Gly Asp Pro Gly Pro
       2000                2005                2010
   Gln Gly Pro Pro Gly Leu Ala Leu Gly Glu Arg Gly Pro Pro Gly
       2015                2020                2025
   Pro Ser Gly Leu Ala Gly Glu Pro Gly Lys Pro Gly Ile Pro Gly
       2030                2035                2040
   Leu Pro Gly Arg Ala Gly Gly Val Gly Glu Ala Gly Arg Pro Gly
       2045                2050                2055
   Glu Arg Gly Glu Arg Gly Glu Lys Gly Glu Arg Gly Glu Gln Gly
       2060                2065                2070
   Arg Asp Gly Pro Pro Gly Leu Pro Gly Thr Pro Gly Pro Pro Gly
       2075                2080                2085
   Pro Pro Gly Pro Lys Val Ser Val Asp Glu Pro Gly Pro Gly Leu
       2090                2095                2100
   Ser Gly Glu Gln Gly Pro Pro Gly Leu Lys Gly Ala Lys Gly Glu
       2105                2110                2115
   Pro Gly Ser Asn Gly Asp Gln Gly Pro Lys Gly Asp Arg Gly Val
       2120                2125                2130
   Pro Gly  Ile Lys Gly Asp Arg  Gly Glu Pro Gly Pro  Arg Gly Gln
       2135                 2140                 2145
   Asp Gly  Asn Pro Gly Leu Pro  Gly Glu Arg Gly Met  Ala Gly Pro
       2150                 2155                 2160
   Glu Gly  Lys Pro Gly Leu Gln  Gly Pro Arg Gly Pro  Pro Gly Pro
       2165                 2170                 2175
   Val Gly  Gly His Gly Asp Pro  Gly Pro Pro Gly Ala  Pro Gly Leu
       2180                 2185                 2190
   Ala Gly  Pro Ala Gly Pro Gln  Gly Pro Ser Gly Leu  Lys Gly Glu
       2195                 2200                 2205
   Pro Gly  Glu Thr Gly Pro Pro  Gly Arg Gly Leu Thr  Gly Pro Thr
       2210                 2215                 2220
   Gly Ala  Val Gly Leu Pro Gly  Pro Pro Gly Pro Ser  Gly Leu Val
       2225                 2230                 2235
   Gly Pro  Gln Gly Ser Pro Gly  Leu Pro Gly Gln Val  Gly Glu Thr
       2240                 2245                 2250
   Gly Lys  Pro Gly Ala Pro Gly  Arg Asp Gly Ala Ser  Gly Lys Asp
       2255                 2260                 2265
   Gly Asp  Arg Gly Ser Pro Gly  Val Pro Gly Ser Pro  Gly Leu Pro
       2270                 2275                 2280
   Gly Pro  Val Gly Pro Lys Gly  Glu Pro Gly Pro Thr  Gly Ala Pro
       2285                 2290                 2295
   Gly Gln  Ala Val Val Gly Leu  Pro Gly Ala Lys Gly  Glu Lys Gly
       2300                 2305                 2310
   Ala Pro  Gly Gly Leu Ala Gly  Asp Leu Val Gly Glu  Pro Gly Ala
       2315                 2320                 2325
   Lys Gly  Asp Arg Gly Leu Pro  Gly Pro Arg Gly Glu  Lys Gly Glu
       2330                 2335                 2340
   Ala Gly Arg Ala Gly Glu Pro Gly Asp Pro Gly Glu Asp Gly Gln
       2345                2350                2355
   Lys Gly Ala Pro Gly Pro Lys Gly Phe Lys Gly Asp Pro Gly Val
       2360                2365                2370
   Gly Val Pro Gly Ser Pro Gly Pro Pro Gly Pro Pro Gly Val Lys
       2375                2380                2385
   Gly Asp Leu Gly Leu Pro Gly Leu Pro Gly Ala Pro Gly Val Val
       2390                2395                2400
   Gly Phe Pro Gly Gln Thr Gly Pro Arg Gly Glu Met Gly Gln Pro
       2405                2410                2415
   Gly Pro Ser Gly Glu Arg Gly Leu Ala Gly Pro Pro Gly Arg Glu
       2420                2425                2430
   Gly Ile Pro Gly Pro Leu Gly Pro Pro Gly Pro Pro Gly Ser Val
       2435                2440                2445
   Gly Pro Pro Gly Ala Ser Gly Leu Lys Gly Asp Lys Gly Asp Pro
       2450                2455                2460
   Gly Val Gly Leu Pro Gly Pro Arg Gly Glu Arg Gly Glu Pro Gly
       2465                2470                2475
   Ile Arg Gly Glu Asp Gly Arg Pro Gly Gln Glu Gly Pro Arg Gly
       2480                2485                2490
   Leu Thr Gly Pro Pro Gly Ser Arg Gly Glu Arg Gly Glu Lys Gly
       2495                2500                2505
   Asp Val Gly Ser Ala Gly Leu Lys Gly Asp Lys Gly Asp Ser Ala
       2510                2515                2520
   Val Ile Leu Gly Pro Pro Gly Pro Arg Gly Ala Lys Gly Asp Met
       2525                2530                2535
   Gly Glu Arg Gly Pro Arg Gly Leu Asp Gly Asp Lys Gly Pro Arg
       2540                2545                2550
   Gly Asp  Asn Gly Asp Pro Gly  Asp Lys Gly Ser Lys  Gly Glu Pro
       2555                 2560                 2565
   Gly Asp  Lys Gly Ser Ala Gly  Leu Pro Gly Leu Arg  Gly Leu Leu
       2570                 2575                 2580
   Gly Pro  Gln Gly Gln Pro Gly  Ala Ala Gly Ile Pro  Gly Asp Pro
       2585                 2590                 2595
   Gly Ser  Pro Gly Lys Asp Gly  Val Pro Gly Ile Arg  Gly Glu Lys
       2600                 2605                 2610
   Gly Asp  Val Gly Phe Met Gly  Pro Arg Gly Leu Lys  Gly Glu Arg
       2615                 2620                 2625
   Gly Val  Lys Gly Ala Cys Gly  Leu Asp Gly Glu Lys  Gly Asp Lys
       2630                 2635                 2640
   Gly Glu  Ala Gly Pro Pro Gly  Arg Pro Gly Leu Ala  Gly His Lys
       2645                 2650                 2655
   Gly Glu  Met Gly Glu Pro Gly  Val Pro Gly Gln Ser  Gly Ala Pro
       2660                 2665                 2670
   Gly Lys  Glu Gly Leu Ile Gly  Pro Lys Gly Asp Arg  Gly Phe Asp
       2675                 2680                 2685
   Gly Gln  Pro Gly Pro Lys Gly  Asp Gln Gly Glu Lys  Gly Glu Arg
       2690                 2695                 2700
   Gly Thr  Pro Gly Ile Gly Gly  Phe Pro Gly Pro Ser  Gly Asn Asp
       2705                 2710                 2715
   Gly Ser  Ala Gly Pro Pro Gly  Pro Pro Gly Ser Val  Gly Pro Arg
       2720                 2725                 2730
   Gly Pro  Glu Gly Leu Gln Gly  Gln Lys Gly Glu Arg  Gly Pro Pro
       2735                 2740                 2745
   Gly Glu  Arg Val Val Gly Ala  Pro Gly Val Pro Gly  Ala Pro Gly
       2750                 2755                 2760
   Glu Arg  Gly Glu Gln Gly Arg  Pro Gly Pro Ala Gly  Pro Arg Gly
       2765                 2770                 2775
   Glu Lys  Gly Glu Ala Ala Leu  Thr Glu Asp Asp Ile  Arg Gly Phe
       2780                 2785                 2790
   Val Arg  Gln Glu Met Ser Gln  His Cys Ala Cys Gln  Gly Gln Phe
       2795                 2800                 2805
   Ile Ala  Ser Gly Ser Arg Pro  Leu Pro Ser Tyr Ala  Ala Asp Thr
       2810                 2815                 2820
   Ala Gly  Ser Gln Leu His Ala  Val Pro Val Leu Arg  Val Ser His
       2825                 2830                 2835
   Ala Glu  Glu Glu Glu Arg Val  Pro Pro Glu Asp Asp  Glu Tyr Ser
       2840                 2845                 2850
   Glu Tyr  Ser Glu Tyr Ser Val  Glu Glu Tyr Gln Asp  Pro Glu Ala
       2855                 2860                 2865
   Pro Trp  Asp Ser Asp Asp Pro  Cys Ser Leu Pro Leu  Asp Glu Gly
       2870                 2875                 2880
   Ser Cys  Thr Ala Tyr Thr Leu  Arg Trp Tyr His Arg  Ala Val Thr
       2885                 2890                 2895
   Gly Ser  Thr Glu Ala Cys His  Pro Phe Val Tyr Gly  Gly Cys Gly
       2900                 2905                 2910
   Gly Asn  Ala Asn Arg Phe Gly  Thr Arg Glu Ala Cys  Glu Arg Arg
       2915                 2920                 2925
   Cys Pro  Pro Arg Val Val Gln  Ser Gln Gly Thr Gly  Thr Ala Gln
       2930                 2935                 2940
   Asp
<210> 3
   <211> 53
   <212> DNA
   <213> Artificial Sequence
<220>
   <223> Description of Artificial Sequence: Synthetic primer
<400> 3
   cgacttgtgt tgggactggc tagcgccacc atgacgctgc ggcttctggt ggc 53
<210> 4
   <211> 22
   <212> DNA
   <213> Artificial Sequence
<220>
   <223> Description of Artificial Sequence: Synthetic primer
<400> 4
   gggcttagct acactgtgcg gg 22
<210> 5
   <211> 24
   <212> DNA
   <213> Artificial Sequence
<220>
   <223> Description of Artificial Sequence: Synthetic primer
<400> 5
   cctgcccagt gtattgtcca aagg 24
<210> 6
   <211> 21
   <212> DNA
   <213> Artificial Sequence
<220>
   <223> Description of Artificial Sequence: Synthetic primer
<400> 6
   ctgctggcat caaggcatct g 21
<210> 7
   <211> 22
   <212> DNA
   <213> Artificial Sequence
<220>
   <223> Description of Artificial Sequence: Synthetic primer
<400> 7
   cccgcacagt gtagctaagc cc 22
<210> 8
   <211> 24
   <212> DNA
   <213> Artificial Sequence
<220>
   <223> Description of Artificial Sequence: Synthetic primer
<400> 8
   cctttggaca atacactggg cagg 24
<210> 9
   <211> 21
   <212> DNA
   <213> Artificial Sequence
<220>
   <223> Description of Artificial Sequence: Synthetic primer
<400> 9
   cagatgcctt gatgccagca g 21
<210> 10
   <211> 53
   <212> DNA
   <213> Artificial Sequence
<220>
   <223> Description of Artificial Sequence: Synthetic primer
<400> 10
   cgtgatttca tttgctacac gtaatcgatt cagtcctggg cagtacctgt ccc 53
<210> 11
   <211> 341
   <212> DNA
   <213> Artificial Sequence
<220>
   <223> Description of Artificial Sequence: Synthetic polynucleotide
<400> 11
   cgctcccaga acatcaccta ccactgcaag aacagcgtgg cctacatgga ccagcagact        60
   ggcaacctca agaaggccct gctcctccag ggctccaacg agatcgagat ccgcgccgag       120
   ggcaacagcc gcttcaccta cagcgtcact gtcgatggct gcacgagtca caccggagcc       180
   tggggcaaga cagtgattga atacaaaacc accaagacct cccgcctgcg catcatcgat       240
   gtggccccct tggacgttgg tgccccagac caggaattcg gcttcgacgt tggccatgtc      300
   tgcttcctgt aaatcgatta cgtgtagcaa atgaaatcac g                           341
<210> 12
   <211> 730
   <212> DNA
   <213> Artificial Sequence
<220>
   <223> Description of Artificial Sequence: Synthetic polynucleotide
<400> 12
   ttcaaatttt cgggggatcg cattagttat taatagtaat caattacggg gtcattagtt 60
   catagcccat atatggagtt ccgcgttaca taacttacgg taaatggccc gcctggttga 120
   ccgcccaacg acccccgccc attgacgtca ataatgacgt atgttcccat agtaacgcca 180
   atagggactt tccattgacg tcaatgggtg gagtatttac ggtaaactgc ccacttggta 240
   gtacatcaag tgtatcatac gccaagtacg ccccctattg acgtcaatga cggtaaatgg 300
   cccgcctggt attatgccca gtacatgacc ttatgggact ttcctacttg gcagtacatc 360
   tgcgtattag tcatcgctat taccatggtg atgcggtttt ggcagtacat caatgggcgt 420
   ggatagcggt ttgactcacg gggatttcca agtctccacc ccattgacgt caatgggagt 480
   ttgttttggc accaaaatca acgggacttt ccaaaatgtc gtaacaactc cgccccattg 540
   acgcaaatgg gcggtaggcg tgtacggtgg gaggtctata taagcagagc tggtttagtg 600
   aaccgtcaga tccgctagca ttgtgtgctt ttcgggccac catgacgctg cggcttctgg 660
   tggccgcgct ctgcgccggg atcctggcag aggcgccccg agtgcgagcc cagcacaggg 720
   agagagtgac 730
<210> 13
   <211> 740
   <212> DNA
   <213> Artificial Sequence
<220>
   <223> Description of Artificial Sequence: Synthetic polynucleotide
<400> 13
   agtgcgagcc cagcacaggg agagagtgac ctgcacgcgc ctttacgccg ctgacattgt 60
   gttcttactg gatggctcct catccattgg ccgcagcaat ttccgcgagg tccgcagctt 120
   tctcgaaggg ctggtgctgc ctttctctgg agcagccagt gcacagggtg tgcgctttgc 180
   cacagtgcag tacagcgatg acccacggac agagttcggc ctggatgcac ttggctctgg 240
   gggtgatgtg atccgcgcca tccgtgagct tagctacaag gggggcaaca ctcgcacagg 300
   ggctgcaatt ctccatgtgg ctgaccatgt cttcctgccc cagctggccc gacctggtgt 360
   ccccaaggtc tgcatcctga tcacgtctct catgcagagg aggaagagcg ggtaccccct 420
   gaggatgatg agtactctga atactccgag tattctgtgg aggagtacca ggaccctgaa 480
   gctccttggg atagtgatga cccctgttcc ctgccactgg atgagggctc ctgcactgcc 540
   tacaccctgc gctggtacca tcgggctgtg acaggcagca cagaggcctg tcaccctttt 600
   gtctatggtg gctgtggagg gaatgccaac cgttttggga cccgtgaggc ctgcgagcgc 660
   cgctgcccac cccgggtggt ccagagccag gggacaggta ctgcccagga ctgagtacct 720
   ttaagaccaa tgacttacaa 740
<210> 14
   <211> 20
   <212> DNA
   <213> Artificial Sequence
<220>
   <223> Description of Artificial Sequence: Synthetic primer
<400> 14
   atggaaaaac gccagcaacg 20
<210> 15
   <211> 20
   <212> DNA
   <213> Artificial Sequence
<220>
   <223> Description of Artificial Sequence: Synthetic primer
<400> 15
   ctgtcaccct tttgtctatg 20
<210> 16
   <211> 24
   <212> DNA
   <213> Artificial Sequence
<220>
   <223> Description of Artificial Sequence: Synthetic primer
<400> 16
   gttgccggac acttcttgtc ctct 24
<210> 17
   <211> 22
   <212> DNA
   <213> Artificial Sequence
<220>
   <223> Description of Artificial Sequence: Synthetic primer
<400> 17
   cccgcacagt gtagctaagc cc 22
<210> 18
   <211> 20
   <212> DNA
   <213> Artificial Sequence
<220>
   <223> Description of Artificial Sequence: Synthetic primer
<400> 18
   agaggccccg aaggacttca 20
<210> 19
   <211> 467
   <212> DNA
   <213> Artificial Sequence
<220>
   <223> Description of Artificial Sequence: Synthetic polynucleotide
<400> 19
   acagcagaga tccagtttgg ttaattaagc attagttatt aatagtaatc aattacgggg 60
   tcattaagac tcggccttag aaccccagta tcagcagaac cgcgtctctc atgcagagga 120
   ggaagagcgg gtaccccctg aggatgatga gtactctgaa tactccgagt attctgtgga 180
   ggagtaccag gaccctgaag ctccttggga tagtgatgac ccctgttccc tgccactgga 240
   tgagggctcc tgcactgcct acaccctgcg ctggtaccat cgggctgtga caggcagcac 300
   agaggcctgt cacccttttg tctatggtgg ctgtggaggg aatgccaacc gttttgggac 360
   ccgtgaggcc tgcgagcgcc gctgcccacc ccgggtggtc cagagccagg ggacaggtac 420
   tgcccaggac tgatcgatac cgtcgacctc gagacctaga aaaacat 467
<210> 20
   <211> 21
   <212> DNA
   <213> Artificial Sequence
<220>
   <223> Description of Artificial Sequence: Synthetic primer
<400> 20
   gtagacataa tagcaacaga c 21
<210> 21
   <211> 21
   <212> DNA
   <213> Artificial Sequence
<220>
   <223> Description of Artificial Sequence: Synthetic primer
<400> 21
   tattgctact tgtgattgct c 21
<210> 22
   <211> 20
   <212> DNA
   <213> Artificial Sequence
<220>
   <223> Description of Artificial Sequence: Synthetic primer
<400> 22
   agaggccccg aaggacttca 20
<210> 23
   <211> 8832
   <212> DNA
   <213> Artificial Sequence
<220>
   <223> Description of Artificial Sequence: Synthetic polynucleotide
<400> 23
   atgacgctgc ggcttctggt ggccgcgctc tgcgccggga tcctggcaga ggcgccccga      60
   gtgcgagccc agcacaggga gagagtgacc tgcacgcgcc tttacgccgc tgacattgtg     120
   ttcttactgg atggctcctc atccattggc cgcagcaatt tccgcgaggt ccgcagcttt     180
   ctcgaagggc tggtgctgcc tttctctgga gcagccagtg cacagggtgt gcgctttgcc     240
   acagtgcagt acagcgatga cccacggaca gagttcggcc tggatgcact tggctctggg     300
   ggtgatgtga tccgcgccat ccgtgagctt agctacaagg ggggcaacac tcgcacaggg     360
   gctgcaattc tccatgtggc tgaccatgtc ttcctgcccc agctggcccg acctggtgtc     420
   cccaaggtct gcatcctgat cacagacggg aagtcccagg acctggtgga cacagctgcc     480
   caaaggctga aggggcaggg ggtcaagcta tttgctgtgg ggatcaagaa tgctgaccct     540
   gaggagctga agcgagttgc ctcacagccc accagtgact tcttcttctt cgtcaatgac     600
   ttcagcatct tgaggacact actgcccctc gtttcccgga gagtgtgcac gactgctggt     660
   ggcgtgcctg tgacccgacc tccggatgac tcgacctctg ctccacgaga cctggtgctg     720
   tctgagccaa gcagccaatc cttgagagta cagtggacag cggccagtgg ccctgtgact     780
   ggctacaagg tccagtacac tcctctgacg gggctgggac agccactgcc gagtgagcgg     840
   caggaggtga acgtcccagc tggtgagacc agtgtgcggc tgcggggtct ccggccactg     900
   accgagtacc aagtgactgt gattgccctc tacgccaaca gcatcgggga ggctgtgagc     960
   gggacagctc ggaccactgc cctagaaggg ccggaactga ccatccagaa taccacagcc    1020
   cacagcctcc tggtggcctg gcggagtgtg ccaggtgcca ctggctaccg tgtgacatgg    1080
   cgggtcctca gtggtgggcc cacacagcag caggagctgg gccctgggca gggttcagtg    1140
   ttgctgcgtg acttggagcc tggcacggac tatgaggtga ccgtgagcac cctatttggc    1200
   cgcagtgtgg ggcccgccac ttccctgatg gctcgcactg acgcttctgt tgagcagacc    1260
   ctgcgcccgg tcatcctggg ccccacatcc atcctccttt cctggaactt ggtgcctgag    1320
   gcccgtggct accggttgga atggcggcgt gagactggct tggagccacc gcagaaggtg    1380
   gtactgccct ctgatgtgac ccgctaccag ttggatgggc tgcagccggg cactgagtac    1440
   cgcctcacac tctacactct gctggagggc cacgaggtgg ccacccctgc aaccgtggtt    1500
   cccactggac cagagctgcc tgtgagccct gtaacagacc tgcaagccac cgagctgccc    1560
   gggcagcggg tgcgagtgtc ctggagccca gtccctggtg ccacccagta ccgcatcatt    1620
   gtgcgcagca cccagggggt tgagcggacc ctggtgcttc ctgggagtca gacagcattc 1680
   gacttggatg acgttcaggc tgggcttagc tacactgtgc gggtgtctgc tcgagtgggt 1740
   ccccgtgagg gcagtgccag tgtcctcact gtccgccggg agccggaaac tccacttgct 1800
   gttccagggc tgcgggttgt ggtgtcagat gcaacgcgag tgagggtggc ctggggaccc 1860
   gtccctggag ccagtggatt tcggattagc tggagcacag gcagtggtcc ggagtccagc 1920
   cagacactgc ccccagactc tactgccaca gacatcacag ggctgcagcc tggaaccacc 1980
   taccaggtgg ctgtgtcggt actgcgaggc agagaggagg gccctgctgc agtcatcgtg 2040
   gctcgaacgg acccactggg cccagtgagg acggtccatg tgactcaggc cagcagctca 2100
   tctgtcacca ttacctggac cagggttcct ggcgccacag gatacagggt ttcctggcac 2160
   tcagcccacg gcccagagaa atcccagttg gtttctgggg aggccacggt ggctgagctg 2220
   gatggactgg agccagatac tgagtatacg gtgcatgtga gggcccatgt ggctggcgtg 2280
   gatgggcccc ctgcctctgt ggttgtgagg actgcccctg agcctgtggg tcgtgtgtcg 2340
   aggctgcaga tcctcaatgc ttccagcgac gttctacgga tcacctgggt aggggtcact 2400
   ggagccacag cttacagact ggcctggggc cggagtgaag gcggccccat gaggcaccag 2460
   atactcccag gaaacacaga ctctgcagag atccggggtc tcgaaggtgg agtcagctac 2520
   tcagtgcgag tgactgcact tgtcggggac cgcgagggca cacctgtctc cattgttgtc 2580
   actacgccgc ctgaggctcc gccagccctg gggacgcttc acgtggtgca gcgcggggag 2640
   cactcgctga ggctgcgctg ggagccggtg cccagagcgc agggcttcct tctgcactgg 2700
   caacctgagg gtggccagga acagtcccgg gtcctggggc ccgagctcag cagctatcac 2760
   ctggacgggc tggagccagc gacacagtac cgcgtgaggc tgagtgtcct agggccggct 2820
   ggagaagggc cctctgcaga ggtgactgcg cgcactgagt cacctcgtgt tccaagcatt 2880
   gaactacgtg tggtggacac ctcgatcgac tcggtgactt tggcctggac tccagtgtcc 2940
   agggcatcca gctacatcct atcctggcgg ccactcagag gccctggcca ggaagtgcct 3000
   gggtccccgc agacacttcc agggatctca agctcccagc gggtgacagg gctagagcct 3060
   ggcgtctctt acatcttctc cctgacgcct gtcctggatg gtgtgcgggg tcctgaggca 3120
   tctgtcacac agacgccagt gtgcccccgt ggcctggcgg atgtggtgtt cctaccacat 3180
   gccactcaag acaatgctca ccgtgcggag gctacgagga gggtcctgga gcgtctggtg 3240
   ttggcacttg ggcctcttgg gccacaggca gttcaggttg gcctgctgtc ttacagtcat 3300
   cggccctccc cactgttccc actgaatggc tcccatgacc ttggcattat cttgcaaagg 3360
   atccgtgaca tgccctacat ggacccaagt gggaacaacc tgggcacagc cgtggtcaca 3420
   gctcacagat acatgttggc accagatgct cctgggcgcc gccagcacgt accaggggtg 3480
   atggttctgc tagtggatga acccttgaga ggtgacatat tcagccccat ccgtgaggcc 3540
   caggcttctg ggcttaatgt ggtgatgttg ggaatggctg gagcggaccc agagcagctg 3600
   cgtcgcttgg cgccgggtat ggactctgtc cagaccttct tcgccgtgga tgatgggcca 3660
   agcctggacc aggcagtcag tggtctggcc acagccctgt gtcaggcatc cttcactact 3720
   cagccccggc cagagccctg cccagtgtat tgtccaaagg gccagaaggg ggaacctgga 3780
   gagatgggcc tgagaggaca agttgggcct cctggcgacc ctggcctccc gggcaggacc 3840
   ggtgctcccg gcccccaggg gccccctgga agtgccactg ccaagggcga gaggggcttc 3900
   cctggagcag atgggcgtcc aggcagccct ggccgcgccg ggaatcctgg gacccctgga 3960
   gcccctggcc taaagggctc tccagggttg cctggccctc gtggggaccc gggagagcga 4020
   ggacctcgag gcccaaaggg ggagccgggg gctcccggac aagtcatcgg aggtgaagga 4080
   cctgggcttc ctgggcggaa aggggaccct ggaccatcgg gcccccctgg acctcgtgga 4140
   ccactggggg acccaggacc ccgtggcccc ccagggcttc ctggaacagc catgaagggt 4200
   gacaaaggcg atcgtgggga gcggggtccc cctggaccag gtgaaggtgg cattgctcct 4260
   ggggagcctg ggctgccggg tcttcccgga agccctggac cccaaggccc cgttggcccc 4320
   cctggaaaga aaggagaaaa aggtgactct gaggatggag ctccaggcct cccaggacaa 4380
   cctgggtctc cgggtgagca gggcccacgg ggacctcctg gagctattgg ccccaaaggt 4440
   gaccggggct ttccagggcc cctgggtgag gctggagaga agggcgaacg tggaccccca 4500
   ggcccagcgg gatcccgggg gctgccaggg gttgctggac gtcctggagc caagggtcct 4560
   gaagggccac caggacccac tggccgccaa ggagagaagg gggagcctgg tcgccctggg 4620
   gaccctgcag tggtgggacc tgctgttgct ggacccaaag gagaaaaggg agatgtgggg 4680
   cccgctgggc ccagaggagc taccggagtc caaggggaac ggggcccacc cggcttggtt 4740
   cttcctggag accctggccc caagggagac cctggagacc ggggtcccat tggccttact 4800
   ggcagagcag gacccccagg tgactcaggg cctcctggag agaagggaga ccctgggcgg 4860
   cctggccccc caggacctgt tggcccccga ggacgagatg gtgaagttgg agagaaaggt 4920
   gacgagggtc ctccgggtga cccgggtttg cctggaaaag caggcgagcg tggccttcgg 4980
   ggggcacctg gagttcgggg gcctgtgggt gaaaagggag accagggaga tcctggagag 5040
   gatggacgaa atggcagccc tggatcatct ggacccaagg gtgaccgtgg ggagccgggt 5100
   cccccaggac ccccgggacg gctggtagac acaggacctg gagccagaga gaagggagag 5160
   cctggggacc gcggacaaga gggtcctcga gggcccaagg gtgatcctgg cctccctgga 5220
   gcccctgggg aaaggggcat tgaagggttt cggggacccc caggcccaca gggggaccca 5280
   ggtgtccgag gcccagcagg agaaaagggt gaccggggtc cccctgggct ggatggccgg 5340
   agcggactgg atgggaaacc aggagccgct gggccctctg ggccgaatgg tgctgcaggc 5400
   aaagctgggg acccagggag agacgggctt ccaggcctcc gtggagaaca aggcctccct 5460
   ggcccctctg gtccccctgg attaccggga aagccaggcg aggatgggaa acctggcctg 5520
   aatggaaaaa acggagaacc tggggaccct ggagaagacg ggaggaaggg agagaaagga 5580
   gattcaggcg cctctgggag agaaggtcgt gatggcccca agggtgagcg tggagctcct 5640
   ggtatccttg gaccccaggg gcctccaggc ctcccagggc cagtgggccc tcctggccag 5700
   ggttttcctg gtgtcccagg aggcacgggc cccaagggtg accgtgggga gactggatcc 5760
   aaaggggagc agggcctccc tggagagcgt ggcctgcgag gagagcctgg aagtgtgccg 5820
   aatgtggatc ggttgctgga aactgctggc atcaaggcat ctgccctgcg ggagatcgtg 5880
   gagacctggg atgagagctc tggtagcttc ctgcctgtgc ccgaacggcg tcgaggcccc 5940
   aagggggact caggcgaaca gggcccccca ggcaaggagg gccccatcgg ctttcctgga 6000
   gaacgcgggc tgaagggcga ccgtggagac cctggccctc aggggccacc tggtctggcc 6060
   cttggggaga ggggcccccc cgggccttcc ggccttgccg gggagcctgg aaagcctggt 6120
   attcccgggc tcccaggcag ggctgggggt gtgggagagg caggaaggcc aggagagagg 6180
   ggagaacggg gagagaaagg agaacgtgga gaacagggca gagatggccc tcctggactc 6240
   cctggaaccc ctgggccccc cggaccccct ggccccaagg tgtctgtgga tgagccaggt 6300
   cctggactct ctggagaaca gggaccccct ggactcaagg gtgctaaggg ggagccgggc 6360
   agcaatggtg accaaggtcc caaaggagac aggggtgtgc caggcatcaa aggagaccgg 6420
   ggagagcctg gaccgagggg tcaggacggc aacccgggtc taccaggaga gcgtggtatg 6480
   gctgggcctg aagggaagcc gggtctgcag ggtccaagag gcccccctgg cccagtgggt 6540
   ggtcatggag accctggacc acctggtgcc ccgggtcttg ctggccctgc aggaccccaa 6600
   ggaccttctg gcctgaaggg ggagcctgga gagacaggac ctccaggacg gggcctgact 6660
   ggacctactg gagctgtggg acttcctgga ccccccggcc cttcaggcct tgtgggtcca 6720
   caggggtctc caggtttgcc tggacaagtg ggggagacag ggaagccggg agccccaggt 6780
   cgagatggtg ccagtggaaa agatggagac agagggagcc ctggtgtgcc agggtcacca 6840
   ggtctgcctg gccctgtcgg acctaaagga gaacctggcc ccacgggggc ccctggacag 6900
   gctgtggtcg ggctccctgg agcaaaggga gagaagggag cccctggagg ccttgctgga 6960
   gacctggtgg gtgagccggg agccaaaggt gaccgaggac tgccagggcc gcgaggcgag 7020
   aagggtgaag ctggccgtgc aggggagccc ggagaccctg gggaagatgg tcagaaaggg 7080
   gctccaggac ccaaaggttt caagggtgac ccaggagtcg ggtcccgggc ctcccctggg 7140
   cctcctggcc ctccaggtgt gaagggagat ctgggcctcc ctggcctgcc cggtgctcct 7200
   ggtgttgttg ggttcccggg tcagacaggc cctcgaggag agatgggtca gccaggccct 7260
   agtggagagc ggggtctggc aggcccccca gggagagaag gaatcccagg acccctgggg 7320
   ccacctggac caccggggtc agtgggacca cctggggcct ctggactcaa aggagacaag 7380
   ggagaccctg gagtagggct gcctgggccc cgaggcgagc gtggggagcc aggcatccgg 7440
   ggtgaagatg gccgccccgg ccaggaggga ccccgaggac tcacggggcc ccctggcagc 7500
   aggggagagc gtggggagaa gggtgatgtt gggagtgcag gactaaaggg tgacaaggga 7560
   gactcagctg tgatcctggg gcctccaggc ccacggggtg ccaaggggga catgggtgaa 7620
   cgagggcctc ggggcttgga tggtgacaaa ggacctcggg gagacaatgg ggaccctggt 7680
   gacaagggca gcaagggaga gcctggtgac aagggctcag ccgggttgcc aggactgcgt 7740
   ggactcctgg gaccccaggg tcaacctggt gcagcaggga tccctggtga cccgggatcc 7800
   ccaggaaagg atggagtgcc tggtatccga ggagaaaaag gagatgttgg cttcatgggt 7860
   ccccggggcc tcaagggtga acggggagtg aagggagcct gtggccttga tggagagaag 7920
   ggagacaagg gagaagctgg tcccccaggc cgccccgggc tggcaggaca caaaggagag 7980
   atgggggagc ctggtgtgcc gggccagtcg ggggcccctg gcaaggaggg cctgatcggt 8040
   cccaagggtg accgaggctt tgacgggcag ccaggcccca agggtgacca gggcgagaaa 8100
   ggggagcggg gaaccccagg aattgggggc ttcccaggcc ccagtggaaa tgatggctct 8160
   gctggtcccc cagggccacc tggcagtgtt ggtcccagag gccccgaagg acttcagggc 8220
   cagaagggtg agcgaggtcc ccccggagag agagtggtgg gggctcctgg ggtccctgga 8280
   gctcctggcg agagagggga gcaggggcgg ccagggcctg ccggtcctcg aggcgagaag 8340
   ggagaagctg cactgacgga ggatgacatc cggggctttg tgcgccaaga gatgagtcag      8400
   cactgtgcct gccagggcca gttcatcgca tctggatcac gacccctccc tagttatgct      8460
   gcagacactg ccggctccca gctccatgct gtgcctgtgc tccgcgtctc tcatgcagag      8520
   gaggaagagc gggtaccccc tgaggatgat gagtactctg aatactccga gtattctgtg      8580
   gaggagtacc aggaccctga agctccttgg gatagtgatg acccctgttc cctgccactg      8640
   gatgagggct cctgcactgc ctacaccctg cgctggtacc atcgggctgt gacaggcagc      8700
   acagaggcct gtcacccttt tgtctatggt ggctgtggag ggaatgccaa ccgttttggg      8760
   acccgtgagg cctgcgagcg ccgctgccca ccccgggtgg tccagagcca ggggacaggt      8820
   actgcccagg ac                                                          8832
<210> 24
   <211> 8832
   <212> DNA
   <213> Homo sapiens
<400> 24
   atgacgctgc ggcttctggt ggccgcgctc tgcgccggga tcctggcaga ggcgccccga      60
   gtgcgagccc agcacaggga gagagtgacc tgcacgcgcc tttacgccgc tgacattgtg     120
   ttcttactgg atggctcctc atccattggc cgcagcaatt tccgcgaggt ccgcagcttt     180
   ctcgaagggc tggtgctgcc tttctctgga gcagccagtg cacagggtgt gcgctttgcc     240
   acagtgcagt acagcgatga cccacggaca gagttcggcc tggatgcact tggctctggg     300
   ggtgatgtga tccgcgccat ccgtgagctt agctacaagg ggggcaacac tcgcacaggg     360
   gctgcaattc tccatgtggc tgaccatgtc ttcctgcccc agctggcccg acctggtgtc     420
   cccaaggtct gcatcctgat cacagacggg aagtcccagg acctggtgga cacagctgcc     480
   caaaggctga aggggcaggg ggtcaagcta tttgctgtgg ggatcaagaa tgctgaccct     540
   gaggagctga agcgagttgc ctcacagccc accagtgact tcttcttctt cgtcaatgac     600
   ttcagcatct tgaggacact actgcccctc gtttcccgga gagtgtgcac gactgctggt     660
   ggcgtgcctg tgacccgacc tccggatgac tcgacctctg ctccacgaga cctggtgctg     720
   tctgagccaa gcagccaatc cttgagagta cagtggacag cggccagtgg ccctgtgact     780
   ggctacaagg tccagtacac tcctctgacg gggctgggac agccactgcc gagtgagcgg     840
   caggaggtga acgtcccagc tggtgagacc agtgtgcggc tgcggggtct ccggccactg     900
   accgagtacc aagtgactgt gattgccctc tacgccaaca gcatcgggga ggctgtgagc     960
   gggacagctc ggaccactgc cctagaaggg ccggaactga ccatccagaa taccacagcc 1020
   cacagcctcc tggtggcctg gcggagtgtg ccaggtgcca ctggctaccg tgtgacatgg 1080
   cgggtcctca gtggtgggcc cacacagcag caggagctgg gccctgggca gggttcagtg 1140
   ttgctgcgtg acttggagcc tggcacggac tatgaggtga ccgtgagcac cctatttggc 1200
   cgcagtgtgg ggcccgccac ttccctgatg gctcgcactg acgcttctgt tgagcagacc 1260
   ctgcgcccgg tcatcctggg ccccacatcc atcctccttt cctggaactt ggtgcctgag 1320
   gcccgtggct accggttgga atggcggcgt gagactggct tggagccacc gcagaaggtg 1380
   gtactgccct ctgatgtgac ccgctaccag ttggatgggc tgcagccggg cactgagtac 1440
   cgcctcacac tctacactct gctggagggc cacgaggtgg ccacccctgc aaccgtggtt 1500
   cccactggac cagagctgcc tgtgagccct gtaacagacc tgcaagccac cgagctgccc 1560
   gggcagcggg tgcgagtgtc ctggagccca gtccctggtg ccacccagta ccgcatcatt 1620
   gtgcgcagca cccagggggt tgagcggacc ctggtgcttc ctgggagtca gacagcattc 1680
   gacttggatg acgttcaggc tgggcttagc tacactgtgc gggtgtctgc tcgagtgggt 1740
   ccccgtgagg gcagtgccag tgtcctcact gtccgccggg agccggaaac tccacttgct 1800
   gttccagggc tgcgggttgt ggtgtcagat gcaacgcgag tgagggtggc ctggggaccc 1860
   gtccctggag ccagtggatt tcggattagc tggagcacag gcagtggtcc ggagtccagc 1920
   cagacactgc ccccagactc tactgccaca gacatcacag ggctgcagcc tggaaccacc 1980
   taccaggtgg ctgtgtcggt actgcgaggc agagaggagg gccctgctgc agtcatcgtg 2040
   gctcgaacgg acccactggg cccagtgagg acggtccatg tgactcaggc cagcagctca 2100
   tctgtcacca ttacctggac cagggttcct ggcgccacag gatacagggt ttcctggcac 2160
   tcagcccacg gcccagagaa atcccagttg gtttctgggg aggccacggt ggctgagctg 2220
   gatggactgg agccagatac tgagtatacg gtgcatgtga gggcccatgt ggctggcgtg 2280
   gatgggcccc ctgcctctgt ggttgtgagg actgcccctg agcctgtggg tcgtgtgtcg 2340
   aggctgcaga tcctcaatgc ttccagcgac gttctacgga tcacctgggt aggggtcact 2400
   ggagccacag cttacagact ggcctggggc cggagtgaag gcggccccat gaggcaccag 2460
   atactcccag gaaacacaga ctctgcagag atccggggtc tcgaaggtgg agtcagctac 2520
   tcagtgcgag tgactgcact tgtcggggac cgcgagggca cacctgtctc cattgttgtc 2580
   actacgccgc ctgaggctcc gccagccctg gggacgcttc acgtggtgca gcgcggggag 2640
   cactcgctga ggctgcgctg ggagccggtg cccagagcgc agggcttcct tctgcactgg 2700
   caacctgagg gtggccagga acagtcccgg gtcctggggc ccgagctcag cagctatcac 2760
   ctggacgggc tggagccagc gacacagtac cgcgtgaggc tgagtgtcct agggccagct 2820
   ggagaagggc cctctgcaga ggtgactgcg cgcactgagt cacctcgtgt tccaagcatt 2880
   gaactacgtg tggtggacac ctcgatcgac tcggtgactt tggcctggac tccagtgtcc 2940
   agggcatcca gctacatcct atcctggcgg ccactcagag gccctggcca ggaagtgcct 3000
   gggtccccgc agacacttcc agggatctca agctcccagc gggtgacagg gctagagcct 3060
   ggcgtctctt acatcttctc cctgacgcct gtcctggatg gtgtgcgggg tcctgaggca 3120
   tctgtcacac agacgccagt gtgcccccgt ggcctggcgg atgtggtgtt cctaccacat 3180
   gccactcaag acaatgctca ccgtgcggag gctacgagga gggtcctgga gcgtctggtg 3240
   ttggcacttg ggcctcttgg gccacaggca gttcaggttg gcctgctgtc ttacagtcat 3300
   cggccctccc cactgttccc actgaatggc tcccatgacc ttggcattat cttgcaaagg 3360
   atccgtgaca tgccctacat ggacccaagt gggaacaacc tgggcacagc cgtggtcaca 3420
   gctcacagat acatgttggc accagatgct cctgggcgcc gccagcacgt accaggggtg 3480
   atggttctgc tagtggatga acccttgaga ggtgacatat tcagccccat ccgtgaggcc 3540
   caggcttctg ggcttaatgt ggtgatgttg ggaatggctg gagcggaccc agagcagctg 3600
   cgtcgcttgg cgccgggtat ggactctgtc cagaccttct tcgccgtgga tgatgggcca 3660
   agcctggacc aggcagtcag tggtctggcc acagccctgt gtcaggcatc cttcactact 3720
   cagccccggc cagagccctg cccagtgtat tgtccaaagg gccagaaggg ggaacctgga 3780
   gagatgggcc tgagaggaca agttgggcct cctggcgacc ctggcctccc gggcaggacc 3840
   ggtgctcccg gcccccaggg gccccctgga agtgccactg ccaagggcga gaggggcttc 3900
   cctggagcag atgggcgtcc aggcagccct ggccgcgccg ggaatcctgg gacccctgga 3960
   gcccctggcc taaagggctc tccagggttg cctggccctc gtggggaccc gggagagcga 4020
   ggacctcgag gcccaaaggg ggagccgggg gctcccggac aagtcatcgg aggtgaagga 4080
   cctgggcttc ctgggcggaa aggggaccct ggaccatcgg gcccccctgg acctcgtgga 4140
   ccactggggg acccaggacc ccgtggcccc ccagggcttc ctggaacagc catgaagggt 4200
   gacaaaggcg atcgtgggga gcggggtccc cctggaccag gtgaaggtgg cattgctcct 4260
   ggggagcctg ggctgccggg tcttcccgga agccctggac cccaaggccc cgttggcccc 4320
   cctggaaaga aaggagaaaa aggtgactct gaggatggag ctccaggcct cccaggacaa 4380
   cctgggtctc cgggtgagca gggcccacgg ggacctcctg gagctattgg ccccaaaggt 4440
   gaccggggct ttccagggcc cctgggtgag gctggagaga agggcgaacg tggaccccca 4500
   ggcccagcgg gatcccgggg gctgccaggg gttgctggac gtcctggagc caagggtcct 4560
   gaagggccac caggacccac tggccgccaa ggagagaagg gggagcctgg tcgccctggg 4620
   gaccctgcag tggtgggacc tgctgttgct ggacccaaag gagaaaaggg agatgtgggg 4680
   cccgctgggc ccagaggagc taccggagtc caaggggaac ggggcccacc cggcttggtt 4740
   cttcctggag accctggccc caagggagac cctggagacc ggggtcccat tggccttact 4800
   ggcagagcag gacccccagg tgactcaggg cctcctggag agaagggaga ccctgggcgg 4860
   cctggccccc caggacctgt tggcccccga ggacgagatg gtgaagttgg agagaaaggt 4920
   gacgagggtc ctccgggtga cccgggtttg cctggaaaag caggcgagcg tggccttcgg 4980
   ggggcacctg gagttcgggg gcctgtgggt gaaaagggag accagggaga tcctggagag 5040
   gatggacgaa atggcagccc tggatcatct ggacccaagg gtgaccgtgg ggagccgggt 5100
   cccccaggac ccccgggacg gctggtagac acaggacctg gagccagaga gaagggagag 5160
   cctggggacc gcggacaaga gggtcctcga gggcccaagg gtgatcctgg cctccctgga 5220
   gcccctgggg aaaggggcat tgaagggttt cggggacccc caggcccaca gggggaccca 5280
   ggtgtccgag gcccagcagg agaaaagggt gaccggggtc cccctgggct ggatggccgg 5340
   agcggactgg atgggaaacc aggagccgct gggccctctg ggccgaatgg tgctgcaggc 5400
   aaagctgggg acccagggag agacgggctt ccaggcctcc gtggagaaca gggcctccct 5460
   ggcccctctg gtccccctgg attaccggga aagccaggcg aggatggcaa acctggcctg 5520
   aatggaaaaa acggagaacc tggggaccct ggagaagacg ggaggaaggg agagaaagga 5580
   gattcaggcg cctctgggag agaaggtcgt gatggcccca agggtgagcg tggagctcct 5640
   ggtatccttg gaccccaggg gcctccaggc ctcccagggc cagtgggccc tcctggccag 5700
   ggttttcctg gtgtcccagg aggcacgggc cccaagggtg accgtgggga gactggatcc 5760
   aaaggggagc agggcctccc tggagagcgt ggcctgcgag gagagcctgg aagtgtgccg 5820
   aatgtggatc ggttgctgga aactgctggc atcaaggcat ctgccctgcg ggagatcgtg 5880
   gagacctggg atgagagctc tggtagcttc ctgcctgtgc ccgaacggcg tcgaggcccc 5940
   aagggggact caggcgaaca gggcccccca ggcaaggagg gccccatcgg ctttcctgga 6000
   gaacgcgggc tgaagggcga ccgtggagac cctggccctc aggggccacc tggtctggcc 6060
   cttggggaga ggggcccccc cgggccttcc ggccttgccg gggagcctgg aaagcctggt 6120
   attcccgggc tcccaggcag ggctgggggt gtgggagagg caggaaggcc aggagagagg 6180
   ggagaacggg gagagaaagg agaacgtgga gaacagggca gagatggccc tcctggactc 6240
   cctggaaccc ctgggccccc cggaccccct ggccccaagg tgtctgtgga tgagccaggt 6300
   cctggactct ctggagaaca gggaccccct ggactcaagg gtgctaaggg ggagccgggc 6360
   agcaatggtg accaaggtcc caaaggagac aggggtgtgc caggcatcaa aggagaccgg 6420
   ggagagcctg gaccgagggg tcaggacggc aacccgggtc taccaggaga gcgtggtatg 6480
   gctgggcctg aagggaagcc gggtctgcag ggtccaagag gcccccctgg cccagtgggt 6540
   ggtcatggag accctggacc acctggtgcc ccgggtcttg ctggccctgc aggaccccaa 6600
   ggaccttctg gcctgaaggg ggagcctgga gagacaggac ctccaggacg gggcctgact 6660
   ggacctactg gagctgtggg acttcctgga ccccccggcc cttcaggcct tgtgggtcca 6720
   caggggtctc caggtttgcc tggacaagtg ggggagacag ggaagccggg agccccaggt 6780
   cgagatggtg ccagtggaaa agatggagac agagggagcc ctggtgtgcc agggtcacca 6840
   ggtctgcctg gccctgtcgg acctaaagga gaacctggcc ccacgggggc ccctggacag 6900
   gctgtggtcg ggctccctgg agcaaaggga gagaagggag cccctggagg ccttgctgga 6960
   gacctggtgg gtgagccggg agccaaaggt gaccgaggac tgccagggcc gcgaggcgag 7020
   aagggtgaag ctggccgtgc aggggagccc ggagaccctg gggaagatgg tcagaaaggg 7080
   gctccaggac ccaaaggttt caagggtgac ccaggagtcg ggtcccgggg ctcccctggg 7140
   cctcctggcc ctccaggtgt gaagggagat ctgggcctcc ctggcctgcc cggtgctcct 7200
   ggtgttgttg ggttcccggg tcagacaggc cctcgaggag agatgggtca gccaggccct 7260
   agtggagagc ggggtctggc aggcccccca gggagagaag gaatcccagg acccctgggg 7320
   ccacctggac caccggggtc agtgggacca cctggggcct ctggactcaa aggagacaag 7380
   ggagaccctg gagtagggct gcctgggccc cgaggcgagc gtggggagcc aggcatccgg 7440
   ggtgaagatg gccgccccgg ccaggaggga ccccgaggac tcacggggcc ccctggcagc 7500
   aggggagagc gtggggagaa gggtgatgtt gggagtgcag gactaaaggg tgacaaggga 7560
   gactcagctg tgatcctggg gcctccaggc ccacggggtg ccaaggggga catgggtgaa 7620
   cgagggcctc ggggcttgga tggtgacaaa ggacctcggg gagacaatgg ggaccctggt 7680
   gacaagggca gcaagggaga gcctggtgac aagggctcag ccgggttgcc aggactgcgt 7740
   ggactcctgg gaccccaggg tcaacctggt gcagcaggga tccctggtga cccgggatcc 7800
   ccaggaaagg atggagtgcc tggtatccga ggagaaaaag gagatgttgg cttcatgggt 7860
   ccccggggcc tcaagggtga acggggagtg aagggagcct gtggccttga tggagagaag 7920
   ggagacaagg gagaagctgg tcccccaggc cgccccgggc tggcaggaca caaaggagag 7980
   atgggggagc ctggtgtgcc gggccagtcg ggggcccctg gcaaggaggg cctgatcggt 8040
   cccaagggtg accgaggctt tgacgggcag ccaggcccca agggtgacca gggcgagaaa 8100
   ggggagcggg gaaccccagg aattgggggc ttcccaggcc ccagtggaaa tgatggctct 8160
   gctggtcccc cagggccacc tggcagtgtt ggtcccagag gccccgaagg acttcagggc 8220
   cagaagggtg agcgaggtcc ccccggagag agagtggtgg gggctcctgg ggtccctgga 8280
   gctcctggcg agagagggga gcaggggcgg ccagggcctg ccggtcctcg aggcgagaag 8340
   ggagaagctg cactgacgga ggatgacatc cggggctttg tgcgccaaga gatgagtcag 8400
   cactgtgcct gccagggcca gttcatcgca tctggatcac gacccctccc tagttatgct 8460
   gcagacactg ccggctccca gctccatgct gtgcctgtgc tccgcgtctc tcatgcagag 8520
   gaggaagagc gggtaccccc tgaggatgat gagtactctg aatactccga gtattctgtg 8580
   gaggagtacc aggaccctga agctccttgg gatagtgatg acccctgttc cctgccactg 8640
   gatgagggct cctgcactgc ctacaccctg cgctggtacc atcgggctgt gacaggcagc 8700
   acagaggcct gtcacccttt tgtctatggt ggctgtggag ggaatgccaa ccgttttggg 8760
   acccgtgagg cctgcgagcg ccgctgccca ccccgggtgg tccagagcca ggggacaggt 8820
   actgcccagg ac 8832
<210> 25
   <211> 21
   <212> DNA
   <213> Artificial Sequence
<220>
   <223> Description of Artificial Sequence: Synthetic primer
<400> 25
   acctgaaagc gaaagggaaa c 21
<210> 26
   <211> 23
   <212> DNA
   <213> Artificial Sequence
<220>
   <223> Description of Artificial Sequence: Synthetic primer
<400> 26
   cacccatctc tctccttcta gcc 23
<210> 27
   <211> 24
   <212> DNA
   <213> Artificial Sequence
<220>
   <223> Description of Artificial Sequence: Synthetic probe
<400> 27
   agctctctcg acgcaggact cggc 24
<210> 28
   <211> 21
   <212> DNA
   <213> Artificial Sequence
<220>
   <223> Description of Artificial Sequence: Synthetic primer
<400> 28
   cactcccaac gaagacaaga t 21
<210> 29
   <211> 22
   <212> DNA
   <213> Artificial Sequence
<220>
   <223> Description of Artificial Sequence: Synthetic primer
<400> 29
   gtctaaccag agagacccag ta 22
<210> 30
   <211> 24
   <212> DNA
   <213> Artificial Sequence
<220>
   <223> Description of Artificial Sequence: Synthetic probe
<400> 30
   tttgtaaacc ggtgcagctg cttt 24
<210> 31
   <211> 21
   <212> DNA
   <213> Artificial Sequence
<220>
   <223> Description of Artificial Sequence: Synthetic primer
<400> 31
   acctgaaagc gaaagggaaa c 21
<210> 32
   <211> 23
   <212> DNA
   <213> Artificial Sequence
<220>
   <223> Description of Artificial Sequence: Synthetic primer
<400> 32
   cacccatctc tctccttcta gcc 23
<210> 33
   <211> 24
   <212> DNA
   <213> Artificial Sequence
<220>
   <223> Description of Artificial Sequence: Synthetic probe
<400> 33
   agctctctcg acgcaggact cggc 24
<210> 34
   <211> 16777
   <212> DNA
   <213> Artificial Sequence
<220>
   <223> Description of Artificial Sequence: Synthetic polynucleotide
<400> 34
   gtcgacggat cgggagatct cccgatcccc tatggtgcac tctcagtaca atctgctctg      60
   atgccgcata gttaagccag tatctgctcc ctgcttgtgt gttggaggtc gctgagtagt     120
   gcgcgagcaa aatttaagct acaacaaggc aaggcttgac cgacaattgc atgaagaatc     180
   tgcttagggt taggcgtttt gcgctgcttc gcgatgtacg ggccagatat acgcgttgac     240
   attgattatt gactagttat taatagtaat caattacggg gtcattagtt catagcccat     300
   atatggagtt ccgcgttaca taacttacgg taaatggccc gcctggctga ccgcccaacg     360
   acccccgccc attgacgtca ataatgacgt atgttcccat agtaacgcca atagggactt     420
   tccattgacg tcaatgggtg gagtatttac ggtaaactgc ccacttggca gtacatcaag     480
   tgtatcatat gccaagtacg ccccctattg acgtcaatga cggtaaatgg cccgcctggc     540
   attatgccca gtacatgacc ttatgggact ttcctacttg gcagtacatc tacgtattag     600
   tcatcgctat taccatggtg atgcggtttt ggcagtacat caatgggcgt ggatagcggt     660
   ttgactcacg gggatttcca agtctccacc ccattgacgt caatgggagt ttgttttggc     720
   accaaaatca acgggacttt ccaaaatgtc gtaacaactc cgccccattg acgcaaatgg     780
   gcggtaggcg tgtacggtgg gaggtctata taagcagcgc gttttgcctg tactgggtct     840
   ctctggttag accagatctg agcctgggag ctctctggct aactagggaa cccactgctt     900
   aagcctcaat aaagcttgcc ttgagtgctt caagtagtgt gtgcccgtct gttgtgtgac     960
   tctggtaact agagatccct cagacccttt tagtcagtgt ggaaaatctc tagcagtggc    1020
   gcccgaacag ggacttgaaa gcgaaaggga aaccagagga gctctctcga cgcaggactc    1080
   ggcttgctga agcgcgcacg gcaagaggcg aggggcggcg actggtgagt acgccaaaaa    1140
   ttttgactag cggaggctag aaggagagag atgggtgcga gagcgtcagt attaagcggg    1200
   ggagaattag atcgcgatgg gaaaaaattc ggttaaggcc agggggaaag aaaaaatata    1260
   aattaaaaca tatagtatgg gcaagcaggg agctagaacg attcgcagtt aatcctggcc    1320
   tgttagaaac atcagaaggc tgtagacaaa tactgggaca gctacaacca tcccttcaga    1380
   caggatcaga agaacttaga tcattatata atacagtagc aaccctctat tgtgtgcatc    1440
   aaaggataga gataaaagac accaaggaag ctttagacaa gatagaggaa gagcaaaaca    1500
   aaagtaagac caccgcacag caagcggccg ctgatcttca gacctggagg aggagatatg    1560
   agggacaatt ggagaagtga attatataaa tataaagtag taaaaattga accattagga    1620
   gtagcaccca ccaaggcaaa gagaagagtg gtgcagagag aaaaaagagc agtgggaata    1680
   ggagctttgt tccttgggtt cttgggagca gcaggaagca ctatgggcgc agcgtcaatg 1740
   acgctgacgg tacaggccag acaattattg tctggtatag tgcagcagca gaacaatttg 1800
   ctgagggcta ttgaggcgca acagcatctg ttgcaactca cagtctgggg catcaagcag 1860
   ctccaggcaa gaatcctggc tgtggaaaga tacctaaagg atcaacagct cctggggatt 1920
   tggggttgct ctggaaaact catttgcacc actgctgtgc cttggaatgc tagttggagt 1980
   aataaatctc tggaacagat ttggaatcac acgacctgga tggagtggga cagagaaatt 2040
   aacaattaca caagcttaat acactcctta attgaagaat cgcaaaacca gcaagaaaag 2100
   aatgaacaag aattattgga attagataaa tgggcaagtt tgtggaattg gtttaacata 2160
   acaaattggc tgtggtatat aaaattattc ataatgatag taggaggctt ggtaggttta 2220
   agaatagttt ttgctgtact ttctatagtg aatagagtta ggcagggata ttcaccatta 2280
   tcgtttcaga cccacctccc aaccccgagg ggacccgaca ggcccgaagg aatagaagaa 2340
   gaaggtggag agagagacag agacagatcc attcgattag tgaacggatc ggcactgcgt 2400
   gcgccaattc tgcagacaaa tggcagtatt catccacaat tttaaaagaa aaggggggat 2460
   tggggggtac agtgcagggg aaagaatagt agacataata gcaacagaca tacaaactaa 2520
   agaattacaa aaacaaatta caaaaattca aaattttcgg gtttattaca gggacagcag 2580
   agatccagtt tggttaatta agcattagtt attaatagta atcaattacg gggtcattag 2640
   ttcatagccc atatatggag ttccgcgtta cataacttac ggtaaatggc ccgcctggtt 2700
   gaccgcccaa cgacccccgc ccattgacgt caataatgac gtatgttccc atagtaacgc 2760
   caatagggac tttccattga cgtcaatggg tggagtattt acggtaaact gcccacttgg 2820
   tagtacatca agtgtatcat acgccaagta cgccccctat tgacgtcaat gacggtaaat 2880
   ggcccgcctg gtattatgcc cagtacatga ccttatggga ctttcctact tggcagtaca 2940
   tctgcgtatt agtcatcgct attaccatgg tgatgcggtt ttggcagtac atcaatgggc 3000
   gtggatagcg gtttgactca cggggatttc caagtctcca ccccattgac gtcaatggga 3060
   gtttgttttg gcaccaaaat caacgggact ttccaaaatg tcgtaacaac tccgccccat 3120
   tgacgcaaat gggcggtagg cgtgtacggt gggaggtcta tataagcaga gctggtttag 3180
   tgaaccgtca gatccgctag cattgtgtgc ttttcgggcc accatgacgc tgcggcttct 3240
   ggtggccgcg ctctgcgccg ggatcctggc agaggcgccc cgagtgcgag cccagcacag 3300
   ggagagagtg acctgcacgc gcctttacgc cgctgacatt gtgttcttac tggatggctc 3360
   ctcatccatt ggccgcagca atttccgcga ggtccgcagc tttctcgaag ggctggtgct 3420
   gcctttctct ggagcagcca gtgcacaggg tgtgcgcttt gccacagtgc agtacagcga 3480
   tgacccacgg acagagttcg gcctggatgc acttggctct gggggtgatg tgatccgcgc 3540
   catccgtgag cttagctaca aggggggcaa cactcgcaca ggggctgcaa ttctccatgt 3600
   ggctgaccat gtcttcctgc cccagctggc ccgacctggt gtccccaagg tctgcatcct 3660
   gatcacagac gggaagtccc aggacctggt ggacacagct gcccaaaggc tgaaggggca 3720
   gggggtcaag ctatttgctg tggggatcaa gaatgctgac cctgaggagc tgaagcgagt 3780
   tgcctcacag cccaccagtg acttcttctt cttcgtcaat gacttcagca tcttgaggac 3840
   actactgccc ctcgtttccc ggagagtgtg cacgactgct ggtggcgtgc ctgtgacccg 3900
   acctccggat gactcgacct ctgctccacg agacctggtg ctgtctgagc caagcagcca 3960
   atccttgaga gtacagtgga cagcggccag tggccctgtg actggctaca aggtccagta 4020
   cactcctctg acggggctgg gacagccact gccgagtgag cggcaggagg tgaacgtccc 4080
   agctggtgag accagtgtgc ggctgcgggg tctccggcca ctgaccgagt accaagtgac 4140
   tgtgattgcc ctctacgcca acagcatcgg ggaggctgtg agcgggacag ctcggaccac 4200
   tgccctagaa gggccggaac tgaccatcca gaataccaca gcccacagcc tcctggtggc 4260
   ctggcggagt gtgccaggtg ccactggcta ccgtgtgaca tggcgggtcc tcagtggtgg 4320
   gcccacacag cagcaggagc tgggccctgg gcagggttca gtgttgctgc gtgacttgga 4380
   gcctggcacg gactatgagg tgaccgtgag caccctattt ggccgcagtg tggggcccgc 4440
   cacttccctg atggctcgca ctgacgcttc tgttgagcag accctgcgcc cggtcatcct 4500
   gggccccaca tccatcctcc tttcctggaa cttggtgcct gaggcccgtg gctaccggtt 4560
   ggaatggcgg cgtgagactg gcttggagcc accgcagaag gtggtactgc cctctgatgt 4620
   gacccgctac cagttggatg ggctgcagcc gggcactgag taccgcctca cactctacac 4680
   tctgctggag ggccacgagg tggccacccc tgcaaccgtg gttcccactg gaccagagct 4740
   gcctgtgagc cctgtaacag acctgcaagc caccgagctg cccgggcagc gggtgcgagt 4800
   gtcctggagc ccagtccctg gtgccaccca gtaccgcatc attgtgcgca gcacccaggg 4860
   ggttgagcgg accctggtgc ttcctgggag tcagacagca ttcgacttgg atgacgttca 4920
   ggctgggctt agctacactg tgcgggtgtc tgctcgagtg ggtccccgtg agggcagtgc 4980
   cagtgtcctc actgtccgcc gggagccgga aactccactt gctgttccag ggctgcgggt 5040
   tgtggtgtca gatgcaacgc gagtgagggt ggcctgggga cccgtccctg gagccagtgg 5100
   atttcggatt agctggagca caggcagtgg tccggagtcc agccagacac tgcccccaga 5160
   ctctactgcc acagacatca cagggctgca gcctggaacc acctaccagg tggctgtgtc 5220
   ggtactgcga ggcagagagg agggccctgc tgcagtcatc gtggctcgaa cggacccact 5280
   gggcccagtg aggacggtcc atgtgactca ggccagcagc tcatctgtca ccattacctg 5340
   gaccagggtt cctggcgcca caggatacag ggtttcctgg cactcagccc acggcccaga 5400
   gaaatcccag ttggtttctg gggaggccac ggtggctgag ctggatggac tggagccaga 5460
   tactgagtat acggtgcatg tgagggccca tgtggctggc gtggatgggc cccctgcctc 5520
   tgtggttgtg aggactgccc ctgagcctgt gggtcgtgtg tcgaggctgc agatcctcaa 5580
   tgcttccagc gacgttctac ggatcacctg ggtaggggtc actggagcca cagcttacag 5640
   actggcctgg ggccggagtg aaggcggccc catgaggcac cagatactcc caggaaacac 5700
   agactctgca gagatccggg gtctcgaagg tggagtcagc tactcagtgc gagtgactgc 5760
   acttgtcggg gaccgcgagg gcacacctgt ctccattgtt gtcactacgc cgcctgaggc 5820
   tccgccagcc ctggggacgc ttcacgtggt gcagcgcggg gagcactcgc tgaggctgcg 5880
   ctgggagccg gtgcccagag cgcagggctt ccttctgcac tggcaacctg agggtggcca 5940
   ggaacagtcc cgggtcctgg ggcccgagct cagcagctat cacctggacg ggctggagcc 6000
   agcgacacag taccgcgtga ggctgagtgt cctagggccg gctggagaag ggccctctgc 6060
   agaggtgact gcgcgcactg agtcacctcg tgttccaagc attgaactac gtgtggtgga 6120
   cacctcgatc gactcggtga ctttggcctg gactccagtg tccagggcat ccagctacat 6180
   cctatcctgg cggccactca gaggccctgg ccaggaagtg cctgggtccc cgcagacact 6240
   tccagggatc tcaagctccc agcgggtgac agggctagag cctggcgtct cttacatctt 6300
   ctccctgacg cctgtcctgg atggtgtgcg gggtcctgag gcatctgtca cacagacgcc 6360
   agtgtgcccc cgtggcctgg cggatgtggt gttcctacca catgccactc aagacaatgc 6420
   tcaccgtgcg gaggctacga ggagggtcct ggagcgtctg gtgttggcac ttgggcctct 6480
   tgggccacag gcagttcagg ttggcctgct gtcttacagt catcggccct ccccactgtt 6540
   cccactgaat ggctcccatg accttggcat tatcttgcaa aggatccgtg acatgcccta 6600
   catggaccca agtgggaaca acctgggcac agccgtggtc acagctcaca gatacatgtt 6660
   ggcaccagat gctcctgggc gccgccagca cgtaccaggg gtgatggttc tgctagtgga 6720
   tgaacccttg agaggtgaca tattcagccc catccgtgag gcccaggctt ctgggcttaa 6780
   tgtggtgatg ttgggaatgg ctggagcgga cccagagcag ctgcgtcgct tggcgccggg 6840
   tatggactct gtccagacct tcttcgccgt ggatgatggg ccaagcctgg accaggcagt 6900
   cagtggtctg gccacagccc tgtgtcaggc atccttcact actcagcccc ggccagagcc 6960
   ctgcccagtg tattgtccaa agggccagaa gggggaacct ggagagatgg gcctgagagg 7020
   acaagttggg cctcctggcg accctggcct cccgggcagg accggtgctc ccggccccca 7080
   ggggccccct ggaagtgcca ctgccaaggg cgagaggggc ttccctggag cagatgggcg 7140
   tccaggcagc cctggccgcg ccgggaatcc tgggacccct ggagcccctg gcctaaaggg 7200
   ctctccaggg ttgcctggcc ctcgtgggga cccgggagag cgaggacctc gaggcccaaa 7260
   gggggagccg ggggctcccg gacaagtcat cggaggtgaa ggacctgggc ttcctgggcg 7320
   gaaaggggac cctggaccat cgggcccccc tggacctcgt ggaccactgg gggacccagg 7380
   accccgtggc cccccagggc ttcctggaac agccatgaag ggtgacaaag gcgatcgtgg 7440
   ggagcggggt ccccctggac caggtgaagg tggcattgct cctggggagc ctgggctgcc 7500
   gggtcttccc ggaagccctg gaccccaagg ccccgttggc ccccctggaa agaaaggaga 7560
   aaaaggtgac tctgaggatg gagctccagg cctcccagga caacctgggt ctccgggtga 7620
   gcagggccca cggggacctc ctggagctat tggccccaaa ggtgaccggg gctttccagg 7680
   gcccctgggt gaggctggag agaagggcga acgtggaccc ccaggcccag cgggatcccg 7740
   ggggctgcca ggggttgctg gacgtcctgg agccaagggt cctgaagggc caccaggacc 7800
   cactggccgc caaggagaga agggggagcc tggtcgccct ggggaccctg cagtggtggg 7860
   acctgctgtt gctggaccca aaggagaaaa gggagatgtg gggcccgctg ggcccagagg 7920
   agctaccgga gtccaagggg aacggggccc acccggcttg gttcttcctg gagaccctgg 7980
   ccccaaggga gaccctggag accggggtcc cattggcctt actggcagag caggaccccc 8040
   aggtgactca gggcctcctg gagagaaggg agaccctggg cggcctggcc ccccaggacc 8100
   tgttggcccc cgaggacgag atggtgaagt tggagagaaa ggtgacgagg gtcctccggg 8160
   tgacccgggt ttgcctggaa aagcaggcga gcgtggcctt cggggggcac ctggagttcg 8220
   ggggcctgtg ggtgaaaagg gagaccaggg agatcctgga gaggatggac gaaatggcag 8280
   ccctggatca tctggaccca agggtgaccg tggggagccg ggtcccccag gacccccggg 8340
   acggctggta gacacaggac ctggagccag agagaaggga gagcctgggg accgcggaca 8400
   agagggtcct cgagggccca agggtgatcc tggcctccct ggagcccctg gggaaagggg 8460
   cattgaaggg tttcggggac ccccaggccc acagggggac ccaggtgtcc gaggcccagc 8520
   aggagaaaag ggtgaccggg gtccccctgg gctggatggc cggagcggac tggatgggaa 8580
   accaggagcc gctgggccct ctgggccgaa tggtgctgca ggcaaagctg gggacccagg 8640
   gagagacggg cttccaggcc tccgtggaga acaaggcctc cctggcccct ctggtccccc 8700
   tggattaccg ggaaagccag gcgaggatgg gaaacctggc ctgaatggaa aaaacggaga 8760
   acctggggac cctggagaag acgggaggaa gggagagaaa ggagattcag gcgcctctgg 8820
   gagagaaggt cgtgatggcc ccaagggtga gcgtggagct cctggtatcc ttggacccca 8880
   ggggcctcca ggcctcccag ggccagtggg ccctcctggc cagggttttc ctggtgtccc 8940
   aggaggcacg ggccccaagg gtgaccgtgg ggagactgga tccaaagggg agcagggcct 9000
   ccctggagag cgtggcctgc gaggagagcc tggaagtgtg ccgaatgtgg atcggttgct 9060
   ggaaactgct ggcatcaagg catctgccct gcgggagatc gtggagacct gggatgagag 9120
   ctctggtagc ttcctgcctg tgcccgaacg gcgtcgaggc cccaaggggg actcaggcga 9180
   acagggcccc ccaggcaagg agggccccat cggctttcct ggagaacgcg ggctgaaggg 9240
   cgaccgtgga gaccctggcc ctcaggggcc acctggtctg gcccttgggg agaggggccc 9300
   ccccgggcct tccggccttg ccggggagcc tggaaagcct ggtattcccg ggctcccagg 9360
   cagggctggg ggtgtgggag aggcaggaag gccaggagag aggggagaac ggggagagaa 9420
   aggagaacgt ggagaacagg gcagagatgg ccctcctgga ctccctggaa cccctgggcc 9480
   ccccggaccc cctggcccca aggtgtctgt ggatgagcca ggtcctggac tctctggaga 9540
   acagggaccc cctggactca agggtgctaa gggggagccg ggcagcaatg gtgaccaagg 9600
   tcccaaagga gacaggggtg tgccaggcat caaaggagac cggggagagc ctggaccgag 9660
   gggtcaggac ggcaacccgg gtctaccagg agagcgtggt atggctgggc ctgaagggaa 9720
   gccgggtctg cagggtccaa gaggcccccc tggcccagtg ggtggtcatg gagaccctgg 9780
   accacctggt gccccgggtc ttgctggccc tgcaggaccc caaggacctt ctggcctgaa 9840
   gggggagcct ggagagacag gacctccagg acggggcctg actggaccta ctggagctgt 9900
   gggacttcct ggaccccccg gcccttcagg ccttgtgggt ccacaggggt ctccaggttt 9960
   gcctggacaa gtgggggaga cagggaagcc gggagcccca ggtcgagatg gtgccagtgg 10020
   aaaagatgga gacagaggga gccctggtgt gccagggtca ccaggtctgc ctggccctgt 10080
   cggacctaaa ggagaacctg gccccacggg ggcccctgga caggctgtgg tcgggctccc 10140
   tggagcaaag ggagagaagg gagcccctgg aggccttgct ggagacctgg tgggtgagcc 10200
   gggagccaaa ggtgaccgag gactgccagg gccgcgaggc gagaagggtg aagctggccg 10260
   tgcaggggag cccggagacc ctggggaaga tggtcagaaa ggggctccag gacccaaagg 10320
   tttcaagggt gacccaggag tcggggtccc gggctcccct gggcctcctg gccctccagg 10380
   tgtgaaggga gatctgggcc tccctggcct gcccggtgct cctggtgttg ttgggttccc 10440
   gggtcagaca ggccctcgag gagagatggg tcagccaggc cctagtggag agcggggtct 10500
   ggcaggcccc ccagggagag aaggaatccc aggacccctg gggccacctg gaccaccggg 10560
   gtcagtggga ccacctgggg cctctggact caaaggagac aagggagacc ctggagtagg 10620
   gctgcctggg ccccgaggcg agcgtgggga gccaggcatc cggggtgaag atggccgccc 10680
   cggccaggag ggaccccgag gactcacggg gccccctggc agcaggggag agcgtgggga 10740
   gaagggtgat gttgggagtg caggactaaa gggtgacaag ggagactcag ctgtgatcct 10800
   ggggcctcca ggcccacggg gtgccaaggg ggacatgggt gaacgagggc ctcggggctt 10860
   ggatggtgac aaaggacctc ggggagacaa tggggaccct ggtgacaagg gcagcaaggg 10920
   agagcctggt gacaagggct cagccgggtt gccaggactg cgtggactcc tgggacccca 10980
   gggtcaacct ggtgcagcag ggatccctgg tgacccggga tccccaggaa aggatggagt 11040
   gcctggtatc cgaggagaaa aaggagatgt tggcttcatg ggtccccggg gcctcaaggg 11100
   tgaacgggga gtgaagggag cctgtggcct tgatggagag aagggagaca agggagaagc 11160
   tggtccccca ggccgccccg ggctggcagg acacaaagga gagatggggg agcctggtgt 11220
   gccgggccag tcgggggccc ctggcaagga gggcctgatc ggtcccaagg gtgaccgagg 11280
   ctttgacggg cagccaggcc ccaagggtga ccagggcgag aaaggggagc ggggaacccc 11340
   aggaattggg ggcttcccag gccccagtgg aaatgatggc tctgctggtc ccccagggcc 11400
   acctggcagt gttggtccca gaggccccga aggacttcag ggccagaagg gtgagcgagg 11460
   tccccccgga gagagagtgg tgggggctcc tggggtccct ggagctcctg gcgagagagg 11520
   ggagcagggg cggccagggc ctgccggtcc tcgaggcgag aagggagaag ctgcactgac 11580
   ggaggatgac atccggggct ttgtgcgcca agagatgagt cagcactgtg cctgccaggg 11640
   ccagttcatc gcatctggat cacgacccct ccctagttat gctgcagaca ctgccggctc 11700
   ccagctccat gctgtgcctg tgctccgcgt ctctcatgca gaggaggaag agcgggtacc 11760
   ccctgaggat gatgagtact ctgaatactc cgagtattct gtggaggagt accaggaccc    11820
   tgaagctcct tgggatagtg atgacccctg ttccctgcca ctggatgagg gctcctgcac    11880
   tgcctacacc ctgcgctggt accatcgggc tgtgacaggc agcacagagg cctgtcaccc    11940
   ttttgtctat ggtggctgtg gagggaatgc caaccgtttt gggacccgtg aggcctgcga    12000
   gcgccgctgc ccaccccggg tggtccagag ccaggggaca ggtactgccc aggactgatc    12060
   gataccgtcg acctcgagac ctagaaaaac atggagcaat cacaagtagc aatacagcag    12120
   ctaccaatgc tgattgtgcc tggctagaag cacaagagga ggaggaggtg ggttttccag    12180
   tcacacctca ggtaccttta agaccaatga cttacaaggc agctgtagat cttagccact    12240
   ttttaaaaga aaagggggga ctggaagggc taattcactc ccaacgaaga caagatatcc    12300
   ttgatctgtg gatctaccac acacaaggct acttccctga ttggcagaac tacacaccag    12360
   ggccagggat cagatatcca ctgacctttg gatggtgcta caagctagta ccagttgagc    12420
   aagagaaggt agaagaagcc aatgaaggag agaacacccg cttgttacac cctgtgagcc    12480
   tgcatgggat ggatgacccg gagagagaag tattagagtg gaggtttgac agccgcctag    12540
   catttcatca catggcccga gagctgcatc cggactgtac tgggtctctc tggttagacc    12600
   agatctgagc ctgggagctc tctggctaac tagggaaccc actgcttaag cctcaataaa    12660
   gcttgccttg agtgcttcaa gtagtgtgtg cccgtctgtt gtgtgactct ggtaactaga    12720
   gatccctcag acccttttag tcagtgtgga aaatctctag cagggcccgt ttaaacccgc    12780
   tgatcagcct cgactgtgcc ttctagttgc cagccatctg ttgtttgccc ctcccccgtg    12840
   ccttccttga ccctggaagg tgccactccc actgtccttt cctaataaaa tgaggaaatt    12900
   gcatcgcatt gtctgagtag gtgtcattct attctggggg gtggggtggg gcaggacagc    12960
   aagggggagg attgggaaga caatagcagg catgctgggg atgcggtggg ctctatggct    13020
   tctgaggcgg aaagaaccag ctggggctct agggggtatc cccacgcgcc ctgtagcggc    13080
   gcattaagcg cggcgggtgt ggtggttacg cgcagcgtga ccgctacact tgccagcgcc    13140
   ctagcgcccg ctcctttcgc tttcttccct tcctttctcg ccacgttcgc cggctttccc    13200
   cgtcaagctc taaatcgggg gctcccttta gggttccgat ttagtgcttt acggcacctc    13260
   gaccccaaaa aacttgatta gggtgatggt tcacgtagtg ggccatcgcc ctgatagacg    13320
   gtttttcgcc ctttgacgtt ggagtccacg ttctttaata gtggactctt gttccaaact    13380
   ggaacaacac tcaaccctat ctcggtctat tcttttgatt tataagggat tttgccgatt    13440
   tcggcctatt ggttaaaaaa tgagctgatt taacaaaaat ttaacgcgaa ttaattctgt 13500
   ggaatgtgtg tcagttaggg tgtggaaagt ccccaggctc cccagcaggc agaagtatgc 13560
   aaagcatgca tctcaattag tcagcaacca ggtgtggaaa gtccccaggc tccccagcag 13620
   gcagaagtat gcaaagcatg catctcaatt agtcagcaac catagtcccg cccctaactc 13680
   cgcccatccc gcccctaact ccgcccagtt ccgcccattc tccgccccat ggctgactaa 13740
   ttttttttat ttatgcagag gccgaggccg cctctgcctc tgagctattc cagaagtagt 13800
   gaggaggctt ttttggaggc ctaggctttt gcaaaaagct cccgggagct tgtatatcca 13860
   ttttcggatc tgatcagcac gtgttgacaa ttaatcatcg gcatagtata tcggcatagt 13920
   ataatacgac aaggtgagga actaaaccat ggccaagttg accagtgccg ttccggtgct 13980
   caccgcgcgc gacgtcgccg gagcggtcga gttctggacc gaccggctcg ggttctcccg 14040
   ggacttcgtg gaggacgact tcgccggtgt ggtccgggac gacgtgaccc tgttcatcag 14100
   cgcggtccag gaccaggtgg tgccggacaa caccctggcc tgggtgtggg tgcgcggcct 14160
   ggacgagctg tacgccgagt ggtcggaggt cgtgtccacg aacttccggg acgcctccgg 14220
   gccggccatg accgagatcg gcgagcagcc gtgggggcgg gagttcgccc tgcgcgaccc 14280
   ggccggcaac tgcgtgcact tcgtggccga ggagcaggac tgacacgtgc tacgagattt 14340
   cgattccacc gccgccttct atgaaaggtt gggcttcgga atcgttttcc gggacgccgg 14400
   ctggatgatc ctccagcgcg gggatctcat gctggagttc ttcgcccacc ccaacttgtt 14460
   tattgcagct tataatggtt acaaataaag caatagcatc acaaatttca caaataaagc 14520
   atttttttca ctgcattcta gttgtggttt gtccaaactc atcaatgtat cttatcatgt 14580
   ctgtataccg tcgacctcta gctagagctt ggcgtaatca tggtcatagc tgtttcctgt 14640
   gtgaaattgt tatccgctca caattccaca caacatacga gccggaagca taaagtgtaa 14700
   agcctggggt gcctaatgag tgagctaact cacattaatt gcgttgcgct cactgcccgc 14760
   tttccagtcg ggaaacctgt cgtgccagct gcattaatga atcggccaac gcgcggggag 14820
   aggcggtttg cgtattgggc gctcttccgc ttcctcgctc actgactcgc tgcgctcggt 14880
   cgttcggctg cggcgagcgg tatcagctca ctcaaaggcg gtaatacggt tatccacaga 14940
   atcaggggat aacgcaggaa agaacatgtg agcaaaaggc cagcaaaagg ccaggaaccg 15000
   taaaaaggcc gcgttgctgg cgtttttcca taggctccgc ccccctgacg agcatcacaa 15060
   aaatcgacgc tcaagtcaga ggtggcgaaa cccgacagga ctataaagat accaggcgtt 15120
   tccccctgga agctccctcg tgcgctctcc tgttccgacc ctgccgctta ccggatacct 15180
   gtccgccttt ctcccttcgg gaagcgtggc gctttctcat agctcacgct gtaggtatct 15240
   cagttcggtg taggtcgttc gctccaagct gggctgtgtg cacgaacccc ccgttcagcc 15300
   cgaccgctgc gccttatccg gtaactatcg tcttgagtcc aacccggtaa gacacgactt 15360
   atcgccactg gcagcagcca ctggtaacag gattagcaga gcgaggtatg taggcggtgc 15420
   tacagagttc ttgaagtggt ggcctaacta cggctacact agaagaacag tatttggtat 15480
   ctgcgctctg ctgaagccag ttaccttcgg aaaaagagtt ggtagctctt gatccggcaa 15540
   acaaaccacc gctggtagcg gtggtttttt tgtttgcaag cagcagatta cgcgcagaaa 15600
   aaaaggatct caagaagatc ctttgatctt ttctacgggg tctgacgctc agtggaacga 15660
   aaactcacgt taagggattt tggtcatgag attatcaaaa aggatcttca cctagatcct 15720
   tttaaattaa aaatgaagtt ttaaatcaat ctaaagtata tatgagtaaa cttggtctga 15780
   cagttaccaa tgcttaatca gtgaggcacc tatctcagcg atctgtctat ttcgttcatc 15840
   catagttgcc tgactccccg tcgtgtagat aactacgata cgggagggct taccatctgg 15900
   ccccagtgct gcaatgatac cgcgagaccc acgctcaccg gctccagatt tatcagcaat 15960
   aaaccagcca gccggaaggg ccgagcgcag aagtggtcct gcaactttat ccgcctccat 16020
   ccagtctatt aattgttgcc gggaagctag agtaagtagt tcgccagtta atagtttgcg 16080
   caacgttgtt gccattgcta caggcatcgt ggtgtcacgc tcgtcgtttg gtatggcttc 16140
   attcagctcc ggttcccaac gatcaaggcg agttacatga tcccccatgt tgtgcaaaaa 16200
   agcggttagc tccttcggtc ctccgatcgt tgtcagaagt aagttggccg cagtgttatc 16260
   actcatggtt atggcagcac tgcataattc tcttactgtc atgccatccg taagatgctt 16320
   ttctgtgact ggtgagtact caaccaagtc attctgagaa tagtgtatgc ggcgaccgag 16380
   ttgctcttgc ccggcgtcaa tacgggataa taccgcgcca catagcagaa ctttaaaagt 16440
   gctcatcatt ggaaaacgtt cttcggggcg aaaactctca aggatcttac cgctgttgag 16500
   atccagttcg atgtaaccca ctcgtgcacc caactgatct tcagcatctt ttactttcac 16560
   cagcgtttct gggtgagcaa aaacaggaag gcaaaatgcc gcaaaaaagg gaataagggc 16620
   gacacggaaa tgttgaatac tcatactctt cctttttcaa tattattgaa gcatttatca 16680
   gggttattgt ctcatgagcg gatacatatt tgaatgtatt tagaaaaata aacaaatagg 16740
   ggttccgcgc acatttcccc gaaaagtgcc acctgac 16777

## Description

### SEQUENCE LISTING

The instant application contains a Sequence Listing which has been submitted electronically in ASCII format Said ASCII copy, created on March 9, 2017, is named 0100-0016PR1_SL.txt and is 92,924 bytes in size.

### TECHNICAL FIELD OF THE INVENTION

The present invention relates to compositions for the treatment of Type VII collagen deficiencies such as dystrophic epidermolysis bullosa comprising autologous genetically modified cells comprising a nucleotide sequence encoding Type VII collagen (C7) or a functional variant thereof.

### BACKGROUND OF THE INVENTION

Type VII collagen (C7) is a protein important for anchoring fibril formation at the dermal-epidermal junction (DEJ), which holds together the layers of skin (Burgeson 1993; Leigh *et al.* 1988). Dystrophic epidermolysis bullosa (DEB) is an inherited disease characterized by a deficiency in C7 resulting in an impairment of anchoring fibrils to adhere between the epidermis and the underlying dermis affecting the skin and organs. Mutations of the COL7A1 gene encoding C7 causes the C7 deficiency. Patients suffering from DEB are highly susceptible to severe blistering. Dominant dystrophic epidermolysis bullosa (DDEB) is characterized by generalized blistering. Transient bullous dermatosis is a form of DDEB, which corrects itself during infancy. Recessive dystrophic epidermolysis bullosa (RDEB) is a debilitating skin-blistering disorder. RDEB inversa is another form of RDEB, where blisters form on areas where skin rubs on skin. Without these fibrils, skin layers separate causing severe blistering, open wounds and scarring in response to any kind of friction, including normal daily activities like rubbing or scratching.

There are currently no curative treatments and patients largely rely on palliative wound care. Preclinical studies employing a number of strategies show encouraging results suggesting that the effects of the disease can be corrected by restoring C7 function in RDEB skin. Initial approaches relied on grafting fragile engineered epidermal tissue and require removal of the skin tissue to be replaced, resulting in scarring (Mavilio *et al.* 2006; Chen, Nat. Genet. 2002; Siprashvili 2010). Other methods have included delivery of the corrective C7 by direct virus-based expression and systemic delivery of recombinant protein (Remington 2009; Woodley *et al.* 2003). All of these potential treatments have a number of drawbacks including cost and biosafety concerns.

The use of replication-defective, self-inactivating (SIN) lentiviral vectors (LV) offers several advantages for gene therapy: 1) ability to transduce both dividing and non-dividing cells, 2) high transduction efficiency, 3) long-term sustained transgene expression, 4) the ability to dispense sequences encoding viral proteins that might trigger an immune response, 5) an increased safety profile due to transcriptionally inactive SIN long terminal repeat (LTR), and 6) an ability to package large transgenes. Safety studies from clinical trials using LV vectors for gene therapy have showed no preferential integration in or near proto-oncogenes or tumor suppressor genes. Patients who have been dosed at 10 billion LV vector-transduced T cells per subject and followed for a median of 4 years, showed no leukemia or other adverse events. Trials with the indications of metachromatic leukodystrophy, Wiskott-Aldrich Syndrome (WAS) and X- adrenoleukodystrophy (ALD), revealed no aberrant clonal expansion seen up to 21 months, 32 months, and 36 months of follow up, respectively (Biffi *et al.* 2013; Aiuti *et al.* 2013).

Treatment with autologous genetically-modified human dermal fibroblasts (GM-HDF) offers a promising alternative based on correcting the hereditary defect ex vivo in the patients' own fibroblasts (Ortiz-Urda *et al.* 2003; Woodley *et al.* 2003). Georgiadis et al. (J. Investigative Dermatology 136:284-92 (2015)) described transduction of RDEB fibroblasts with a self-inactivating-lentiviral vector encoding codon-optimized COL7A1. It has been reported that hCOL7A1 gene-corrected fibroblasts are better than keratinocytes at supplying C7 to the basement membrane zone (BMZ) in mice with RDEB skin grafts (Goto *et al.* 2006). In addition, injection of fibroblasts can be more easily completed than grafting of keratinocytes.

### SUMMARY OF THE INVENTION

As described but not claimed herein, treating a patient suffering from a Type VII collagen (C7) deficiency may compise obtaining cells from the dermis or epidermis, preferably fibroblasts or keratinocytes, of a C7-deficient patient, contacting the cells with a transducing lentiviral vector particle comprising the *COL7A1* gene or a functional variant thereof to form an autologous genetically modified cell comprising the *COL7A1* gene or functional variant thereof having a vector copy number wherein said lentiviral vector particle has a transducing vector copy number in the range of 0.1 to 5.0 copies per cell, culturing said autologous genetically modified cell, and administering the genetically modified cells to the C7-deficient patient. The administration may be done by injection, preferably intradermal injection. The C7 deficiency may be dystrophic epidermolysis bullosa (DEB), such as recessive dystrophic epidermolysis bullosa (RDEB) or dominant dystrophic epidermolysis bullosa (DDEB). Subtypes of RDEB may also be treated including Hallopeau-Siemens, non-Hallopeau-Siemens RDEB, RDEB inversa, pretibial RDEB, acral RDEB, and RDEB centripetalis.

The transducing lentiviral vector particle may be constructed from a transfer lentiviral vector comprising (a) a modified 5' long terminal repeat in LTR, wherein the promoter of the modified 5' LTR is a cytomegalovirus promoter, (b) the *COL7A1* gene or a functional variant thereof, (c) at least one lentiviral central polypurine tract, (d) a hepatitis B virus post- transcriptional regulatory element (PRE), and (e) a modified 3' LTR, wherein the modified 3' LTR comprises a deletion relative to the wild-type 3' LTR, wherein the *COL7A1* gene or functional variant is incorporated into the cells to form genetically modified cells having a functional *COL7A1* gene or functional variant thereof.

There may be at least two lentiviral central polypurine tract elements. The PRE may be woodchuck hepatitis virus post-transcriptional regulatory element (WPRE). Preferably, the transfer lentiviral vector used to construct the transducing vector is selected from the group consisting of pSMPUW and pFUGW. Preferably, the transducing lentiviral vector particle is INXN-2004 or INXN-2002.

Administration of the genetically modified fibroblasts autologous to the C7-deficient patient may be done in any suitable manner, including by injection, topically, orally, or embedded in a biocompatible matrix.

The present invention is directed to an autologous genetically modified fibroblast from a patient suffering from a dystrophic epidermolysis bullosa (DEB) or pseudosyndactyly transduced with a lentiviral vector, wherein the lentiviral vector is replication defective and self-inactivating and is derived from the human immunodeficiency virus (HIV-1) which is pseudotyped with a heterologous VSV-G envelope protein instead of the HIV-1 envelope protein, comprising a nucleotide sequence encoding a functional *COL7A1* gene or a functional variant thereof, and expressing type VII collagen, wherein transduction of an autologous population of fibroblast cells with the VSV-G pseudotyped lentiviral vector results in a transducing vector copy number in the range of 0.1 to 5.0 copies per cell, wherein the transducing lentiviral vector comprises (a) a modified 5' long terminal repeat in LTR, wherein the promoter of the modified 5' LTR is a cytomegalovirus promoter, (b) the functional *COL7A1* gene or functional variant thereof, (c) at least one lentiviral central polypurine tract element, and (d) a modified 3' LTR, wherein the modified 3' LTR lacks a post-transcriptional regulatory element (PRE), wherein the functional *COL7A1* gene or functional variant thereof is incorporated into the fibroblast to form a genetically modified fibroblast having the functional *COL7A1* gene or functional variant thereof.

The self-inactivating lentiviral vector may be formed from a transfer vector comprising (a) a modified 5' long terminal in LTR, wherein the promoter of the modified 5' LTR is a cytomegalovirus promoter, (b) the *COL7A1* gene or a functional variant thereof, (c) at least one lentiviral central polypurine tract element, (d) a hepatitis B virus post-transcriptional regulatory element (PRE), and (e) a modified 3' LTR, wherein the modified 3' LTR comprises a deletion relative to the wild-type 3' LTR. The vector may comprise at least two lentiviral central polypurine tract elements and the woodchuck hepatitis virus post-transcriptional regulatory element (WPRE). The vector of the invention includes the vector designated INXN-2004 and comprising the sequence of IGE308 plasmid; or the vector designated INXN-2002.

Another embodiment of the invention is directed to pharmaceutical compositions comprising an autologus genetically modified fibroblast according to the claims. The fibroblast may be obtained from a patient suffering from RDEB or DDEB transduced with a lentiviral vector designated INXN-2004 comprising the sequence of IGE308 plasmid; or transduced with a lentiviral vector designated INXN-2002.

Cells may be transduced in vitro or ex vivo using the vectors, such as INXN-2002 and INXN-2004.

Transduced autologous fibroblasts using vectors INXN-2002 or INXN-2004 may be obtained and propagated according to methods described in U.S. Patent No. 8,728,819 and International Patent Application.

WO2008/027984 entitled "Methods for culturing minimally-passaged fibroblasts and uses thereof".

Described but not claimed herein is the use of concentrated lentivirus for transduction of human autologous dermal cells, such as fibroblasts and/or keratinocytes.

Described but not claimed herein is the transduction of human autologous dermal cells, such as fibroblasts and/or keratinocytes via centrifugation with lentivirus.

Described but not claimed herein is the use of both concentrated lentivirus and centrifugation for transduction of human autologous dermal cells, such as fibroblasts and/or keratinocytes.

Described but not claimed herein is the use of human autologous dermal cells, such as fibroblasts and/or keratinocytes, which have been contacted with lentivirus in two or more separate transduction processes.

Described but not claimed herein is the use of human autologous dermal cells, such as fibroblasts and/or keratinocytes, which have been contacted with lentivirus in two or more separate transduction processes, wherein the cells are cultured and passaged one, two, or three times, before the second or any subsequent transduction process.

Described but not claimed herein is the medical use for treatment of patients suffering from pseudosyndactyly comprising administering to said patient an autologous population of cells obtained from said patient transduced with a lentiviral vector particle comprising a functional *COL7A1* gene or its functional variant thereof, and expressing type VII collagen.

Described but not claimed herein are medical uses for treating, inhibiting or reducing the blistering of a dystrophic epidermolysis bullosa (DEB) or treating lesions in patients suffering from RDEB comprising administering the autologous genetically modified cells of the present invention.

Described but not claimed herein is an isolated population of genetically modified fibroblasts autologous to a C7-deficient patient, such as DDEB or RDEB patient, transduced with a vector particle comprising a functional *COL7A1* gene or functional variant thereof and expressing type VII collagen, as well as methods of making these isolated populations.

Described but not claimed herein is a method of increasing the integrated transgene copy number per cell in genetically modified human dermal fibroblasts or keratinocytes comprising contacting a transducing lentiviral vector comprising a nucleotide sequence encoding a *COL7A1* gene or a functional variant thereof with a human dermal fibroblast or keratinocyte obtained from a C7-deficient patient to form a transduction composition. The transduction composition is subjected to spinoculation to form transduced human dermal fibroblast or keratinocyte, wherein the integrated transgene copy number of the transduced human dermal fibroblasts or keratinocytes is higher relative to a transduction composition not subjected to spinoculation. A super-transduction step may be performed, wherein the transduced cells are further contacted with a second transducing lentiviral vector to form a second trandusction composition, which is optionally subjected to spinoculation. The transducing lentiviral vector may INXN-2002 or INXN-2004, preferably INXN-2004. It has been found that the the transduced human dermal fibroblast or keratinocytes have at least or about 1, 2, 5, 10, 20, 25, 27, 28, 29, 30, 35, 40, 45, or 50-fold greater integrated transgene copy number per cell relative to a transduction composition not subjected to spinoculation or super-transduction. An integrated transgene copy number per cell may be at least 0.05, 0.09, 0.41, or 0.74 or a range of between about 0.1 to about 5, 0.1 to about 1, 0.4 to about 1, or 0.4 to about 0.75. Expression has been found to be increased using the transduction methods described herein. For example, it has been found that expression of C7 has increased by 10, 25, 50, 100, 150, or 200-fold relative to transduced human dermal fibroblasts or keratinocytes not subjected to spinoculation or a second transduction.

### BRIEF DESCRIPTION OF THE DRAWINGS

A more complete understanding of the present invention may be obtained by reference to the accompanying drawings, when considered in conjunction with the subsequent detailed description.
Figure 1A depicts type VII collagen (C7) trimers, which form anchoring fibrils. The *COL7A1* gene encodes a 290-kDa alpha chain and three of the chains form a triple helix (trimer). Image from Bruckner- Tuderman L. Molecular Therapy (2008) 17:6-7.
Figure 1B depicts the general structure of normal and RDEB skin. C7 anchoring fibrils bind to other collagens, extracellular matrix proteins, and Lam332 to mediate attachment of the dermis to the epidermis. Absence of anchoring fibrils can lead to blister formation.

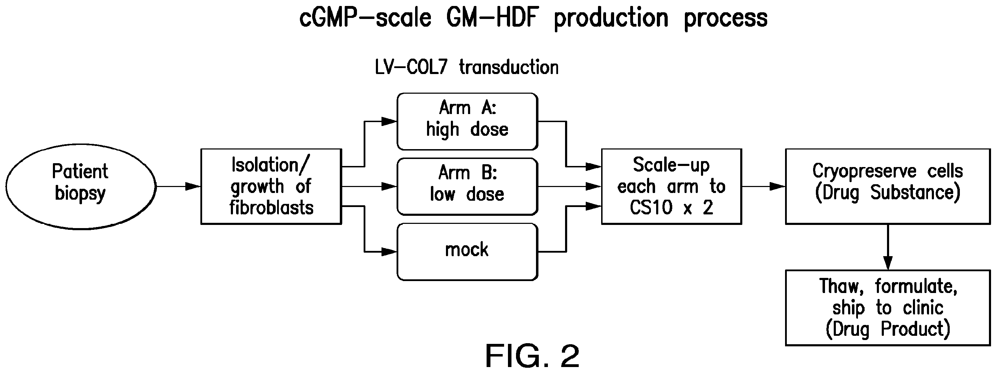

Figure 2 describes a cGMP-scale GM-HDF production process according to one aspect of the present invention. A C7 expression cassette was cloned into a self-inactivating lentivirus backbone. A pilot-scale production of lentiviral vector (INXN-2002) comprising *COL7A1* gene (LV-COL7) with a titer of ~9 x 10⁶ IU/mL was generated for use in the cGMP-scale production process. Fibroblasts were isolated from RDEB patient biopsies, grown, then split into three arms for mock, high-, and low-dose LV-COL7 transduction. Each arm of fibroblasts was grown to 2 x CS10 scale then cryopreserved (Drug Substance). For patient treatment, Drug Substance vials are thawed and formulated (Drug Product), then shipped back to the clinic for wound-site injection of the originating patient.

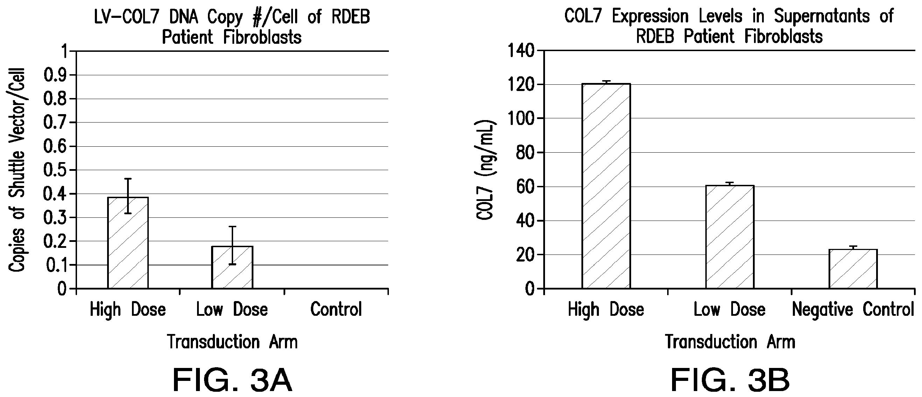

Figure 3A provides the LV-COL7 (for INXN-2002) copy number per cell. Drug Substance vials were thawed and assayed for LV-COL7 DNA copies per cell using qPCR. Primers were specific for the LV shuttle vector. Results demonstrate dose-dependent levels of copies per cell with an average of 0.38 and 0.18 copies from the High and Low Dose arms, respectively.
Figure 3B provides the C7 expression levels produced by RDEB patient fibroblasts transduced with LV-COL7: Drug Substance vials were thawed and cultured for 3 days. Conditioned cell culture supernatants were collected and assayed for C7 levels by ELISA. Results show virus dose-dependent protein expression that ranges from 60 to 120 ng/mL C7 in LV-COL7-transduced cells.

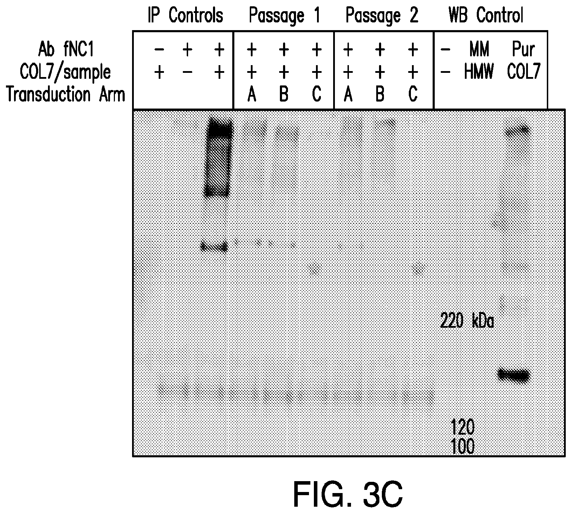

Figure 3C displays the trimeric form of C7 produced by RDEB patient fibroblasts transduced with LV- COL7: Conditioned cell culture supernatants were also used in an immunoprecipitation assay. Immunoprecipitated C7 was separated on non-denaturing SDS-PAGE and visualized by western blot. The C7 produced by RDEB fibroblasts was predominantly trimeric with LV-COL7-transduced cells expressing more COL7 than mock-transduced cells (Arm C). Some lower molecular weight species showing immunoreactivity, were also observed.

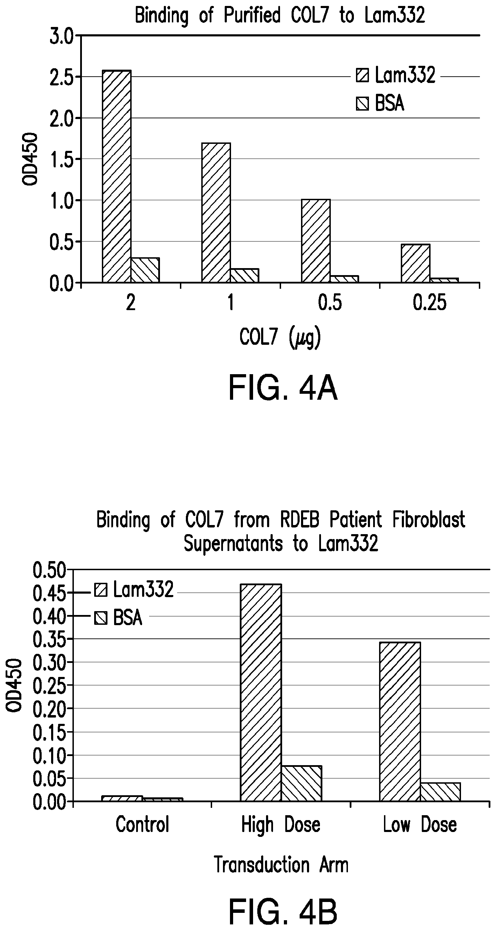

Figure 4A depicts binding of purified COL7 to Lam332. COL7 was expressed in CHO-DG44 cells, was purified by size exclusion chromatography, and was assayed for preferential binding to Lam332 as compared to BSA to establish the assay (increase in OD450 corresponds to increase in COL7 binding to Laminin332).
Figure 4B depicts Lam332 binding of COL7 in LV-COL7-transduced fibroblasts (as transduced by the INXN-2002 vector) culture supernatants. Drug Substance vials were thawed and cultured for 3 days. Conditioned cell culture supernatants were collected and assayed for binding to Lam332 compared with BSA control. Results show virus dose-dependent binding to Lam332.

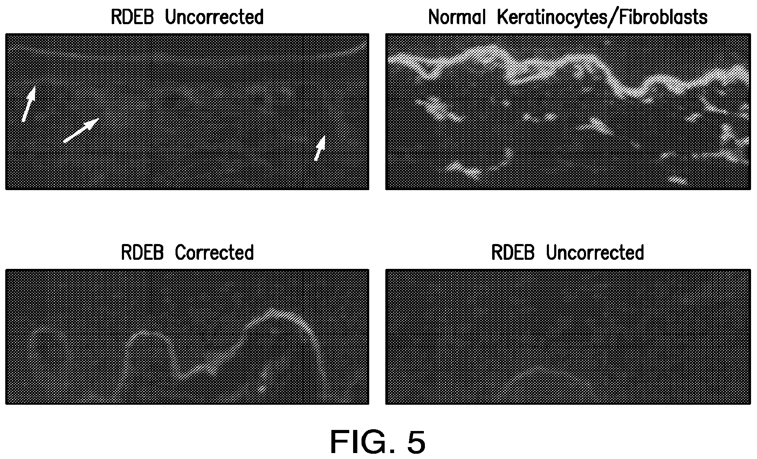

Figure 5 depicts composite RDEB skin grafts on the dorsum of SCID mice that were injected intradermally with 1 x 10⁶ GM-HDF (as transduced by the INXN-2002 vector) and analyzed by immunofluorescent staining with human COL7 specific antibodies. Representative images are shown. Localization of COL7 was observed in composite grafts (n=4) generated with RDEB keratinocytes at Day 10-post intradermal injection of 1 x 10⁶ GM-HDF. Positive control grafts generated from normal keratinocytes and fibroblasts showed intense COL7 staining and negative control grafts did not show COL7 staining at baseline measurements (arrows at DEJ for negative baseline comparison).
Figure 6 provides a schematic of the pSMPUW lentiviral expression plasmid vector.
Figure 7 compares the features of the pSMPUW lentiviral expression vector relative to 3rd generation lentivirus expression vectors.
Figure 8 provides a schematic of the pFUGW lentiviral expression plasmid vector.
Figure 9 depicts an electron micrograph of HIV-1 virus particles.
Figure 10 provides the immunoprecipitation results for TR8 GM-HDFs detecting the formation of C7 trimers.
Figure 11A provides the immunoprecipitation results for TR10 and ER1 GM-HDFs detecting the formation of C7 trimers.
Figure 11B provides the immunoprecipitation results for TR9 detecting the formation of C7 trimers.
Figure 12 shows the results of Lam332 binding results by C7 as expressed by GM-HDFs.
Figure 13 provides a schematic of the lentiviral vector plasmid construct, which encodes *a COL7A1* gene.
Figure 14 presents a schematic of the proviral RNA genome structure of the INXN-2002 lentiviral vector.
Figure 15 shows a schematic depicting the details of the mature INXN-2002 virus particle.
Figure 16 provides a schematic of the *in vitro* immortalization assay to assess the potential for insertional genotoxicity of INXN-2002.
Figure 17 represents typical cell morphology and structures of FXC-007 in culture.
Figure 18 provides a graph of the C7 expression levels as determined by ELISA for particular dosages of the TR8 GM-HDFs.
Figure 19 provides the western blot showing the immunoprecipitation of C7 trimers against NC1-specific antibodies as produced by FCX-007 GM-HDFs.
Figure 20 shows the assay readout (optical density at 450 nm (OD450)) for a binding assay to detect the interaction of C7 with laminin 332 or bovine serum albumin (BSA)-coated wells. Bound C7 was detecting using a C7 NC1-specific antibody and an HRP-conjugated secondary antibody.
Figure 21A graphically represents the amount of cell migration of normal and RDEB patient fibroblasts transduced with the LV-COL7 over time.
Figure 21B provides images of the cell migration of normal and RDEB patient fibroblasts transduced with the LV-COL7 over time.
Figure 22 provides the schematic for the cloning of human *COL7A1* gene.
Figure 23 provides the schematic of the cloning of the *COL7A1* gene into the pSMPUW expression vector to produce the INXN-2002 lentiviral vector transfer plasmid, IGE230.
Figure 24 provides the schematic representation of the construction of the INXN-2004 lentiviral vector transfer plasmid (IGE308).
Figure 25A schematically depict the gene elements of the IGE308 plasmid.
Figure 25B schematically depict the gene elements of the IGE308 plasmid.
Figure 26 provides the immunofluorescence analysis of GM-HDF injected composite grafts.

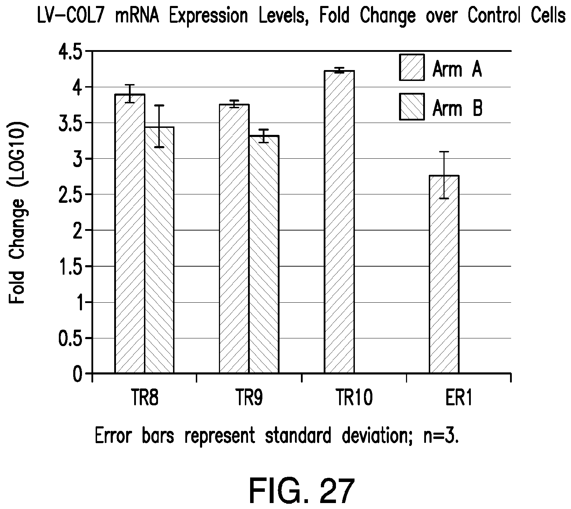

Figure 27 provides a graph representing the LV-COL7 transcript levels as measured by its fold change value over control cells.
Figure 28 provides representative images of C7 staining for INXN-2002 and LV-HA-COL7-transduced RDEB fibroblasts.
Figure 29 provides a graphical representation of the C7 protein levels as measured in the cell supernatants of GM-HDFs transduced by TR8, TR9, T10 and ER1.
Figure 30 graphically represents the correction of hypermotility of GM-HDFs over time for TR8, TR9, TR10 and ER1.
Figure 31 depicts representative indirect immunofluorescence (IF) images to examine C7 protein expression by GM-HDF cells using an antibody specific for C7 at two magnifications, 20x and 5x. (Only Arm A was tested for TR12.1 and TR13. Only Arm A of TR8 is shown for comparison of previous processes.)
Figure 32 provides the percentage of C7-positive (C7+) cells of GM-HDFs for each training run arm of TR11, TR12.1 and TR13.
Figure 33 provides a graph representation of the amount of C7 protein levels expressed for TR8, TR11, TR12.1 and 13. Error bars represent standard deviation; n=3. Only Arm A results are shown for TR8, TR12.1, and TR13.
Figure 34 provides the SDS-PAGE/immunoblots for the detection of C7 trimers (a) TR11, (b) TR12.1 and TR13 GM-HDFs.
Figure 35 depicts a graph of C7 expressed by GM-HDF that bind to Laminin 332 (Lam332) for TR11, TR12.1 and TR13.
Figure 36 provides the results of cell migration assays depicting the correction of hypermotility (% migration) over time for GM-HDFs for TR11, TR12.1 and TR13. The hypermotility of normal human dermal fibroblasts (NHDF) and a control fibroblasts from recessive dystrophic epidermolysis bullosa cells are provided.

### DETAILED DESCRIPTION OF THE INVENTION

Unless defined otherwise, all technical and scientific terms and any acronyms used herein have the same meanings as commonly understood by one of ordinary skill in the art in the field of this invention. Although any compositions, methods, kits, and means for communicating information similar or equivalent to those described herein can be used to practice this invention, the preferred compositions, methods, kits, and means for communicating information are described herein.

In order that the present invention may be more readily understood, certain terms are herein defined. Additional definitions are set forth throughout the detailed description.

The use of the word "a" or "an" when used in conjunction with the term "comprising" in the claims and/or the specification may mean "one," but it is also consistent with the meaning of "one or more," "at least one," and "one or more than one."

Throughout this application, the term "about" is used to indicate that a value includes the inherent variation of error for the device, the method being employed to determine the value, or the variation that exists among the study subjects. Typically, the term is meant to encompass approximately or less than 1%, 2%, 3%, 4%, 5%, 6%, 7%, 8%, 9%, 10%, 11%, 12%, 13%, 14%, 15%, 16%, 17%, 18%, 19% or 20% variability depending on the situation.

The use of the term "or" in the claims is used to mean "and/or" unless explicitly indicated to refer to alternatives only or the alternatives are mutually exclusive, although the disclosure supports a definition that refers to only alternatives and "and/or."

As used in this specification and claim(s), the words "comprising" (and any form of comprising, such as "comprise" and "comprises"), "having" (and any form of having, such as "have" and "has"), "including" (and any form of including, such as "includes" and "include") or "containing" (and any form of containing, such as "contains" and "contain") are inclusive or open-ended and do not exclude additional, unrecited elements or method steps. It is contemplated that any embodiment discussed in this specification can be implemented with respect to any method or composition of the invention, and vice versa. Furthermore, compositions of the invention can be used to achieve methods of the invention.

Nucleic acids and/or nucleic acid sequences are "homologous" when they are derived, naturally or artificially, from a common ancestral nucleic acid or nucleic acid sequence. Proteins and/or protein sequences are homologous when their encoding DNAs are derived, naturally or artificially, from a common ancestral nucleic acid or nucleic acid sequence. The homologous molecules can be termed homologs. For example, any naturally occurring proteins, as described herein, can be modified by any available mutagenesis method. When expressed, this mutagenized nucleic acid encodes a polypeptide that is homologous to the protein encoded by the original nucleic acid. Homology is generally inferred from sequence identity between two or more nucleic acids or proteins (or sequences thereof). The precise percentage of identity between sequences that is useful in establishing homology varies with the nucleic acid and protein at issue, but as little as 25% sequence identity is routinely used to establish homology. Higher levels of sequence identity, e.g., 30%, 40%, 50%, 60%, 70%, 80%, 90%, 95% or 99% or more can also be used to establish homology. Methods for determining sequence identity percentages (e.g., BLASTP and BLASTN using default parameters) are described herein and are generally available.

The terms "identical" or "sequence identity" in the context of two nucleic acid sequences or amino acid sequences of polypeptides refers to the residues in the two sequences which are the same when aligned for maximum correspondence over a specified comparison window. A "comparison window", as used herein, refers to a segment of at least about 20 contiguous positions, usually about 50 to about 200, more usually about 100 to about 150 in which a sequence may be compared to a reference sequence of the same number of contiguous positions after the two sequences are aligned optimally. Methods of alignment of sequences for comparison are well-known in the art. Optimal alignment of sequences for comparison may be conducted by the local homology algorithm of Smith and Waterman (1981) Adv. Appl. Math. 2:482; by the alignment algorithm of Needleman and Wunsch (1970) J. Mol. Biol. 48:443; by the search for similarity method of Pearson and Lipman (1988) Proc. Nat. Acad. Sci U.S.A. 85:2444; by computerized implementations of these algorithms (including, but not limited to CLUSTAL in the PC/Gene program by Intelligentics, Mountain View Calif., GAP, BESTFIT, BLAST, FASTA, and TFASTA in the Wisconsin Genetics Software Package, Genetics Computer Group (GCG), 575 Science Dr., Madison, Wis., U.S.A.); the CLUSTAL program is well described by Higgins and Sharp (1988) Gene 73:237-244 and Higgins and Sharp (1989) CABIOS 5:151-153; Corpet et al. (1988) Nucleic Acids Res. 16:10881-10890; Huang et al (1992) Computer Applications in the Biosciences 8:155-165; and Pearson et al. (1994) Methods in Molecular Biology 24:307-331. Alignment is also often performed by inspection and manual alignment.

In one class of embodiments, the polypeptides herein are at least 70%, generally at least 75%, optionally at least 80%, 85%, 90%, 98% or 99% or more identical to a reference polypeptide, or a fragment thereof, e.g., as measured by BLASTP (or CLUSTAL, or any other available alignment software) using default parameters. Similarly, nucleic acids can also be described with reference to a starting nucleic acid, e.g., they can be 50%, 60%, 70%, 75%, 80%, 85%, 90%, 98%, 99% or more identical to a reference nucleic acid or a fragment thereof, e.g., as measured by BLASTN (or CLUSTAL, or any other available alignment software) using default parameters. When one molecule is said to have certain percentage of sequence identity with a larger molecule, it means that when the two molecules are optimally aligned, said percentage of residues in the smaller molecule finds a match residue in the larger molecule in accordance with the order by which the two molecules are optimally aligned.

The term "substantially identical" as applied to nucleic acid or amino acid sequences means that a nucleic acid or amino acid sequence comprises a sequence that has at least 90% sequence identity or more, preferably at least 95%, more preferably at least 98% and most preferably at least 99%, compared to a reference sequence using the programs described above (preferably BLAST) using standard parameters. For example, the BLASTN program (for nucleotide sequences) uses as defaults a word length (W) of 11, an expectation (E) of 10, M=5, N=-4, and a comparison of both strands. For amino acid sequences, the BLASTP program uses as defaults a word length (W) of 3, an expectation (E) of 10, and the BLOSUM62 scoring matrix (see Henikoff & Henikoff, Proc. Natl. Acad. Sci. USA 89:10915 (1992)). Percentage of sequence identity is determined by comparing two optimally aligned sequences over a comparison window, wherein the portion of the polynucleotide sequence in the comparison window may comprise additions or deletions (i.e., gaps) as compared to the reference sequence (which does not comprise additions or deletions) for optimal alignment of the two sequences. The percentage is calculated by determining the number of positions at which the identical nucleic acid base or amino acid residue occurs in both sequences to yield the number of matched positions, dividing the number of matched positions by the total number of positions in the window of comparison and multiplying the result by 100 to yield the percentage of sequence identity. Preferably, the substantial identity exists over a region of the sequences that is at least about 50 residues in length, more preferably over a region of at least about 100 residues, and most preferably the sequences are substantially identical over at least about 150 residues. In a most preferred embodiment, the sequences are substantially identical over the entire length of the coding regions.

A "functional variant" of a protein disclosed herein can, for example, comprise the amino acid sequence of the reference protein with at least or about 1, 2, 3, 4, 5, 6, 7, 8, 9, 10, 11, 12, 13, 14, 15, 16, 17, 18, 19, or 20 conservative amino acid substitutions. The phrase "conservative amino acid substitution" or "conservative mutation" refers to the replacement of one amino acid by another amino acid with a common property. A functional way to define common properties between individual amino acids is to analyze the normalized frequencies of amino acid changes between corresponding proteins of homologous organisms (Schulz, G. E. and Schirmer, R. H., Principles of Protein Structure, Springer-Verlag, New York (1979)). According to such analyses, groups of amino acids may be defined where amino acids within a group exchange preferentially with each other, and therefore resemble each other most in their impact on the overall protein structure (Schulz, G. E. and Schirmer, R. H., supra). Examples of conservative mutations include amino acid substitutions of amino acids within the sub-groups above, for example, lysine for arginine and vice versa such that a positive charge may be maintained; glutamic acid for aspartic acid and vice versa such that a negative charge may be maintained; serine for threonine such that a free --OH can be maintained; and glutamine for asparagine such that a free --NH₂ can be maintained. In one embodiment of the present invention, a *COL7A1* gene encodes for the C7 protein or a functional variant thereof, provided that the variant is capable of anchoring the fibrils between the epidermis and dermis.

Alternatively or additionally, the functional variants can comprise the amino acid sequence of the reference protein with at least one non-conservative amino acid substitution. "Non-conservative mutations" involve amino acid substitutions between different groups, for example, lysine for tryptophan, or phenylalanine for serine, etc. In this case, it is preferable for the non-conservative amino acid substitution to not interfere with, or inhibit the biological activity of, the functional variant. The non-conservative amino acid substitution may enhance the biological activity of the functional variant, such that the biological activity of the functional variant is increased as compared to the reference sequence.

Proteins disclosed herein (including functional portions and functional variants thereof) may comprise synthetic amino acids in place of one or more naturally-occurring amino acids. Such synthetic amino acids are known in the art, and include, for example, aminocyclohexane carboxylic acid, norleucine, α-amino n-decanoic acid, homoserine, S-acetylaminomethyl-cysteine, trans-3- and trans-4-hydroxyproline, 4-aminophenylalanine, 4-nitrophenylalanine, 4-chlorophenylalanine, 4-carboxyphenylalanine, β-phenylserine β-hydroxyphenylalanine, phenylglycine, α-naphthylalanine, cyclohexylalanine, cyclohexylglycine, indoline-2-carboxylic acid, 1,2,3,4-tetrahydroisoquinoline-3-carboxylic acid, aminomalonic acid, aminomalonic acid monoamide, N'-benzyl-N'-methyl-lysine, N',N'-dibenzyl-lysine, 6-hydroxylysine, ornithine, α-aminocyclopentane carboxylic acid, α-aminocyclohexane carboxylic acid, α-aminocycloheptane carboxylic acid, α-(2-amino-2-norbornane)-carboxylic acid, α,γ-diaminobutyric acid, α,β-diaminopropionic acid, homophenylalanine, and α-tert-butylglycine.

The invention includes the vectors INXN-2002; and IGE308 also referred to herein as INXN-2004, as well as vectors substantially identical and/or homologous thereto as well as functional variants thereof.

The term "patient" or "subject" refers to mammals, including humans and animals.

The term "treating" refers to reducing or alleviating the symptoms, and/or preventing relapses and/or the progression of DEB, including RDEB and/or DDEB.

The term "autologous cells" refers to cells obtained and then returned to the same individual. In particular, cells may be removed from the body of a DEB patient, which may subsequently be genetically modified, cultivated to proliferate, and then returned to the body of the patient from which they were originally removed.

The term "transduction" refers to the delivery of a gene(s) using a viral or retroviral vector by means of viral infection rather than by transfection.

The term "transducing lentiviral vector" or "transducing lentiviral vector particle" refers to the infectious lentiviral vector particles formed from the co-transfection of a packaging cell line with the lentiviral expression/transfer plasmid vector comprising the *COL7A1* gene or functional variant thereof, a packaging vector(s), and an envelope vector. The transducing lentiviral vector is harvested from the supernatant of the producer cell culture after transfection. Suitable packaging cell lines are known in the art and include, for example, the 293T cell line.

The term "lentiviral vector" refers to a vector containing structural and functional genetic elements outside the LTRs that are primarily derived from a lentivirus.

The term "self-inactivating vector" (SIN) refers to vectors in which the 3' LTR enhancer-promoter region, known as the U3 region, has been modified (e.g., by deletion or substitution) to prevent viral transcription beyond the first round of viral replication. Consequently, the vectors are capable of infecting and then integrating into the host genome only once, and cannot be passed further. SIN vectors greatly reduce risk of creating unwanted replication-competent virus since the 3' LTR U3 region has been modified to prevent viral transcription beyond the first round of replication, eliminating the ability of the virus to be passed.

The term "pharmaceutically acceptable carrier" is employed herein to refer to liquid solutions which are, within the scope of sound medical judgment, suitable for use in contact with the autologous, genetically-modified cells without affecting their activity, and without being toxic to the tissues of human beings and animals or causing irritation, allergic response, or other complications, commensurate with a reasonable benefit/risk ratio. A useful pharmaceutically acceptable carrier may be an injectable solution which is biocompatible with the autologous, genetically-modified cells and does not reduce their activity or cause their death.

Described but not claimed herein is an autologous, genetically-modified cell therapy for C7-deficient patients. "C7-deficient patients" either lack or have substantially reduced production of type VII collagen (C7), which is important for anchoring fibril formation at the dermal-epidermal junction (DEJ), resulting in dystrophic epidermolysis bullosa (DEB). Such therapy may comprise harvesting DEB patient cells, such as fibroblasts or keratinocytes, genetically modifying the harvested cells to insert a functional *COL7A1* gene or functional variant thereof, expanding the genetically modified cells, and administering a population of the genetically-modified cells back into the DEB patient.

Cells may be harvested from a patient suffering from DEB. Fibroblasts may be used. The cells may be obtained through known methods, including from patient skin samples, scrapings, and biopsies, for example. Cells may be obtained from the non-blistering skin of a DEB patient. Alternatively, cells may be obtained from the blistering skin of a DEB patient. The cells harvested from a DEB patient have either a mutated, non-functional or missing *COL7A1* gene. These cells may be cultured using standard cell culture techniques. The harvested patient's cells may be subsequently treated *ex vivo* with genetic material encoding a functional type VII collagen gene (C7) or functional variant thereof.

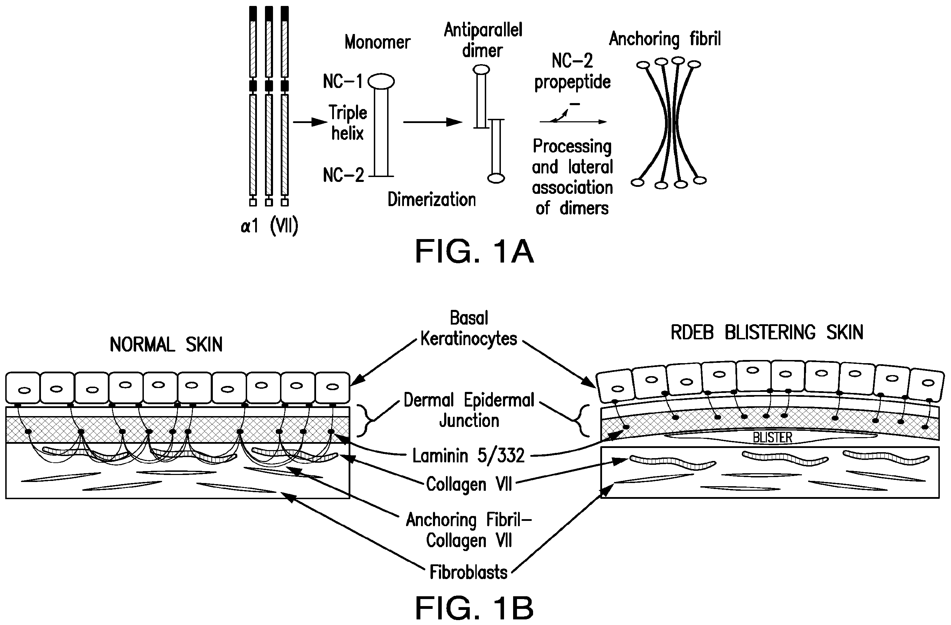

The *COL7A1* gene has the nucleic acid, SEQ ID NO:1, which encodes for a 290 kDa alpha chain, wherein three chains form a triple helix (trimer), as depicted in Figure 1A. C7 has the amino acid sequence of SEQ ID NO: 2 and is the protein important for anchoring fibril formation at the dermal-epidermal junction (DEJ). Mutations in the C7 gene cause an absence or reduction of C7, which make up anchoring fibrils that maintain binding of the epidermis to the dermis. C7 anchoring fibrils bind to other collagens, extracellular matrix proteins, and Lam332 that mediate the attachment of the dermis to the epidermis, as shown in Figure 1B. The absence of anchoring fibrils leads to blistering of the skin. Vectors comprising nucleic acids and polypeptides substantially identical to *COL7A1* and C7 respectively may be used. Vectors that comprise nucleic acids that encode a functional variant of the COL7A1 or C7 protein may be used, provided that the variant is capable of anchoring the fibrils between the epidermis and dermis. Only the open reading frame of the *COL7A1* gene, which encodes the C7 protein or a functional variant thereof may be used. The *COL7A1* nucleic acid may be codon-optimized for increased expression of C7 in the transduced cell, as is known in the art. According to the invention, the lentiviral vector comprises a nucleotide sequence encoding a functional COL7A1 gene or a functional variant thereof.

In accordance with the present invention, the *COL7A1* is transduced into the harvested cells using a transducing lentiviral vector, which is replication defective and self-inactivating. Self-inactivating lentiviral vectors are derived from the human immunodeficiency virus (HIV-1), which are pseudotyped with a heterologous VSV-G envelope protein instead of the HIV-1 envelope protein. As previously reported, a 400 bp deletion is introduced to the U3 region of the LTR resulting in a self-inactivating (SIN) vector (Zuffery 1998). The vectors of the present invention are distinguished from 2nd and 3rd generation SIN vectors. In particular, the vectors of the present invention have been modified to enhance the safety features and increase the cloning capacity as further described herein.

Vectors used to construct the transducing lentiviral vectors of the present invention are introduced via transfection or infection into a packaging cell line. The packaging cell line produces transducing lentiviral vector particles that contain the vector genome. After cotransfection of the packaging vectors, transfer vector, and an envelope vector to the packaging cell line, the recombinant virus is recovered from the culture media and titered by standard methods used by those of skill in the art. Thus, the packaging constructs can be introduced into human cell lines by calcium phosphate transfection, lipofection or electroporation, generally together with a dominant selectable marker, such as kanamycin, neomycin, DHFR, or Glutamine synthetase, followed by selection in the presence of the appropriate drug and isolation of clones. The selectable marker gene can be linked physically to the packaging genes in the construct.

Stable cell lines wherein the packaging functions are configured to be expressed by a suitable packaging cell are known. For example, see U.S. Pat. No. 5,686,279; and Ory et al., (1996), which describe packaging cells. The packaging cells with a lentiviral vector incorporated in them form producer cells. Producer cells are thus cells or cell-lines that can produce or release packaged infectious viral particles carrying the therapeutic gene of interest. An example of a suitable lentiviral vector packaging cell lines includes 293 cells.

The transducing lentiviral vector may be constructed from a lentiviral expression plasmid vector comprising (a) a modified 5' long terminal repeat (LTR), wherein the promoter of the modified 5' LTR is a cytomegalovirus promoter, (b) the *COL7A1* gene, (c) at least one lentiviral central polypurine tract element, and (d) a modified 3' LTR, wherein the modified 3' LTR comprises a deletion relative to the wild-type 3' LTR and comprises a deletion of a hepatitis B virus post- transcriptional regulatory element (PRE).

The deleted hepatitis B virus post- transcriptional regulatory element (PRE) may be a woodchuck post-transcriptional regulatory element PRE (WPRE).

The lentiviral expression plasmid vector may comprise two or more lentiviral central polypurine tract (cPPT) elements. Incorporation of cPPT element(s) has been found to increase gene expression levels.

The 3' LTR may be modified to delete a 400 bp fragment (as disclosed by Zuffery 1998), instead of the commonly used 133 bp deletion of standard 3rd generation SIN LV vectors. The 400 bp deletion is believed to increase safety by preventing read-through transcription as well increase viral titer because the vector transcript is more stable in packaging cells.

The lentiviral expression plasmid may incorporate a hepatitis B virus post-transcriptional regulatory element (PRE), which is preferably woodchuck post-transcriptional regulatory element PRE (WPRE). WPRE has been found to increase the viral titer of the vector.

The addition of the cPPT, and modified 3'LTR deletion has been shown to improve the function of the lentiviral vector comprising the *COL7A1* gene of the present invention to increase gene expression levels, viral titer, and safety.

The present invention further includes the use of lentiviral transfer vectors in which certain elements common for 3rd generation lentiviral expression vectors are deleted. In particular, safety concerns for use of lentiviral vectors in humans hinge on the fear of generating a replication-competent lentivirus, which may arise from recombination between split genomes of 3rd generation lentiviral vectors. (Tareen *et al.,* 2013.). The gag sequence and/or the rev-responsive elements (RRE) may be deleted from the viral vector, in order to reduce sequence homology with other helper plasmids and thereby increase safety to humans. Accordingly, the gag sequence may be absent in the vector. Alternatively, both the gag sequence and RRE may be absent in the vector.

The starting materials to generate the lentiviral vectors of the present invention is preferably a lentiviral expression plasmid vector that comprises a cPPT and PRE that can accommodate the insertion of the large *COL7A1* gene (8.89 kbp) or a functional variant thereof. The starting material to construct the transducing lentiviral vector of the present invention may be is selected from the lentiviral expression plasmid vectors, pSMPUW (Cell Biolabs, Inc., San Diego, CA) and pFUGW (Addgene, Cambridge, MA).

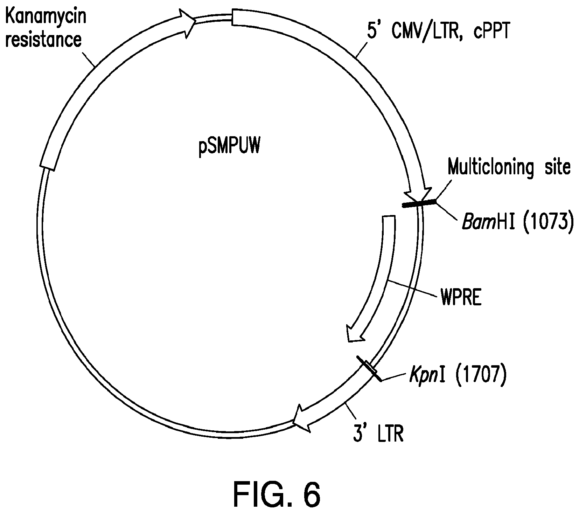

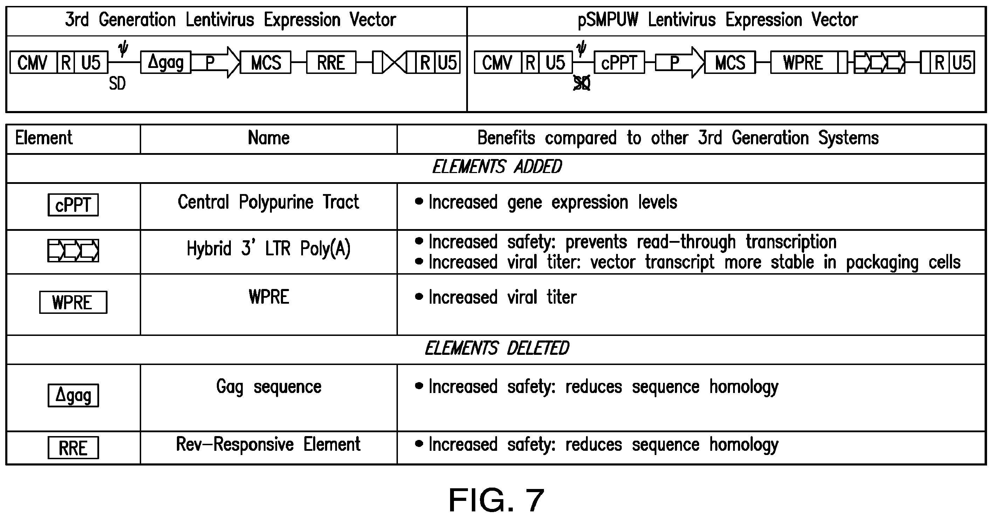

The pSMPUW lentiviral expression plasmid vector may be selected for construction of the transducing lentiviral vector. Figure 6 shows a schematic of the genetic elements in the pSMPUW lentiviral expression plasmid vector. Features of the pSMPUW lentiviral expression vector have been modified from 3rd generation lentivirus expression vectors in order to enhance gene expression levels and safety features, as depicted in Figure 7. In particular, the pSMPUW lentiviral expression vector encodes for a multicloning site (MCS) followed by the Woodchuck Hepatitis Virus Post-transcriptional regulatory element (WPRE). The residual gag (Δgag) and the RRE element were removed from the pSMPUW vector construct. Additionally, the pSMPUW vector construct utilized a larger 400 bp deletion in the 3'LTR U3 region instead of the commonly used 133 bp deletion in a standard 3rd generation SIN LV vector. Wild type *COL7A1* gene was incorporated into the pSMPUW vector.

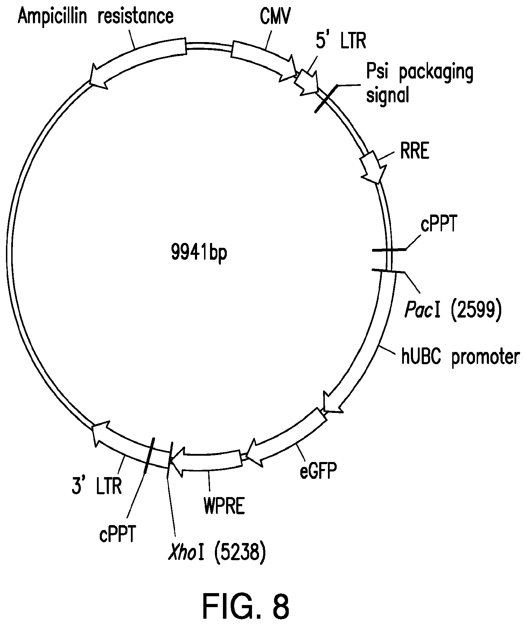

Alternatively, the pFUGW lentiviral expression plasmid may be selected for construction of the transducing lentiviral vector. Figure 8 shows a schematic of the genetic elements in this plasmid vector. Features of the pFUGW vector include a RRE, two cPPT elements, and a WPRE element, which may improve lentiviral production and transgene expression.

The WPRE element may be deleted from the lentiviral vector.

Exemplary transducing lentiviral vector particles of the present invention are designated INXN-2002 (vector transfer plasmid - IGE230) or INXN-2004 (vector transfer plasmid - IGE308), or a substantially identical vector comprising functional variants thereof.

The cells harvested from the patient suffering from a dystrophic epidermolysis bullosa (DEB) or pseudosyndactyly are transduced with a lentiviral vector according to the claims. The lentiviral vector is a self-inactivating vector. The copy number of the integrated transgene can be assessed using any known methods. For example, copy number may be determined through quantitative PCR, multiplex ligation-dependent probe amplification, fluorescent in situ hybridization (FISH), microarray-based copy number screening, and conventional karyotyping. The number of copies of the transgene integrated into each cell may be modulated by the virus dose given to the cells during production. The integrated transgene copy number per cell in the RDEB harvested cells transduced with a *COL7A1*-containing vector is dose dependent.

Cells harvested from the DEB patient and transformed with the functional *COL7A1* gene will have a functional *COL7A1* gene and exhibit normal cell morphologies. These cells may be caused to proliferate or expanded in culture using standard cell culture techniques.

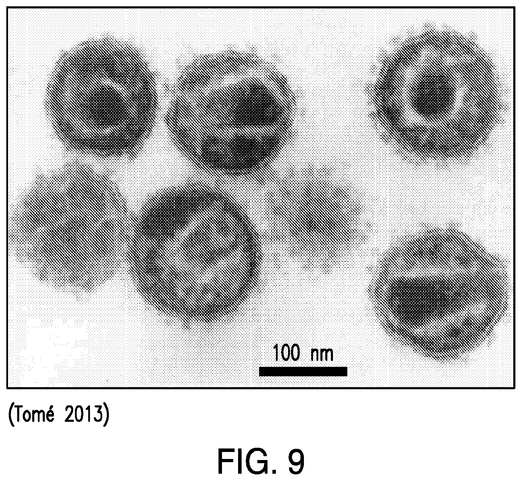

Normal fibroblast morphological characteristics are observed among harvested fibroblasts transduced with *COL7A1*-transducing vectors within the scope of the present invention. For example, normal fibroblast morphologies include cells displaying elongated, fusiform or spindle appearance with slender extensions. Normal morphologies further include cells appearing larger, flattened stellate cells which may have cytoplasmic leading edges. Figure 9 displays an example of normal cell morphology for fibroblasts transduced with a *COL7A1-*transducing vector.

The production of C7 produced by harvested DEB patient cells transduced with a *COL7A1*-transducing vector has also been observed. In particular, the formation of C7 trimers is important in the assembly of anchoring fibrils. It has been found that DEB patient cells transduced with a *COL7A1*-transducing vector are capable of forming the C7 trimers with the correct structure, size, and function.

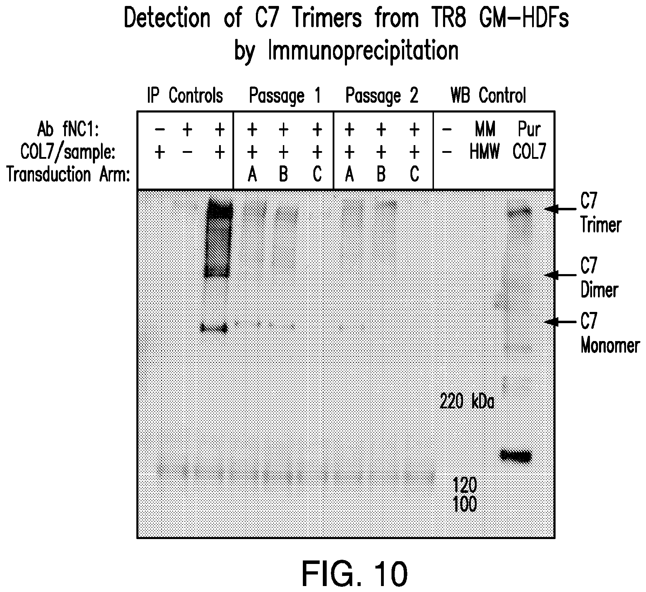

It is possible to verify that the C7 expressed by fibroblasts transduced with a *COL7A1*-transducing vector will be capable of forming anchoring fibrils using immunoprecipitation with an anti-C7 specific antibody. For example, the anti-C7 specific antibody, fNC1, may be used for selective capture and the concentration of C7 from culture supernatants for detection by SDS-PAGE/immunoblot. For example, Figures 9 and 10 demonstrate that C7 produced by fibroblasts transduced with a *COL7A1*-transducing vector were predominantly trimeric.

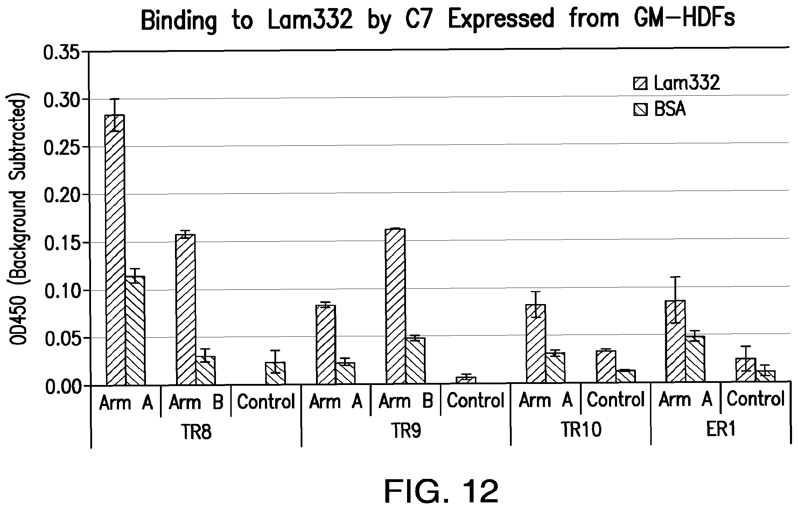

The function of C7 may be assessed using known methods, such as using a laminin binding assay or cell migration assay. C7 has been shown to bind immobilized extracellular matrix (ECM) components, including fibronectin, Laminin 332 (Lam332), COL1, and COL4 (Chen, et al., 2002a). The interaction between C7 and Lam332 occurs through the NH2-terminal NC1 domain of C7 and is dependent upon the native conformation of both Lam332 and C7 NC1 (Rousselle, et al., 1997). The association between C7 and Lam332 is important for establishing correct Lam332 architecture at the dermal-epidermal junction. Such organization is important for interactions with extracellular ligands and cell surface receptors, and for cell signaling (Waterman, et al., 2007). An ELISA using an antibody against the C7 NC1 domain to detect binding of C7 to purified Lam332 has been developed. Although a C7/Lam332 binding ELISA has already been described in the literature (Chen, et al., 2002a), to our knowledge it has never been used to test C7 present in the supernatants of transduced cells. Results in Figure 12 show dose-dependent binding to Lam332 by C7 expressed by GM-HDFs from the Training and Engineering runs.

In addition, a cell migration assay can be used to assess functional C7 activity. Previous studies have shown that RDEB fibroblasts and keratinocytes show an increase in motility relative to their normal counterparts, and that normal motility can be restored by expression of C7 (Chen, et al., 2000; Chen, et al., 2002b; Cogan, et al. 2014; Baldeschi et al., 2003). Suitable assay includes the colloidal gold salt migration assay to measure the migration of fibroblasts and keratinocytes, or a wound healing assay to measure the migration of keratinocytes. Cells having functional C7 activity will exhibit a reduced motility relative to RDEB cells in such assays.

The present invention is directed, in one aspect, to pharmaceutical formulations comprising the autologous, genetically-modified cells according to the caims. These cells may be present in any amount suitable for the delivery to the patient in which the cells were originally harvested. For example, that cells may be present in a cell concentration of 1.0 - 5.0 x 10⁷ cells/mL, 1.0 - 4.0 x 10⁷ cells/mL, 1.0 - 3.0 x 10⁷ cells/ mL, or 1.0 - 2.0 x 10⁷ cells/mL. The cells may be present in a suspension suitable to sustain the viability of the cells, such as in Dulbecco's Modified Eagle's Medium (DMEM). In particular, the viability of the cells in the formulation are present in an amount of ≥ 60%, 70%, 75% or 80%. Suitable excipients may also be present in the formulation, such as phosphate buffered saline which may be used to wash the cells from thawed vials containing the autologous, genetically-modified. Preferably, no phenol red is present in the final formulation.

The autologous genetically modified cells of the present invention have a transducing vector copy number in the range of 0.1 to 5.0 copies per cells. It has been found that transduced human dermal fibroblast or keratinocytes have at least or about 1, 2, 5, 10, 20, 25, 27, 28, 29, 30, 35, 40, 45, or 50-fold greater integrated transgene copy number per cell relative to a transduction composition not subjected to spinoculation or super-transduction. Moreover, the C7 protein expression values of the FCX-007 cells of the present invention may be ≥ 300, 350, 400, 450, 500, 550, or 600 ng/day/E6 cell. The C7 protein expression values of the autologous genetically-modified cells of the present invention may be ≥ 500 ng/day/E6 cell. It has also found that expression of C7 has increased by 10, 25, 50, 100, 150, or 200-fold relative to transduced human dermal fibroblasts or keratinocytes not subjected to spinoculation or a second transduction.

The multiplicity of infection (MOI) refers to the number of vector particles per cell used in transduction. The desired transducing vector copy number has been achieved even as the MOIs decreases, as shown in the examples below. For example, MOIs of ≤ 15, < 14, ≤ 13, ≤ 12, ≤ 11, or ≤ 10. Preferably, the MOI is between about 1 to 10.

The formulations of the present invention may be used to treat various maladies of patients suffering from Type VII deficiency. In particular, the formulations of the present invention are suitable to treat DEB, including DDEB or RDEB. Subtypes of RDEB may also be treated, including but not limited to Hallopeau-Seiemens, non-Hallopeau-Siemens RDEB, RDEB inversa, pretibial RDEB, acral RDEB, and RDEB centripetalis.

Accordingly, in one embodiment, the autologous genetically modified fibroblast according to the claimed invention is from a patient suffering from DEB, wherein the DEB is recessive dystrophic epidermolysis bullosa (RDEB). In specific embodiments, the RDEB is Hallopeau-Siemens RDEB, non-Hallopeau-Siemens RDEB, RDEB inversa, pretibial RDEB, acral RDEB, or RDEB centripetalis.

In another embodiment, the autologous genetically modified fibroblast according to the claimed invention is from a patient suffering from DEB, wherein the DEB is dominant dystrophic epidermolysis bullosa (DDEB).

Described but not claimed herein are medical uses for the treatment, reduction, prevention, and/or inhibition of various symptoms attributed to C7 deficiencies. For example, DEB is known to cause scarring after blisters, which may cause contracture deformities, swallowing difficulty if the mouth and esophagus are affected, fusion of fingers and toes, and limited mobility. Therefore, medical uses may be for the treatment, reduction, inhibition, and/or prevention of pseudosyndactyly, also known as mitten hand syndrome. Further medical uses may be for the treatment, reduction, inhibition, and/or prevention of deep fibrosis and/or scarring associated with RDEB and/or DDEB, which may result in milia, joint contractures, generalized soft tissue fibrosis, organ fibrosis, corneal lesions, scarring plaques, scarring alopecia, nail dystrophy, ankyloglosia, and increased frequency of dental caries, for example. A further medical use may be for the treatment, reduction, prevention, and/or inhibition of oral mucosa lesions and gastrointestinal lesions associated with RDEB. A further medical use may be for the treatment, prevention, reduction and/or inhibition of blisters associated with DEB patients.

Administration of the autologous genetically modified cells may be done at any appropriate time as one skilled in the art would be capable of determining based on the needs of the patient. For example, administration may be done once, once a day, once a month, once a quarter, or 1-2 times per year.

The formulation of the present invention containing the autologous genetically modified cells may be administered to the patient by any known method, including but not limited to injection, topically, orally, or embedded in a biocompatible matrix. The injection may be, e.g., parenteral, intradermal, subcutaneous, intramuscular, intravenous, intraosseous, intraarterial, ocular and intraperitoneal or direct injection into a specific organ/tissue, e.g. prostate or liver. Topically, the formulation may be administered directly to an affected site, such as at the site of a lesion. Debridement of the affected tissue can precede the direct application of the formulation to the site. Alternatively, the formulation may be encapsulated in a suitable delivery system, such as in a polymer capsule, or embedded in a biocompatible matrix or graft, e.g., collagen matrix, in a hydrogel, skin graft, or a mesh. In specific embodiments, the autologous genetically modified fibroblast of the present invention is embedded in a biocompatible matrix.

The autologous genetically modified cells of the present invention may be administered solely or in combination with other treatments for a patient suffering from DEB. Examples of therapeutic agents used for treating DEB include topical care, such as Zorblisa, skin grafts, antiinflammatories, antibodies, other potential gene or cell-based therapies. The modified cells of the present invention may be administered for the skin or muoscal areas where bone marrow transplant therapies (or other system cellular therapies such as mesenchymal stem cells) did not provide sufficient therapy. The C7-expressing ability of the autologous genetically modified cells is preferably not impaired by a combination with other therapeutic agents.

Described but not claimed herein is a method of enhancing or increasing the integrated transgene copy number per cell in the genetically modified cells obtained from C7-deficient patients and transduced according to the present disclosure. In particular, it has been found that certain steps during transduction substantially enhanced the copy number of the transgene in these cells. Cells obtained from the C7-deficient patient may be contacted with a lentiviral vector of the present invention and that composition may be subjected to spinoculation, also known as spin transduction. In this manner, the lentiviral vectors are spun onto the targeted cells. The addition of spinoculation has unexpectedly increased the copy number per cells in the genetically modified human dermal fibroblasts by a level of ≥ 2-fold relative to a transduction without spinoculation or transduction. However, in order to identify additional methods to increase copy number, it has unexpectedly been found that a second transduction step (or super-transduction) in which the targeted cells are passaged through the first transduction with spinoculation, and then subsequently passaged through a second transduction optionally with spinoculation. In this manner, it has been found that this super-transduction with spinoculation increases the copy number by an additional 2-fold increase relative to the original traditional transduction method that did not include spinoculation or super-transduction.

It has been found that changing the lentiviral vector from INXN-2002 to INXN-2004 increased the transgene copy number in the genetically modified human dermal fibroblasts by 4-fold relative to traditional transduction without spinoculation or super-transduction.

Through extensively evaluating integrated transgene copy numbers, it has been found the cumulative change of adding spinoculation, adding super-transduction with spinoculation, and changing from INXN-2002 to INXN-2004, relative to the original transduction process that did not include spinoculation or super-transduction, unexpectedly increased the copy number > 27-fold (*see* Example 9: comparing TR12.1 and TR3 to TR8 in original studies (which did not include spinoculation or super-transduction). Accordingly, an increased copy number of the transduced cells from the C7-deficient patient has been achieved.

### EXAMPLES

FCX-007 is a suspension of live, autologous human dermal fibroblast cells genetically modified using either a lentiviral vector (INXN-2002) (as shown in Example 1) or a lentiviral vector (INXN-2004) (as shown in Example 2) to express the human collagen type 7 protein.

### EXAMPLE 1

### A. Elucidation of Structure and Characteristics of FCX-007 transduced with Lentiviral Vector INXN-2002

The FCX-007 cells derived from the lentiviral vector INXN-2002 cells are suspended in a cryopreservation medium consisting of Iscove's Modified Dulbecco's Medium (IMDM) without fetal bovine serum (50.0%), Profreeze-CDMTM (42.5%) and dimethyl sulfoxide (DMSO) (7.5%). The structural features for FCX-007 Drug Substance (DS) include the primary structure of the autologous human dermal fibroblast (HDF) cells and the structure of the lentiviral vector used to transduce and gene modify the HDF cells.

### B. Lentiviral Vector (INXN-2002)

INXN-2002 Lentiviral Vector (LV), which is used to transduce and to introduce the human collagen 7A1 gene into the HDF cells, is a recombinant lentiviral vector encoding the human collagen 7A1 gene. INXN-2002 LV is a self-inactivating (SIN) lentiviral vector that is constructed based on the human immunodeficiency virus type 1 (HIV-1) pseudotyped with a heterologous VSV-G envelope protein. The virus particle for INXN-2002 lentiviral vector is approximately 120 nm in diameter and is comprised of numerous proteins with two copies of a single-stranded RNA genome. A specific molecular formula, molecular weight, or stereochemistry is not available.

Structural features for INXN-2002 LV include the primary structure of the RNA viral genome and the structure of the viral particle. The primary structure of the RNA genome of INXN-2002 is determined by the full nucleotide sequencing of the viral genome. The viral particles structure is deduced from the particle structure of HIV-1 with added VSV-G protein pseudotyping. An overview of the nucleic acid structure and the structure of the viral particle are provided below.

### C. INXN-2002 RNA Viral Genome Structure

INXN-2002 LV is a self-inactivating (SIN) lentiviral vector that is constructed based on the human immunodeficiency virus type 1 (HIV-1) pseudotyped with a heterologous VSV-G envelope protein instead of the HIV-1 envelope protein. A 400 bp deletion is introduced to the U3 region of the LTR resulting in a self-inactivating (SIN) vector (Zuffery 1998). The pSMPUW lentiviral expression plasmid vector (Cell Biolabs, Inc., San Diego, CA) was selected for construction of the INXN-2002 LV. Figure 6 shows a schematic of the genetic elements in the pSMPUW lentiviral expression plasmid vector.

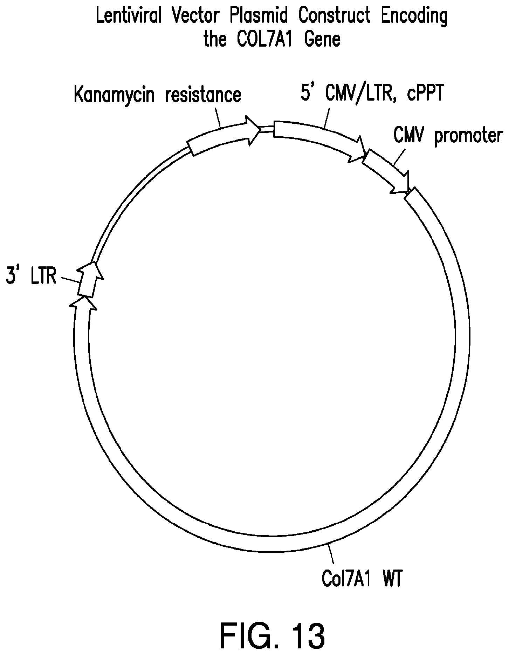

The coding elements between the 5' and 3' LTRs of the HIV-1 virus are fully gutted. The vector encodes for a multicloning site followed by the Woodchuck Hepatitis Virus Posttransscriptional Regulatory Element (WPRE). In order to maximize the cloning capacity of the vector, these elements were removed by digesting the vector with BamHI from the multicloning site and KpnI at the 5' end of the 3' left terminal repeat. The COL7A1 gene expression cassette with a CMV promoter is cloned into the digested vector by single strand annealing to generate the lentiviral vector plasmid construct encoding the COL7A1 gene as shown in Figure 13.

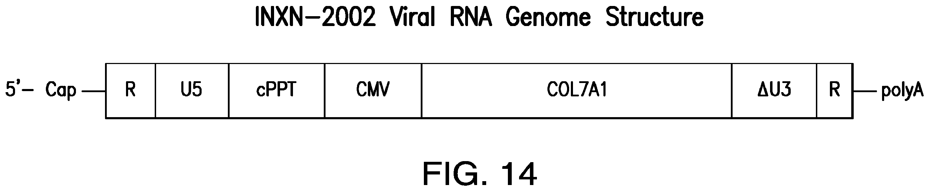

The lentiviral vector plasmid construct is co-transfected into HEK293T cells with three helper plasmids (pCMV-G, pCMV-Rev2 and pCgp) to produce the INXN-2002 lentiviral vector particles. pCMV-G plasmid provides the VSV-G pseudotyping surface protein, pCMV-Rev2 provides the HIV-1 Rev protein for efficient RNA transport and packaging, and pCgp provides the structural and viral enzyme proteins for production of the INXN-2002 lentiviral vector particles. Figure 14 shows a schematic of the proviral RNA genome structure of the INXN-2002 lentiviral vector.

### D. INXN-2002 Lentiviral Vector Particle Structure

INXN-2002 lentiviral vector is constructed based on a HIV-1 derived vector backbone and has a similar viral particle structure to the HIV-1 virus. Figure 9 shows an electron micrograph of HIV-1 particles.

The HIV-1 particle has a spherical shape of approximately 120 nm in diameter, with an estimated molecular weight of 277MDa (Carlson 2008). The particle has a lipid bilayer membrane stubbed with envelope protein. The envelope protein interacts with the receptors on the target cells for infection and delivery of the RNA viral genome to the target cells. A cone shaped nucleo-core, where two strands of viral RNA genome is housed, can be observed inside the virus particle. The cone shaped nucleo-core is formed by the viral capsid protein.

For INXN-2002, VSV-G (glycoprotein of the vesicular stomatitis virus (VSV-G)) is used as a substitute for the HIV-1 envelope proteins resulting in improved vector stability, target cell tropism, and transduction efficiency (Cronin 2005).

During production, INXN-2002 viral particles assemble and bud out from the surface of transfected HEK293T cells. The VSV-G protein is provided by the pCMV-G helper plasmid, the vector core and enzyme proteins are provided by the pCgp helper plasmid, and the Rev protein, which is needed for efficient RNA genome transport and packaging into the viral particle, is provided by the pCMV-Rev2 plasmid. It is noted that all the other HIV-1 accessory proteins including Vpu, Vif, Vpr, Nef, and Tat, are deleted from the INXN-2002 vector.

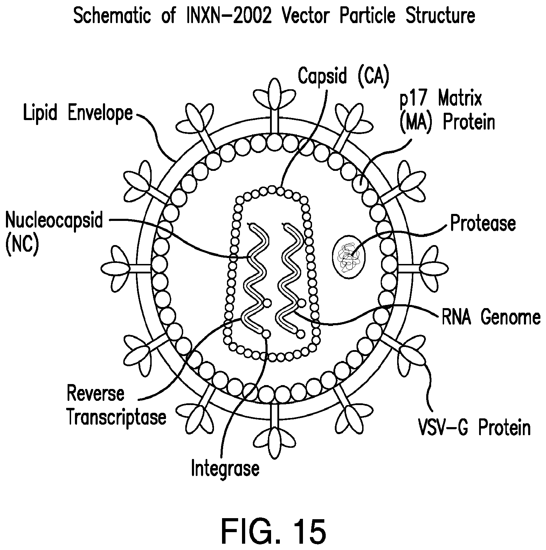

After budding from the producer cell surface, the protease enzyme packaged inside the virus particle cleaves the Gag precursor protein into its constituent proteins (MA, CA, NC), to convert the immature virion into a mature infectious INXN-2002 vector particle. Figure 15 shows a schematic depicting the details of the mature INXN-2002 virus particle.

Two strands of the INXN-2002 RNA genome are packaged inside the cone shaped core formed by the Capsid protein (CA). The nucleocapsid (NC) protein forms a stable complex with the RNA genome inside the capsid core. The matrix protein forms a coat on the inner surface of the membrane. The virus buds through the cell plasma membrane spiked with the VSV-G envelope protein and forms the lipid envelope. Based on the recent three dimensional analysis of HIV-1 virus particle structure (Carlson 2008), Table 1 shows the component proteins that make up the INXN-2002 lentiviral vector and a brief description of the function of each of the components.

**Table 1: INXN-2002 Lentiviral Vector Major Component Proteins**

| **Component Proteins** | **Protein MW (kD)** | **Protein functions** |
|---|---|---|
| Capsid (CA) | 24 | Forms the nucleo-core |
| Matrix (MA) | 17 | Forms the protein coat on the inner surface of the lipid membrane |
| Nucleocapsid (NC) | 7 | Forms a stable complex with the RNA genome |
| VSV-G | 69 | Pseudotyped envelope protein interacts and binds to receptors on target cells for transduction to deliver the vector RNA genome to the cells |
| Protease | 11 | Plays an important role in the maturation of the INXN-2002 vector by cleaving the Gag proteins to its functional constituents, CA, MA, and NC |
| Reverse transcriptase | 66/51 | Builds a DNA copy of the viral RNA genome |
| Integrase | 31 | Inserts the DNA copy of the viral RNA genome into the infected cell genome |

### E. INXN-2002 Characterization

Table 2 provides a list of the characterization assays and specifications for INXN-2002 manufactured by City of Hope. Characterization assay results are provided in the attached CoA for INXN-2002 lot number 0786-240-0002-1, the lot intended for use in manufacture of the FCX-007 clinical product.

**Table 2: Characterization of INXN-2002**

| **Category** | **Assay** | **Test Method** | **Lab** | **SOP** | **Target Specification** |
|---|---|---|---|---|---|
| Identity | Vector identity | RT-PCR | CoH | QC-SOP-0843 | Band of correct size detected |
| | Vector insert stability | Southern blot analysis | Indiana University | VP-10-17.16 | Report result |
| Potency | Viral antigen Physical Titer (p24) | ELISA | Indiana University | VP-10-05.06 | Report result |
| | TU titer | H1299 transduction with qPCR readout | BioReliance | 016135.BSV | Report result |
| | C7 expression (Potency) | ELISA | BioReliance | 016136.BSV | Report result |
| Appearance and pH | Appearance | Visual inspection | CoH | QC-SOP-0734 | Opaque solution |
| | pH | pH meter | CoH | QC-SOP-0675 | 6.9 - 7.8 |

### F. Characterization of INXN-2002 by an in vitro Immortalization Assay

The potential for insertional genotoxicity of INXN-2002 was evaluated using an in vitro immortalization (IVIM) assay. The test was conducted at Cincinnati Children's Hospital Medical Center, Division of Experimental Hematology & Cancer Biology (CCHMC).

The principle of the IVIM test is based on the understanding that normal Lineage negative (Lin- ) bone marrow (BM) cells will stop proliferating after 3-4 weeks in vitro, but in the presence of certain vector integrations which cause upregulation of proto-oncogenes, some clones will continue to proliferate after 5 or more weeks. The number of such immortalized clones is representative of the oncogenic potential of the vector. The immortalized clones are expanded and further analyzed for stem cell markers by FACS, vector copy number by qPCR or the site of integration by LAM-PCR. Insertion in common integration sites (cis) such as Evi1 or Prdm 16 is frequently observed in immortalized clones generated in IVIM assays (Calmels et al., 2005; Modlich et al., 2008).

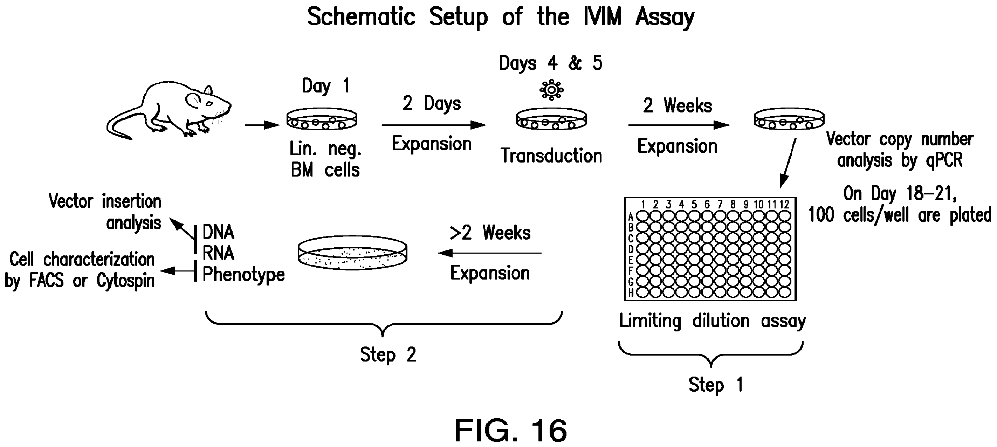

Lineage-negative (Lin-) bone marrow (BM) cells from C57BL/6 mice were isolated from complete BM by magnetic sorting using lineage-specific antibodies. The Lin- BM cells were then cultured and stimulated in complete growth medium for 2 days. On Day 4, cells were transduced with INXN-2002 vector in a 48-well plate coated with RetroNectin (10µg/cm², Takara). Figure 16 shows a schematic setup of the IVIM assay.

To enhance the INXN-2002 transduction efficiency on Lin- BM cells, INXN-2002 vector was concentrated approximately 14-fold using a spin concentrator. For transduction, 80 µL of the concentrated INXN-2002 was added to each well containing 1.5 x 10⁵ cells with 150 µL of transduction medium. The plate was centrifuged for 1000g at 32°C for 70 minutes (spinocculation) to further enhance the transduction efficiency. Cells were incubated at 37°C, 5% CO₂ incubator O/N.

In order to achieve a high INXN-2002 vector copy number in the transduced Lin- BM cells, INXN-2002 transduction was repeated on Day 5, Day 6, and Day 7 for a total of 4 consecutive transductions.

The transduced Lin- BM cells were expanded for 10 days and cell concentration was adjusted to 2 - 4 x 10⁵ cells/ml every two days, and fresh medium was provided as needed. On Day 10 post transduction, cells were harvested and counted. A portion of the cells was then submitted for DNA isolation and qPCR to determine the vector copy number (VCN). The remaining cells were placed back into culture for potential plating of the IVIM assay between days 18 - 21.

Table 3 shows the pilot test run result of INXN-2002 transduced Lin- BM cells harvested on Day 10.

**Table 3: Day 10 Post-Transduction Lin- BM Cells Analysis**

| **Test Article** | **Total Cells** | **Cell Viability** | **Vector Copy per Cell** |
|---|---|---|---|
| INXN-2002 | 1.5 x 10⁶ | 89% | 0.09 |
| Control vector-1 | 2.7 x 10⁶ | 95% | 0.81 |
| Control vector-2 | 2.1 x 10⁶ | 91% | 3.58 |
| Mock | 3.0 x 10⁶ | 88% | 0.0 |

Despite the use of concentrated INXN-2002 vector and 4x consecutive spinocculation transduction, the vector copy number reached in the Lin- BM cells was significantly lower than the targeted copy number of 1-3 expected for the IVIM test. The pilot test was terminated.

Because of the low vector copy numbers experienced in the IVIM assays as well as the low vector copy numbers achieved in all the RDEB fibroblast cell transduction runs, it is believed that this lot of INXN-2002 poses minimal risk of insertional genotoxicity when used to transduce RDEB fibroblast cells. No further analysis of this lot of INXN-2002 by the IVIM assay was pursued.

### G. FCS-007 Transduced with INXN-2002

The human dermal fibroblast cells used for the manufacture of FCX-007 are derived from a live skin biopsy. The biopsy is digested using Liberase (Roche) to release the dermal fibroblast cells and then the cells are expanded in culture using standard cell culture techniques. At the completion of culture expansion after INXN-2002 transduction, the cells are harvested and washed, then formulated to contain 1.0 - 3.0 x 10⁷cells/mL. The DS is tested for purity and confirmed to contain ≥ 98% fibroblasts by CD90 staining with cell viability of ≥ 85%.

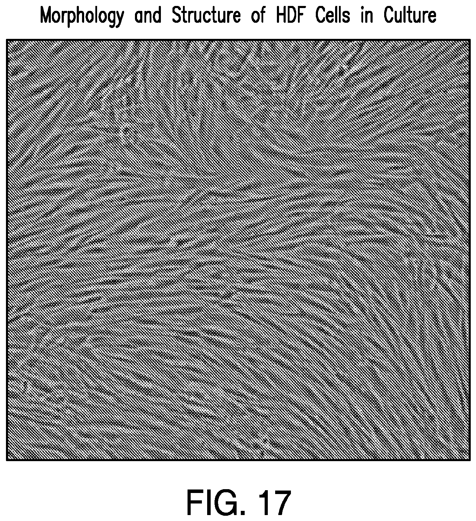

FCX-007 cells transduced with the INXN-2002 LV in the DS formulation display typical fibroblast morphologies when cultured on tissue culture surfaces. Specifically, cells may display an elongated, fusiform or spindle appearance with slender extensions, or cells may appear as larger, flattened stellate cells which may have cytoplasmic leading edges. A mixture of these morphologies may also be observed. Figure 17 shows a typical cell morphology and structure of FCX-007 DS in culture.

The cells express proteins characteristic of normal fibroblasts including the fibroblast specific marker, CD90 (Thy-1), a 35 kDa cell-surface glycoprotein, and the extracellular matrix proteins, such as various types of collagen.

### H. FCX-007 Derived from Lentiviral Vector INXN-2002: Drug Substance Release Characterization

FCX-007 Drug Substance is characterized for release at three stages during manufacturing: in- process, bulk harvest, and after cryopreservation (DS). Table 4 provides a list of the characterization assays and specifications for release of FCX-007 DS manufactured by PCT. Characterization assay results for the finished Drug Substance are provided in the attached CoTs for Training Run 8 Arm A, Arm B and Arm C (Non-Transduced Control).

**Table 4: FCX-007 Drug Substance Release Characterization**

| **Stage** | **Assay** | **Test Method** | **SOP** | **Specification** |
|---|---|---|---|---|
| In-Process | Morphology | Microscopic evaluation | PCT SOP-0334 | Pass |
| | Cell confluence | Microscopic evaluation | PCT SOP-0334 | Pass |
| Bulk Harvest | Cell count | Hemacytometer | PCT SOP-0329 | ≥1E9 |
| | Cell viability | Hemacytometer | PCT SOP-0329 | ≥ 85% |
| Drug Substance-Cryovial | Cell Count | Hemacytometer | PCT SOP-0329 | 1.0-2.7E7 cells/mL |
| | Cell Viability | Hemacytometer | PCT SOP-0329 | >/= 85% |
| | Purity | FACS | PCT SOP WI-1067 | >/= 98% CD-90+ |
| | COL7A1 gene copy # | qPCR | BioReliance109011.BSV | Report result |
| | C7 expression | ELISA | PCT SOP-0338 | Report result |

### I. Additional FCX-007 DS Derived from INXN-2002 Characterization

Additional characterization of FCX-007 has been performed on the training production runs to confirm expression of functional C7. The representative assessments shown below are from Training Run 8 (TR8) where cells were transduced with either a high dose (3.4 IU/cell) or low dose (1.7 IU/cell) of LV-COL7, or were mock-transduced. These transduction arms are also referred to as Arm A, B, and C, respectively.

### J. FCX-007 DS Transduced with INXN-2002: C7 Expression Level

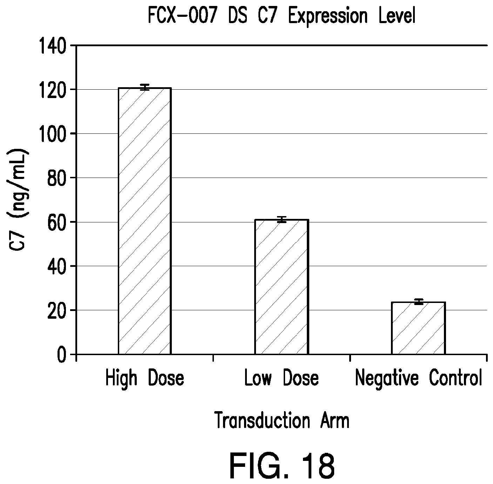

An enzyme-linked immunofluorescence assay (ELISA) was developed for the purpose of quantifying C7 expression by FCX-007. For this assay, TR8 Drug Substance vials (high dose, low dose, and mock-transduced) were thawed and cultured for 3 days and conditioned cell culture supernatants were collected and assayed for C7. Results in Figure 18 show virus dose-dependent protein expression that ranges from 60 to 120 ng/mL C7 in LV-COL7-transduced cells.

### K. FCX-007 DS Transduced with INXN-2002: C7 Trimer Formation

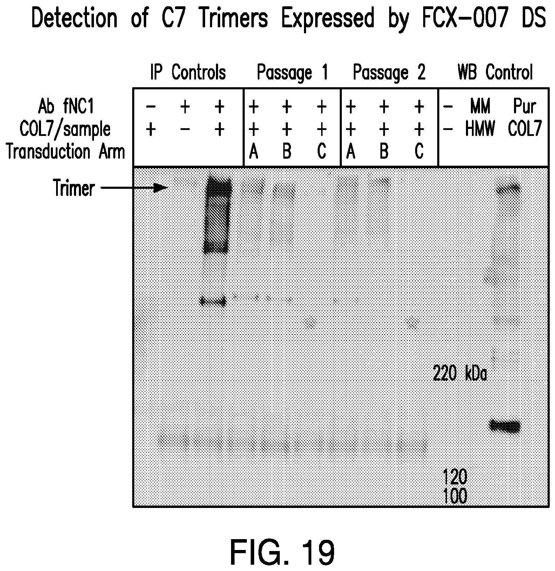

Anchoring fibrils are formed from the assembly of C7 trimers. The proper expression and formation of C7 trimers by FCX-007 can be detected by immunoprecipitation of C7 followed by non-denaturing SDS-PAGE/immunoblot analyses. For Figure 19, C7 was immunoprecipitated from FCX-007 cell culture supernatants at passage 1 and passage 2 post- thaw using a NC1-specific antibody and was separated on non-denaturing SDS-PAGE then visualized by western blot. The C7 produced by RDEB fibroblasts was predominantly trimeric (red arrow; ~870 kDa) with LV-COL7-transduced cells (Transduction Arms A and B) expressing more C7 than mock-transduced cells (Arm C, starred). Monomeric (290 kDa) and dimeric (580 kDa) forms were also observed. Assay controls included immunopreciptation of purified C7 (Pur COL7) and immunoprecipitation without antibody or test sample (IP Controls).

### L. FCX-007 Transduced with INXN-2002: C7 Binding to Lam332

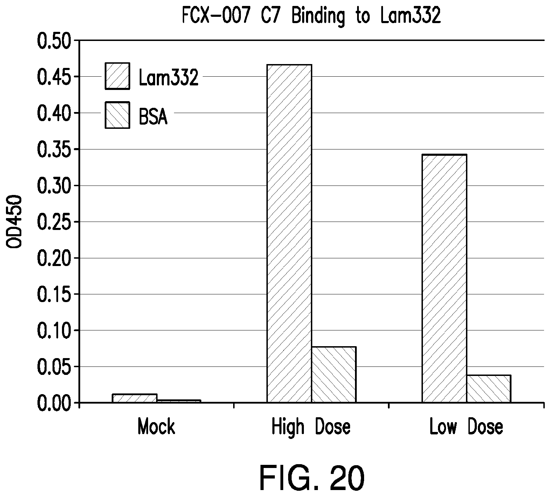

C7 interacts with Laminin332 at the dermal/epidermal junction (DEJ). The interaction between C7 and Lammin332 is important for anchoring fibril functionality (Chen 2002, Rousselle 1997, Waterman 2007). A binding assay for detection of this interaction was developed at Intrexon. Drug Substance vials (high dose, low dose, and mock-transduced) were thawed and cultured for 2 days. Conditioned cell culture supernatants were collected and were incubated with Lam332 or bovine serum albumin (BSA)-coated wells and bound C7 was detected using a C7 NC1- specific antibody and an HRP-conjugated secondary antibody. The assay readout is optical density at 450 nm (OD450). Results in Figure 20 show virus dose-dependent binding to Lam332 compared with a BSA control.

### M. FCX-007 Derived from INXN-2002 Migration Assessment

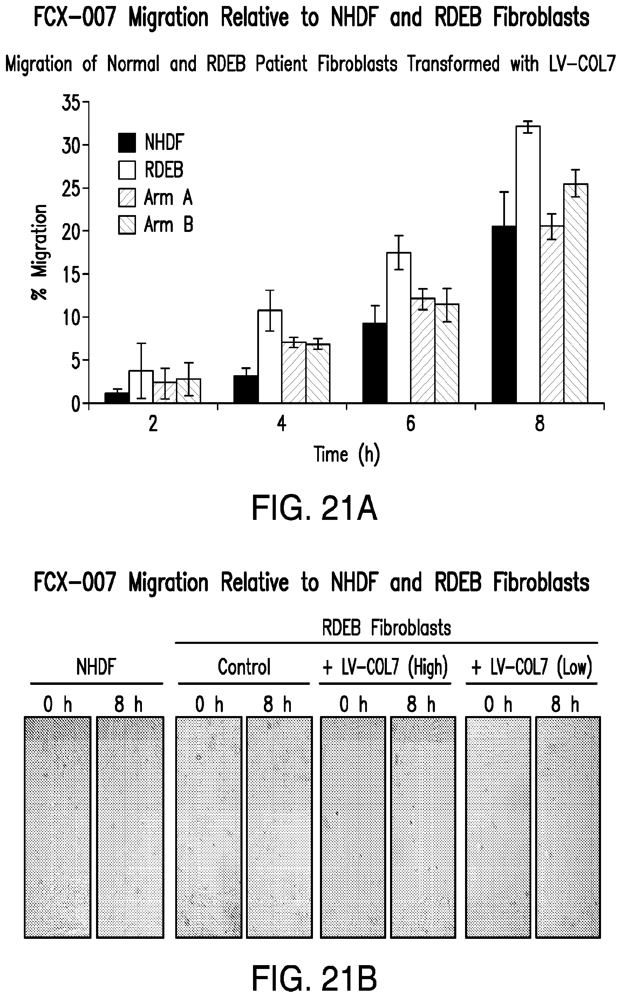

A migration assay was developed to assess function of C7. Chen 2000 has demonstrated that RDEB patient skin cells migrate faster than normal skin cells into an artificial wound margin created on tissue culture vessels, and that application of C7 can restore the migration rate. Normal human dermal fibroblasts (NHDFs; Lonza) and cells from FCX-007 Drug Substance vials were thawed and cultured. Cells were seeded into culture dishes with an insert to prevent cell adherence in a small strip of the culture dish. The strip was then removed and the rate at which the cells migrated into the open area was monitored by microscopy and quantified using the irregular shape delineation plugin for ImageJ software. Figures 21A-21B show both the percent migration (A) and the images of migration (B) for this assay. The results show that the mock-transduced RDEB patient fibroblasts migrate into the open area faster than NHDFs, and transduction with LV-COL7 reverts the patient cells to a rate of migration similar to NHDFs. These results are consistent with those described by Chen 2000.

### Example 2: FCX-007 Transduced with INXN-2004 Lentiviral Vector

FCX-007 is an autologous fibroblast cell product genetically modified by INXN-2004 lentiviral vector (LV-COL7) to express the human collagen 7 protein (C7). The materials used to manufacture FCX-007 DS/INXN-2004 are described below are similar to that set forth above in Example 1, except that the IGE-230 LV-COL7 vector plasmid was used for the production of INXN-2002. IGE-308 LV-COL7 vector plasmid was used to make the INXN-2004 vector. The same helper plasmids, pCMV-G, pCMV-Rev2, and pCgp were used to co-transfect a 293 WCB cell line.
INXN-2004 Lentiviral Vector
IGE308 LV-COL7 Vector Transfer Plasmid

The IGE308 plasmid was constructed using standard molecular cloning methods. The construction process involved cloning of the human *COL7A1* gene and introduction of the cloned *COL7A1* gene into a lentiviral vector (SIN) backbone, pFUGW, to produce the IGE308 LV-COL7 vector transfer plasmid.

### Cloning of the Human COL7A1 Gene

The *COL7A1* gene cloned into the IGE308 LV-COL7 vector transfer plasmid is the same *COL7A1* gene cloned into INXN-2002 (IGE230 vector transfer plasmid).

Primers were designed and produced with the Takara PrimeScript reverse transcriptase to amplify the *COL7A1* gene from human genomic cDNA. Four primer pairs, as shown in Table 5 were designed, each to amplify approximately 2 kb of the *COL7A1* gene.

**Table 5: Primer Sets used to Amplify the Human COL7A1 Gene**

| **Forward Primer** | **Primer Sequence** | **Reverse Primer** | **Primer Sequence** | **Expected Amplicon Size (bp)** |
|---|---|---|---|---|
| CollF1 (SEQ ID NO: 3) | | CollR3 (SEQ ID NO: 7) | | 1753 |
| CollF3 (SEQ ID NO: 4) | | CollR2 (SEQ ID NO: 8) | | 2059 |
| CollF2 (SEQ ID NO: 5) | | CollR4 (SEQ ID NO: 9) | | 2127 |
| CollF4 (SEQ ID NO: 6) | | CollR1 (SEQ ID NO: 10) | | 2993 |

Products were amplified with two primer pairs (CollF2/CollR4 and CollF4/CollR1) designed to amplify the 5' end 3790 base pairs (bp) of *COL7A1* from human genomic cDNA along with a 5' overhang for cloning into the expression vector. The two 5' PCR products (2127 bp and 2993 bp) were joined by overlap extension PCR to generate a 3790 bp final product.

In order to clone the remainder of the gene, a fragment of DNA (341bp, Col1A1-3, Table 6 was synthesized encompassing the final 322 bp of the *COL7A1* gene with overlaps to the PCR amplified 5' 3790bp gene fragment and the cloning vector.

**Table 6: Col1A1-3 Fragment Sequence**

| **Fragment** | **Sequence** |
|---|---|
| Col1A1-3 (SEQ ID NO: 11) | |

The two *COL7A1* gene fragments (3790 bp and Col1A1-3) were assembled into an inducible expression vector (VVN-257673) that had previously been digested with NheI and ClaI using the In-Fusion HD Cloning Kit. Bacterial clones were screened by PCR with primers specific to the backbone vector and *COL7A1* sequence to identify candidates for sequencing. Positive clones were confirmed by digestion and *COL7A1* fragment DNA sequencing. The resulting plasmid was named VVN-4311835.

VVN-4311835 and a *COL7A1* expression plasmid (SC300011) purchased from Origene were digested with BstZ17I and SapI. A 6276 bp fragment of the *COL7A1* gene was cloned from SC300011 into VVN-4311835. The resulting plasmid, VVN-4311835 (used the same plasmid number), was completely sequenced and the *COL7A1* sequence was confirmed to be complete and without mutations.

VVN-4311835 plasmid was digested with NheI and ClaI, two restriction enzymes with sites just outside of the *COL7A1* coding sequence. The excised *COL7A1* gene was cloned into a constitutive expression vector, VVN-257231 which was also digested with NheI and ClaI, to produce VVN-4319958. VVN-4319958 expresses the full length human *COL7A1* under the control of the constitutive CMV promoter.

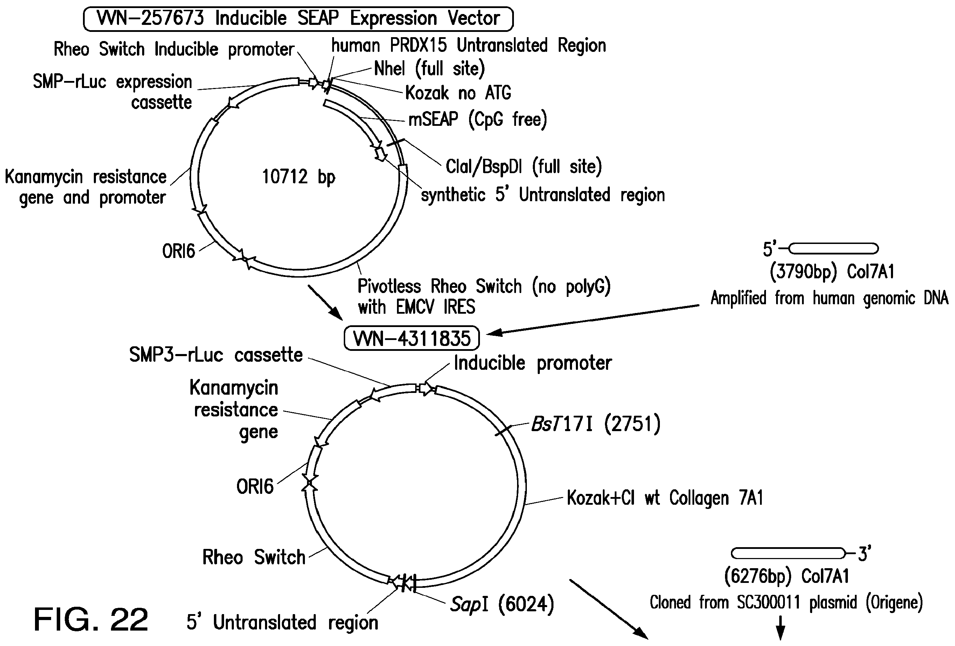

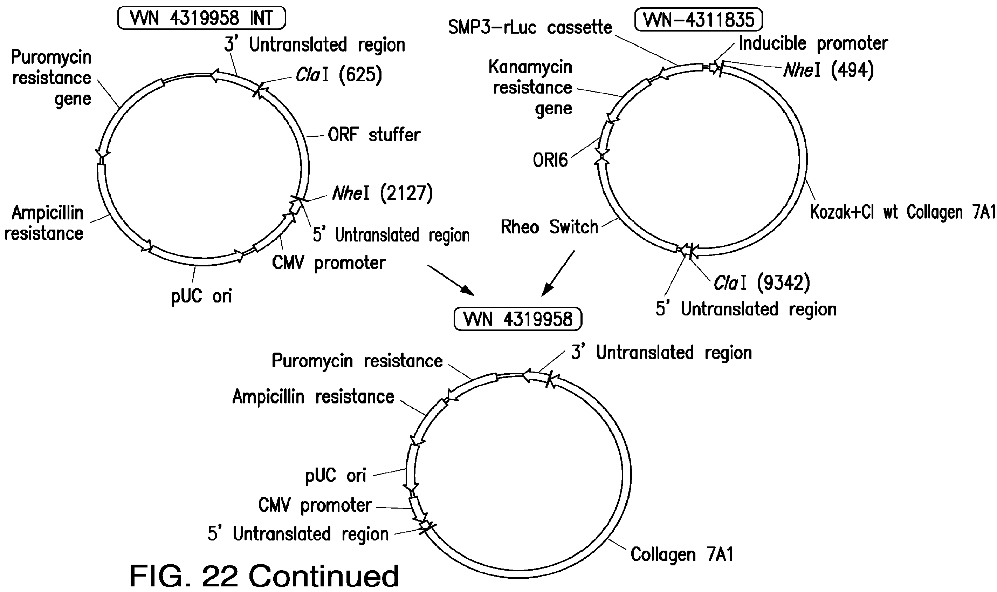

Figure 22 provides a schematic for the cloning of the human *COL7A1* gene.

### Introduction of the Cloned COL7A1 Gene into a Lentiviral Vector (SIN)

The pSMPUW lentiviral expression vector (VPK-211, Cell Biolabs, Inc., San Diego, CA) was initially selected for construction of INXN-2002 lentiviral vector encoding the *COL7A1* gene based on its enhanced safety features and large cloning capacity.

Figure 7 compares the pSMPUW vector to a standard 3rd generation SIN LV vector. The pSMPUW vector encodes for a multicloning site (MCS) followed by the Woodchuck Hepatitis Virus Post-transcriptional regulatory element (WPRE). The residual gag (Δgag) and the RRE element were removed from the pSMPUW vector construct. Additionally, the pSMPUW vector construct utilized a larger 400 bp deletion in the 3'LTR U3 region instead of the commonly used 133 bp deletion in a standard 3rd generation SIN LV vector.

Details of the cloning of the *COL7A1* gene into the pSMPUW lentiviral expression vector for construction of INXN-2002 lentiviral vector were described above in Example 1, and are further described below.

An insert was generated containing a CMV promoter followed by a Kozak sequence and a truncated version of the *COL7A1* gene with BclI and SapI restriction sites to use for introduction of the entire coding sequence. This insert was synthesized in two fragments, CColG and ColG2 (IDT) (Table 8). These two fragments were assembled with the digested pSMPUW vector using the In-Fusion HD Cloning Kit. Positive clones were identified using colony PCR with a primer specific to the plasmid backbone (63968-3R), and a primer specific to the *COL7A1* gene (ColS14). Plasmids from positive clones were purified and sequence confirmed to generate IGE228. IGE228 was digested with FspI and BamHI.

Initial attempts to clone the *COL7A1* gene using the BclI and SapI sites were unsuccessful. An alternate strategy was devised using a PCR product as a linker to bypass the BclI restriction site. Primers EPF5 and CollR3 were used to generate a PCR product from the plasmid template VVN-4319958. This product was digested with BamHI, which cuts 48 bp upstream of the 5' end of *COL7A1,* and FspI, which cuts 1630 bp into the 5' end of *COL7A1* to generate the 5' linker.

The wild type *COL7A1* gene was cut with FspI and SapI from VVN-4319958. This fragment was ligated to the digested IGE228 and the 5' linker to generate a lentivirus construct for the expression of *COL7A1,* VVN-4580853 (also named IGE230). Positive clones were identified using the primers ColS13, specific to *COL7A1* and 63968-3R, specific to the lentiviral backbone. The plasmid was sequenced from the CMV promoter to the 3' end of *COL7A1.*

Table 7 below shows the synthetic gene sequences and primers used for introduction of the *COL7A1* gene into the pSMPUW lentiviral expression vector.

**Table 7: Synthetic Gene Elements and Primers**

| **Gene Element / Primer Name** | **Sequence** |
|---|---|
| CColG (SEQ ID NO: 12) | |
| ColG2 (SEQ ID NO: 13) | |
| 63968-3R (SEQ ID NO: 14) | ATGGAAAAACGCCAGCAACG |
| ColS14 (SEQ ID NO: 15) | CTGTCACCCTTTTGTCTATG |
| EPF5 (SEQ ID NO: 16) | GTTGCCGGACACTTCTTGTCCTCT |
| CollR3 (SEQ ID NO: 17) | CCCGCACAGTGTAGCTAAGCCC |
| ColS13 (SEQ ID NO: 18) | AGAGGCCCCGAAGGACTTCA |

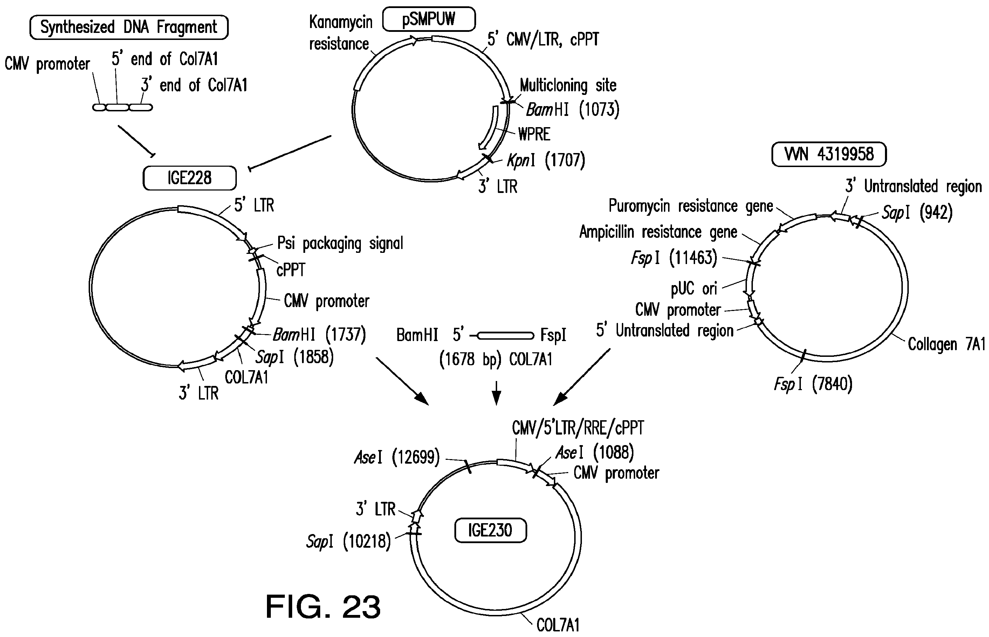

Figure 23 provides a schematic of the cloning of the *COL7A1* gene into the pSMPUW expression vector to produce the INXN-2002 lentiviral vector transfer plasmid, IGE230.

Results from continuing development indicated that deletion of the RRE element and the use of a large 400 bp deletion in the 3'LTR U3 region in the pSMPUW vector construct, combined with the requirement to package a large *COL7A1* gene (8.8 kbp), resulted in a negative impact on LV-COL7 vector production (infectious titer) and subsequent transduction and C7 protein expression in RDEB fibroblast cells.

A 3^{rd} GEN SIN vector, pFUGW (FUGW, plasmid #14883, www.addgene.org) was selected to construct a second generation LV-COL7 vector, INXN-2004. INXN-2004 was constructed to improve the vector copy number. Figure 8 provides a schematic of the genetic elements in the pFUGW lentiviral expression plasmid vector.

The pFUGW vector contains a RRE, two cPPT elements, as well as a WPRE (woodchuck hepatitis virus posttranscriptional regulatory element) element for achieving improved lentiviral vector production and transgene expression. The IGE308 lentiviral vector transfer plasmid was constructed to maximize the transgene cloning capacity to accommodate the insertion of the large *COL7A1* gene (8.8 kbp).

The WPRE element, the hUBC promoter and the GFP reporter gene were removed through digestion of the vector with PacI and XhoI. An insert was generated containing the 5' end of the CMV promoter followed by a small fragment of the 3' end of *COL7A1* gene. This insert (ColCN) was synthesized at IDT as a G-Block fragment (sequence in Table 8). The fragment was assembled with the digested pFUGW vector using a Gibson cloning derivative method. Positive clones were identified using colony PCR with primers specific to the plasmid backbone (FugwQCF and FugwQCR). Plasmids from positive clones were purified and sequence confirmed to generate IGE301.

IGE301 was then digested with BaeI. IGE230 was digested with AseI and SapI to create a fragment containing the 3' end of the CMV promoter, the 5' UTR, and the majority of the *COL7A1* gene. The fragment was transferred to the BaeI-digested IGE301 vector using a Gibson cloning derivative method. Positive clones were identified using the primers ColS13, specific to *COL7A1* and FugwQCR, specific to the lentiviral backbone. Plasmids from positive clones were purified and sequence-confirmed to generate IGE308.

Table 8 below provides the synthetic gene sequences and primers used for introduction of the *COL7A1* gene into the pFUGW lentiviral expression vector.

Plasmids were Maxi-prepped with the Qiagen MaxiPrep Kit per the manufacturer's protocol.

**Table 8: Synthetic Gene Elements and Primers used in the Final Steps of IGE308 Creation**

| **Gene Element / Primer Name** | **Sequence** |
|---|---|
| ColCN (g-block) (SEQ ID NO: 19) | |
| FugwQCF (SEQ ID NO: 20) | GTAGACATAATAGCAACAGAC |
| FugwQCR (SEQ ID NO: 21) | TATTGCTACTTGTGATTGCTC |
| ColS13 (SEQ ID NO: 22) | AGAGGCCCCGAAGGACTTCA |

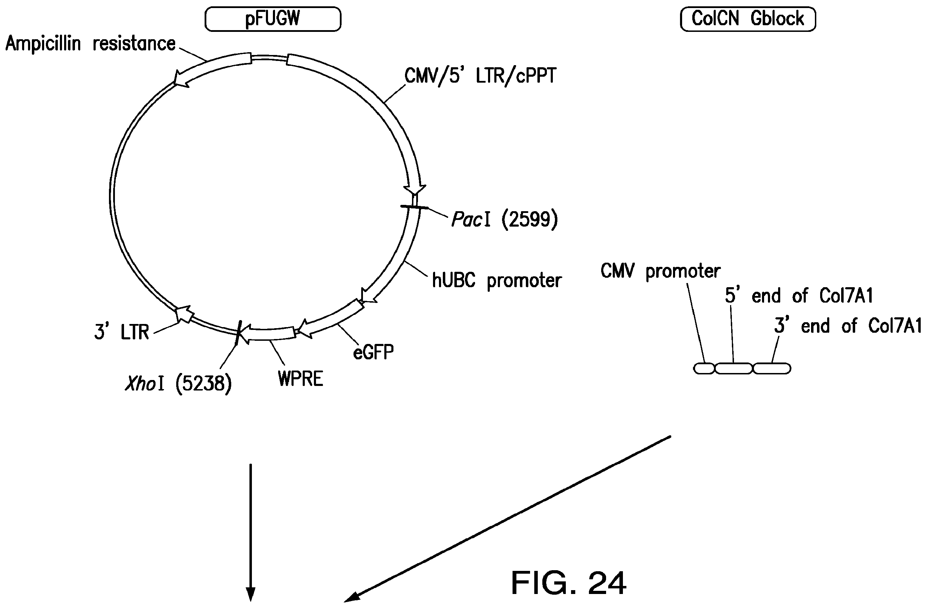

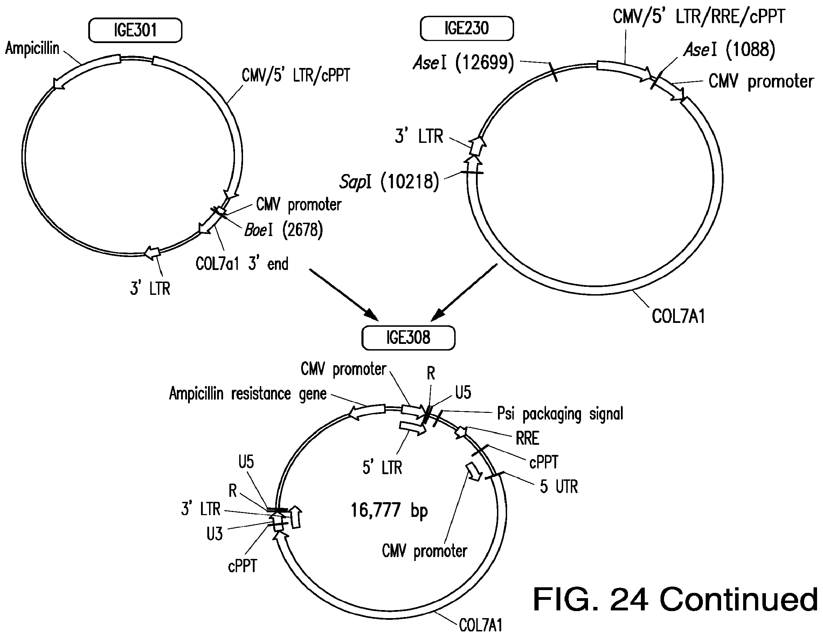

Figure 24 provides a schematic representation of the construction of the INXN-2004 lentiviral vector transfer plasmid (IGE308).

### Production of IGE-308 Plasmid

The IGE308 plasmid was produced using a process consisting of five steps: transformation, glycerol stock production, scale up, capture, diafiltration and formulation.

Transformation: a seed stock of the IGE308 plasmid was provided to Aldevron for transformation into the competent *E. coli* (DH10B). Transformation plates were stored at 34°C which is the standard for lentiviral constructs.

Glycerol stock production - Seed cultures were created by picking isolated colonies from the transformation plate. Each culture was mini prepped and the best seed culture, as determined via agarose gel, was used to make the working glycerol stocks. Scale Up - One 500 mL culture was grown in each of the following media types: Rapid Growth media and Maximum Yield media (for a total of two 500 mL cultures) in order to compare media. The cultures were inoculated using the glycerol stocks created in step two and a full panel of QC assays was run on each prep. This also allowed Aldevron to test the stability of the plasmid in each media type.

Based on the initial QC results Rapid Growth media was chosen for growth of the transformed *E. coli* in shaker flasks at 34°C. A total of 8L of culture was prepared.

Capture - The 8 L of *E. coli* culture was lysed and initially purified using DMAE anion exchange chromatography which produced a total of 833.8 mg plasmid. An additional purification step was run using HIC (Hydrophobic Interaction Chromatography) chromatography over OS resin. This step yielded 663 mg of purified DNA.

Concentration and Formulation - The purified plasmid was then adjusted to the final buffer and concentration via serial diafiltration. The final buffer used was TE.

A total of 200 mg of IGE308 plasmid was shipped at a concentration of 1.4 mg/mL. The IGE308 plasmid was analyzed prior to further manufacture of the INXN-2004.

### Analysis of IGE308 Plasmid Sequence

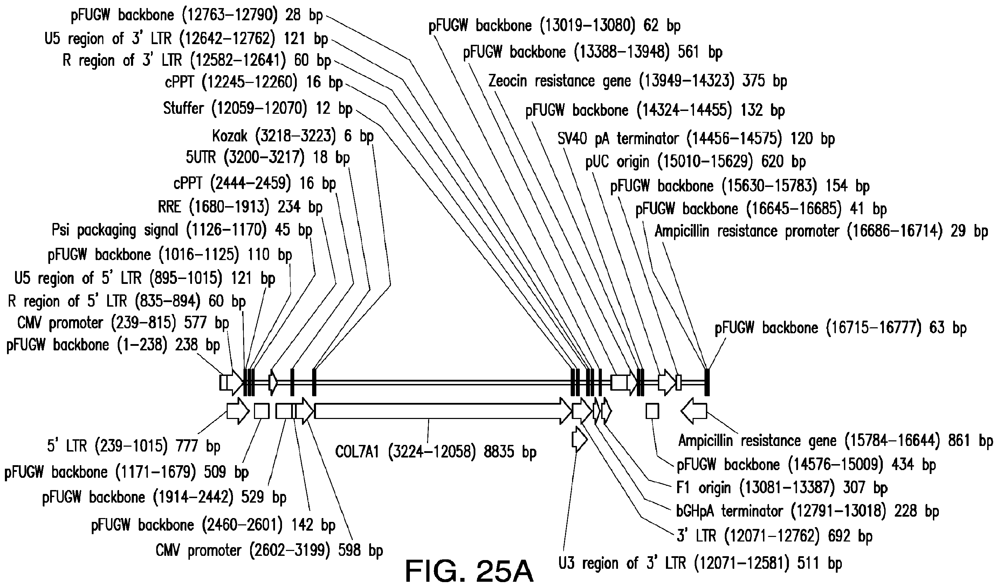

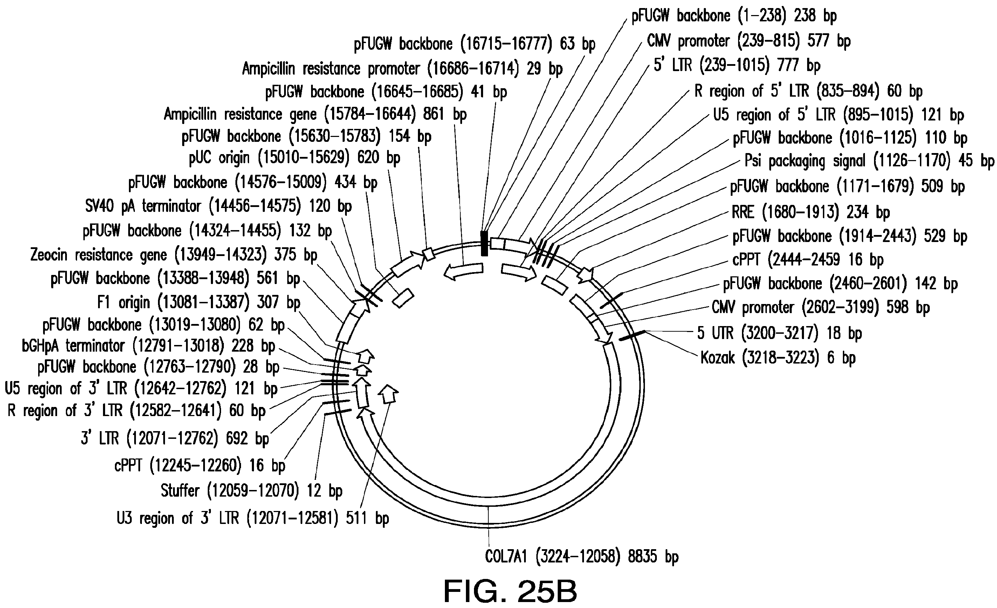

The IGE308 plasmid used for INXN-2004 manufacture was fully sequenced at SeqWright under GLP conditions using primer walking and oligonucleotide synthesis to yield 4-fold, bi-directional sequence coverage. The IGE308 plasmid has a size of 16,777 bp. The sequence showed 100% match to the IGE308 construction reference sequence provided to SeqWright. Gene elements in the IGE308 plasmid are illustrated in Figures 25A-25B. Table 9 provides a list of gene elements with their corresponding functions.

**Table 9: Size and Functions of Gene Elements in IGE308 Plasmid**

| **Location** | **Size (bp)** | **Element** | **Function** |
|---|---|---|---|
| 1-238 | 238 | pFUGW backbone | Backbone element |
| 239-815 | 577 | CMV promoter | Promoter to drive expression of the proviral genome |
| 239-1015 | 777 | 5' LTR | Proviral genome packaging and integration |
| 835-894 | 60 | R region of 5' LTR | Repeat region in 5' and 3' LTRs, transcription initiation site |
| 895-1015 | 121 | U5 region of 5' LTR | Unique 5' sequence contains the Tat binding site and packaging sequences of HIV |
| 1016-1125 | 110 | pFUGW backbone | Backbone element |
| 1126-1170 | 45 | Psi packaging signal | RNA target site for packaging the viral RNA genome into viral capsid during replication |
| 1171-1679 | 509 | pFUGW backbone | Backbone element |
| 1680-1913 | 234 | RRE | Facilitates mRNA transcript nucleus export when bound to Rev protein |
| 1914-2443 | 529 | pFUGW backbone | Backbone element |
| 2444-2459 | 16 | cPPT | Recognition site for proviral DNA synthesis, increases transduction efficiency and transgene expression |
| 2460-2601 | 142 | pFUGW backbone | Backbone element |
| 2602-3199 | 598 | CMV promoter | Promoter to drive the expression of *COL7A1* gene inside the LV-COL7 vector |
| 3200-3217 | 18 | 5UTR | Synthetic 5'UTR fragment to improve the translation of the *COL7A1 (derived from* Rahnella aquatilis, ATCC 33071) |
| 3218-3223 | 6 | Kozak | Translation enhancement |
| 3224-12058 | 8835 | Collagen 7A1 ORF with TGA stop codon | Collagen 7A1 ORF encoding the human wild type collagen 7 protein |
| 12059-12070 | 12 | Stuffer | Remnant sequence resulted from DNA cloning |
| 12071-12762 | 692 | 3'LTR | Polyadenylation signal and proviral genome packaging and integration |
| 12071-12581 | 511 | U3 region of 3' LTR | SIN deletion (133 bp) located in this region |
| 12245-12260 | 16 | cPPT | Recognition site for proviral DNA synthesis, increases transduction efficiency and transgene expression |
| 12582-12641 | 60 | R region of 3' LTR | Repeat region in 5' and 3' LTRs, transcription initiation site |
| 12642-12762 | 121 | U5 region of 3' LTR | Unique 5' sequence contains the Tat binding site and packaging sequences of HIV |
| 12763-12790 | 28 | pFUGW backbone | Backbone element |
| 12791-13018 | 228 | bGHpA terminator | Sequence to stop transcription of DNA |
| 13019-13080 | 62 | pFUGW backbone | Backbone element |
| 13081-13387 | 307 | F1 origin | F1 phage origin of replication |
| 13388-13948 | 561 | pFUGW backbone | Backbone element |
| 13949-14323 | 375 | Zeocin resistance gene | Mammalian selection marker |
| 14324-14455 | 132 | pFUGW backbone | Backbone element |
| 14456-14575 | 120 | SV40 pA terminator | Sequence to stop transcription of DNA |
| 14576-15009 | 434 | pFUGW backbone | Backbone element |
| 15010-15629 | 620 | pUC origin | Bacterial DNA replication start |
| 15630-15783 | 154 | pFUGW backbone | Backbone element |
| 15784-16644 | 861 | Ampicillin resistance gene | Plasmid selection marker |
| 16645-16685 | 41 | pFUGW backbone | Backbone element |
| 16686-16714 | 29 | Ampicillin resistance promoter | Promoter to drive expression of the ampicillin resistance gene |
| 16715-16777 | 63 | pFUGW backbone | Backbone element |

The *COL7A1* gene sequence in the IGE308 plasmid (without the stop codon TGA) was compared to the GenBank Homo sapiens collagen, type VII, alpha 1 (*COL7A1*) sequence (NM_000094.3). Sequence alignment is provided in Appendix 4. Three silent point mutations were identified as listed in Table 10 below. No sequence gap was identified. The three silent point mutations have no impact on the encoded C7 protein.

**Table 10: Sequence Comparison of COL7A1 in IGE308 and GenBank Consensus**

| **Nucleotide Position** | ***COL7A1* in IGE308** | **GenBank Consensus (NM_000094.3)** | **Impact** |
|---|---|---|---|
| 6040 | G | A | Silent point mutation |
| 8674 | A | G | Silent point mutation |
| 8731 | G | C | Silent point mutation |

### Helper Plasmids, pCMV-G, pCMV-Rev2 and pCgp

The three helper plasmids used for INXN-2004 LV vector production, pCMV-G, pCMV-Rev2 and pCgp, were provided by CoH.

### 293T Working Cell Bank (WCB)

The 293T WCB used for INXN-2004 LV vector production was provided by CoH.

### Other Starting Materials for INXN-2004 Manufacture

Other starting materials used for INXN-2004 LV vector production were also provided by CoH.

### Biopsy Tissue

Three 3-4 mm punch skin biopsies (dermis and epidermis layers) are collected from an un-blistered area of the RDEB subject's body using standard aseptic practices. The biopsies are collected by the treating physician, placed into a vial containing cold, sterile phosphate buffered saline (PBS), and shipped via next day delivery in a refrigerated Infectious Shipper container, which is appropriate for shipping potentially biohazardous tissue and is designed to maintain a temperature of 2-8°C.

### a. Reagents, Solvents, and Auxiliary Materials

### i. Reagents and Solvents

Two animal-sourced reagents are used in the FCX-007 Drug Substance manufacturing process: Fetal Bovine Serum (FBS) and Trypsin-EDTA solution. Complete Growth Medium

Complete Growth Medium (CGM) is used in FCX-007 manufacturing during all the cell culture expansion steps. CGM is prepared by mixing 18 L of IMDM in a 20 L bag with 2 L of FBS through aseptic transfer. The formulated CGM bag is stored in a 5±3°C refrigerator.

### a. Initiation Growth Medium

Initiation Growth Medium (IGM) is used in FCX-007 manufacturing for the initial seeding of fibroblasts after biopsy digestion into a T-75 cell culture flask in the presence of antibiotics. Initiation Growth Medium is prepared fresh by adding 0.6 mL of the100-fold concentrated antibiotic solution (GA) aseptically inside an ISO5 BSC to 58.2 mL of CGM in a 500 mL square media bottle. Final concentrations are 0.4 mg/mL gentamicin, 0.295 µg/mL amphotericin B. GSH solution (1.2 mL of 50X solution) is also added to the 500 mL square media bottle. The bottles are closed and inverted 3-5 times to mix. The IGM is warmed inside a 37°C incubator for at least 60 minutes before immediate use.

The Initiation Growth Medium is prepared fresh immediately prior to use and therefore no expiration date is applied and no Quality Control release tests are performed.

### b. Transduction Medium

Transduction Medium (TM) is used during the INXN-2004 transduction of fibroblast cells. Transduction Medium is prepared fresh inside an ISO5 BSC by adding 40 mL IMDM to a 250 mL centrifuge tube containing 10 mL CGM to dilute the FBS concentration to 2% in the medium. The 250 mL tube is mixed and placed inside a 37.0°C Incubator to warm until needed for INXN-2004 transduction.

The Transduction Medium is prepared fresh immediately prior to use and therefore no expiration date is applied and no Quality Control release tests are performed.

### c. GSH Solution

GSH is supplemented to the Initiation Growth Medium (IGM) and CGM used in the early stage T-75 and T-175 fibroblast cell cultures to enhance cell growth. A 50X GSH stock solution is prepared inside an ISO5 BSC by dissolving 51.1 g of GSH into 1 L of IMDM. The solution is 0.22 µm filtered. The filtered solution is aliquoted into 50 mL centrifuge tubes at 20 mL per tube and stored frozen at -80°C for further use.

### d. RetroNectin^{™} Solution

RetroNectin^{™} is used in the FCX-007 production process to enhance the INXN-2004 transduction efficiency on fibroblast cells. Inside an ISO5 BSC, using a 5 mL syringe and a 18g needle, 2.5 mL WFI is aseptically transferred into a vial of clinical grade RetroNectin^{®} to reconstitute RetroNectin^{®} at 1.0 mg/mL. RetroNectin^{®} is dissolved thoroughly by swirling gently. The entire contents of the RetroNectin^{®} vial are withdrawn using the attached 5 mL syringe. The 18g needle is aseptically replaced with a 0.22 µm syringe filter onto the syringe, and the reconstituted RetroNectin^{®} is sterile filtered into a 250 mL centrifuge tube. Using an appropriately sized pipette, 122.5 mL PBS is transferred to the reconstituted RetroNectin^{®} in the 250 mL centrifuge tube to dilute the RetroNectin^{®} to 20µg/mL, which is then mixed well using a pipette. Using an appropriately sized pipette, 6.3 mL of the diluted RetroNectin^{®} solution is added to each of T-25 flasks to coat the surface with RetroNectin^{®} at 5 µg/cm². For the first round of INXN-2004 transduction, 5x to 10x T-25 flasks are prepared. For INXN-2004 super transduction, 18x T-25 flasks are prepared.

The RetroNectin^{™} solution is prepared fresh immediately prior to use and therefore no expiration date is applied and no Quality Control release tests are performed.

### Cryopreservation Medium

Cryopreservation Medium is a two-fold concentrated solution that is added to harvested and washed fibroblasts during the FCX-007 manufacturing process to formulate the cell seed stock and FCX-007 Drug Substance for storage in liquid nitrogen. Cryopreservation Medium is prepared by mixing 0.85 volume ProFreeze^{™}-CDM (2X) with 0.15 volume DMSO to obtain one volume Cryopreservation Medium.

The Cryopreservation Medium is prepared fresh immediately prior to use and therefore no expiration date is applied and no Quality Control release tests are performed.

### Example 3: Training Run 8, 9 and 10 and Enhanced Biopsy Enzymatic Digestion

### Training Run 8 and Enhanced Biopsy Enzymatic Digestion

In the above TRs, the biopsy digestion method was suspected to not be able to achieve effective digestion of the 3-4mm sized biopsy tissue. In TR8, the application of additional shear force during the digestion process was incorporated into the process to improve the overall digestion efficiency. The digestion process was modified to the following: pulse vortex the centrifuge tube at maximum setting for 5 seconds, every 15 ± 2 minutes during the digestion, returning the centrifuge tube to the orbital shaker after each vortex; at the end of the 60 minutes of incubation, pulse vortex the centrifuge tube at maximum setting for 10 seconds.

A biopsy from an RDEB donor was processed and digested using the enhanced digestion method. Cells from the digestion were seeded into a T-75 flask using culture medium supplemented with GSH. Cells showed good growth and reached 90% confluence on day 14 for passaging. To evaluate the INXN-2002 transduction conditions, cells harvested from the T-75 flask were seeded into 3x T-175 flasks, as Control, Arm A, and Arm B. Cells in all three arms were expanded, transduced with the 10 L pilot LV-COL7 vector in 1-CS (the control arm was mock transduced), and then further expanded into 2x 10-CS before being harvested for cryopreservation.

**Table 11: Cell Growth and Cell Yields in Training Run 8**

| **Activity** | **Days in Culture** | **Viable Cells** | **Cell Viability** | **Note** |
|---|---|---|---|---|
| Biopsy digestion, seed into one T-75 flask, with 35 mL culture medium, 37°C and 5% CO₂ | 0 | 3.4 x 10⁵ | 99% | Good cell growth was observed in the T-75 flask; approximately 90% confluence on Day 14. |
| Passage 1: to three T-175 flasks with 50 mL culture medium, 37°C and 10% CO₂ | 14 | 6.5 x 10⁶ | 97% | Flask 1: mock transduction control |
| | | | | Flask 2: LV transduction (Arm A) |
| | | | | Flask 3: LV transduction (Arm B) |
| Passage 2: to 1-CS and LV transduction | | | | Cells were harvested from each of the T-175 flask and expanded into corresponding 1-CS for LV-COL7 transduction (CoH Pilot LV-COL7). |
| Control | 20 | 1.4 x 10⁷ | 98% | |
| Arm A | 20 | 1.0 x 10⁷ | 97% | |
| Arm B | 20 | 1.0 x 10⁷ | 98% | |
| Passage 3: to 10-CS | | | | Cells from each arm were harvested and expanded to one corresponding 10-CS. |
| Control | 26 | 6.1 x 10⁷ | 99% | |
| Arm A | 27 | 6.6 x 10⁷ | 99% | |
| Arm B | 26 | 6.4 x 10⁷ | 99% | |
| Passage 4: to 2x 10-CS | | | | Cells from each arm were harvested and expanded to two corresponding 10-CS. |
| Control | 35 | 4.0 x 10⁸ | 99% | |
| Arm A | 39 | 3.3 x 10⁸ | 98% | |
| Arm B | 38 | 3.8 x 10⁸ | 98% | |
| Harvest: 2x10-CS | | | | Cells were harvested from each of the 2x10-CS for cryopreservation and testing. |
| Control | 45 | 6.9 x 10⁸ | 95% | |
| Arm A | 47 | 4.0 x 10⁸ | 98% | |
| Arm B | 46 | 5.3 x 10⁸ | 97% | |

The total number of cells harvested from the 10-CS was considered adequate for further production of FCX-007 drug product for injection. The harvested cells were tested for COL7A1 gene copy number (transduction efficiency), C7 protein expression, cell viability, and cell purity. Table 12 provides the analysis results for the harvested cells from all three arms.

**Table 12: Analysis of Cells Harvested from Training Run 8**

| **Condition** | **INXN-2002 Transduction MOI (IU/cell)¹** | **COL7 A1gene copy/cell²** | **C7 protein expression² (ng/mL)** | **Purity² (% CD90⁺)** | **Cell Viability (%)** | **Endotoxin** |
|---|---|---|---|---|---|---|
| Arm A | 3.4 | 0.021 | 59.4 | 100% | 95% | Pass |
| Arm B | 1.7 | 0.017 | 46.6 | 100% | 93% | Pass |
| Control | Mock | BLQ³ | BLQ³ | 100% | 96% | Pass |

| | | | | | | |
|---|---|---|---|---|---|---|
| ¹ INXN-2002 IU titer: 9.2 x 10⁶ IU/mL which was determined at Intrexon using an early development stage H1299 infectious titer assay. ² Details of the assays are provided in Section 3.2.S.4. ³ BLQ: below limit of quantitation | | | | | | |

A Multiplicity of Infection (MOI) dose dependent increase in COL7A1 gene copy number and C7 protein expression was observed. Details of the INXN-2002 transduction development and optimization are described below. Although low INXN-2002 IU titer limited the transduction MOI, and the subsequent COL7A1 gene copy number, C7 protein expression was observed to be biologically relevant in the preclinical setting as evidenced by the in vitro and in vivo results. Overall, the results show that RDEB fibroblast cells can be transduced with INXN-2002 and express functional C7 protein.

The Arm A cell product from TR8 was used for Proof of Concept studies and toxicology and biodistribution studies.

### Training Run 9

A biopsy from an RDEB donor was processed and digested using the enhanced digestion method. Cells from the digestion were seeded into a T-75 flask using culture medium supplemented with GSH. Similar to TR8, cells showed good growth and reached 90% confluence on Day 19 for passaging. To evaluate the LV-COL7 transduction conditions, cells harvested from the T-75 flask were seeded into 3x T-175 flasks, as Control, Arm A, and Arm B. Cells in all three arms were expanded, transduced with LV-HA-COL7 vector (containing an HA tag in the construct) in 1-CS (the control arm was mock transduced), then further expanded into 2x 10-CS before being harvested. The harvested cells were tested for COL7A1 gene copy number (transduction efficiency), C7 protein expression, and cell purity. Table 13 provides the cell growth in TR9.

**Table 13: Cell Growth and Cell Yields in Training Run 9**

| **Activity** | **Days in Culture** | **Viable Cells** | **Cell Viability** | **Note** |
|---|---|---|---|---|
| Biopsy digestion, seed into one T-75 flask, with 35 mL culture medium, 37°C and 5% CO₂ | 0 | NA | NA | Good cell growth was observed in the T-75 flask; approximately 90% confluence on Day 19. |
| Passage 1: to three T-175 flasks with 50 mL culture medium, 37°C and 10% CO₂ | 19 | 4.5 x 10⁶ | 99% | Flask 1: mock transduction control |
| | | | | Flask 2: LV transduction (Arm A) |
| | | | | Flask 3: LV transduction (Arm B) |
| Passage 2: to 1-CS and LV transduction | | | | Cells were harvested from each of the T-175 flask and expanded into corresponding 1-CS for LV-COL7 transduction (CoH Pilot LV-HA-COL7, a research vector) 1. |
| Control | 26 | 1.0 x 10⁷ | 99% | |
| Arm A | 25 | 7.1 x 10⁶ | 97% | |
| Arm B | 25 | 7.8 x 10⁶ | 98% | |
| Passage 3: to 10-CS | | | | Cells from each arm were harvested and expanded to one corresponding 10-CS. |
| Control | 33 | 3.8 x 10⁷ | 99% | |
| Arm A | 32 | 5.3 x 10⁷ | 98% | |
| Arm B | 31 | 2.5 x 10⁷ | 97% | |
| Passage 4: to 2x 10-CS | | | | Cells from each arm were harvested and expanded to two corresponding 10-CS.² |
| Control | 46 | 2.6 x 10⁸ | 97% | |
| Arm A | 42 | 3.6 x 10⁸ | 98% | |
| Arm B | 45 | 2.8 x 10⁸ | 97% | |
| Harvest: 2x10-CS | | | | Cells were harvested from each of the 2x10-CS for cryopreservation and testing. |
| Control | 61 | 7.6 x 10⁸ | 100% | |
| Arm A | 53 | 5.3 x 10⁸ | 97% | |
| Arm B | 60 | 5.5 x 10⁸ | 98% | |

| | | | | |
|---|---|---|---|---|
| 1 LV-HA-COL7 vector is a research vector which incorporated an HA tag to the COL7A1 sequence in the INXN-2002 LV vector. ² The total number of cells harvested from the 10-CS is considered adequate for further production of FCX-007 drug product for injection. | | | | |

Table 14 provides the analysis results for the harvested cells from all three arms.

**Table 14: Analysis of Cells Harvested from Training Run 9**

| **Condition** | **LV-HA-Col7 Transductio n MOI (IU/cell)¹** | **Col7 Gene Copy/Cell²** | **Col7 Protein Expression (ng/mL)** | **Purity² (% CD90⁺)** | **Cell Viability (%)** | **Endotoxin** |
|---|---|---|---|---|---|---|
| Arm A | 1.0 | 0.009 | 71.4 | 100% | 92% | Pass |
| Arm B | 1.9 | 0.021 | 91.6 | 99% | 95% | Pass |
| Control | Mock | BLQ³ | BLQ³ | 100% | 92% | Pass |

| | | | | | | |
|---|---|---|---|---|---|---|
| ¹ LV-HA-Col7 vector titer: 2.0 x 10⁶ IU/mL which was determined at Intrexon using an early development stage H1299 infectious titer assay. ² Details of the assays are provided in Section 3.2.S.4. ³ BLQ: below limit of quantitation | | | | | | |

An MOI dose dependent increase in COL7A1 gene copy number and C7 protein expression was again observed. Again, the results show that RDEB fibroblast cells can be successfully grown, transduced with LV-HA-COL7 vector, and express C7 protein.

### Training Run 10 and Scale-Up to Six 10-CS

According to the proposed clinical protocol (See Module 5), 4 x 10⁸ FCX-007 drug product cells are needed for a single treatment dose, with a possibility of repeat dosing. To meet the projected FCX-007 product needs, it is necessary to scale-up the final cell expansion step to six 10-CSs based on the cell yields attained in TR8 and TR9.

A biopsy from an RDEB donor was processed and digested using the enhanced digestion method. Cells from the digestion were seeded into a T-75 flask using culture medium supplemented with GSH. Cells showed good growth and reached 90% confluence on Day 19 for passaging. Cells harvested from the T-75 flask were seeded into 2x T-175 flasks, one for INXN-2002 transduction, and one for cell substrate control. Cells from one flask were expanded, transduced with INXN-2002 in 1-CS, and then further expanded into 6x 10-CS before being harvested. Table 15 provides the cell growth in TR10.

**Table 15: Cell Growth and Cell Yields in Training Run 10**

| **Activity** | **Days in Culture** | **Viable Cells** | **Cell Viability** | **Note** |
|---|---|---|---|---|
| Biopsy digestion, seed into one T-75 flask, with 35mL culture medium, 37°C and 5% CO₂ | 0 | NA | NA | Good cell growth was observed in the T-75 flask; approximately 90% confluence on Day 19. |
| Passage 1: to two T-175 flasks with 50mL culture medium, 37°C and 10% CO₂ | 19 | 3.5 x 10⁶ | 98% | Flask 1: LV transduction |
| | | | | Flask 2: control cells for analysis |
| Passage 2: to 1-CS and LV transduction | 30 | 4.6 x 10⁶ | 96% | Cells from Flask 1 were harvested and expanded into 1-CS for LV-COL7 transduction (GMP grade INXN-2002). |
| Passage 3: to 10-CS | 38 | 2.1 x 10⁷ | 97% | Cells were harvested and expanded to one 10-CS. |
| Passage 4: to 6x 10-CS | 60 | 1.9 x 10⁸ | 95% | Cells were harvested and expanded to six 10-CS. |
| Harvest: 6x 10-CS | 75 | 5.8 x 10⁸ | 99% | Cells were harvested from the 6x10-CS for cryopreservation and testing. |

Relative to cells in TR8 and TR9, cells in TR10 grew slower and yielded fewer cells at each passage step, indicating potential variability among different RDEB donors.

The total number of cells harvested from the 6x 10-CS is considered adequate for production of one dosage of FCX-007 drug product for injection.

Table 16 provides the analysis results for the harvested cells.

**Table 16: Analysis of Cells Harvested from TR10**

| **INXN-2002 Transduction MOI (IU/cell)¹** | **COL7 Gene Copy/Cell²** | **C7 Protein Expression² (ng/mL)** | **Purity² (% CD90⁺)** | **Cell Viability (%)** | **Endotoxin** |
|---|---|---|---|---|---|
| 2.0 | 0.013 | 60.09 | 100% | 92% | Pass |

| | | | | | |
|---|---|---|---|---|---|
| ¹ INXN-2002 IU titer: 9.2 x 10⁶ IU/mL which was determined at Intrexon using an early development stage H1299 infectious titer assay. ² Details of the assays are provided in Section 3.2.S.4. | | | | | |

### Example 4

### INXN-2002 Transduction Development and Optimization

Lentiviral transduction of dermal fibroblast cells was initially developed and optimized using normal human dermal fibroblast cells (NHDF; Lonza, CC-2511) cultured in 96-well plates with a model lentiviral GFP (GeneCopoeia, LP-EGFP-LV105-0205).

Using GeneCopoeia's LV transduction protocol as a starting point, initial optimization evaluated conditions including cell density at time of transduction, transduction culture volume, use of RetroNectin^{™}, super-infection (re-transducing cells with virus on two consecutive days), time of cell plating (at time of transduction versus one day prior to transduction), and serum content in transduction media. The optimal transduction procedure was then implemented in a GMP production setting. Details of the studies are described below.

### A. Effect of Cell Seeding Conditions and RetroNectin^{™} Coating for LV Transduction

NHDFs in exponential growth phase were harvested and seeded into 96-well plates on two different dates at different seeding densities. One set of non tissue culture treated 96-well plates were coated with RetroNectin^{™} per the manufacturer's recommendation (the RetroNectin^{™} coating density was 20 µg/cm²). For cells seeded one day before LV transduction (Day -1), the spent medium is removed and fresh medium (100 µL) with the LV vector is added to the wells on Day 0 for transduction. For the day of transduction condition (Day 0), cells and LV vector in 100 µL of fresh medium is added simultaneously to the wells. The MOI used was 2000 vp/cell for all conditions. After an overnight incubation, the medium was removed and the cells were fed with 100 µL fresh medium for further culture. At ninety-six hours post-transduction, the cells were harvested for transduction efficiency analysis by FACS analysis of GFP signal on a BD LSRII and analyzed using FlowJo software (v.10). Table 17 provides the LV transduction efficiencies under the different conditions.

**Table 17: Effect of Cell Seeding Condition and RetroNectin^{™} Coating on LV Transduction of Fibroblast Cells (% GFP Positive Cells)**

| **Cell Seeding Density (Cells/Well; Cells/Cm²)¹** | **Without RetroNectin^{™} Coating** | | **With RetroNectin^{™} Coating** | |
|---|---|---|---|---|
| | Day -1 Cell Seeding | Day 0 Cell Seeding | Day -1 Cell Seeding | Day 0 Cell Seeding |
| 3000; 9375 | 6.8% | 11.2% | NC | 22.6% |
| 1500; 4688 | 3.3% | 7.2% | NC | 18.2% |

| | | | | |
|---|---|---|---|---|
| ¹ Surface area for a 96-well plate well: 0.32 cm² NC = Not conducted | | | | |

The results demonstrate that it is not necessary to pre-seed the cells on the day before LV transduction. Cells and LV vector can be added simultaneously at the time of transduction, which is convenient for GMP manufacturing operations. Coating the culture surface with RetroNectin^{™} prior to LV transduction significantly increased the LV transduction efficiency. The higher cell seeding density of approximately 1 x 10⁴ cells/cm² is desired during LV transduction, as the lower cell seeding density resulted in lower LV transduction efficiency.

### B. Effect of MOI and Super-Transduction on LV Transduction Efficiency

In this study, the effects of MOI and super-transduction on LV transduction of fibroblast cells were examined.

A set of non tissue culture treated 96-well plates was pre-coated with RetroNectin^{™} (20 µg/cm²) before being used for transduction. At the time of transduction, 3000 cells with LV vector in a volume of 100 µL were added to a well. After an overnight incubation, the medium was removed and the cells were fed with 100 µL fresh medium for further culture. For the super-transduction, the culture medium was removed from the wells one day after the initial transduction (approximately 24 hours), and the same amount of LV vector in 100 µL fresh medium was added to the wells. After 3 hours of transduction, the medium was removed and the cells were fed with 100 µL fresh medium for further culture. Ninety-six hours post-transduction, the cells were harvested for transduction efficiency analysis by FACS analysis of GFP signal on a BD LSRII and analyzed using FlowJo software (v.10). Table 18 provides the LV transduction efficiencies under the different conditions.

**Table 18: Effect of MOI and Super-Transduction on LV Transduction of Fibroblast Cells (% GFP Positive Cells)**

| **MOI (vp/cell)** | **Without Super-Transduction** | **With Super-Transduction** |
|---|---|---|
| 0 | 1.6% | 2.0% |
| 200 | 4.3% | 5.8% |
| 500 | 7.7% | 10.0% |
| 1000 | 15.3% | 16.3% |
| 2000 | 22.6% | 27.5% |

An increasingly higher MOI resulted in higher transduction efficiencies with and without super-transduction. Super-transduction resulted in an incremental increase in LV transduction efficiency. However, the increase was not considered significant, and was not implemented in the GMP production setting.

### C. LV Transduction Culture Volume and FBS Concentration

Lentiviral vector particles first need to make contact with fibroblast cells in culture in order to achieve transduction. Like other viral particles, LV particles in solution follow Brownian motion, and a productive transduction is generally a random event. Reduction of culture medium depth to a minimum volume or the use of spino-transduction has been shown to improve viral vector transduction on target cells (Nyberg-Hoffman 1997). The effect of transduction volume/culture depth and medium FBS concentration on LV transduction of fibroblast cells were evaluated.

A set of non tissue culture treated 96-well plates was pre-coated with RetroNectin^{™} (20µg/cm²) before being used for transduction. At the time of transduction, 3000 cells with LV vector in a volume of 100 µL or 50 µL of medium with 10% FBS were added to the wells. The same transduction conditions were repeated in medium with 2% FBS. After an overnight incubation, the medium was removed and the cells were all fed with 100 µL fresh medium with 10% FBS for further culture. The MOI used was 2000 vp/cell for all conditions. Ninety-six hours post-transduction, the cells were harvested for transduction efficiency analysis by FACS analysis of GFP signal on a BD LSRII and analyzed using FlowJo software. Table 19 provides the LV transduction efficiencies under the different conditions.

**Table 19: Effect of Culture Volume (Depth) and FBS on LV Transduction (% GFP Positive Cells)**

| **Transduction Medium Volume in 96-well plate (µL)** | **Transduction Medium Depth (mm)** | **Transduction Medium FBS %** | |
|---|---|---|---|
| | | **2%** | **10%** |
| 50 | 1.6 | 46.7% | 46.2% |
| 100 | 3.1 | 31.8% | 27% |

As expected, reducing the transduction medium volume (depth) resulted in a noticeable increase in LV transduction efficiency. Additionally, results indicate that using a 2% FBS transduction medium is beneficial for LV transduction. Based on these results, a 2% FBS transduction medium was incorporated into the GMP manufacturing process, while keeping the transduction medium volume to a minimum, approximately 60 mL in 1-CS. In the GMP manufacturing process for FCX-007, a 1-layer CellSTACK^{®} with a surface area of 636 cm² will be used for LV transduction of fibroblast cells. It was determined that it is feasible to use a transduction volume of 60 mL (depth 0.9mm) without causing detrimental effect (e.g. drying) on the cells during the 3 hours transduction period.

### D. Evaluations of RetroNectin^{™} Coating and the Type of Culture Surface

RetroNectin^{™} coating of the culture surface significantly increased the LV transduction of fibroblast cells. The RetroNectin^{™} manufacturer recommends a RetroNectin^{™} coating amount in the range of 4 µg/cm² to 20 µg/cm². To minimize the amount of RetroNectin^{™} used without negatively impacting the LV transduction of fibroblast cells, the amount of RetroNectin^{™} used for coating was evaluated.

Furthermore, the RetroNectin^{™} manufacturer recommends use of a non tissue culture treated plastic surface for RetroNectin^{™} coating. However, culture flasks and CellSTACKs^{®} used for fibroblast cell culture are all tissue culture-treated. Both tissue culture treated and non tissue culture treated 96-well plates were coated with different amounts of RetroNectin^{™} and evaluated for LV transduction of fibroblast cells.

Both non tissue culture treated and tissue culture treated 96-well plates were pre-coated with different amounts of RetroNectin^{™} (20 µg/cm², 10 µg/cm², and 5 µg/cm² ) before being used for transduction. At the time of transduction, 3000 cells with LV vector in a volume of 50 µL of transduction medium with 2% FBS were added to each well. After 3 hours transduction, the medium was removed and the cells were all fed with 100 µL fresh medium with 10% FBS for further culture. The MOI used was 2000 vp/cell for all conditions. Ninety-six hours post-transduction, the cells were harvested for transduction efficiency analysis by FACS analysis of GFP signal on a BD LSRII and analyzed using FlowJo software. Table 20 provides the LV transduction efficiencies under the different conditions.

**Table 20: Effect of RetroNectin^{™} Amount and Culture Surface Type on LV Transduction of Fibroblast Cells (% GFP Positive Cells)**

| **Type of Culture Surface** | **Amount of RetroNectin^{™} Used for Coating (µg/cm²)** | | |
|---|---|---|---|
| | **5** | **10** | **20** |
| Tissue culture treated | 27.8% | 25.8% | 28.1% |
| Non tissue culture treated | 38.2% | 38.8% | 38.5% |

Comparable efficiencies of LV transduction of fibroblast were observed for all three doses of RetroNectin^{™} used for coating. As a result, a RetroNectin^{™} coating density of 5 µg/cm² was selected for use in the FCX-007 production process. Although lower LV transduction efficiency was observed in the tissue culture treated surface, the difference is not considered significant enough to prevent the use of tissue culture treated flasks in the INXN-2002 transduction step of the FCX-007 manufacturing process.

### Summary of the Development and Optimization of Fibroblast LV Transduction

Based on the study results described above using the LV-GFP model vector for fibroblast cell transduction, the following LV transduction protocol was selected for INXN-2002 transduction of fibroblast cells derived from RDEB donor:

Pre-coat the culture surface with RetroNectin^{™} at 5 µg/cm². Tissue culture treated flasks can be used for RetroNectin^{™} coating.

Add cells and LV vector simultaneously at the time of transduction, with a cell seeding density of approximately1x10⁴ cells/cm².

Use a high MOI which does not cause toxic effect on cells to achieve high transduction efficiency (10 mL of INXN-2002 LV-Col7 vector per transduction).

Transduce for 3 hours at 37°C in a transduction medium with 2% FBS, and then change or feed cells with a medium which contains 10% FBS.

Use a low transduction medium volume, 50 µL/well for a 96-well plate or 60 mL for a 1-layer CellSTACK^{®}, for high transduction efficiency.

Super-transduction is not required.

### F. INXN-2002 LV Transduction of Fibroblast Cells

Based on the LV transduction protocol developed above, INXN-2002 was used to transduce normal human fibroblast cells (NHDF, Lonza, CC-2511) in a 96-well plate. Considering the relative low titer of the INXN-2002, three doses of INXN-2002 were used for transduction, 12.5 µL (1:4 dilution), 3.1 µL (1:16 dilution), and 0.8 µL (1:64 dilution). The transduced cells were passaged three times to ensure stable integration of the LV-COL7 vector into the transduced cell genome. At passage 3, genomic DNA was extracted from the cells and analyzed by qPCR using a primer specific to the LV-COL7 vector sequence. The transduction efficiency was quantified as gene copy numbers per cell. Table 21 provides the transduction efficiency as measured by gene copy number per cell.

**Table 21: INXN-2002 Transduction of Fibroblast Cells**

| **INXN-2002 Vector Dosing¹** | | | **Gene Copy Number per Cell²** |
|---|---|---|---|
| **Dilution factor** | **Vector volume per well of 96-well plate (µL)** | **Calculated MOI (IU/cell)** | |
| 1/4 | 12.5 | 40 | 0.08 |
| 1/16 | 3.1 | 10 | 0.86 |
| 1/64 | 0.8 | 2.5 | 0.11 |

| | | | |
|---|---|---|---|
| ¹ INXN-2002 IU titer: 9.2 x 10⁶ IU/mL which was determined at Intrexon using an early development stage H1299 infectious titer assay. ² Gene copy number per cell was determined at Intrexon using an early development stage qPCR assay, which resulted in an approximately 10-fold higher gene copy number compared to the fully developed assay that was transferred to BioReliance for FCX-007 product analysis. The difference was due to the use of a different qPCR primer set and circular plasmid standards in the early development stage assay. | | | |

At the highest dose of INXN-2002 vector, ¼ dilution (MOI= 40), a significant toxic effect on cells were observed: most of the cells did not recover from the transduction step, resulting in a minimal gene copy number per cell at the end of the cell expansion. The optimal INXN-2002 MOI was approximately 10 IU/cell, resulting in a gene copy number of 0.86.

### INXN-2002 LV Transduction in Training Runs

Following the LV transduction protocol (Section [0260] ) and the INXN-2002 transduction vector dosing/MOI results from the 96-well studies, INXN-2002 transduction of RDEB fibroblast cells was conducted at scale in TR8, TR9, and TR10. The 1-layer CellSTACK^{®} used for INXN-2002 transduction was pre-coated with RetroNectin^{™} at 5 µg/cm². At the time of transduction, cells and INXN-2002 were added simultaneously in 60 mL of transduction medium with 2% FBS to the 1-layer CellSTACK^{®}. After 3 hours in the incubator at 37°C, the cells were fed with 130 mL of complete growth medium with 10% FBS. The transduced cells were passaged twice, first to one 10-layer CellSTACK^{®}, and then to two or six 10-layer CellSTACKs^{®} for cell harvest. Gene copy numbers in the harvested cells were analyzed by qPCR. Table 22 provides the INXN-2002 transduction results from the executed Training Runs.

**Table 22: Summary of LV-COL7 Transduction in Training Runs**

| **Training Run** | **LV-COL7 Vector** | **LV-COL7 Vector Titer (IU/mL)** | **Arm** | **Amount of Vector Used for Transduction (mL) (dilution factor)** | **Number of Cells for Transduction** | **MOI¹ (IU/cell)** | **Gene Copy per Cell²** |
|---|---|---|---|---|---|---|---|
| 8 | INXN-2002 LV-COL7 (Pilot) | 9.2x10⁶ | A | 3.75 (1/16) | 1.0 x 10⁷ | 3.4 | 0.021 |
| | | | B | 1.88 (1/32) | 1.0 x 10⁷ | 1.7 | 0.017 |
| | | | Control | 0 | 1.4 x 10⁷ | 0 | BLQ₁ |
| 9 | LV-HA-COL7 (Pilot) | 2.0x10⁶ | A | 3.75 (1/16) | 7.1 x 10⁶ | 1.0 | 0.009 |
| | | | B | 7.5 (1/8) | 7.4 x 10⁶ | 1.9 | 0.021 |
| | | | Control | 0 | 1.0 x 10⁷ | 0 | BLQ³ |
| 10 | INXN-2002 LV-COL7 (GMP) | 2.3x10⁶ | TR10 | 4 (1/15) | 4.6 x 10⁶ | 2.0 | 0.013 |

| | | | | | | | |
|---|---|---|---|---|---|---|---|
| 1 MOI was determined based on the INXN-2002 LV-Col7 vector IU titer determined at Intrexon using an early development stage H1299 infectious titer assay, which resulted in approximately 10-fold higher titer value compared to the fully developed assay that was transferred to BioReliance for FCX-007 product analysis. The difference was due to the use of a different qPCR primer set and circular plasmid standards in the early development stage assay. ² Gene copy per cell was quantified using the fully developed qPCR assay, which was transferred to BioReliance for FCX-007 product analysis ³ BLQ: Below the limit of quantitation | | | | | | | |

Following the LV transduction protocol developed using the small scale 96-well plates, successful INXN-2002 transduction of RDEB fibroblast cells was achieved at scale for FCX-007 manufacture. Gene copy numbers in the transduced cells increased with an increased MOI of transduction. Because of the low titer of INXN-2002, gene copy numbers in the transduced, harvested cell product were relatively low. In order to produce FCX-007 cell product with a feasibly high gene copy number, 10 mL of INXN-2002 was selected for GMP production transduction, as it caused minimal toxic effects on cells during transduction.

### Engineering Run

In preparation for GMP manufacturing of FCX-007, and finalization of the production Master Batch Records (MBR), an engineering run was executed using a biopsy from an RDEB donor with approved production batch records. The GMP grade INXN-2002 LV vector was used for transduction. Table 23 provides cell growth figures from the Engineering Run.

**Table 23: Cell Growth and Cell Yields in Engineering Run**

| **Activity** | **Days in Culture** | **Viable Cells** | **Cell Viability** | **Note** |
|---|---|---|---|---|
| Biopsy digestion, seed into one T-75 flask, with 35mL culture medium, 37°C and 5% CO₂ | 0 | 9.6 x 10⁵ | 92% | Good cell growth in the T-75 flask, reached approximately 90% confluence on Day 19 |
| Passage 1: to one T-175 flask and one T-25 flask, 37°C and 10% CO₂ | 20 | 3.5 x 10⁶ | 99% | T-175 Flask: LV transduction |
| | | | | T-25 Flask: control cells for analysis |
| Passage 2: to 1-CS and LV transduction | 29 | 9.9 x 10⁶ | 98% | Cells from T-175 Flask were harvested and expanded into 1-CS for LV-COL7 transduction (GMP grade INXN-2002) |
| Passage 3: to 10-CS | 38 | 3.3 x 10⁷ | 98% | Cells were harvested and expanded to one 10-CS. |
| Passage 4: to 6x 10-CS | 49 | 3.1 x 10⁸ | 96% | Cells were harvested and expanded to six 10-CS. |
| Harvest: 6x 10-CS | 60 | 9.6 x 10⁸ | 97% | Cells were harvested from the 6x10-CS for cryopreservation and testing |

Cell growth and yields from the ER are comparable to those in TR8 and TR9, and are noticeably faster and higher relative to TR10, indicating cell growth variability among different RDEB donors. The harvested cells were filled and cryopreserved as shown in Table 24.

**Table 24: FCX-007 Drug Substance Vials Filled in Engineering Run**

| **Vials** | **Fill Volume (mL)** | **Number of Vials Filled** | **Purpose** |
|---|---|---|---|
| 2 mL cryovial | 1.2 | 10 | Bulk Drug Substance |
| 5 mL cryovial | 4.5 | 6 | Bulk Drug Substance |
| 2 mL cryovial | 1.2 | 2 | Sterility test |
| 2 mL cryovial | 0.6 | 4 | QC test |
| 2 mL cryovial | 0.6 | 6 | Bulk Drug Substance Stability Test |

The total number of cells harvested from the 6x 10-CS is considered adequate for production of two doses of FCX-007 drug product for injection.

The harvested cells were tested according to the proposed FCX-007 Drug Substance Specifications. Table 25 provides the analysis results for the harvested cells.

**Table 25: FCX-007 Drug Substance Engineering Run Analysis**

| **Test¹** | **Test Method** | **Lab** | **SOP** | **Specifications** | **Test Result** |
|---|---|---|---|---|---|
| Mycoplasma | USP<63> | BioReliance | 102063GMP.BSV | Negative | Negative |
| Replication Competent Lentivirus | C8166 cell | BioReliance | 009130GMP.BUK | No RCL detected | ND⁴ |
| Sterility | USP<71> | BioReliance | 510120GMP.BSV | No Growth | No growth |
| Endotoxin | Endosafe | PCT | SOP-0249 | ≤ 5.0 EU/mL | < 2.00 EU/mL |
| Cell Count | Hemacytometer | PCT | SOP-0329 | 1.0 - 3.0x10⁷ cells/mL | 1.6x10⁷ cells/mL |
| Cell Viability | Hemacytometer | PCT | SOP-0329 | ≥ 85% viability | 94% |
| Purity | FACS Calibur | PCT | SOP-1067 | ≥ 98% CD 90⁺ | 100% |
| Residual VSV-G Protein | VSV-G ELISA | Intrexon' | VSV-G ELISA | Report Result | BLQ |
| COL7A Gene Copy Number | qPCR | Intrexon' | COL7A Gene Copy Number | Report Result | 0.025 copies/cell |
| COL7A Gene Expression | Col 7 ELISA | Intrexon³ | COL7A Gene Expression | Report Results | 66.4 ng/mL |

| | | | | | |
|---|---|---|---|---|---|
| ¹ Details of the assays are provided in Section 3.2.S.4.2. ² Intrexon in house assay. Assays are being transferred to BioReliance for GMP release test ³ Intrexon in house assay. Assays are being transferred to PCT for GMP release test ⁴ RCL testing was not performed on the cell product material as the material was not used for further studies | | | | | |

### Example 5 - Description of Manufacturing Process Development to Improve LV-Col7 Transduction Efficiency

FCX-007 cell products manufactured using the process described above had low levels of gene modification efficiency as indicated by the low gene copy number and C7 expression levels when INXN-2002 vector was used for cell transduction. Two approaches were pursued to increase the LV-Col7 transduction efficiency on RDEB fibroblast cells: 1) development of a new LV-Col7 lentiviral vector (INXN-2004) which offers better vector titer and improved stable transgene (collagen VII) expression in the transduced fibroblast cells, and 2) further optimization of the LV-Col7 transduction process on fibroblast cells. Details of the development of the new INXN-2004 vector are described in Section 3.2.S.2.3. Studies in further optimization of the LV-Col7 transduction process, as well as cell expansion procedures are described below.

### Spin Transduction (Spinoculation)

Spin transduction involves centrifugation of viral vectors onto the target cells under a g force environment. Spin transduction (spinoculation) has been reported in the literature to increase retroviral vector transduction efficiency on target cells (J Virol Methods. 1995 Aug;54(2-3):131-43. Centrifugal enhancement of retroviral mediated gene transfer. Bahnson AB1, Dunigan JT, Baysal BE, Mohney T, Atchison RW, Nimgaonkar MT, Ball ED, Barranger JA. and Hum Gene Ther. 1994 Jan;5(1):19-28. Improved methods of retroviral vector transduction and production for gene therapy. Kotani H1, Newton PB 3rd, Zhang S, Chiang YL, Otto E, Weaver L, Blaese RM, Anderson WF, McGarrity GJ.).

RDEB fibroblast cells from the previous TR8 were cultured. Cells in exponential growth phase were harvested and used for this study. The previous INXN-2002 vector lots were used for transduction. For spin transduction, 1x10³ cells with different MOIs of INXN-2002 in 50µL of transduction media (IMDM + 2%FBS) were added into each well of a 96-well tissue culture coated plates which were pre-coated with RetroNectin (5 µg/cm²). The plates containing cells plus INXN-2002 vector were centrifuged at 1300g, 4°C for 1.5 hours. After centrifugation, the plates were placed into a 37°C incubator to allow the cells to recover and grow for 3 hours. After which, 100 µl of complete culture media (IMDM +10FBS+GSH) was added to each well. The plates were returned to the incubator for further culture. The standard non-spin transduction described above was used as a control. After transduction, cells were passaged three times, 3-5 days culture between each cell passage. At the last passage culture supernatants were harvested to evaluate C7 protein expression levels, while the cells were harvested for gene copy number per cell analysis. Table 26 shows the Col7 expression levels for the different transduction conditions.

**Table 26: C7 Expression Level**

| **INXN-2002** | **MOI (IU/cell)** | **Vector Dilution used for Transduction** | **Transduction Method** | |
|---|---|---|---|---|
| | | | **Spin Transduction** | **Non-spin transduction** |
| GMP lot | 6.31 | | 257.4±62.17 | 311.75±70.89 |
| | 1.60 | | 539.50±70.49 | 251.27±22.41 |
| | 0.4 | | 243.94±3.40 | 207.51±25.26 |
| Pilot lot | 30 | | 327.76±21.69 | 238.56±27.93 |
| | 15 | | 391.59±42.82 | 208.39±16.80 |
| | 7.5 | | 269.07±25.51 | 214.23±16.83 |

Table 27 provides the vector copy number per cells of the harvested cells.

**Table 27: Vector Copy Number per Cell**

| **INXN-2002** | **MOI (IU/cell)** | **Vector dilution used for transduction** | **Transduction Method** | |
|---|---|---|---|---|
| | | | **Spin Transduction** | **Non-spin transduction** |
| GMP lot | 6.31 | | 0.11±0.02 | 0.10±0.02 |
| | 1.60 | | 0.11±0.01 | 0.06±0.00 |
| | 0.4 | | 0.10±0.03 | 0.04±0.01 |
| Pilot lot | 30 | | 0.09±0.01 | 0.06±0.02 |
| | 15 | | 0.09±0.02 | 0.05±0.01 |
| | 7.5 | | 0.05±0.03 | 0.07±0.02 |

| | | | | |
|---|---|---|---|---|
| INXN-2002, IU titer: 2.36 x 10⁶ IU/mL (determined at Intrexon ) INXN-2002, IU titer: 2.36 x 10⁶ IU/mL (determined at Intrexon ) | | | | |

The results show a modest increase in both Col7 production levels and vector copy number per cell using the spin transduction method, especially at the lower MOIs and at the INXN-2002 vector dilutions tested.

### Optimization of Spin Transduction Parameters

The spin transduction parameters, including centrifugation time, speed and temperature, were evaluated with an aim to further increase the INXN-2002 transduction efficiency. The same cell culture condition as described above was used in this study. In the initial experiment, centrifugation temperature was examined. 96-well plates containing cells plus INXN-2002 vector were centrifuged at either 4°C or 25°C (RT) for 1.5 hours at 1300g. This was followed by another experiment where 96-well plates containing cells plus INXN-2002 vector were centrifuged at 4°C or 25°C (RT) for 1 or 2 hours, at either a high speed of 1300g, or a low speed of 300g, to examine the effect of centrifugation speed on INXN-2002 transduction efficiency. In both experiments, cells were returned to a 37°C incubator for incubation post centrifugation. Three hours later, 100 µl of complete culture media was added to each well. Cells were passaged three times before being harvested for Col7 expression analysis in the culture supernatant and vector copy number per cell analysis in the harvested cells.

Table 28 provides the effect of spin transduction temperature on Col7 expression levels and vector copy number per cell. The standard non-spin transduction was included as a control.

Again, increased INXN-2002 transduction, as indicated by higher C7 protein expression and vector copy number per cell, was demonstrated using spin transduction compared to the standard no spin transduction. Spin transduction temperature did not demonstrate impact on the overall transduction efficiency.

Table 29 below provides the effect of centrifugation speed as well as temperature and time on INXN-2002 transduction efficiency.

**Table 28: The Effect of Spin Transduction Temperature on INXN-2002 Transduction Efficiency**

| **INXN-2002 MOI** | **C7 Expression (ng C7/day/E6 cells)** | | | **Vector Copy Number per Cell** | | |
|---|---|---|---|---|---|---|
| | **No spin** | **Spin Transduction** | | **No Spin** | **Spin Transduction** | |
| | | **4°C** | **RT** | | **4°C** | **RT** |
| 18 | 105.84±11.69 | 177.36±24.98 | 313.59±39.13 | 0.07±0.04 | 0.07±0.02 | 0.08±0.03 |
| 6 | 117.82±14.79 | 196.26±23.75 | 143.61±19.01 | 0.02±0.01 | 0.08±0.03 | 0.05±0.00 |
| 2 | 50.05±13.60 | 136.55±6.82 | 105.57±17.90 | 0.01±0.00 | 0.02±0.00 | 0.03±0.02 |

| | | | | | | |
|---|---|---|---|---|---|---|
| INXN-2002 IU titer: 2.36 x 10⁶ IU/mL (determined at Intrexon ) | | | | | | |

**Table 29: Effect of Spin Transduction Temperature, Time, and Speed on INXN-2002 Transduction Efficiency**

| **INXN-2002 MOI** | **C7 Expression (ng C7/day/E6 cells)** | | | | | | | | **Vector Copy Number per Cell** | | | | | | | |
|---|---|---|---|---|---|---|---|---|---|---|---|---|---|---|---|---|
| Temp | 4°C | | | | RT | | | | 4°C | | | | RT | | | |
| Speed (xg) | 1300 | | 300 | | 1300 | | 300 | | 1300 | | 300 | | 1300 | | 300 | |
| Time ( hr) | 1 | 2 | 1 | 2 | 1 | 2 | 1 | 2 | 1 | 2 | 1 | 2 | 1 | 2 | 1 | 2 |
| 3.8 | 208.88 ±13.61 | 247.0 3±10. 09 | 221. 49±4 .08 | 310. 93±0 .41 | 359.4 7±3.8 5 | 201.0 6±5.3 7 | 168. 98t3 34 | 201. 82±9 .41 | 0.08 ±0. 01 | 0.07 ±0. 01 | 0.06 ±0. 01 | 0.09 ±0. 03 | 0.11 ±0. 01 | 0.06 ±0. 01 | 0.07 ±0. 01 | 0.07 ±0. 02 |
| 1.9 | 180.34 ±12.33 | 265.4 4±1.3 3 | 213. 83±0 .58 | 301. 82±1 1.54 | 240.6 1±2.6 8 | 180.3 6±3.1 0 | 212. 76±3 .19 | 177. 16±5 .92 | 0.06 ±0. 02 | 0.10 ±0. 02 | 0.05 ±0. 00 | 0.06 ±0. 01 | 0.10 ±0. 02 | 0.07 ±0. 02 | 0.06 ±0. 01 | 0.05 ±0. 01 |

| | | | | | | | | | | | | | | | | |
|---|---|---|---|---|---|---|---|---|---|---|---|---|---|---|---|---|
| INXN-2002 IU titer: 2.36 x 10⁶ IU/mL (determined at Intrexon ) | | | | | | | | | | | | | | | | |

The results indicate a modest improvement in INXN-2002 transduction efficiency, as indicated by C7 protein expression and vector copy number, when spin transduction was conduced at RT, 1300g, for 1 hour. The significance of the improvement is not known and the initially developed spin transduction parameters (centrifugation at 1300g for 1.5 hours at 4°C) were selected for future study use.

### Super Transduction to Further Increase INXN-2002 Transduction Efficiency

Super transduction (a second transduction) was evaluated in the early development of INXN-2002 transduction, which is described above. Super transduction was evaluated again in combination with spin transduction to further increase INXN-2002 transduction efficiency on fibroblast cells. Unlike the previous method, in the current study's super transduction was performed on cells one passage after the initial spin transduction. The inclusion of the one cell passage is expected to allow the cells to recover from the initial spin transduction and become more receptive to the 2^{nd} super transduction.

Similarly, RDEB fibroblast cells from the previous TR8 in exponential growth phase were harvested and used for this study. The optimized spin transduction method described above, centrifugation at 1300g for 1.5 hours at 4°C, was used for INXN-2002 transduction. The transduced cells were passaged once at 72-96 hours post the initial spin transduction. After 72-96 hours of culture in the second passage, cells were harvested and re-transduced using the same spin transduction conditions. After the super spin transduction, cells were passaged an additional three times before being harvested for Col7 expression analysis in the culture supernatant, and vector copy number per cell analysis in the harvested cells. The INXN-2002 vector was used for the study. Table 30 provides the effect of super spin transduction on INXN-2002 transduction efficiency.

**Table 30: Effect of Super Spin Transduction**

| **INXN-2002 MOI** | **C7 Protein Expression** | | **Vector Copy Number per Cell** | |
|---|---|---|---|---|
| | **One Round of Spin Transduction** | **Super Spin Transduction** | **One Round of Spin Transduction** | **Super Spin Transduction** |
| 1.9 | 257.8±3.37 | 431.9±95.5 | 0.07±0.03 | 0.22±0.07 |

| | | | | |
|---|---|---|---|---|
| INXN-2002 IU titer: 2.36 x 10⁶ IU/mL (determined at Intrexon ) | | | | |

Super spin transduction resulted in a significant increase in INXN-2002 transduction efficiency on RDEB fibroblast cells. C7 protein expression increased approximately 1.7-fold and the vector copy number per cell increased approximately 3-fold.

The data demonstrate a 40% increase in Col7 expression and a 60% increase in copy number per cell with the additional transduction. Due to this increase, super-infection was added to the production protocol.

### Transduction with the INXN-2004 Vector

Concurrent with the development of the improved transduction method, a new LV-Col7 vector (referenced as "INXN-2004") based on a different lentiviral vector backbone (that is, pFUGW) was also developed.

### INXN-2004 Single Spin Transduction

RDEB fibroblast cells from the previous TR8 in exponential growth phase were harvested and used for this study. In the first study, single round of spin transduction as described above was carried out using a pilot lot of INXN-2004 vector. The standard no-spin transduction was included in the study as a control. Cells were passaged three times before being harvested for Col7 expression analysis in the culture supernatant, and vector copy number per cell analysis in the harvested cells. Table 31 provides the Col7 expression levels and vector copy number per cell using the INXN-2004 vector transduction.

**Table 31: C7 Protein Expression and Vector Copy Number per Cell using INXN-2004 Transduction**

| **INXN-2004 MOI** | **C7 Protein Expression** | | **Vector Copy Number per Cell** | |
|---|---|---|---|---|
| | **No Spin Transduction** | **Spin Transduction** | **No Spin Transduction** | **Spin Transduction** |
| 17 | 2312.46±0.03 | 3960.58±0.02 | 0.16±0.03 | 0.05±0.02 |
| 4.25 | 1668.46±0.06 | 3602.10±0.06 | 0.21±0.06 | 0.27±0.06 |
| 1.06 | 724.12±0.03 | 2566.88±0.10 | 0.06±0.03 | 0.21±0.10 |
| 0.27 | 168.61±0.00 | 1936.72±0.00 | 0.02±0.00 | 0.13±0.00 |

| | | | | |
|---|---|---|---|---|
| INXN-2004 IU titer: 2.36 x 10⁶ IU/mL (determined at Intrexon ) | | | | |

Relative to the INXN-2002 vector, a significant improvement in transduction efficiency was achieved with use of INXN-2004 in combination with the spin transduction method. Additionally, the INXN-2004 vector appears to be significantly more active in transducing RDEB fibroblast cells even under a no-spin transduction condition, as indicated by the approximately 10-fold higher C7 protein expression and vector copy number per cell. The results support the use of the pFUGW base for construction of the new INXN-2004 vector.

Consistent to what is reported above, spin transduction appears to exacerbate the INXN-2004 vector toxic effect on fibroblast cells at higher MOI conditions, as indicated by the lower C7 expression levels and vector copy numbers at MOI17. This does not impact FCX-007 manufacturing as the use of a lower MOI is demonstrated to be as effective.

### INXN-2004 Super Spin Transduction

In the second study, super spin transduction (as described above) was used for INXN-2004 transduction. The scale of transduction was increased from a 96-well plate to a T-25 flask. The transduction parameters remained unchanged, including the same RetroNectin^{™} coating density of 5µg/cm2, and the same cell density at transduction of 1x10⁴ cells/cm². Transduction volume in the T-25 flask was 4.2 mL. For transduction, the T-25 flask containing the cells and virus was centrifuged at 4°C, for 1.5 hours at 1300g. Cells were returned to a 37°C incubator and incubated for three hours. After the initial incubation, 4.2 mL of complete culture media was added to the flask for further incubation. Cells were passaged three times before being harvested for Col7 expression analysis in the culture supernatant, and vector copy number per cell analysis in the harvested cells. Table 32 provides the effect of super spin transduction on INXN-2004 vector transduction efficiency. A pilot lot of INXN-2004 vector was used for the study.

**Table 32: Effect of Super Spin Transduction for the INXN-2004 Vector**

| **INXN-2004 MOI** | **C7 Protein Expression** | | **Vector Copy Number per Cell** | |
|---|---|---|---|---|
| | **Single Spin Transduction** | **Super Spin Transduction** | **Single Spin Transduction** | **Super Spin Transduction** |
| 0.11 | 3178±20.53 | 4029±59.67 | 0.12±0.007 | 0.57±0.134 |

| | | | | |
|---|---|---|---|---|
| INXN-2004 IU titer: 2.36 x 10⁶ IU/mL (determined at Intrexon ) | | | | |

As observed for the previous INXN-2002 vector, super spin transduction resulted in a significant increase, approximately 5-fold, in vector copy number per cell in the transduction cells, despite a relatively modest increase in Col7 protein expression level, under a low MOI transduction condition.

### Summary of Transduction Development and Optimization

Significant improvements in LV-Col7 transduction efficiency on RDEB fibroblast cells was achieved in continued process development, both with the previous INXN-2002 and the new INXN-2004 vector. The improvements are to enable the manufacturing of a biologically potent FCX-007 cell product. Based on the manufacturing process development study results, the following transduction procedure was chosen for manufacturing of FCX-007 using INXN-2004 vector for transduction.

Pre-coat culture surface with RetroNectin at 5µg/cm². Tissue culture-treated flasks are used for RetroNectin^{™} coating.

Add cells and INXN-2004 vector simultaneously at the time of transduction. Cell seeding density is approximately 1 x 10⁴ cells/cm².

Centrifuge cells and INXN-2004 vector transduction mixture in the transduction vessel at 4°C, for 1.5 hours at 1300g.

Incubate cells for 1.5- 2.0 hours at 37°C post transduction in transduction medium with 2%FBS, and then change or feed cells with complete culture medium with 10%FBS.

Use a low transduction medium volume (50µL/well for a 96-well plate, or 0.35 mL/cm²).

Perform a super-transduction on cells after one passage post the initial transduction.

### Example 6 - Analysis of RDEB Genetically Modified Human Dermal Fibroblasts Transduced by INXN-2002

RDEB Genetically Modified Human Dermal Fibroblasts (GM-HDF) derived from INXN-2002 lentiviral vector was analyzed in order to:

Evaluate localization of COL7 expression in established composite human skin grafts (prepared on porcine dermis) comprised of RDEB keratinocytes by ID injection.

### Assess potential carcinogenicity/tumorgenicity in composite RDEB human skin grafted (prepared on porcine dermis) on SCID mice treated with RDEB GM-HDF

An initial attempt to evaluate GM-HDF (derived from INXN-2002) function by seeding fibroblasts on the reticular side of composite graft cultures was unsuccessful, possibly due to inadequate migration of GM-HDF into the devitalized porcine dermis or insufficient time for adequate diffusion and deposition of rC7 at Day 19. An alternative approach was used to evaluate function by direct ID injection of existing grafts in six back-up animals on study. RDEB composite grafts prepared on porcine dermis appear to be more robust than anticipated, making direct injection into established RDEB skin grafts possible. This approach more closely mimics the disease and clinical route of administration.

This study report presents data from an initial evaluation of ID injection of GM-HDF into established RDEB grafts. Additional studies to achieve long term objectives for in vivo pharmacology are planned.

### A. STUDY MATERIALS

### A.1 Test Article(s)

Test article was prepared and tested for Drug Substance release specifications prior to shipment. The test article for skin graft injection was prepared one day prior to each scheduled day of treatment. GM-HDF was thawed, washed, resuspended in DMEM, and evaluated against release specifications. The GM-HDF was shipped for overnight delivery and administered within 48 hours. Non-corrected RDEB HDF (mock transduced) was isolated and expanded from the identical patient as the GM-HDF by the same methods.

The test article and cells to be used for preparation of composite skin grafts was shipped frozen. Cells used for skin graft preparation will be thawed and cultured for testing.

Lot number TR8 (Non-Transduced Control) was used as the RDEB-HDF negative control. Lot number TR8 Arm A (GM-HDF-LV-COL7) was used as the GM-HDF test article.

Test article analysis was performed at the manufacturing facility prior to shipment. Remaining test article will be stored at -70°C for 1 year. The test article will be stored in Dulbecco's Modified Eagle Medium (DMEM).

### A.2. Other Chemicals and Materials

A list of other materials is provided below in Table 33.

**Table 33: Other Study Materials**

| **Description** | **Source** |
|---|---|
| Normal Porcine Skin | Porcine cadaver |
| RDEB keratinocytes | Human skin biopsy |
| RDEB fibroblasts | Human skin biopsy |
| Normal keratinocytes | Human skin biopsy |
| Normal fibroblasts | Human skin biopsy |

Group 1 (Negative Control) Cells: Untransduced RDEB cells from the donor from training run 8 were thawed, and passaged once prior to transfection. Two days after passaging the cells, the cells were collected and 1.2 x 10⁶ cells were transfected in a 50ml conical tube in low serum growth (ATCC PCS-201-030 and PCS-201-041) media using the lipid based transfection agent Transfex (ATCC) with COL7 plasmid VVN 4317513. After a 10 minutes incubation with complexed Transfex, DNA and Optimem (LifeTech), 1 x 10⁶ transfected cells were transferred to a T150 flask, and 2 x 10⁵ cells were placed in a T25 and placed in a 37°C 5% CO2 incubator for 16 hours, at which time the media was removed and replace with full growth media (IMDM (Sigma), 920mg/L of L-Glutathione Reduced (Sigma) 10% heat inactivated FBS (Atlantic Biologicals) and 1XGlutamax (LifeTech)). Cells were then returned to the 37°C 5%CO2 incubator until time of shipment. At time of shipment all cell flask was filled to capacity, such that all parts of the flask were in contact with media regardless of the flask orientation.

### B. STUDY DESIGN

### B.1. Randomization

Due to high failure rates of RDEB human skin grafts, mice in these groups of animals are not randomized.

### B.2. Justification for Species and Number on Study

The NOD. CB.17-PRKDC scid/scid mouse is the standard species for use in proof of concept preclinical efficacy/toxicology studies and is a rodent species of choice for such evaluations using the human xenograft models. This study was designed to minimize the number of animals on study that would provide sufficient data to evaluate the test articles in.

### B.3. Route of Administration

Fibroblasts will be administered by intradermal injection for normal skin grafts. Composite grafts will be administered cells by seeding prior to grafting.

### B.4. Dose Administration

Intradermal cell injections of lentivirus transduced (COL7A1-corrected) RDEB GM-HDF (using INXN-2002) and negative controls (vehicle or RDEB-HDF) will be done with a 30-gauge needle. The injection will be performed by first piercing the skin, then directing the needle as superficially as possible back upward toward the surface.

Composite grafts will be administered cells by seeding prior to grafting according to protocol outlined in the in vitro study with the distinction that the composite culture system will not be lifted to air-fluid interface prior to grafting.

### B.4.1. Preparation of Fibroblasts for Injection (Composite Grafts)

Fibroblasts were removed from the liquid nitrogen storage at one week prior to injection, thawed in 37°C water bath until a small ice crystal remains, then disinfected and transferred to BSC. Vials were resuspended in 10ml of PBS wash volume and spun for 10 minutes at 1000 RPM. The supernatant was discarded and the cells were washed in DMEM+10% FBS 1x Antibiotic media and re-spun for 10minutes at 1000RPM. The pellet was resuspended in appropriate volume and plated on 15cm TC dishes. Media was changed every other day and passaged at 80% confluence until grafts are ready to inject. On the day of injection, fibroblasts were trypsinized, neutralized in DMEM+10% FBS media and spun 10min at 10000 RPM, the pellet of cells was resuspended and an aliquot of cells was taken, cells were counted with trypan blue stain to determine viability. The cells were resuspended such that 1.0 x 10⁶ cells in 50uL volume is drawn up into syringe. A 30g needle was attached and placed at 4°C until mouse was ready tobe injected.

**Table 34: Composite Skin Grafts (Fibroblast Seeding)**

| **Grp** | **N** | **Sex** | **Composite Skin Graft** | | **Number of Cells** | | **Collection of Specimens** |
|---|---|---|---|---|---|---|---|
| | | | **Keratinocytes** | **Fibroblasts** | **Keratinocytes** | **Fibroblasts** | |
| 1 | 3 | M | WT | WT | 1 x 10⁶ | 1 x 10⁶ | Day 15 |
| 2 | 3 | M | RDEB Keratinocytes | RDEB HDF | 1 x 10⁶ | 1 x 10⁶ | Day 15 |
| 3 | 3 | M | RDEB Keratinocytes | RDEB-GM-HDF | 1 x 10⁶ | 1 x 10⁶ | Day 15 |
| 4 | 3 | M | RDEB Keratinocytes | RDEB - GM-HDF | 1 x 10⁶ | 5 x 10⁵ | Day 15 |
| 5 | 3 | M | RDEB Keratinocytes | RDEB - GM-HDF | 1 x 10⁶ | 2.5 x 10⁵ | Day 15 |
| 6 | 3 | M | RDEB Keratinocytes | RDEB - GM-HDF | 1 x 10⁶ | 1.25 x 10⁵ | Day 15 |

**Table 35: Composite Skin Grafts (Fibroblast Intradermal Injections)**

| **Grp** | **N** | **Sex** | **Test Article** | **Dose** | **Injection** | **Graft Description** |
|---|---|---|---|---|---|---|
| 1 | 2 | M | RDEB-HDF | 1 x 10⁶ | Composite Graft | 1 million RDEB GM-HDF, 1 million RDEB KC |
| 2 | 4 | M | RDEB-GM-HDF | 1 x 10⁶ | Composite Graft | 0.125 million RDEB GM-HDF, 1 million RDEB KC |

**Table 36: Study Groups for the Evaluation of ID-Injected Xenografts**

| **Condition** | **Graft Pre-Seeding Prep Conditions** | | **Injected Fibroblasts** | **Date of Harvest** |
|---|---|---|---|---|
| **Control Groups** | **Keratinocytes** | **Fibroblasts** | | |
| Normal | Normal KC | Normal FB | N/A | Day 19 post-grafting |
| FDEB | RDEB | RDEB | N/A | Day 19 post-grafting |

| **Experimental Groups** | | | | |
|---|---|---|---|---|
| 1A | RDEB | TR8/A 1 million | TR8 nontransduced | 10 days post-injection |
| 1B | RDEB | TR8/A 1 million | TR8 nontransduced | 10 days post-injection |
| 2A | RDEB | TR8/A 0.125 mil | TR8/ArmA | 10 days post-injection |
| 2B | RDEB | TR8/A 0.125 mil | TR8/ArmA | 10 days post-injection |
| 2C | RDEB | TR8/A 0.125 mil | TR8/ArmA | 10 days post-injection |
| 2D | RDEB | TR8/A 0.125 mil | TR8/ArmA | 10 days post-injection |

### C. Skin Grafts

### C.1 RDEB Human Skin Grafts

### C.1.1 Fibroblasts

Genetically engineered human fibroblasts (RDEB GM-HDF) produced by lentiviral gene transfer, including the INXN-2002 lentiviral vector, along with normal fibroblasts and non-corrected RDEB fibroblasts as controls, are the cell types used for initially infusing dermis skin. Cells are cultured onto devitalized dermis to form skin equivalents approximately 2 cm square. Regenerated skin composites consist of human keratinocytes cultured atop fibroblast-infused human devitalized dermis.

### Isolation and culture of primary keratinocytes

Wild-type keratinocyte cells are isolated from neonatal foreskin, whereas primary RDEB keratinocytes are isolated from patient skin samples. The latter is obtained as a 6mm punch biopsy submerged in 15 mL of keratinocyte growth medium 50/50V in a 15 mL centrifuge tube shipped on wet ice. No bubbles can be present in shipped samples. Medium 50/50V contains 50% medium 154 (Invitrogen) supplemented with HKGS (0.2% bovine pituitary extract [BPE], bovine insulin [5 mg/mL], hydrocortisone [0.18 mg/mL], bovine transferrin [5 mg/mL], human epidermal growth factor [0.2 ng/mL]) and 50% keratinocyte SFM (KSFM; Invitrogen) supplemented with recombinant human EGF1-53 BPE. Antibiotics AV100 (amikacin/vancomycin) are added to reduce bacterial contamination.

Separate dermis and epidermis by treating with 25 U/mL dispase (Becton Dickinson) overnight at 4°C. Epidermis is then peeled from dermis and placed in a 15 mL centrifuge tube with 5 mL TrypLE 10x. Epidermal "peel" is incubated in a 37°C water bath for 15-30 min with gentle agitation every 5 min. TrypLE is neutralized with equal volume DTI (defined trypsin inhibitor) or DMEM+10%FBS. Cells are pelleted at 1200 rpm and plated in 12 mL 50/50V onto T75 Corning ^{®} Purecoat^{™}collagen 1 mimetic flasks. In the event of PureCoat^{™} flask unavailability from manufacturer, 1ml of collagen 1 coating solution per 10cm plastic dish for 15 minutes at 37°C can be used instead. Collagen 1 coating is a 0.2 uM filtered solution of Vitrogen 100 Collagen (1ml), HEPES (2ml), BSA (100uL of 100mg/ml) and 1x HBSS (100ml). The excess collagen coating is aspirated prior to cell seeding. Flasks are swirled to ensure an even distribution of cells and placed in a 37°C 5% CO₂ humidified incubator for at least one night before any manipulation or observation. Cells are allowed to adapt to tissue culture conditions for 3 days. After adaptation, medium is changed once every 2 days or as needed. Keratinocytes are passaged onto normal tissue culture plastic when they are approximately 70% confluent.

In the 50/50V media keratinocytes grow well for five passages. Keratinocytes are utilized within the first four passages.

### Preparation of porcine devitalized dermis

Obtain sheets of split thickness porcine skin. This may be prepared in similar fashion to devitalized human dermis (as above) from porcine skin harvested by dermatome. Wash the porcine dermis 3x in sterile PBS containing 1x penicillin/streptomycin/Amphotericin/gentamicin and incubate in PBS with 1M NaCl and 2x antibiotics at 37°C for 72 hours. Separate epidermis and discard, wash 3x with 1XPBS containing antibiotics to remove excess salt and gentamicin in solution.

Confirm sterility by culturing a piece of porcine dermis in 50/50V or KGM for 4 days. Dermis can be stored at 4°C in antibiotic solution, changing the PBS weekly. Dermis should not be stored for more than a few months before use.

### Organotypic Culture Seeding Fibroblasts

Cut devitalized dermis into 2 cm square pieces. Place dermis basement membrane side down into 6 well culture plates and allow dermis to dry slightly.

Seed fibroblasts into each well, centrifuge 1200 rpm for 5 minutes and return to 37°C with 5% humidified CO₂ for 3-4 days, changing media every day. During this time, fibroblasts attach to the dermis and begin to migrate into the tissue.

### Seeding Keratinocytes

After the fibroblasts have migrated through the dermis, carefully detach dermis and transfer to annular dermal support (ADS). Add a thin layer of Matrigel to seal and secure dermis in device. Add 5 mL KGM to stromal compartment of ADS. KGM is a 3:1 mixture of DMEM:Ham's F12 supplemented with FBS (10%), adenine (1.8 x 10-4 M), hydrocortisone (0.4 µg/mL), insulin (5 µg/mL), cholera toxin (1 x 10-10 M), EGF (10 ng/mL), transferrin (5 µg/mL), and triiodo-L-thyronine (1.36 ng/mL). Trypsinize, count, and seed keratinocytes in a total volume of 100 µL of KGM per ADS. Return ADS assembly to incubator. Do not disturb ADS for at least 24hrs. The keratinocytes at the top of the dermis must remain at the air liquid interface. Change the KGM in the lower chamber every day. Organotypic culture will continue for 1-2 weeks.

### Grafting Organotypic Culture

Organotypic cultures will be ready to graft after 7-10 days. Grafting will be conducted as described above for normal human skin grafts. Bandages and sutures will be removed after 7-10 days and animal will be rewrapped with fresh bandaging for another week. Thereafter graft will be exposed to air to mature.

### Harvesting Grafts and Sectioning

Humanely sacrifice mice and carefully harvest graft. Bisect graft and cut an additional 1 mm slice. Trim slice down to a 1 mm square and send for immunoelectron microscopy (IEM). Freeze remaining halves in OCT on dry ice while noting the central edge. Sectioning will start at this edge. One half will be sectioned for immediate analysis and remaining half will be stored for later analysis. Cut ten 8 µm sections starting from the central edge and sequentially label. Discard the next forty sections. Repeat until end of graft is reached. Air dry and fix with ice cold 50/50 acetone/methanol.

### Injection of Established RDEB Composite Grafts

Fibroblasts will be intradermally injected directly into the skin following of the established graft. Injections will be no greater than 50 µL in volume.

Injections were performed on mice previously grafted with composite grafts as described herein: Devitalized porcine dermis was seeded by centrifugation with variable dosage of RDEB GM- HDF and grown in tissue culture for 1 week. Subsequently, the dermis was flipped over and 1 million RDEB keratinocytes were seeded and allowed to grow in culture for one more week. After this two week period in tissue culture as described above, the grafts were placed on SCID mice and sutured in place, bandaged for 13 days. The dressings were removed at that time, and at 38-41 days post grafting, grafts were injected with 50uL volume of fibroblasts.

### D. EXPERIMENTAL PROCEDURES

### D.1. Localization of COL7

Immunofluorescent microscopy was used to evaluate expression of type VII collagen. For immunofluorescence (IF) analyses of skin cultures with anti-human C7 specific antibody NP185.

### D.1.2. Methods for NP 185 Antibody Immunofluorescence Microscopy

Composite grafts were harvested from SCID mice immediately after CO₂ euthanasia was performed. The grafts were bisected and placed in OCT blocks. Frozen blocks of tissue were sectioned on a Leica CM1850 cryostat at 8µM thickness at -21°C and fixed in ice cold 50% methanol/50% acetone. Slides were rehydrated and washed in 1XPBS three times, incubated in primary antibody (NP185 10ug/ml; mouse anti-Human Collagen VII) for 1 hour at room temperature. Slides were washed and incubated in Alexa 488 tagged goat anti-mouse IgG antibody (1:400, 1hour, Room Temperature, life technologies) and a nuclear Hoescht 33342 counterstain (Life technologies). Samples were washed three times in 1xPBS, mounted using fluoromount prior to imaging. Images were taken on a Zeiss Observer.Z1 fluorescence microscope at 20X magnification.

Grafts harvested at Day 19 were included as positive and negative control arms. Experimental arms were injected Day 38, 39, or 41 and harvested Day 48, 49, or 51 (Table 37).

**Table 37: Graft, Injection, and Harvest Days**

| **Study Arm** | **N** | **Day of Graft** | **Day of Injection** | **Day of Harvest** |
|---|---|---|---|---|
| Control Arms | | | | |
| Positive (Normal keratinocytes, normal fibroblasts) | 1 | 0 | N/A | 19 |
| Negative (RDEB keratinocytes, RDEB fibroblasts) | 1 | 0 | N/A | 19 |

| Experiment Arms | | | | |
|---|---|---|---|---|
| Non-transduced RDEB fibroblasts | 2 | 0 | 41 | 51 |
| GM-HDF | 4 | 0 | 38 (n=3) or 39 (n=1) | 48 (n=3) or 49 (n=1) |

### E. DATA ANALYSIS

IF images were prepared and provided to the PI in a blinded fashion. Data was analyzed and interpreted by the PI prior to unblinding.

### F. RESULTS

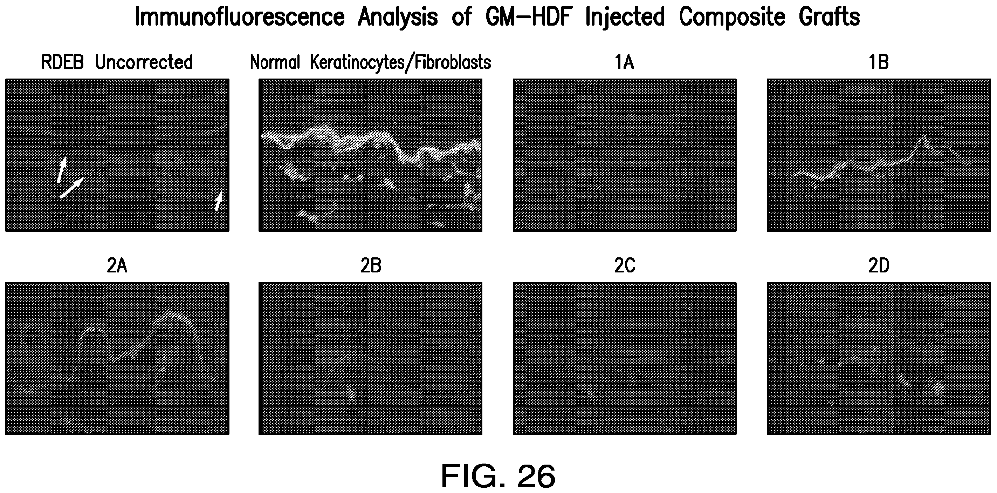

Representative results from the IF analysis of the harvested graft tissue are presented in Figure 26.

C7 staining at DEJ was visualized in all NP185 staining conditions excluding the negative control (image "RDEB uncorrected" - see arrows at DEJ for negative baseline comparison). With regard to the positive controls, there was intense DEJ staining seen in the DEJ of normal keratinocytes/fibroblasts seeded grafts. There was a small focus of positive staining seen in the group 1 injected with uncorrected fibroblasts (Image 1b) in a small section that could represent previously seeded corrected fibroblasts, however the remainder of the uncorrected fibroblast injected grafts in both mice appear to be less intense (as represented in image 1a). There was C7 staining seen at the DEJ in the representative images of four mouse grafts injected with corrected fibroblasts (4a-d) were observed even after only 10-days post-injection.

No tumors were observed in any grafts during the course of the study.

### G. CONCLUSIONS

RDEB GM-HDF TR8 intradermally injected at the approximate proposed clinical dose produced C7 that localized to the DEJ in vivo in composite grafts of RDEB keratinocytes on devitalized pig dermis. Additionally results from seeding of GM-HDF in composite grafts prior to grafting indicate that GM-HDF could persist in expression C7 that localizes to the DEJ. This model can be used to characterize the localization, durability, persistence and phenotype correction approximating the effect of GM-HDF predictive of the clinical dose.

Results of these studies show intradermal injections of autologous GM-HDF as an effective treatment of RDEB in patients.

### Example 7 - Non-GLP In Vitro GM-HDF Cell Characterization and Proof-of-Concept Assessments

The objectives of this study are the following: (a) characterize the GM-HDFs produced using the anticipated at-scale GMP production method to be used for the treatment of RDEB patients; (b) assess copy number of integrated LV and expression levels of C7; and (c) confirm the functionality of C7 expressed by the GM-HDFs

### A. STUDY MATERIALS

### A.1. Test Article(s)

GM-HDFs from Training runs 8, 9, and 10, and the Engineering run (as described above) were characterized.

**Table 38: General production information for TR8, TR9, TR10, and ER1**

| **Production Run** | **Arm** | **MOI (IU/cell)** | **LV-COL7 and TU titer** | **Production Scale** |
|---|---|---|---|---|
| TR8 | A | 3.4 | INXN-2002 (Pilot) 9.2x10⁶ IU/mL | 2 x 10-layer CellSTACKs^{®} |
| | B | 1.7 | | |
| | Control ¹ | 0 | | |
| TR9 | A | 1.0 | LV-HA-COL7 (Pilot) 2.0x10⁶ IU/mL | 2 x 10-layer CellSTACKs^{®} |
| | B | 1.9 | | |
| | Control¹ | 0 | | |
| TR10² | A | 2.0 | INXN-2002 (GMP) 2.3x10⁶ IU/mL | 6 x 10-layer CellSTACKs^{®} |
| | Control¹ | 0 | | |
| ER1² | A | 2.6 | INXN-2002 (GMP) 2.3x10⁶ IU/mL | 6 x 10-layer CellSTACKs^{®} |

| | | | | |
|---|---|---|---|---|
| ¹Control arm cells were mock-transduced ²Only one LV-COL7 transduction arm was generated for TR10 and for ER1 | | | | |

### B. Primer and Probe sequences used in qPCR analyses

**Table 39: Primer and Probe Sequences used for qPCR Assays**

| **Name** | **Sequence¹** | **Assay** |
|---|---|---|
| PR13843 forward² | ACCTGAAAGCGAAAGGGAAAC (SEQ ID NO:25) | LV-COL7 copy number |
| PR13843 reverse² | CACCCATCTCTCTCCTTCTAGCC (SEQ ID NO: 26) | LV-COL7 copy number |
| PR13843 probe² | | LV-COL7 copy number |
| PR13653 forward | CACTCCCAACGAAGACAAGAT (SEQ ID NO: 28) | COL7A1 mRNA expression |
| PR13653 reverse | GTCTAACCAGAGAGACCCAGTA (SEQ ID NO: 29) | COL7A1 mRNA expression |
| PR13653 probe | | COL7A1 mRNA expression |

| | | |
|---|---|---|
| ¹Primers/probes purchased from IDT. ²LV-specific primer/probe sequences derived from Greenberg et al. (2006). | | |

### C. EXPERIMENTAL PROCEDURES

### C.1 LV-COL7 Copy Number

Nucleic acid isolation was performed using Qiagen's AllPrep kit according to the manufacturer's instructions. gDNA isolated from 3x10⁵ GM-HDF cells was normalized to 12.5 ng/µl. 8 µl of the normalized gDNA was used in a 20 µL assay (10 µL Taqman^{®} Gene Express, 1.8 µL nuclease-free water, 0.06 µL of 100 µM forward primer, 0.06 µL of 100 µM reverse primer, 0.04 µL of 100 µM Taqman ^{®} probe). A standard curve of serially diluted linearized C7 lentiviral shuttle vector (1e6 copies/reaction to 5 copies/reaction), plus 4 µL of human gDNA (1.5e4 cells/reaction) were also assayed in the 20 µL assay mentioned above. The Taqman ^{®} assay used was PR13843. 8 µL of an additional standard curve of commercial human gDNA (Clontech) (1.5e4 cells/reaction to 2e2 cells/reaction) in a 20 µL assay (10 µL Taqman^{®} Gene Express, 1.0 L nuclease-free water, 1 µL of 20X ACTB primer/probe set) was also performed. All samples were run on an ABI7900 using the following cycling parameters: 2 minutes at 50°C, 10 minutes at 95°C, and 40 cycles of 15 sec at 95°C and 1 minute at 60°C.

### C.2 COL7A1 RT-qPCR

Vials of GM-HDF cells were thawed and gDNA and RNA were isolated using Qiagen's AllPrep^{™} kit. RNA (800 µg) was used to generate cDNA using Quanta's qScript^{™} per manufacturer's instructions. The cDNA and gDNA were then tested using Taqman^{®} assay PR13653 as described in above. Data were normalized to housekeeping gene ACTB (dCT), and then compared to Control data (ddCT). The resulting ddCT is converted to fold change using the formula 2^-(ddCT).

### C.3 C7 Immunofluorescence

For immunofluorescence analyses, 1.2 x 10⁴ GM-HDFs were allowed to attach to PDL/Lamin- coated coverslips in 24-well plates overnight and then fixed and permeabilized with a 50%/50% mix of methanol/acetone. The coverslips were washed 3 times with 1X PBS and then blocked with 10% goat serum in PBS for 30 minutes at room temperature. After three additional washes with PBS, the coverslips were incubated with 1.25 µg/mL fNC1 antibody in 1% goat serum/PBS, followed by 3 additional washes with PBS and incubation with 5 µg/mL Alexa Fluor^{®} 555- conjugated goat anti-rabbit IgG in 1% goat serum/PBS for 1 hour at room temperature. Coverslips were stained with NucBlue^{®} Live Cell Stain Ready Probes Reagent before being mounted onto slides. Images were acquired on a Zeiss Axio Observer microscope at 20X magnification using an exposure time of 290 ms. NHDFs or GM-HDFs were fixed, permeabilized, and stained with NucBlueLive^{®} Cell Stain to visualize nuclei (blue) or with the fNC1 antibody and Alexa Fluor^{®} 555-congugated goat anti-rabbit IgG (5 µg/mL) to visualize C7 expression (red). Images were acquired at 20X magnification using an exposure time of 290 ms.

### C.4 C7 Protein ELISA

Briefly, a standard curve of purified His-NC1 fragment (9.8 - 625 ng/mL) or collected supernatants containing C7 protein (from GM-HDF in culture for three to five days) were immobilized to a Nunc MaxiSorp ^{®} 96-well plate overnight at 4°C. Standards and samples were tested in the same sample matrix (20% RDEB fibroblast conditioned media). Coated wells were washed with PBST and blocked with 3% BSA/PBS for 1 hour at 37°C. Detection was accomplished using a polyclonal anti-NC1 Ab (fNC1, 0.5 µg/mL) followed by incubation with secondary antibody donkey anti-rabbit IgG HRP (Jackson ImmunoResearch, 0.08 µg/mL). Bound antibodies were detected via colorimetric development with TMB substrate solution. Following quenching of the reaction, absorbance was measured at 450 nm on the SpectraMax^{®} Plus 384 (Molecular Devices).

### C.5 Immunoprecipitation of C7 trimers

First, magnetic Protein G beads were washed with 1 X PBS-T. Beads were bound to the magnet for a minimum of 2 minutes prior to removal of supernatant and in all subsequent steps. Following the washes, the beads were coated with 5 µg of anti-C7 fNC1 and incubated for 10 minutes with rotation (Glas-Col, setting 30 ~ 14 rpm at room temperature). Beads were bound to the magnet to remove the supernatant and washed with Ab binding/Wash buffer. Supernatants were collected from GM-HDFs in culture for three to five days. C7 containing supernatant was added to the bead/C7 supernatant mix and was incubated overnight at 4° C with rotation (setting 30 ~ 14 rpm). The next day the beads were bound to the magnet and washed three times using Wash buffer. Target antigen was eluted in 20 µL of elution buffer (50 mM glycine, pH 2.8, and 10 µl of 4X Loading Dye). The samples were denatured at 70°C for 10 minutes. 12 µl of a total of 30 µl (remaining sample was stored at 4°C) were then loaded (per well) into a 12 well 3-8% Tris Acetate gel and run for 4 hours at 150 volts. The gel was removed from the cassette and soaked in Transfer Buffer containing 10% methanol for 20 minutes. Overnight wet transfer of the gel to a nitrocellulose membrane was performed at 15 volts at 4°C. The next day, the voltage was increased to 50 volts for 30 min on ice. Upon completion of transfer, the blot was blocked using 5% milk in TBS-T with agitation (Labnet ProBlot^{™} Rocker 25, setting 60 rpm) for 2 hours at room temperature and incubated with commercially available anti-C7 LH7.2 (0.25 µg/mL) with agitation for 2 hours at room temperature. The blot was washed 3 times with 1X TBS-T (5 min each) with agitation before being probed with HRP-conjugated goat anti-mouse IgG (0.1 µg/mL) with agitation for 1 hour at room temperature. The blot was developed using Lumiglo Ultra^{™}Chemiluminescent substrate and Fujifilm LAS 3000 Imaging System.

### C.6 Lam332 Binding

96-well MaxiSorp^{™} plates were coated with 1 µg Lam332 or BSA in 100 mM carbonate buffer, pH 9.3 overnight at 4°C. The plates were rinsed five times with PBS-T, and then blocked with 1% BSA in PBS-T at room temperature for 1 hour. Coated wells were rinsed five times with PBS-T and incubated with supernatants from transduced cells overnight at 4°C. Following three more washes with PBS-T, bound C7 was incubated with the fNC1 antibody (0.5 µg/mL, PBS-T) at room temperature for 3 hours, followed by incubation with HRP-conjugated donkey anti- rabbit IgG (0.8 µg/mL final concentration in PBS-T) at room temperature for 2 hrs. Detection of the C7-bound antibodies was via a colorimetric reaction using TMB and measured by reading the absorbance of the product at 450nm on a SpectraMax^{®} Plus 384 plate reader (Molecular Devices). All incubations were carried out on an orbital shaker (GeneMate) at a speed setting of 2.

### C.7. Cell Migration Assay

CytoSelect^{™} 24-well wound-healing assay kit from Cell Biolabs, Inc. was used for the cell migration assay. GM-HDFs (0.8 x 10⁵) were seeded in the presence of a wound insert in 24-well plates and grown for 48 hours. The wound insert was then removed and the cells were further cultured in reduced (1%) serum media containing 15 µg/mL type I collagen (COL1) for 8 hours. Brightfield images of the wounds were acquired 0, 2, 4, 6, and 8 hours after removal of the wound insert using the Celigo^{®} Imaging Cytometry System (Cyntellect). The surface area of the wound at each time point was measured using ImageJ (National Institutes of Health). The data is reported as the percentage of migration (% Migration) covering the wound area.

### C.7. DATA ANALYSIS

qPCR was performed using an ABI 7900 instrument utilizing SDS2.3 software. The experimental data were viewed and exported in SDS 2.4 software. ImagJ software was used to quantitate percent cell migration for the assay described above. ImageJ is an open source image processing program developed by the Nation Institute of Health and designed for scientific multidimensional images.

### D. RESULTS

### D.1. Characterization of C7 Expression

### D.1.1. LV-COL7 Copy Number and Transcript Levels

Taqman^{®} assays were designed against various regions of the viral shuttle vector. Of the Taqman^{®} assays tested, designs targeting the C7 coding sequence demonstrated unacceptable levels of background amplification. The Taqman^{®} assay selected, PR13843, was targeted to a sequence found at the 5' end of the LV backbone as described in Greenberg et al. (2006).

GM-HDF training run cells were obtained, washed with dPBS, and resuspended in RLT buffer. Qiagen's AllPrep kit was used to isolate the DNA (for copy number) and RNA (for mRNA transcript level) from the drug substance vials from each arm of each training run. The isolated DNA and RNA were then subjected to qPCR and RT-qPCR assays as described above in Section C.1 and C.2, respectively. Table 40 shows the copy number/cell from Training Runs 8, 9, and 10 (TR8, TR9 and TR10) and the Engineering Run (ER1). The data demonstrate that the number of copies of transgene integrated into each cell can be modulated by the virus dose given to the cells during production, and is less than 1 copy/cell. Similarly, the LV-COL7 transcript levels, shown in Figure 40, also correlate with the virus dose and copy number, with Arms A and B from the training runs expressing higher levels (3 to 5 LOG) of the lentiviral C7 sequence compared to Control cells.

**Table 40: LV-COL7 Copy Number in GM-HDF Production Run Cells**

| **Production Run** | **LV-COL7 Used** | **Arm** | **MOI (IU/cell)** | **Gene Copy per Cell** |
|---|---|---|---|---|
| TR8 | INXN-2002 (Pilot) | A | 3.4 | 0.021±0.007 |
| | | B | 1.7 | 0.017±0.007 |
| | | Control¹ | 0 | BLQ² |
| TR9 | LV-HA-COL7 (Pilot) | A | 1.0 | 0.009±0.002 |
| | | B | 1.9 | 0.021±0.002 |
| | | Control ¹ | 0 | BLQ² |
| TR10³ | INXN-2002 (GMP) | A (3) | 2.0 | 0.013±0.002 |
| | | Control¹ | 0 | BLQ² |
| ER1³ | INXN-2002 (GMP) | A (3) | 2.6 | 0.025±0.005 |
| | | Control¹ | 0 | BLQ² |

| | | | | |
|---|---|---|---|---|
| ¹ Control arms were mock-transduced and were not exposed to LV-COL7 ² BLQ: below limit of quantitation ³ Only a single transduction arm was generated for TR10 and for ER1 | | | | |

### D.1.2. C7 Protein Expression

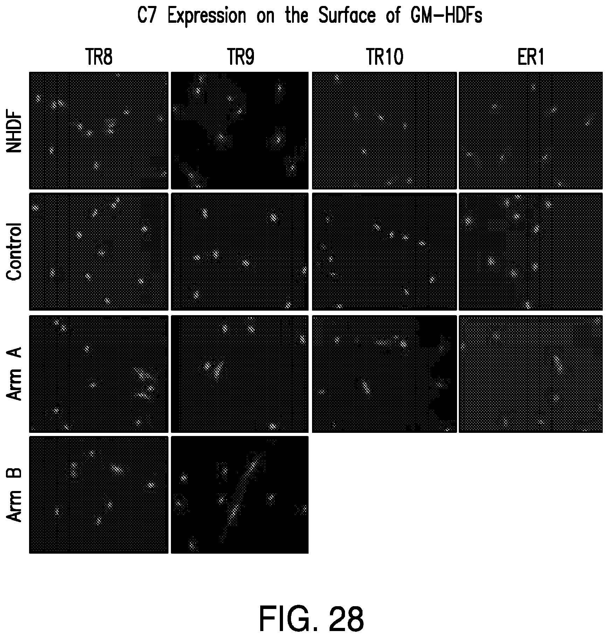

Indirect immunofluorescence (IF) was used to examine C7 protein expression by GM-HDF cells using an antibody specific for the C7. Normal human dermal fibroblasts (NHDFs) were used as a positive control for this assay and the control arms of the training runs were used as negative controls. In the representative images shown in Figure 28 below, a small percentage (<10%) of INXN-2002 and LV-HA-COL7-transduced RDEB fibroblasts show C7 staining, which is consistent with the copy number of integrated LV (Table 18). The signal from the GM-HDFs that are C7-positive is brighter than the signal from NHDFs, indicating greater amounts of C7 produced by GM-HDFs.

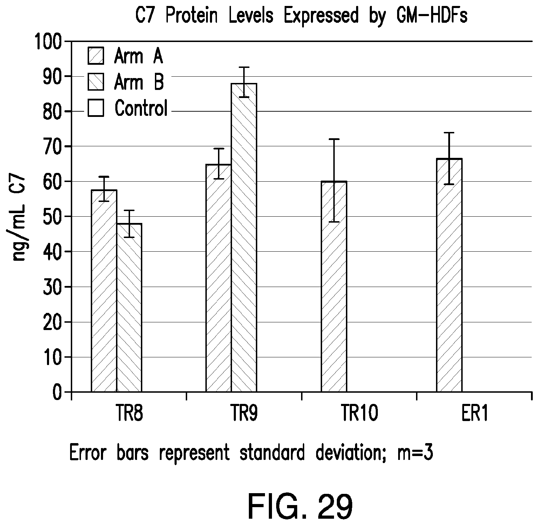

A direct ELISA was developed by Intrexon to measure C7 secreted by GM-HDFs into the cell culture media. This assay, as described above in Section C.4, utilized an NC1 region-specific polyclonal antibody. ELISA assay results of the GM-HDFs from the three training runs show that C7 expression is LV dose dependent with expression levels ranging from 45-90 ng/mL of cell supernatants (Figure 29).

### D.2. Functional Assessment of C7 Expressed by GM-HDFs 9.2.1. Formation of C7 Trimers

The formation of C7 trimers is important in the assembly of anchoring fibrils. To verify that the C7 expressed by GM-HDFs will be capable of forming anchoring fibrils, we assessed whether or not the expressed C7 formed trimers using immunoprecipitation (IP) with an anti-C7 specific antibody (fNC1) for selective capture, then concentration of C7 from culture supernatants for detection by SDS-PAGE/immunoblot.

The optimal conditions for immunoprecipitation were established by testing a commercial C7 antibody and the fNC1 C7 antibody. The coating of protein G beads with fNC1 resulted in higher levels of C7 binding compared to coating with the commercially available antibody. Controls used in this assay include a no antibody control to rule out non-specific binding of C7 to the beads, as well as antibody coated beads incubated with and without purified C7 spiked into conditioned media as controls for specificity of C7 isolated from GM-HDF culture supernatants.

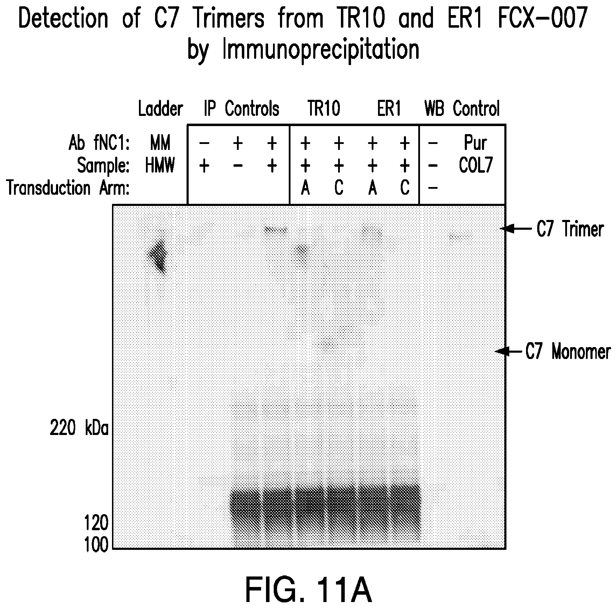

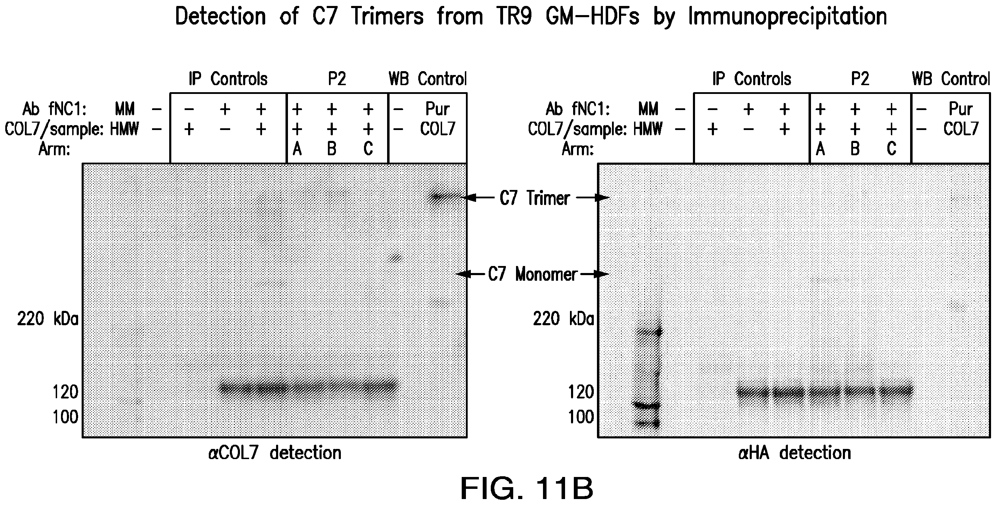

Immunoprecipitation results for TR8 and for TR10 and ER1 showed that the C7 produced by GM-HDF was predominantly trimeric (Figure 10, and Figure 11, respectively). Some lower molecular weight species showing immunoreactivity were also observed, and included the dimeric and 290 kDa monomeric forms. IP of C7 from GM-HDFs of TR9 was detected on the immunoblot at low levels by both an anti-C7 antibody (left panel, Figure 11B) as well as an antibody specific for the HA tag incorporated into the C7 protein (right panel, Figure 11B).

### D.2.2. Lam332 Binding by C7 Expressed from GM-HDFs

C7 has been shown to bind immobilized extracellular matrix (ECM) components, including fibronectin, Laminin 332 (Lam332), COL1, and COL4 (Chen, et al., 2002a). The interaction between C7 and Lam332 occurs through the NH2-terminal NC1 domain of C7 and is dependent upon the native conformation of both Lam332 and C7 NC1 (Rousselle, et al., 1997). The association between C7 and Lam332 is important for establishing correct Lam332 architecture at the dermal-epidermal junction. Such organization is important for interactions with extracellular ligands and cell surface receptors, and for cell signaling (Waterman, et al., 2007). Intrexon developed an ELISA using an antibody against the C7 NC1 domain to detect binding of C7 to purified Lam332. Although a C7/Lam332 binding ELISA has already been described in the literature (Chen, et al., 2002a), to our knowledge it has never been used to test C7 present in the supernatants of transduced cells. Results in Figure 12 show dose-dependent binding to Lam332 by C7 expressed by GM-HDFs from the Training and Engineering runs.

### D.2.3. Cell Migration Assay

Previous studies have shown that RDEB fibroblasts and keratinocytes show an increase in motility relative to their normal counterparts, and that normal motility can be restored by expression of C7 (Chen, et al., 2000; Chen, et al., 2002b; Cogan, et al. 2014; Baldeschi et al., 2003). Most of these studies employed either the colloidal gold salt migration assay to measure the migration of fibroblasts and keratinocytes, or a wound-healing assay to measure the migration of keratinocytes. Here we used the CytoSelect^{™} 24-well wound-healing assay kit (Cell Biolabs, Inc.) to measure the motility of NHDFs and GM-HDFs.

Immunofluorescent staining for C7 revealed that only a small percentage (<10%) of transduced cells expressed high levels of the protein, raising the question as to whether a small number of C7-producing cells could have a global effect on cell migration. To determine if the presence of C7 in the culture media is sufficient to reverse the RDEB hyper-motility phenotype, the wound healing assay was carried out on GM-HDFs from the three training runs, with NHDFs as a positive control and the mock-transduced Control (RDEB) arms as the negative control.

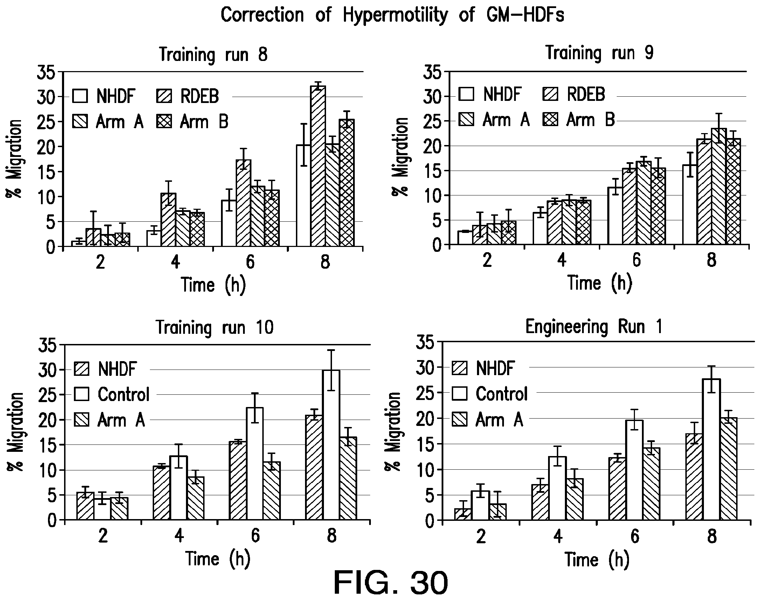

Control fibroblasts from TR8, TR10, and ER1 exhibit a hyper-motility phenotype that is reversed by the expression of C7 (Figure 30). However, the Control fibroblasts from TR9 do not exhibit a significant hyper-motility phenotype, making it difficult to investigate the functionality of HA- tagged C7 in this assay.

### CONCLUSIONS

The integrated transgene copy number per cell in GM-HDFs was dependent on the virus dose ranging from 0.009 to 0.03 transgene copies per cell.

Dose-dependent C7 expression from the GM-HDF cells was confirmed by qRT-PCR, immunofluorescence staining, and ELISA.

The structure of the C7 expressed by the GM-HDF cells was confirmed to be predominantly trimeric by immunoprecipitation/SDS-PAGE/immunoblot analysis for TR8, TR10, and ER1. TR9 showed minimal trimer formation.

The C7 produced from the TR8 GM-HDF cells was demonstrated to be functional by binding to Laminin332 in an in vitro binding assay and by correction of the hypermotility phenotype of Control RDEB cells in an in vitro migration assay. TR9 showed binding to Laminin332, however, migration assay results were equivocal. TR9 HA-GM-HDFs generated from transduction with LV-HA-COL7 showed low levels of trimer, less binding to Lam332, and lack of clear hypermotility correction.

Due to lack of confirmation of in vitro functionality of HA-C7 expressed by TR9 HA- GM-HDFs, TR8 cells were chosen for in vitro and in vivo hybrid pharmacology/toxicology evaluations. This eliminated the ability to assess expression, localization and persistence in normal human skin or RDEB composite grafts prepared with devitalized human skin. Ultimately, composite grafts prepared with devitalized porcine dermis were used for these studies. Immunostaining with human specific anti-C7 antibody was used to distinguish rC7 form native C7 in this approach.

### EXAMPLE 8 - DRUG PRODUCT

FCX-007 Drug Product consists of a sterile suspension of each patient's own living autologous fibroblast cells, which are gene modified using INXN-2004 LV-COL7 to encode the human wild type *COL7A1* gene, formulated in Dulbecco's Modified Eagle's Medium (DMEM) without phenol red.

### Formulation Development

Development of FCX-007 formulation and cell dosage was based on Fibrocell's manufacturing experience with azficel-T (LAVIV^{®}); a live, autologous fibroblast cell product for injection. Fibroblast cells have been successfully formulated in DMEM at a cell concentration range of 0.5 - 3.0 x 10⁷ cells/mL without viscosity concerns (azficel-T BLA #125348). Data suggest viscosity becomes a concern for product injection when cell concentration reaches 5.8 x 10⁷ cells/mL. As a result, DMEM at a cell concentration of 1.0-3.0 x 10⁷ cells/mL was selected as the formulation for FCX-007 DP.

### Excess

Up to ten FCX-007 DP vials are shipped to the clinic as one dose for treatment injection. At the time of administration, the DP vial is gently inverted three times, and then 1 mL from each vial is drawn aseptically into a sterile 1 mL syringe with a 21 gauge needle. The 21 gauge needle is then replaced with a 30 gauge needle for intradermal administration. The process is repeated for the remaining 9 product vials.

FCX-007 DP vials are filled with 1.2 mL of the fibroblast cell suspension into a 2.0 mL capacity vial for a recoverable volume of 1.0 mL. The 0.2 mL excess is intended to ensure that 1.0 mL can be obtained from the vial at the time of administration in the clinic.

### a. Physicochemical and Biological Properties

### i. Physicochemical Properties

FCX-007 DP is presented as a gene modified autologous fibroblast cell suspension in DMEM without phenol red with a 1.2 mL fill in a 2 mL cryovial. The cells are demonstrated by assays to be a living culture. Table 41 shows the physicochemical properties of FCX-007 DP.

**Table 41: Physicochemical Properties of FCX-007 DP**

| **Physicochemical Property** | **Value** |
|---|---|
| Cell Count | 1.0-3.0 x 10⁷ cells |
| Cell Viability | ≥ 70% |

### Biological Properties

FCX-007 is a sterile autologous fibroblast cell product that is genetically modified to express the human collagen 7 protein (C7). INXN-2004 vector copy number per cell and expression of the C7 protein are unique biological properties of FCX-007 DP. FCX-007 DP is a live cell suspension product that is produced from FCX-007 DS by washing the thawed FCX-007 DS cells in PBS and DMEM buffers. INXN-2004 vector copy number per cell and C7 protein expression are analyzed on the FCX-007 DS prior to release to manufacture FCX-007 DP Table 42 shows the biological properties of FCX-007 DP.

**Table 42: Biological Properties of FCX-007 DP**

| **Biological Property** | **Value** | **Stage Measured** |
|---|---|---|
| Cell Type Purity / Identity (fibroblast) | ≥ 98% CD 90+ | Drug Substance |
| INXN-2002 Vector Copy Number | 0.1 - 5.0 | Drug Substance |
| C7 Protein Expression | ≥ 500ng/day/E6 cells | Drug Substance |
| Sterility | No Growth (Sterile) | Drug Product |

### BATCH FORMULA

To prepare the FCX-007 Drug Product, FCX-007 Drug Substance vials are removed from the vapor phase of liquid nitrogen frozen storage, thawed, and pooled inside an ISO 5 BSC. The cells are washed with phosphate buffered saline (PBS), followed by a wash with Dulbecco's Modified Eagle's Medium (DMEM) without phenol red before being suspended in DMEM without phenol red to a target concentration of 1.0 - 3.0 x 10⁷ cells/mL. The formulated cells are then filled into 2 mL cryovials at 1.2 mL/vial. Drug Product material prepared for each injection treatment is defined as a batch. The proposed treatment batch size is up to ten (10) 2 mL vials containing 1.2 mL of cell suspension.

Table 43 provides a list of all components used in the Drug Product manufacturing process.

**Table 43: Composition and Quantity of FCX-007 Drug Product Batch**

| **Component** | **Function** | **Quality Standard** | **Quantity per DP Vial¹** | **Quantity per Batch (10 vials per batch)** |
|---|---|---|---|---|
| FCX-007 DS | Drug Substance | Drug Product Certificate of Analysis | 1.2-3.6 x 10⁷ cells | 1.2 - 3.6 x 10⁸ cells |
| PBS | Diluent used for washing Drug Substance cells | Manufacturer 's Certificate of Analysis | Minimal residual trace² | Minimal residual trace² |
| DMEM without phenol red | Buffer for cell suspension | Manufacturer 's Certificate of Analysis | 1.2 mL | 12 mL |

| | | | | |
|---|---|---|---|---|
| ¹ Vial contains 1.2 mL of FCX-007 cell suspension ² PBS wash residual is diluted with DMEM without phenol red such that trace amounts of PBS is possible but not quantifiable | | | | |

### DESCRIPTION OF MANUFACTURINIG PROCESS AND PROCESS CONTROLS

### b. Manufacturing Process Flow Diagram

Manufacturing of the FCX-007 Drug Product commences when FCX-007 Drug Substance vials are removed from liquid nitrogen storage and thawed using a ThermoMixer^{®}. In an ISO5 BSC the thawed FCX-007 DS cells are pooled into a 250 mL centrifuge tube containing >5x bulk volume of phosphate buffered saline (PBS) wash buffer for re-suspension. The cells are centrifuged at 1000 rpm for 10 minutes at 5 ± 3°C and the PBS wash buffer is removed. This is followed by a wash with > 5x bulk volume of Dulbecco's Modified Eagle Medium (DMEM) by re-suspension and centrifugation at 1000 rpm for 10 minutes at 5 ± 3°C. The DMEM wash buffer is removed. The washed cells are re-suspended in DMEM without phenol red to a target concentration of 2.0 x 10⁷ cells/mL. The cell suspension is manually filled into sterile 2.0 mL cryovials in an ISO5 BSC to a volume of 1.2 mL per vial to produce the FCX-007 DP vials. The Drug Product vials are stored at 2-8°C until released by QA for shipment in a Credo Cube^{™} shipper to the clinical site.

### Manufacturing Process Description

FCX-007 Drug Product is manufactured as indicated above. INXN-2002 is a synonymous name for FCX-007.

### Step 1: Removal of FCX-007 Drug Substance Vials from Liquid Nitrogen Storage and Lot Verification

The FCX-007 Drug Substance, which is stored in a liquid nitrogen freezer, is requested from Materials Management. FCX-007 Drug Substance vials are then pulled for this batch of FCX-007 Drug Product production before being transferred to the cleanroom suite.

### Step 2: Thaw Cryovials

A Line Clearance of the cleanroom suite is performed by QA per SOP-0099 GMP Production Line Clearance/GTP Line Cleaning in order to release the room for processing use. Operators initiate room, personnel, and BSC environmental monitoring (EM) per SOP-0129 (In Process Environmental and Personnel Monitoring) prior to processing and non-viable EM sampling in the BSC during processing.

The FCX-007 DS cyrovials are disinfected and thawed in a ThermoMixer^{®} equilibrated to 37°C until cell suspension is almost completely thawed with a sliver of ice crystals remaining. The thawed vials are transferred to a BSC.

### Step 3: PBS Wash

Inside the BSC, using a pipette, transfer approximately 150 mL of PBS (> 5 volumes of the FCX-007 DS cell suspension) into a 250 mL centrifuge tube. The contents of the thawed cryovials are transferred into the 250 mL centrifuge tube. Use the diluted cell suspension to rinse the pipette. Use a pipette to transfer 3.0 mL of fresh PBS to each cryovial as a rinse. Transfer the rinse to a 250 mL centrifuge tube. Swirl the tube gently to mix the cell suspension. Transfer the centrifuge tube to a centrifuge and centrifuge the cells at 1000 RPM for 10 minutes at 5 ± 3°C. After centrifugation, remove the PBS wash supernatant from the tube.

### i. Step 4: DMEM Wash

Using a pipette, add approximately 200 mL of DMEM without phenol red (> 5 volumes of the FCX-007 DS cell suspension) to the tube to re-suspend the cell pellet. Swirl the tube gently to mix the cell suspension. Transfer the centrifuge tube to a centrifuge and centrifuge the cells at 1000 RPM for 10 minutes at 5 ± 3°C. After centrifugation, using a pipette, transfer 0.9 mL of the supernatant from the centrifuge bottle into each of 2 separate 2 mL cryovials (1.8 mL total) labeled "Lot, QC-Sterility". Set the cryovials labeled for sterility aside in the BSC. Aspirate the remaining DMEM wash supernatant from the centrifuge tube.

### Step 5: Cell Pellet Re-suspension in DMEM

The volume of DMEM used to re-suspend the cell pellet is calculated as:
Volume of thawed FCX-007 DS cell suspension divided by 1.5
The 1/1.5 volume factor compensates for possible cell loss during the wash steps, in order to result in a cell concentration in the specified range of 1.0-3.0 x 10⁷ cells/mL after re-suspension.

Use a pipette to transfer the calculated amount of DMEM to re-suspend the cell pellet in the centrifuge tube. Use a pipette to transfer 0.1 mL of the cell suspension into a 2 mL cryovial labeled "Lot, QC- Count and Viability" and submit the cryovial to QC for cell counting. Use a pipette to measure the remaining total cell suspension volume and place the cell suspension into a 2-8°C refrigerator for later final fill use.

### Step 5a: Re-dilution (if needed)

If the QC cell concentration is above 3.0 x 10⁷cells/mL, perform an additional dilution using fresh DMEM to target a cell concentration of 2.0 x 10⁷ cells/mL. Re-submit a cell suspension sample to QC for cell count to confirm final cell concentration.

### Step 6: Final Fill

Retrieve the cells from the refrigerator and transfer the cells to the BSC for final fill. Use a pipette to fill the well-mixed cell suspension manually into 2 mL cryovials in the following order:
Fill 0.1 mL of cell suspension into the first sterility sample vial (added to retained sample from Step 4)
Fill the product vials at 1.2 mL per vial
Fill 0.1 mL of cell suspension into the second sterility sample vial (added to retained sample from Step 4)
Fill the QC vial at ≤1.8 mL per vial
Submit the sterility and QC vials for testing.
Step 7: Store DP Vials for Release and Shipment
Hold the DP vials at 5 ± 3°C for release and shipment to the clinical site.

### Description of the In-Process Controls

### Environmental Controls

All cell culture manipulations are carried out in a certified ISO 5 Biological Safety Cabinets (BSCs) within an ISO7 environmental background using aseptic techniques. The final Drug Product 2 mL cryovial containers are purchased pre-sterilized from Corning. The operators performing the final fill are qualified for aseptic fill operations.

### In-Process Controls and Testing

The process controls and testing performed for release of FCX-007 Drug Product are described below.

### Step 1

The correct FCX-007 Drug Substance lot is verified by QA before use in FCX-007 DP manufacture.

### Step 2

The temperature of the ThermoMixer^{®} used to thaw FCX-007 Drug Substance vials is controlled at 37°C. The content of each vial is thawed until a sliver of ice still remains in the vial to prevent overheating.

### Step 3

The PBS wash volume is controlled at ≥ 5 volumes of the FCX-007 DS cell pool to ensure adequate washing of the cells. The centrifugation conditions are controlled at 1000 rpm, 10 minutes and 5±3°C to ensure sufficient cell pelleting.

### Step 4

Similarly to the PBS wash step, the DMEM wash volume is controlled at ≥ 5 volumes of the FCX-007 DS cell pool to ensure adequate washing of the cells. The centrifugation conditions are controlled at 1000 rpm, 10 minutes and 5±3°C to ensure sufficient cell pelleting. Two 900 µL aliquots of DMEM wash supernatant are taken for use in sterility testing of the final product.

### Step 5 and 5(a) (if needed)

The re-suspended cell concentration is controlled at 1.0 -3.0 x 10⁷ cells/mL, and viability ≥ 70%.

### Step 6

Fill volume of the FCX-007 DP vials is controlled at 1.2 mL fill per vial. A final cell count and viability measurement, as well as Gram stain is performed on the filled QC vial. The two sterility vials are submitted for sterility testing. The results of this sterility test are not received until after Drug Product release, so release of DP is done on the basis of a negative Gram Stain. The use of a rapid microbial test in this situation is indicated in Guidance for FDA Reviewers and Sponsors "Content and Review of Chemistry, Manufacturing, and Control (CMC) Information for Human Somatic Cell Therapy Investigation New Drug Applications (INDs)" of April 2008.

### ii. Control of Materials

The FCX-007 Drug Product manufacturing process utilizes single use, disposable products that will come in contact with raw materials (e.g. pipets, centrifuge tubes, and cryovials) to remove cleaning and carryover issues from consideration. Raw materials selected for use in the manufacturing process are acceptable for pharmaceutical processing as being USP VI compliant. All disposable items are purchased pre-sterilized by gamma irradiation. The PBS and DMEM wash buffers are purchased sterile from commercial suppliers. Steps in the process have the appropriate process parameters identified, such as time and temperature.

### EXAMPLE 9 - In Vitro Characterization of Optimized GM-HDF

This example describes characterization of GM-HDFs generated using an enhanced LV transduction procedure the LV-COL7 construct INXN-2004. Three training runs (TR11, TR12.1, and TR13) were executed to generate cells for the analyses at GMP-scale. TR11 was transduced with the LV-COL7 vector INXN-2002 and was used to evaluate a centrifugation-enhanced (spinoculation) LV transduction procedure with and without a second transduction step (super-transduction). TR12.1 and TR13 were transduced with the LV-COL7 vector INXN-2004 utilizing enhanced transduction procedures as determined through evaluation of TR11.

GM-HDFs from each training run were characterized using assays to assess results such as copy number of integrated LV-COL7, protein expression of C7, and functionality of the C7 expressed by GM-HDFs.

LV integration and C7 expression levels increased first with the addition of the super-transduction step and increased further with use of the LV-COL7 INXN-2004. C7 expressed from GM-HDFs from all three training runs assembled into the trimeric form and bound to Laminin332, which is important in the formation of anchoring fibrils. Expression of C7 by the GM-HDFs was able to reverse hypermotility observed in RDEB fibroblasts. GM-HDFs from TR12.1 and 13 were selected to be used in *in vivo* GLP toxicology studies.

Objectives in this study were to (a) demonstrate improvement in copy number of integrated LV and expression of C7 resulting from 1) an enhanced transduction procedure incorporated into the at-scale GMP production method and 2) adoption of the INXN-2004 LV-COL7 construct and (b) to confirm the functionality of C7 expressed by the GM-HDFs

### 1. STUDY MATERIALS

### 1.1. Test Article(s)

GM-HDFs from Training Runs (TR) 11, 12.1, and 13 were characterized.

**Table 44: General production information for TR11, TR12.1, and TR13**

| **Production Run** | **LV-COL7 and TU titer** | **Arm** | **MOI (IU/cell)** | **Super-transduction** | **Production Scale** |
|---|---|---|---|---|---|
| TR11 | INXN-2002 (GMP) | A | 0.77 | Yes | 2 x 10-layer CellSTACKs^{®} |
| | | B | 0.77 | No | |
| | 1.2x10⁵ IU/mL | Control¹ | 0 | n/a | |
| TR12.1 | INXN-2004 (GMP) | A | 0.12 | Yes | 2 x 10-layer CellSTACKs^{®} |
| | 1.4x10⁵ IU/mL | B | 0.12 | No | T-175² |
| TR13 | INXN-2004 (GMP) | A | 0.12 | Yes | 2 x 10-layer CellSTACKs^{®} |
| | 1.4x10⁵ IU/mL | B | 0.12 | No | T-175² |

| | | | | | |
|---|---|---|---|---|---|
| ¹ Control arm cells were mock-transduced ² Arm B of TR12.1 and TR13 were for research only. Passaging of these arms terminated early and, thus, did not complete the full-scale production process. | | | | | |

### 1.2. Primer and Probe Sequences

**Table 45: Primer and Probe Sequences used for qPCR Assays**

| **Name** | **Sequence¹** | **Assay** |
|---|---|---|
| PR13843 forward² | ACCTGAAAGCGAAAGGGAAAC (SEQ ID NO: 31) | LV-COL7 copy number |
| PR13843 reverse² | CACCCATCTCTCTCCTTCTAGCC (SEQ ID NO: 32) | LV-COL7 copy number |
| PR13843 probe² | | LV-COL7 copy number |

| | | |
|---|---|---|
| ¹ Primers/probes purchased from IDT. ² LV-specific primer/probe sequences derived from Greenberg *et al.* (2006). | | |

### 2. EXPERIMENTAL PROCEDURES

### 2.1. LV-COL7 Copy Number

Nucleic acid isolation was performed using Qiagen's AllPrep kit according to the manufacturer's instructions. gDNA was isolated from 6.7x10⁵ GM-HDF cells, and 8 µL of gDNA was used in a 20 µL assay (10 µL Taqman^{®} Gene Express, 1.8 µL nuclease-free water, 0.06 µL of 100 µM forward primer, 0.06 µL of 100 µM reverse primer, 0.04 µL of 100 µM Taqman^{®} probe). A standard curve of serially diluted linearized LV-COL7 lentiviral shuttle vector (1e6 copies/reaction to 5 copies/reaction), plus 4 µL of human gDNA (1.5e4 cells/reaction) were also assayed in the 20 µL assay mentioned above. The Taqman^{®} assay used was PR13843. 8 µL of an additional standard curve of commercial human gDNA (Clontech) (1.5e4 cells/reaction to 2e2 cells/reaction) in a 20 µL assay (10 µL Taqman^{®} Gene Express, 1.0 L nuclease-free water, 1 µL of 20X ACTB primer/probe set) was also performed. All samples were run in triplicate on an ABI7900HT using the following cycling parameters: 2 minutes at 50°C, 10 minutes at 95°C, and 40 cycles of 15 sec at 95°C and 1 minute at 60°C.

### 2.2. C7 Immunofluorescence

For immunofluorescence analyses, 1.2 x 10⁴ GM-HDFs were allowed to attach to PDL/Lamin-coated coverslips in 24-well plates overnight and then fixed and permeabilized with a 50%/50% mix of methanol/acetone. The coverslips were washed 3 times with 1X PBS and then blocked with 10% goat serum in PBS for 30 minutes at room temperature. After three additional washes with PBS, the coverslips were incubated with 1.25 µg/mL of a polyclonal fNC1 antibody (α-fNC1) in 1% goat serum/PBS, followed by 3 additional washes with PBS and incubation with 5 µg/mL Alexa Fluor^{®} 555-conjugated goat anti-rabbit IgG in 1% goat serum/PBS for 1 hour at room temperature. Coverslips were stained with NucBlue^{®} Live Cell Stain Ready Probes Reagent before being mounted onto slides. Images were acquired on a Zeiss Axio Observer microscope at 20X magnification using an exposure time of 290 ms. NHDFs or GM-HDFs were fixed, permeabilized, and stained with NucBlue^{®} Live Cell Stain to visualize nuclei (blue) or with the fNC1 antibody and Alexa Fluor^{®} 555-congugated goat anti-rabbit IgG (5 µg/mL) to visualize C7 expression (red). Images were acquired at 20X and at 5X magnification using an exposure time of 290 ms.

### 2.3. C7 Flow Cytometry

GM-HDFs (~500,000 cells) were collected into each well of a 96-well plate V-bottom plate, concentrated by centrifugation (1500 rpm, 5 min, room temp.), and the culture media supernatants removed. The cells were then resuspended in 200 µL of CytoFix, transferred to a 96-well V-bottom plate, and incubated for 30 min at 4°C. Following fixation, the fixative is then removed by centrifugation (1500 rpm, 5 min, 4°C) and the cells permeabilized with 200 µL ice-cold 100% methanol for 30 min on ice. Cells were then held at -20°C prior to staining procedure. For staining, permeabilized cells were washed twice with 200 µL incubation buffer (0.5 g BSA in 100 mL PBS), with centrifugation (300 rpm, 5 min, room temp.) in between washes to remove the buffer. Washed cells were resuspended in 100 µL of incubation buffer containing α-fNC1 diluted 1:400 and incubated for 1hour at room temp. Cells were then washed twice as described above and resuspended in 100 µL of incubation buffer containing AlexaFluor 555-Rabbit IgG secondary antibody diluted 1:2000 and incubated for 30 min at room temp. Cells were then washed twice as described above and resuspended in 100 µL of PBS. C7-expressing cells were enumerated by flow cytometry using BD LSRII and BD FACSDiva Software (V6.2) with the following parameters: FSC Voltage = 484, SSC Voltage = 209, PE = 500.

### 2.4. C7 Protein ELISA

Briefly, a standard curve of purified His-NC1 fragment (9.8 - 625 ng/mL) or collected supernatants containing C7 protein (from GM-HDF in culture for three to five days) were immobilized to a Nunc MaxiSorp^{®} 96-well plate overnight at 4°C. Standards and samples were tested in the same sample matrix (10% RDEB fibroblast conditioned media). Coated wells were washed with PBST and blocked with 3% BSA/PBS for 1 hour at 37°C. Detection was accomplished using α-fNC1 Ab (0.5 µg/mL) followed by incubation with secondary antibody donkey anti-rabbit IgG HRP (Jackson ImmunoResearch, 0.08 µg/mL). Bound antibodies were detected via colorimetric development with TMB substrate solution. Following quenching of the reaction, absorbance was measured at 450 nm on the SpectraMax^{®} Plus 384 (Molecular Devices).

### 2.5. Immunoprecipitation of C7 trimers

First, magnetic Protein G beads were washed with 1 X PBS-T. Beads were bound to the magnet for a minimum of 2 minutes prior to removal of supernatant and in all subsequent steps. Following the washes, the beads were coated with 5 µg of α-fNC1 and incubated for 10 minutes with rotation (Glas-Col, setting 30 ~ 14 rpm at room temperature). Beads were bound to the magnet to remove the supernatant and washed with Ab binding/Wash buffer. Supernatants were collected from GM-HDFs in culture for three to five days. C7 containing supernatant was added to the bead/C7 supernatant mix and was incubated overnight at 4° C with rotation (setting 30 ~ 14 rpm). The next day the beads were bound to the magnet and washed three times using Wash buffer. Target antigen was eluted in 20 µL of elution buffer (50 mM glycine, pH 2.8, and 10 µL of 4X Loading Dye). The samples were denatured at 70°C for 10 minutes. 12 µL of a total of 30 µL (remaining sample was stored at 4°C) were then loaded (per well) into a 12 well 3-8% Tris Acetate gel and run for 4 hours at 150 volts. The gel was removed from the cassette and soaked in Transfer Buffer containing 10% methanol for 20 minutes. Overnight wet transfer of the gel to a nitrocellulose membrane was performed at 15 volts at 4°C. The next day, the voltage was increased to 50 volts for 30 min on ice. Upon completion of transfer, the blot was blocked using 5% milk in TBS-T with agitation (Labnet ProBlot^{™} Rocker 25, setting 60 rpm) for 2 hours at room temperature and incubated with commercially available anti-C7 LH7.2 (0.25 µg/mL) with agitation for 2 hours at room temperature. The blot was washed 3 times with 1X TBS-T (5 min each) with agitation before being probed with HRP-conjugated goat anti-mouse IgG (0.1 µg/mL) with agitation for 1 hour at room temperature. The blot was developed using Lumiglo Ultra^{™} Chemiluminescent substrate and Fujifilm LAS 3000 Imaging System.

### 2.6. Lam332 Binding

96-well MaxiSorp^{™} plates were coated with 1 µg Lam332 or BSA in 100 mM carbonate buffer, pH 9.3 overnight at 4°C. The plates were rinsed five times with PBS-T, and then blocked with 1% BSA in PBS-T at room temperature for 1 hour. Coated wells were rinsed five times with PBS-T and incubated with supernatants from transduced cells overnight at 4°C. Following three more washes with PBS-T, bound C7 was incubated with the α-fNC1 (0.5 µg/mL, PBS-T) at room temperature for 3 hours, followed by incubation with HRP-conjugated donkey anti-rabbit IgG (0.8 µg/mL final concentration in PBS-T) at room temperature for 2 hrs. Detection of the C7-bound antibodies was via a colorimetric reaction using TMB and measured by reading the absorbance of the product at 450nm on a SpectraMax^{®} Plus 384 plate reader (Molecular Devices). All incubations were carried out on an orbital shaker (GeneMate) at a speed setting of 2.

### 2.7. Cell Migration Assay

CytoSelect^{™} 24-well wound-healing assay kit from Cell Biolabs, Inc. was used for the cell migration assay. GM-HDFs (0.8 x 10⁵) were seeded in the presence of a wound insert in 24-well plates and grown for 48 hours. The wound insert was then removed and the cells were further cultured in reduced (1%) serum media containing 15 µg/mL type I collagen (COL1) for 8 hours. Brightfield images of the wounds were acquired 0, 2, 4, 6, and 8 hours after removal of the wound insert using the Celigo^{®} Imaging Cytometry System (Cyntellect). The surface area of the wound at each time point was measured using ImageJ (National Institutes of Health). The data is reported as the percentage of migration (% Migration) covering the wound area.

### 3. DATA ANALYSIS

qPCR was performed using an ABI 7900 instrument utilizing SDS2.3 software. The experimental data were viewed and exported in SDS 2.4 software. FlowJo V10 software was used to analyze Flow Cytometry data. ImagJ software was used to quantitate percent cell migration for the assay described in Section 2.7. ImageJ is an open source image processing program developed by the Nation Institute of Health and designed for scientific multidimensional images.

### 4. RESULTS

### 4.1. Characterization of C7 Expression

The overall objectives of this evaluation were to increase copy number and protein expression through a comparison of transduction methods and LV vectors. Specific objectives were to:
1. Evaluate the effect of super-transduction on integrated copy number
2. Evaluate the effect of spinoculation on integrated copy number
3. Compare copy number values between INXN-2002 and INXN-2004 under super-transduced and spinoculated conditions.

Two GMP lots of LV-COL7 constructs were used for these studies: the INXN-2002 and the construct INXN-2004 as described in this Example.

### 4.1.1. LV-COL7 Copy Number

GM-HDF training run cells were obtained and copy number was determined as described above in Section 2.1. Table 46 below provides the copy number/cell from Training Runs utilizing the new transduction methods. Initial optimization implemented the addition of centrifugation during the transduction step ("spinoculation"). Standard transduction had been used previously with INXN-2002 and resulted in integrated copy numbers ranging from 0.013 to 0.025. Addition of only the spinoculation step for transduction with INXN-2002 resulted in a ≥2-fold increase in the integrated copy number to 0.05 per cell (TR11 Arm B, Table 46) compared to TR8 results from previous studies.

To further enhance the integrated copy number, a second transduction ("super-transduction") was added to the GM-HDF production process. Addition of super-transduction, also utilizing spinoculation, further increased integrated copy numbers of INXN-2002 by approximately 2-fold (TR11 Arm A, Table 46).

Small-scale research studies (not shown) demonstrated a second-generation INXN-2004 LV-COL7 construct, which utilizes an alternative 3^{rd}-generation self-inactivating LV backbone, had the potential to achieve higher integrated copy numbers, resulting in higher expression levels, while utilizing a lower multiplicity of infection (MOI) than used for INXN-2002. When used at GMP-scale with the optimized transduction procedure (spinoculations with super-transduction), INXN-2004 had further improved integrated LV copy numbers of approximately 4- to 8-fold over the INXN-2002 construct to achieve average copy numbers of 0.41 to 0.74 (TR12.1 and TR13, respectively, Table 46).

Of note, Arms B of TR12.1 and TR13 were not tested for copy numbers as they were not passaged through the entire production process (Table 44). Accurate copy number assessments require cells that have been passaged several times following transduction to ensure that episomal copies of LV, which can artificially inflate measured copy numbers, no longer remain.

**Table 46: LV-COL7 Copy Number in GM-HDF Production Run Cells**

| **Production Run** | **LV-COL7 Used** | **Arm** | **MOI (IU/cell)** | **Super-transduction¹** | **Gene Copy per Cell** |
|---|---|---|---|---|---|
| TR8² | INXN-2002 (pilot) | A | 3.4 | No | 0.021 ±0.005 |
| TR11 | INXN-2002 (GMP) | A | 0.77 | Yes | 0.09±0.03 |
| | | B | 0.77 | No | 0.05±0.01 |
| | | Control³ | n/a | No | BLQ⁴ |
| TR12.1⁵ | INXN-2004 (GMP) | A | 0.12 | Yes | 0.41±0.19 |
| TR13⁵ | INXN-2004 (GMP) | A | 0.12 | Yes | 0.74±0.10 |

| | | | | | |
|---|---|---|---|---|---|
| ¹ Only indicated arms were super-transduced; all TR11, TR12.1, and TR13 arms were spinoculated ² TR8 Arm A data is presented as a comparator for previous results presented in RDEB-ADSO-2 ³ Control arm was mock-transduced and was not exposed to LV-COL7 ⁴ BLQ: below lower limit of quantitation ⁵ Arm B for both TR12.1 and TR13 was not carried through the full production process and not tested for LV-COL7 copy numbers | | | | | |

### 4.1.2. C7 Protein Expression

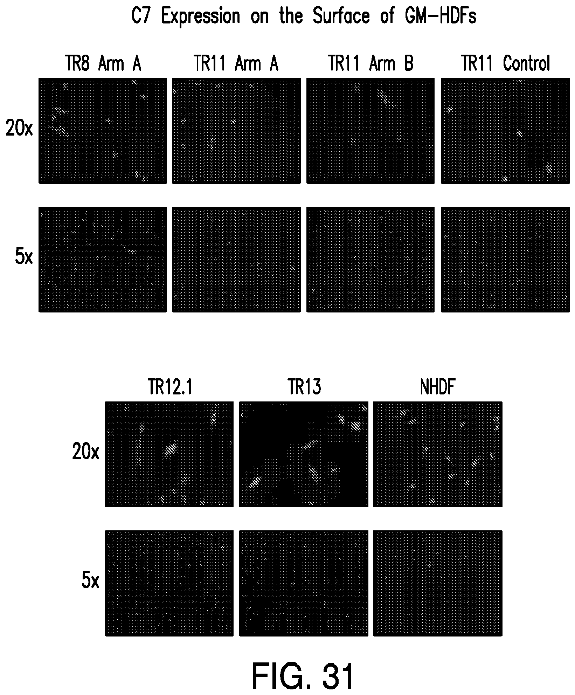

Indirect immunofluorescence (IF) was used to examine C7 protein expression by GM-HDF cells using an antibody specific for the C7. TR11 Arms A & B, and only Arm A of TR12.1 and TR13 were analyzed by IF. Normal human dermal fibroblasts (NHDFs) were used as a positive control and the control arm of TR11 was used as a negative control. Representative images at two magnifications, 20x and 5x are shown below in Figure 31. Also included as a comparator for the original process are IF images of cells from TR8 Arm A which had integrated LV-COL7 copy numbers of 0.015 per cell. Consistent with the copy number of integrated LV presented in Table 46 above, only a small number of TR11 GM-HDFs were visualized with C7 antibodies, though still greater than the number of C7-positive cells from TR8. Furthermore, the higher copy numbers measured from TR12.1 and TR13 GM-HDFs correlate to higher numbers of cells positive for C7 detection. Of note, the signal from the GM-HDFs that are C7-positive is brighter than the signal from NHDFs, suggesting greater amounts of C7 produced by GM-HDFs per cell.

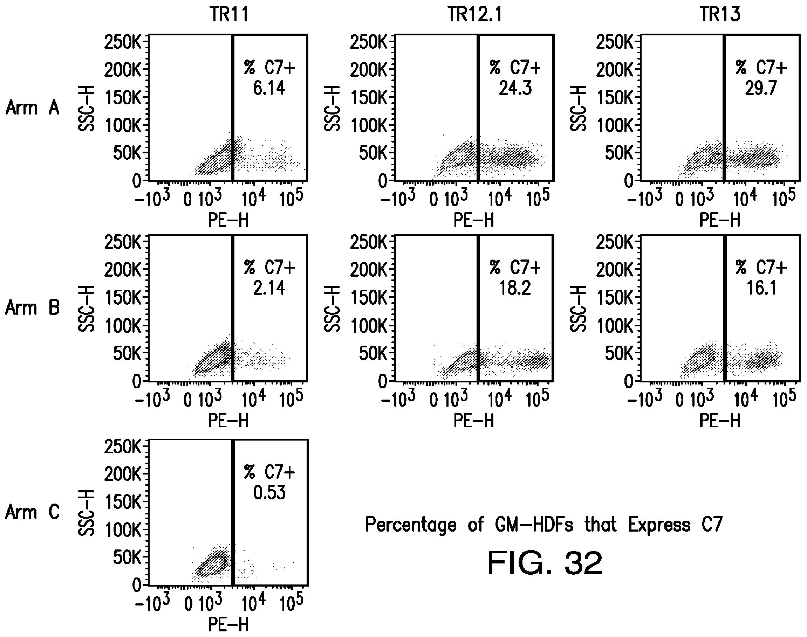

The specific number of cells expressing C7 was then quantified using a flow cytometry assay. As described in Section 2.3, this assay utilized an NC1 region-specific polyclonal antibody. For this experiment, Arm B cells from TR12.1 and TR13 were included to compare the single transduction procedure (Arm B) with the super-transduction procedure (Arm A) using second-generation LV-COL7 construct (INXN-2004). As shown in Figure 32 below, the percentage of C7-positive (C7+) cells for each training run arm is consistent with the qualitative number of cells expressing C7 as detected by IF (Figure 32), and correlates to the measured copy number (Table 46). Comparison of C7+ cells between Arm A and Arm B for TR12.1 and TR13 confirms the near doubling of expression levels that would be predicted by the addition of a second transduction.

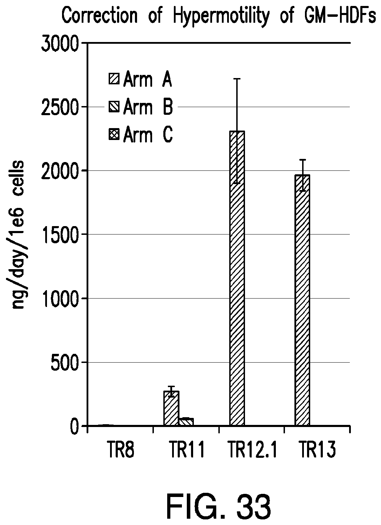

A direct ELISA was used to measure C7 secreted by GM-HDFs into the cell culture media. This assay, as described in Section 2.4, also utilized the NC1 region-specific polyclonal antibody. ELISA assay results of the GM-HDFs from the three training runs show that C7 expression is dependent on the level of integrated LV copy numbers with expression levels reaching approximately 2300 ng/day/1e6 cells (Figure 33). Compared to TR8 Arm A as a representative result from previous studies (RDEB-ADSO-2), this represents a >200-fold increase in C7 expression (Figure 33).

### 4.2. Functional Assessment of C7 Expressed by GM-HDFs

### 4.2.1. Formation of C7 Trimers

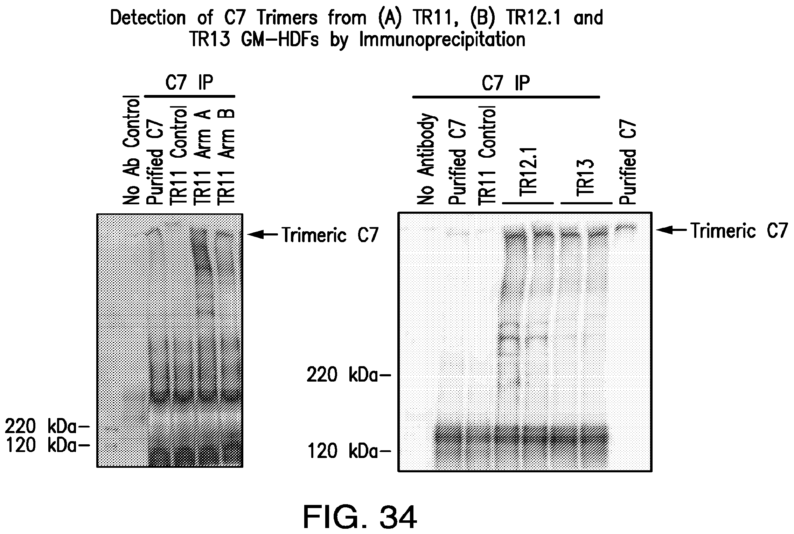

The formation of C7 trimers is essential in the assembly of anchoring fibrils (Bruckner-Tuderman, 1999). To verify that the C7 expressed by GM-HDFs will be capable of forming anchoring fibrils, GM-HDFs were assessed for the formation of C7 trimers using immunoprecipitation (IP) with an anti-C7 specific antibody (fNC1) for selective capture, then concentration of C7 from culture supernatants for detection by SDS-PAGE/immunoblot. Purified recombinant C7 was used as a positive control and the capture step without C7-specific antibody was used as a negative control. IP results for the three training runs showed that the C7 produced by the GM-HDFs was predominantly trimeric (Figure 34) with some lower molecular weight species showing immunoreactivity present, likely the dimeric and monomeric forms of C7.

### 4.2.2. Lam332 Binding by C7 Expressed from GM-HDFs

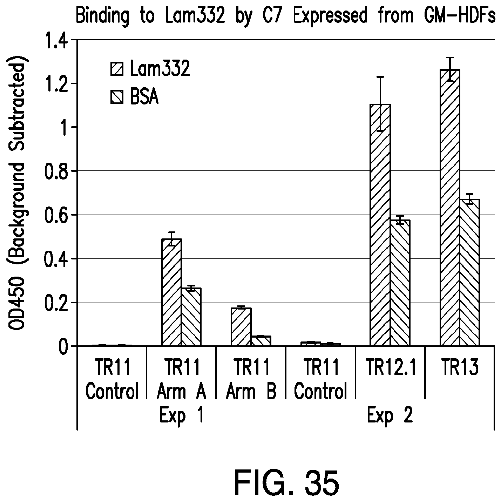

C7 has been shown to bind immobilized extracellular matrix (ECM) components, including fibronectin, Laminin 332 (Lam332), COL1, and COL4 (Chen, et al., 2002a). The interaction between C7 and Lam332 occurs through the NH2-terminal NC1 domain of C7 and is dependent upon the native conformation of both Lam332 and C7 NC1 (Rousselle, et al., 1997). The association between C7 and Lam332 is important for establishing correct Lam332 architecture at the dermal-epidermal junction. Such organization is critical for interactions with extracellular ligands and cell surface receptors, and for cell signaling (Waterman, et al., 2007). Intrexon developed an ELISA using an antibody against the C7 NC1 domain to detect binding of C7 to purified Lam332. Results from two experiments, presented in Figure 35, show copy number-dependent binding to Lam332 by C7 expressed by GM-HDFs from the three Training runs. TR11 Control cells were used as a negative control in both experiments.

### 4.2.3. Cell Migration Assay

Previous studies have shown that RDEB fibroblasts and keratinocytes show an increase in motility relative to their normal counterparts, and that normal motility can be restored by expression of C7 (Chen, et al., 2000; Chen, et al., 2002b; Cogan, et al. 2014; Baldeschi et al., 2003). Most of these studies employed either the colloidal gold salt migration assay to measure the migration of fibroblasts and keratinocytes, or a wound-healing assay to measure the migration of keratinocytes. In this Example a CytoSelect^{™} 24-well wound-healing assay kit (Cell Biolabs, Inc.) was used to measure the motility of NHDFs and GM-HDFs.

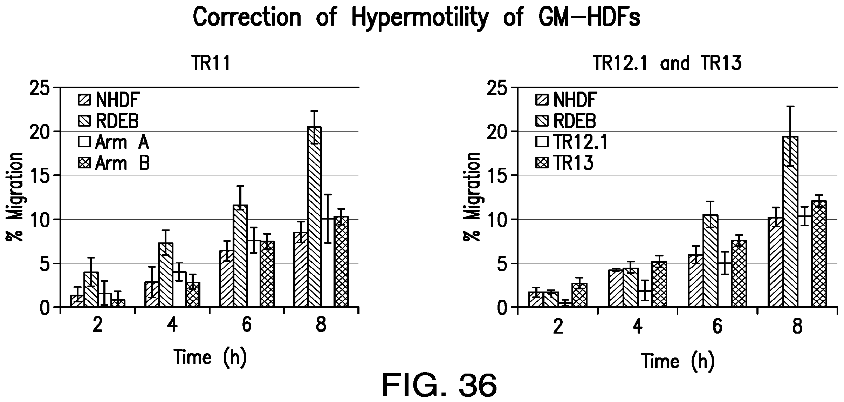

To determine whether the presence of C7 in the culture media is sufficient to reverse the RDEB hyper-motility phenotype, the wound healing assay was carried out on GM-HDFs from the three training runs, with NHDFs as a positive control and the mock-transduced Control (RDEB) arms as the negative control. Control fibroblasts from TR11 Arm C (RDEB) exhibited a hyper-motility phenotype that is reversed by the expression of C7 (Figure 36). Interestingly, the levels of phenotype reversal was not dependent upon the integrated copy number/C7 expression levels, suggesting that even low levels of C7 expression are enough to reverse the hyper-motility phenotype.

### 5. CONCLUSIONS

The integrated transgene copy number per cell in GM-HDFs was improved by both the addition of enhanced transduction methods (spinoculation and super-transduction) and a change to the LV-COL7 construct INXN-2004, with levels as high as 0.74 copies per cell achieved. The fold-improvements achieved with each process change are as follows:
- The addition of spinoculation: ≥2-fold
- The addition of super-transduction (with spinoculation): ~2-fold
- The change from INXN-2002 to INXN-2004: >4-fold
- The total cumulative change from original process: average of >27-fold (TR12.1 and TR13 compared to TR8 used in original studies)

Integrated copy number-dependent C7 expression from the GM-HDF cells was confirmed by immunofluorescence staining, flow cytometry, and ELISA. A >200-fold improvement in C7 expression was achieved relative to TR8 results presented in IND submission 16582 Serial 0000.

The structure of the C7 expressed by the GM-HDF cells was confirmed to be predominantly trimeric by immunoprecipitation/SDS-PAGE/immunoblot analysis.

In addition, the C7 produced from the GM-HDF cells was confirmed to be functional with binding to Laminin332 *in vitro* and by correction of the hypermotility phenotype of control RDEB cells in an *in vitro* migration assay.

### APPENDIX 4

| | |
|---|---|
| *COL7A1* Genbank | |
| *COL7A1* Genbank | |
| *COL7A1* Genbank | |
| *COL7A1* Genbank | |
| *COL7A1* Genbank | |
| *COL7A1* Genbank | |
| *COL7A1* Genbank | |
| *COL7A1* Genbank | |
| *COL7A1* Genbank | |
| *COL7A1* Genbank | |
| *COL7A1* Genbank | |
| *COL7A1* Genbank | |
| *COL7A1* Genbank | |
| *COL7A1* Genbank | |
| *COL7A1* Genbank | |
| *COL7A1* Genbank | |
| *COL7A1* Genbank | |
| *COL7A1* Genbank | |
| *COL7A1* Genbank | |
| *COL7A1* Genbank | |
| *COL7A1* Genbank | |
| *COL7A1* Genbank | |
| *COL7A1* Genbank | |
| *COL7A1* Genbank | |
| *COL7A1* Genbank | |
| *COL7A1* Genbank | |
| *COL7A1* Genbank | |
| *COL7A1* Genbank | |
| *COL7A1* Genbank | |
| *COL7A1* Genbank | |
| *COL7A1* Genbank | |
| *COL7A1* Genbank | |
| *COL7A1* Genbank | |
| *COL7A1* Genbank | |
| *COL7A1* Genbank | |
| *COL7A1* Genbank | |
| *COL7A1* Genbank | |
| *COL7A1* Genbank | |
| *COL7A1* Genbank | |
| *COL7A1* Genbank | |
| *COL7A1* Genbank | |
| *COL7A1* Genbank | |
| *COL7A1* Genbank | |
| *COL7A1* Genbank | |
| *COL7A1* Genbank | |
| *COL7A1* Genbank | |
| *COL7A1* Genbank | |
| | |
| *COL7A1* Genbank | |
| *COL7A1* Genbank | |
| *COL7A1* Genbank | |
| *COL7A1* Genbank | |
| *COL7A1* Genbank | |
| *COL7A1* Genbank | |
| *COL7A1* Genbank | |
| *COL7A1* Genbank | |
| *COL7A1* Genbank | |
| *COL7A1* Genbank | |
| *COL7A1* Genbank | |
| *COL7A1* Genbank | |
| *COL7A1* Genbank | |
| *COL7A1* Genbank | |
| *COL7A1* Genbank | |
| *COL7A1* Genbank | |
| *COL7A1* Genbank | |
| *COL7A1* Genbank | |
| *COL7A1* Genbank | |
| *COL7A1* Genbank | |
| *COL7A1* Genbank | |
| *COL7A1* Genbank | |
| *COL7A1* Genbank | |
| *COL7A1* Genbank | |
| *COL7A1* Genbank | |
| *COL7A1* Genbank | |
| *COL7A1* Genbank | |
| *COL7A1* Genbank | |
| *COL7A1* Genbank | |
| *COL7A1* Genbank | |
| *COL7A1* Genbank | |
| *COL7A1* Genbank | |
| *COL7A1* Genbank | |
| *COL7A1* Genbank | |
| *COL7A1* Genbank | |
| *COL7A1* Genbank | |
| *COL7A1* Genbank | |
| *COL7A1* Genbank | |
| *COL7A1* Genbank | |
| *COL7A1* Genbank | |
| *COL7A1* Genbank | |
| *COL7A1* Genbank | |
| *COL7A1* Genbank | |
| *COL7A1* Genbank | |
| *COL7A1* Genbank | |
| *COL7A1* Genbank | |
| *COL7A1* Genbank | |
| *COL7A1* Genbank | |
| *COL7A1* Genbank | |
| *COL7A1* Genbank | |
| *COL7A1* Genbank | |
| *COL7A1* Genbank | |
| *COL7A1* Genbank | |
| *COL7A1* Genbank | |
| *COL7A1* Genbank | |
| *COL7A1* Genbank | |
| *COL7A1* Genbank | |
| *COL7A1* Genbank | |
| *COL7A1* Genbank | |
| *COL7A1* Genbank | |
| *COL7A1* Genbank | |
| *COL7A1* Genbank | |
| *COL7A1* Genbank | |
| *COL7A1* Genbank | |
| *COL7A1* Genbank | |
| *COL7A1* Genbank | |
| *COL7A1* Genbank | |
| *COL7A1* Genbank | |
| *COL7A1* Genbank | |
| *COL7A1* Genbank | |
| *COL7A1* Genbank | |
| *COL7A1* Genbank | |
| *COL7A1* Genbank | |
| *COL7A1* Genbank | |
| *COL7A1* Genbank | |
| *COL7A1* Genbank | |
| *COL7A1* Genbank | |
| *COL7A1* Genbank | |
| | |
| *COL7A1* Genbank | |
| *COL7A1* Genbank | |
| *COL7A1* Genbank | |
| *COL7A1* Genbank | |
| *COL7A1* Genbank | |
| *COL7A1* Genbank | |
| *COL7A1* Genbank | |
| *COL7A1* Genbank | |
| *COL7A1* Genbank | |
| *COL7A1* Genbank | |
| *COL7A1* Genbank | |
| *COL7A1* Genbank | |
| *COL7A1* Genbank | |
| *COL7A1* Genbank | |
| *COL7A1* Genbank | |
| *COL7A1* | |
| Genbank | |
| *COL7A1* Genbank | |
| *COL7A1* Genbank | |
| *COL7A1* Genbank | |
| *COL7A1* Genbank | |
| *COL7A1* Genbank | |
| *COL7A1* Genbank | |
| *COL7A1* Genbank | ACTGCCCAGGAC 8832 (SEQ ID NO: 23) |
| | ACTGCCCAGGAC 8832 (SEQ ID NO: 24) |
| | ************ |

The following is a comparative analysis of 60010743-ITX-00001_CONTIG SEQUENCE (SEQ ID NO:34) relative to IGE308_REFSEQUENCE (SEQ ID NO: 34).

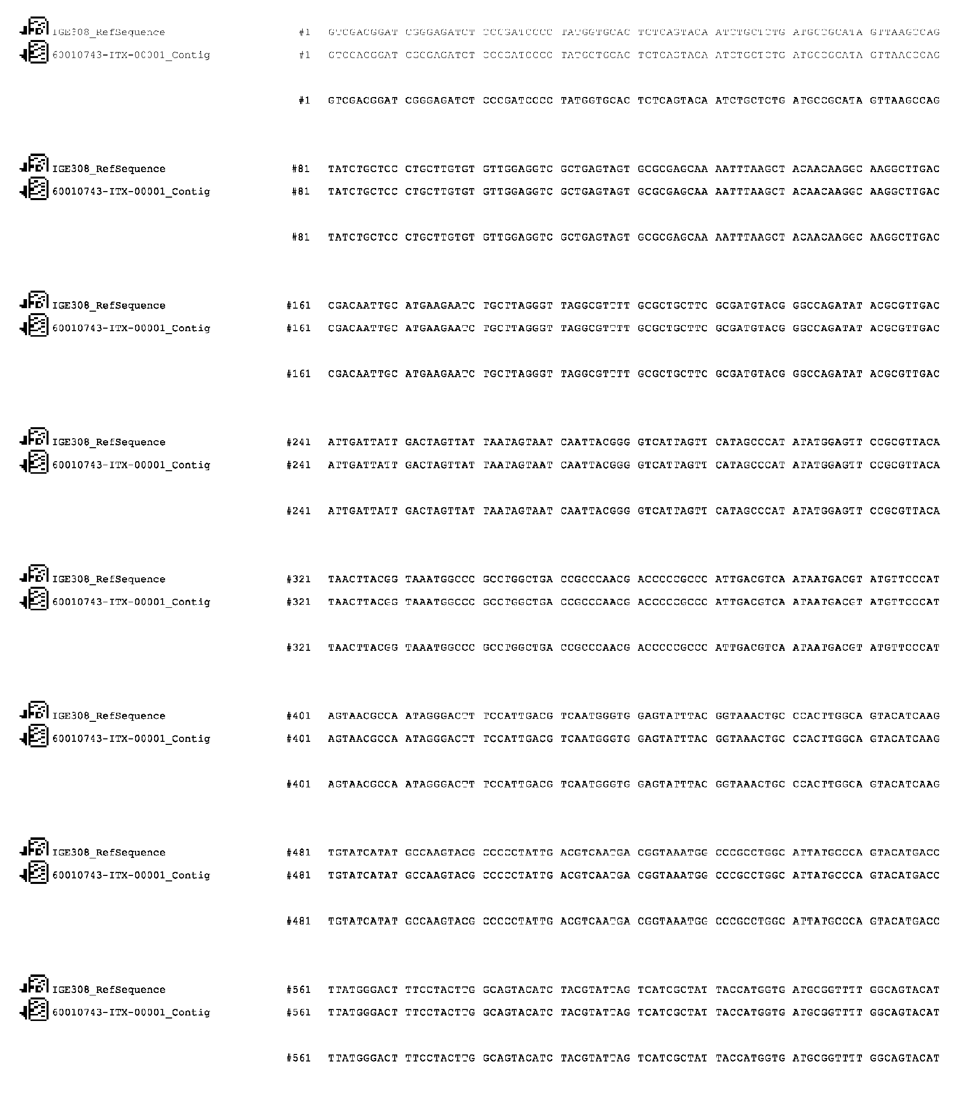

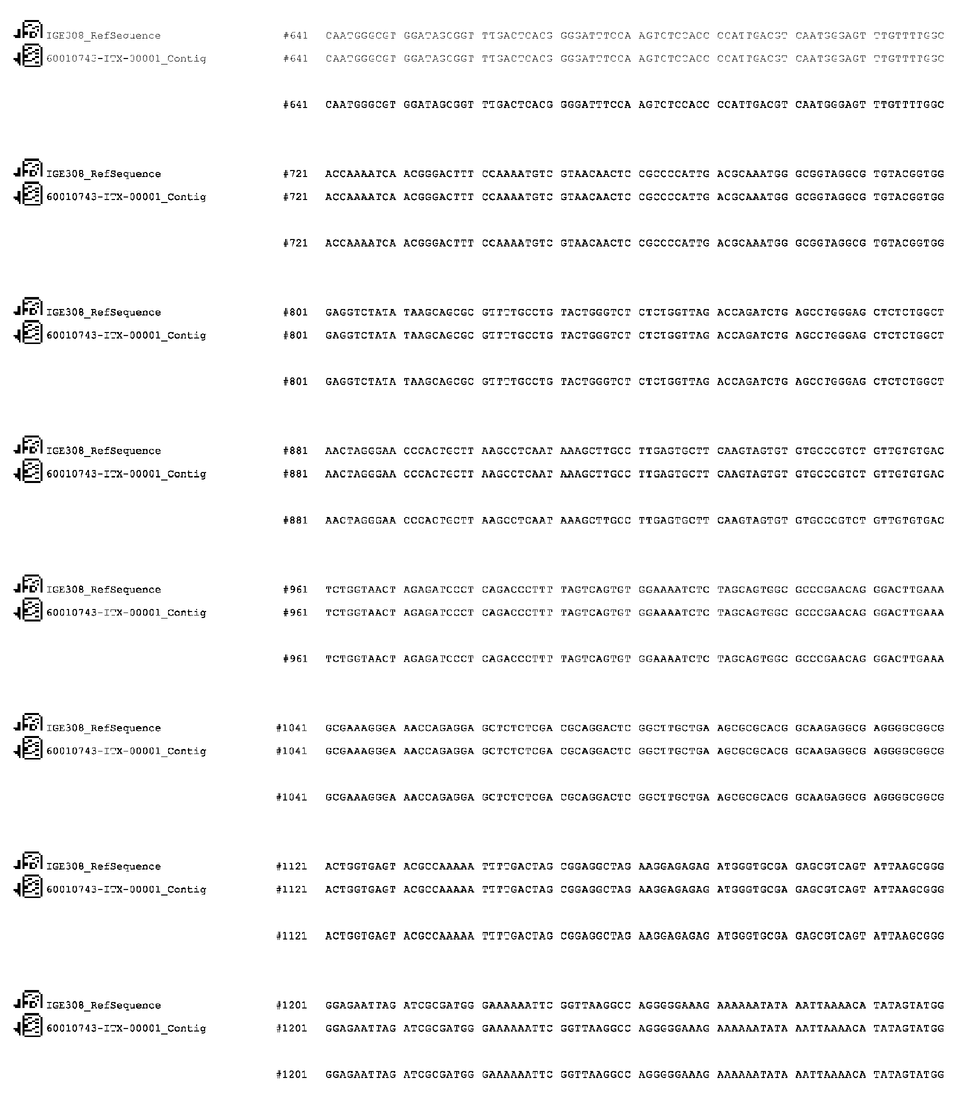

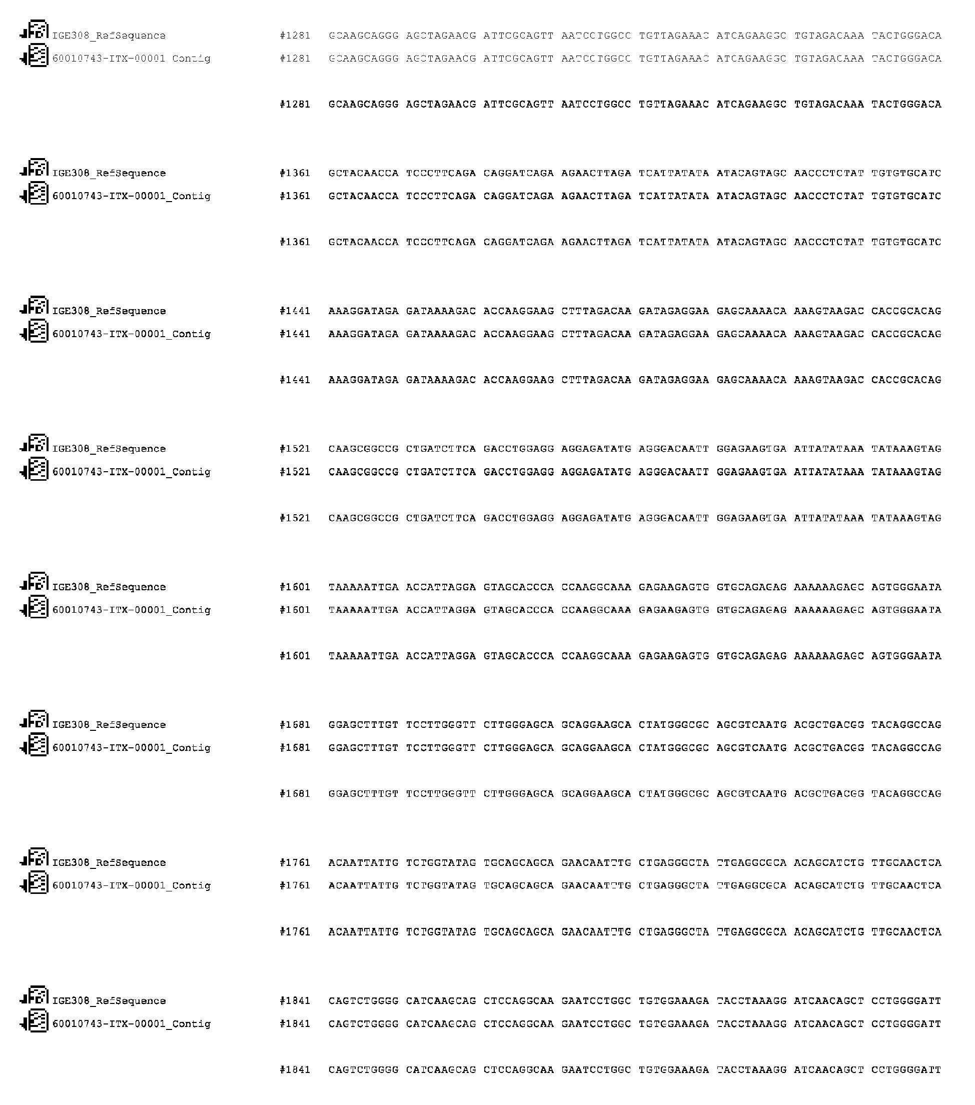

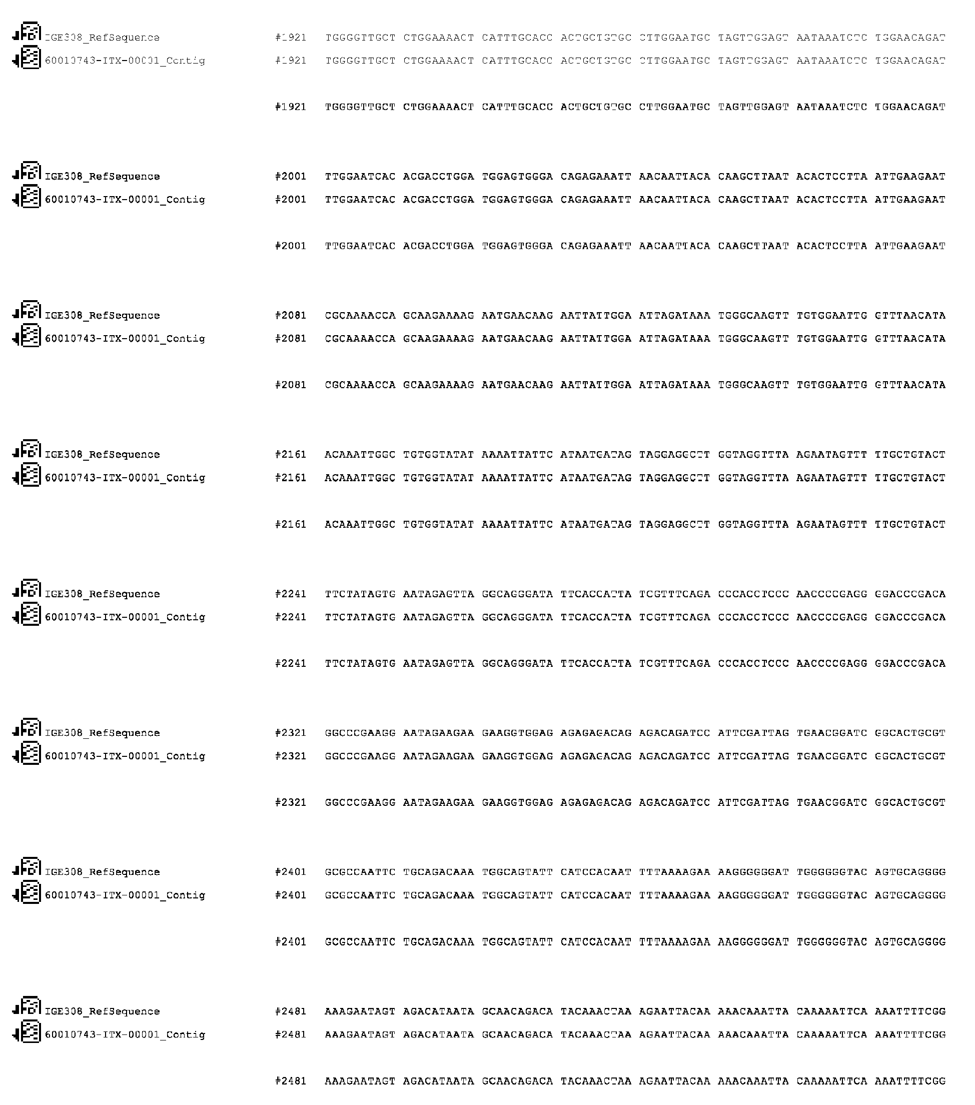

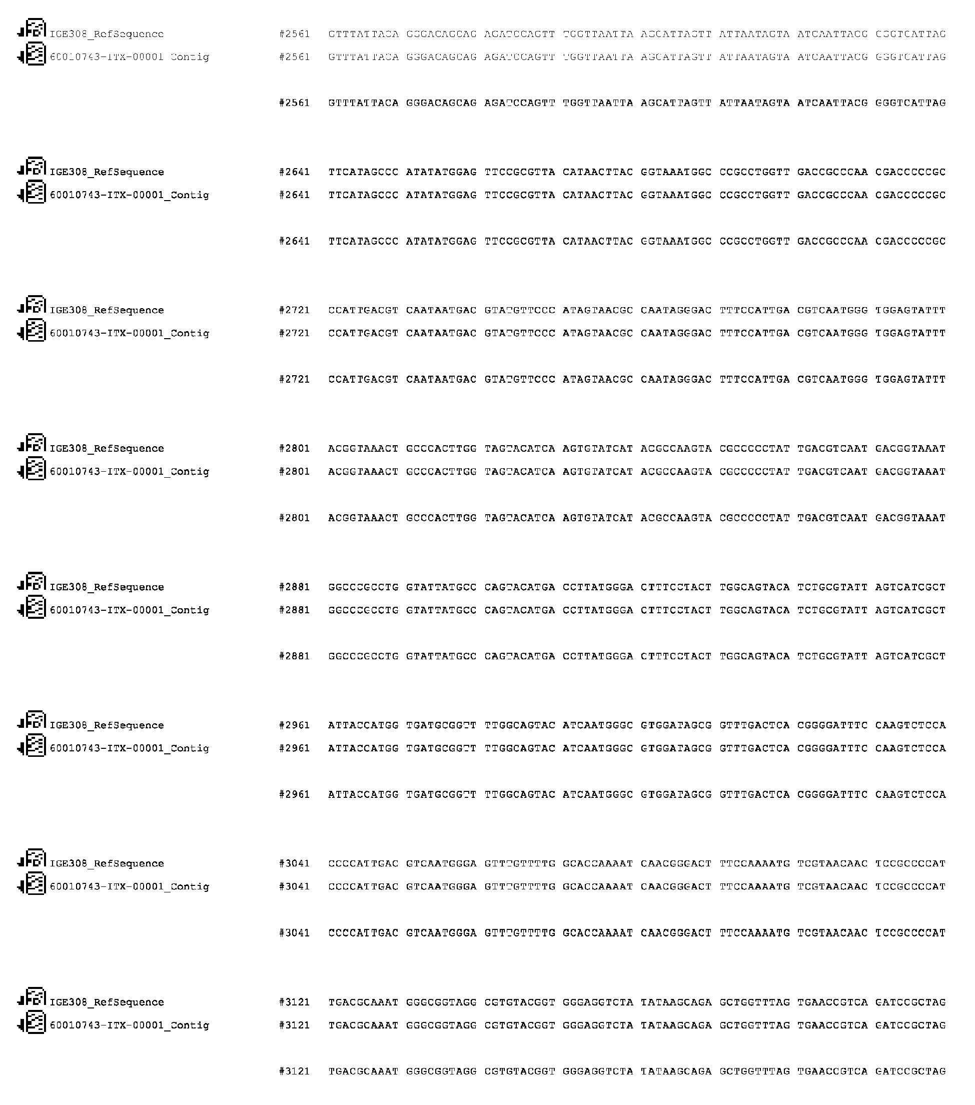

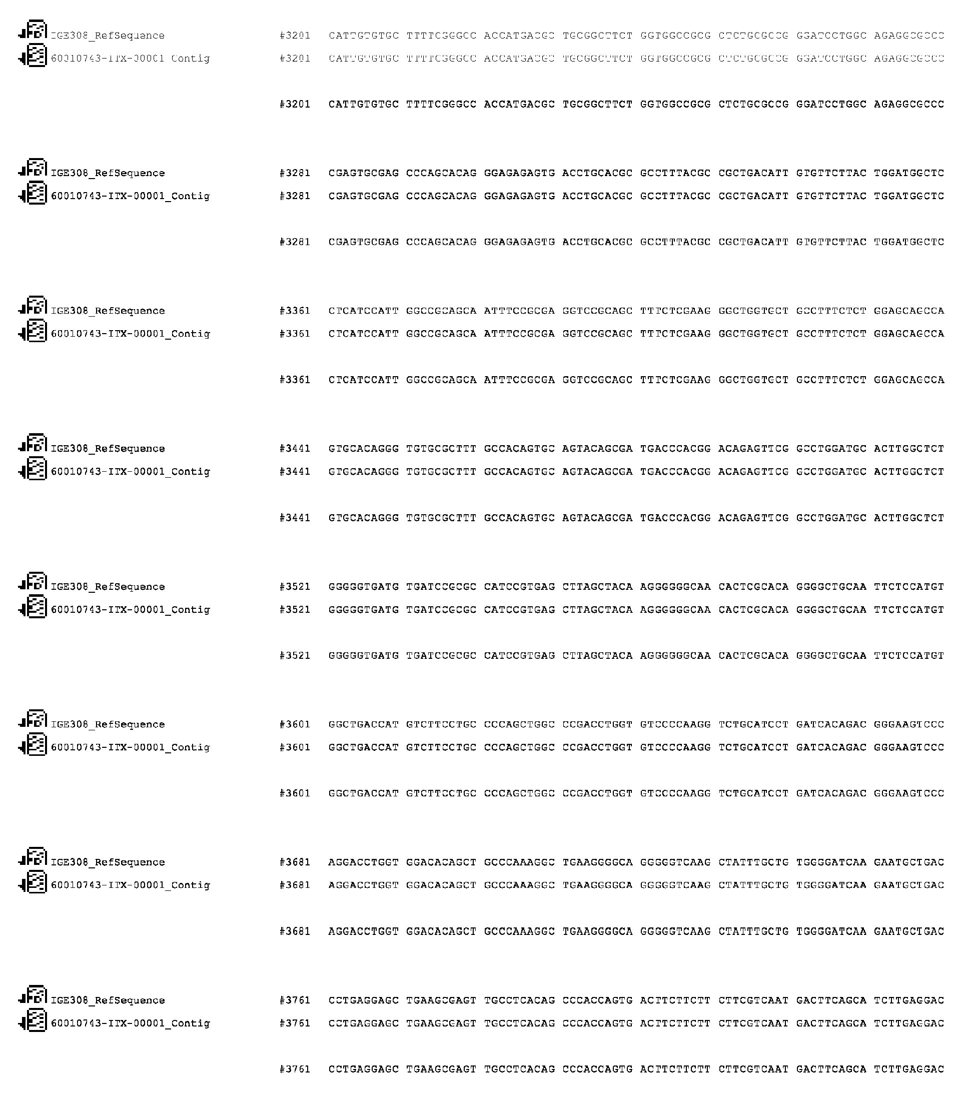

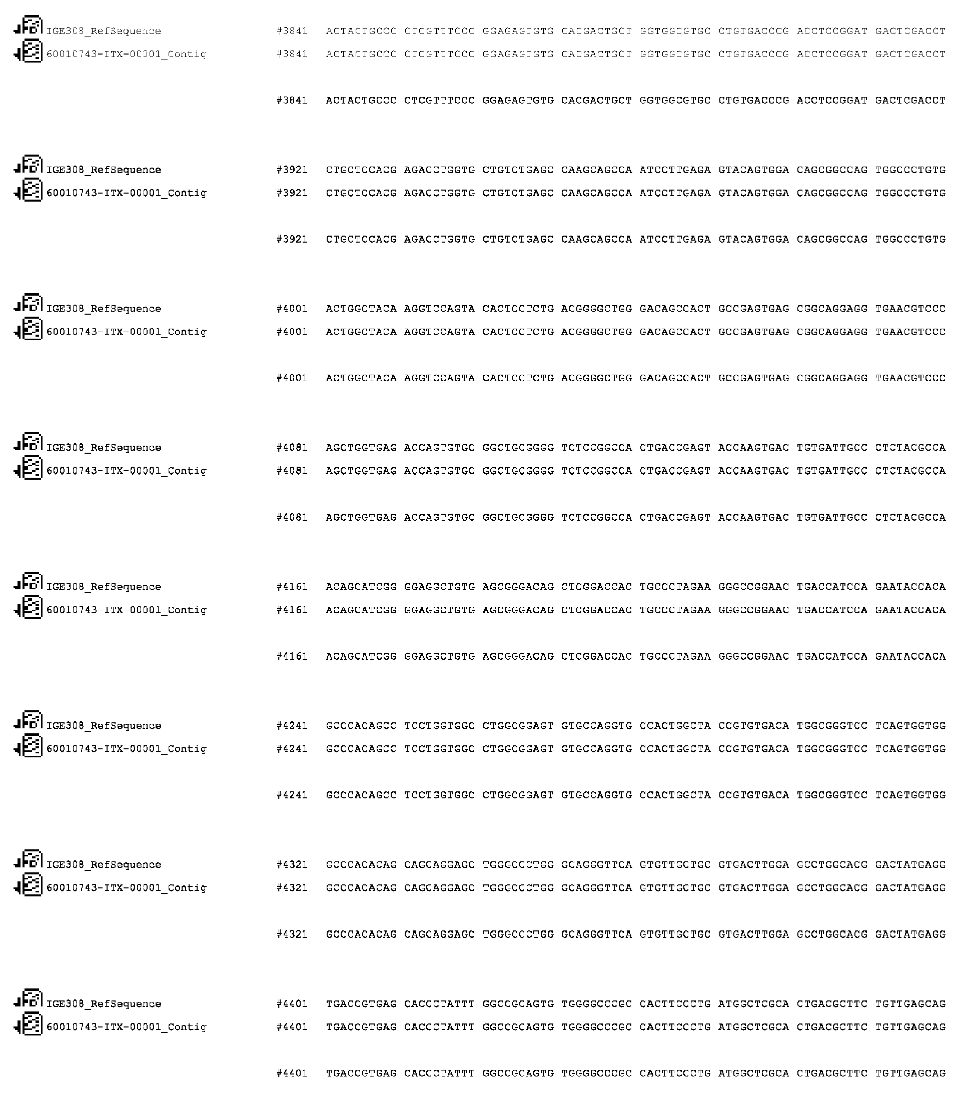

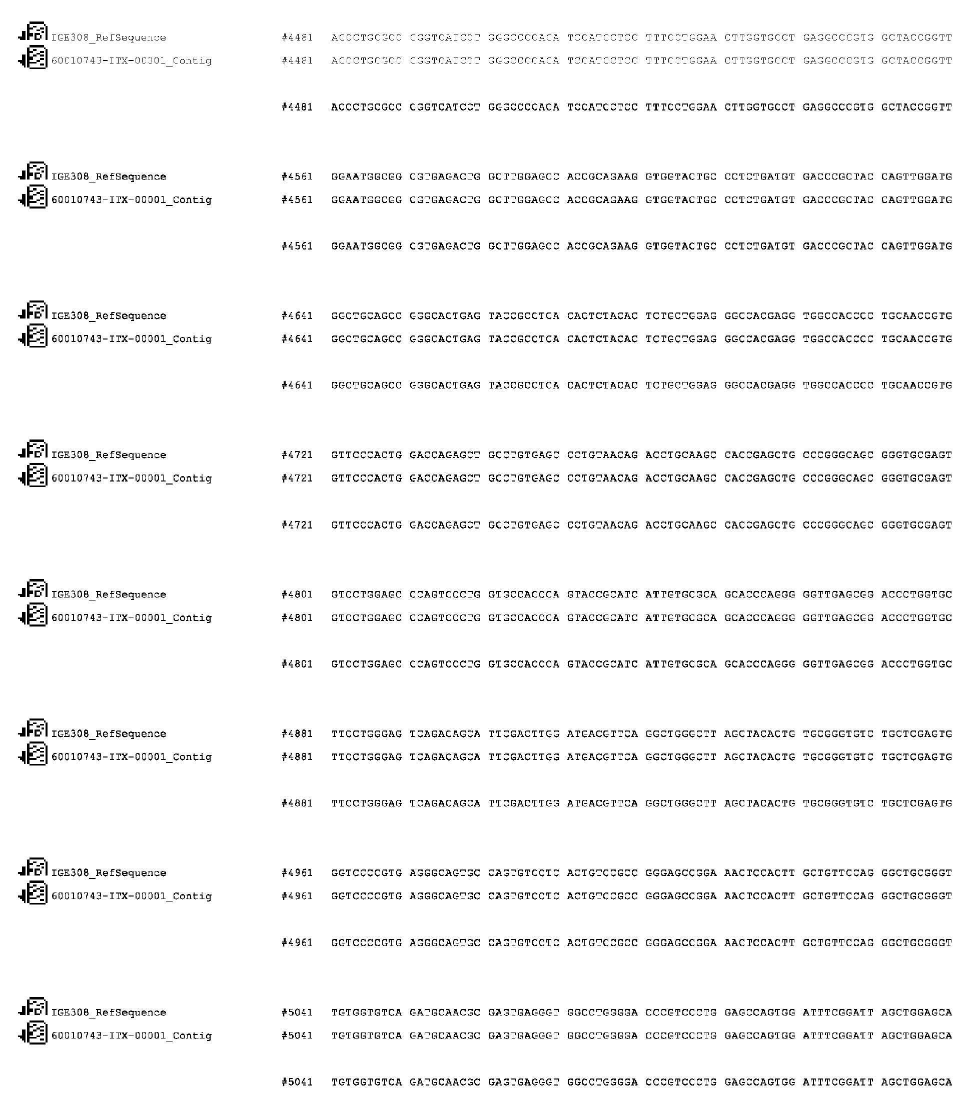

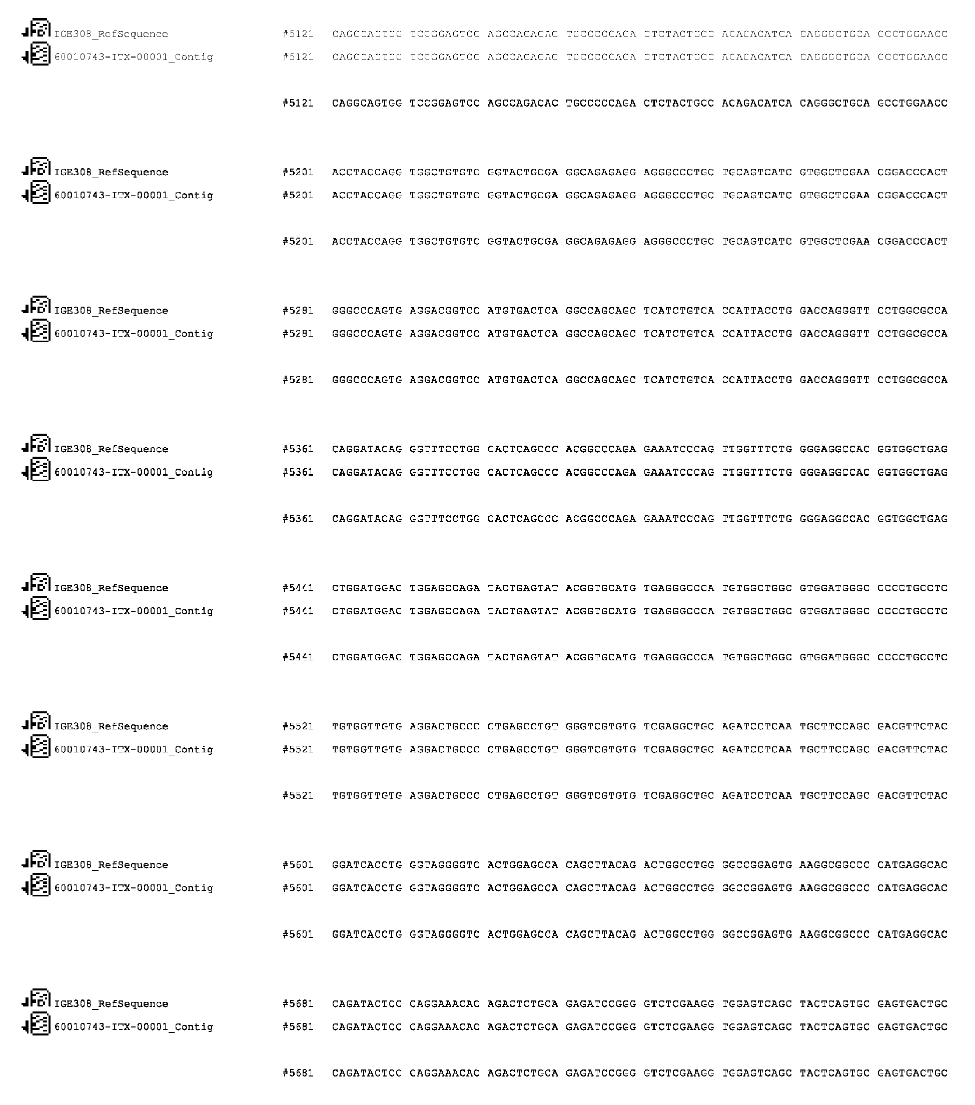

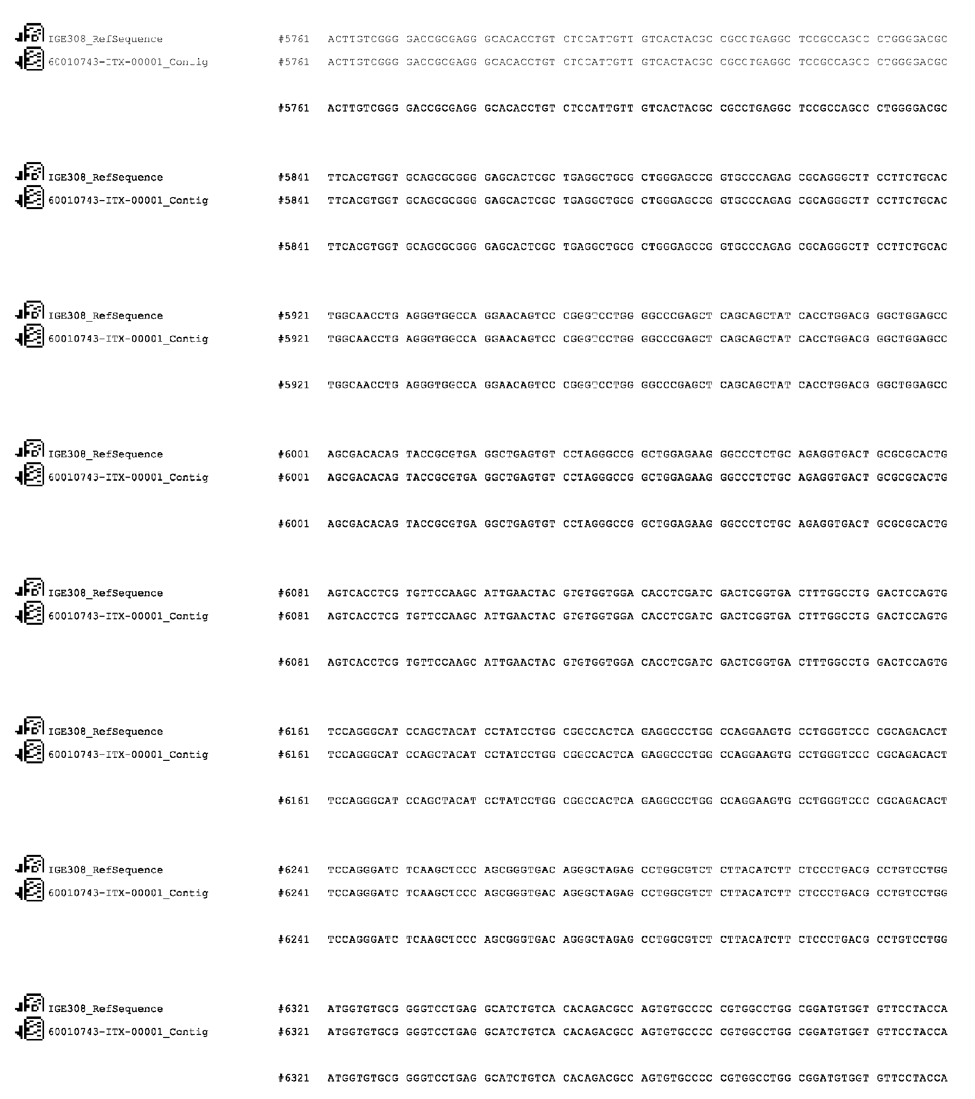

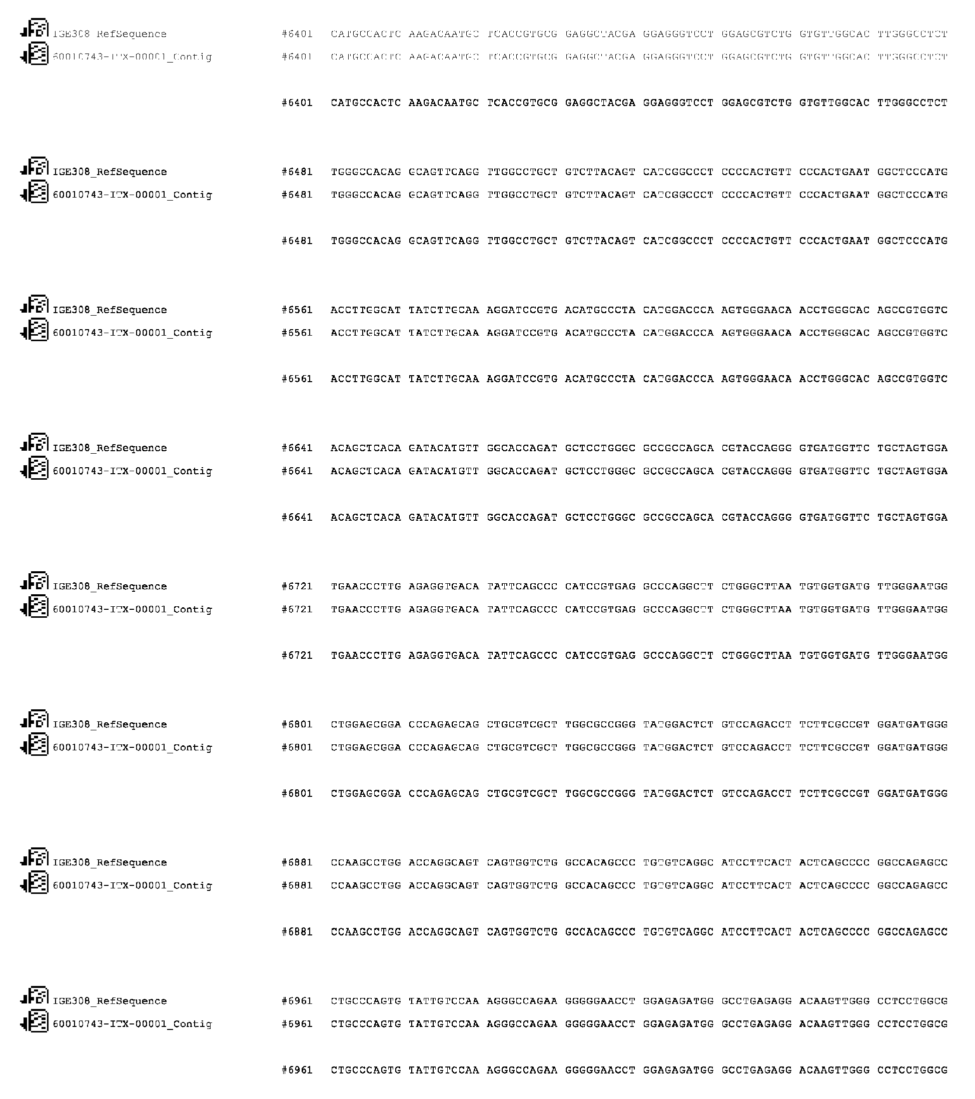

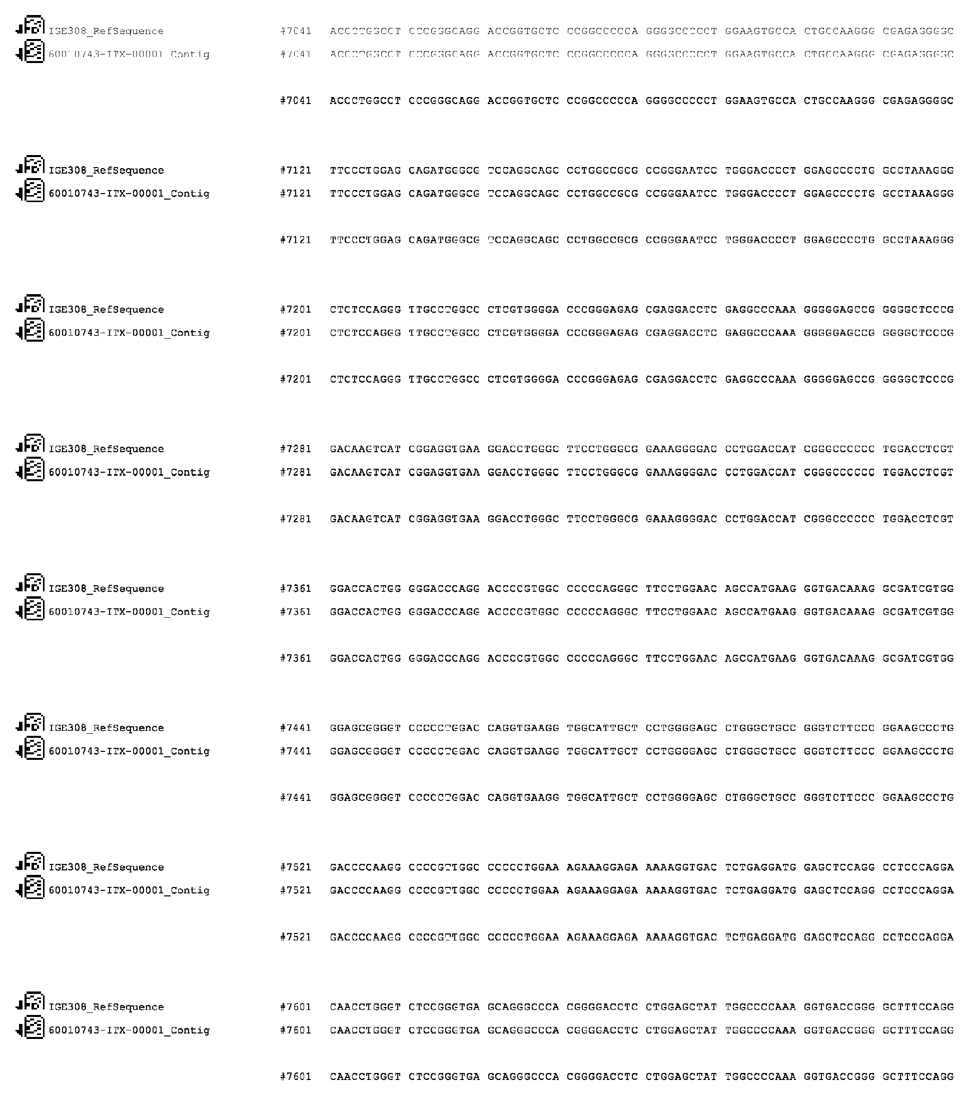

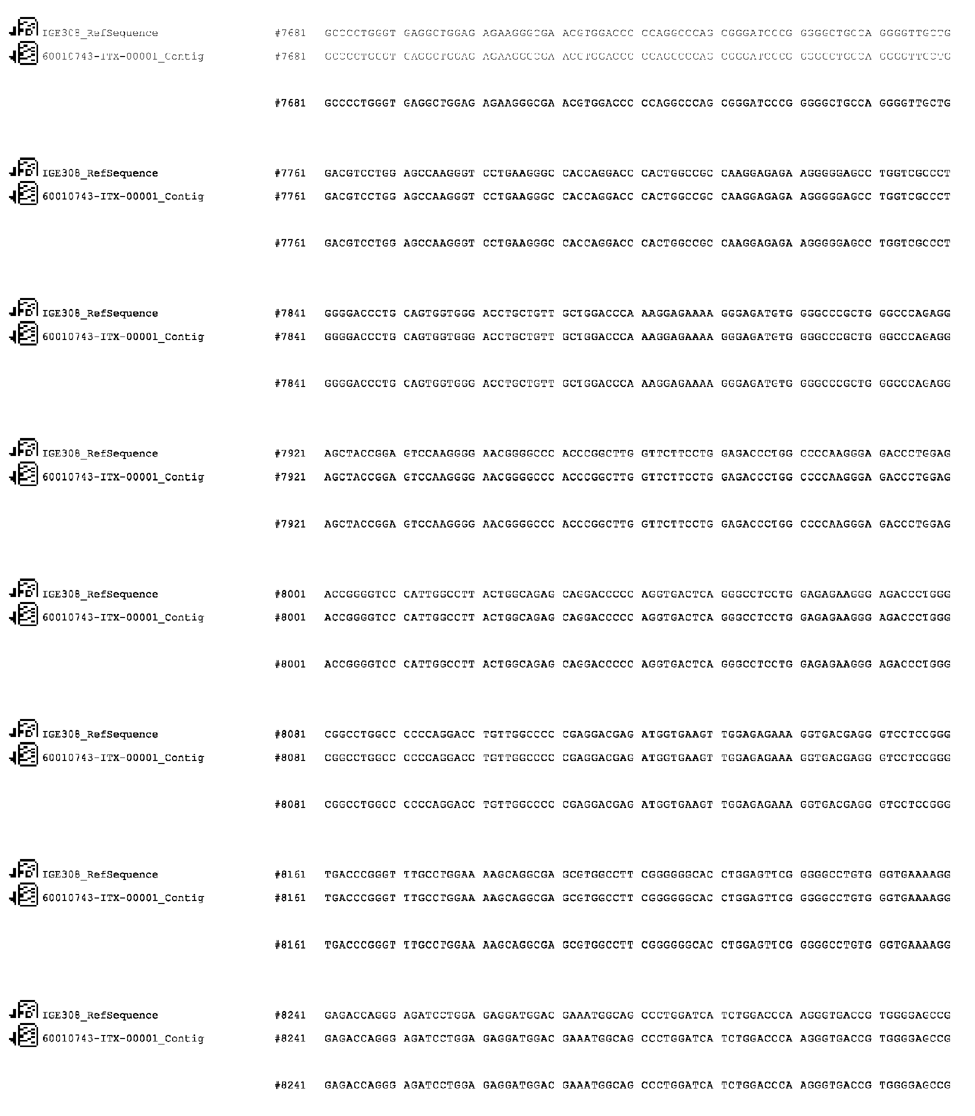

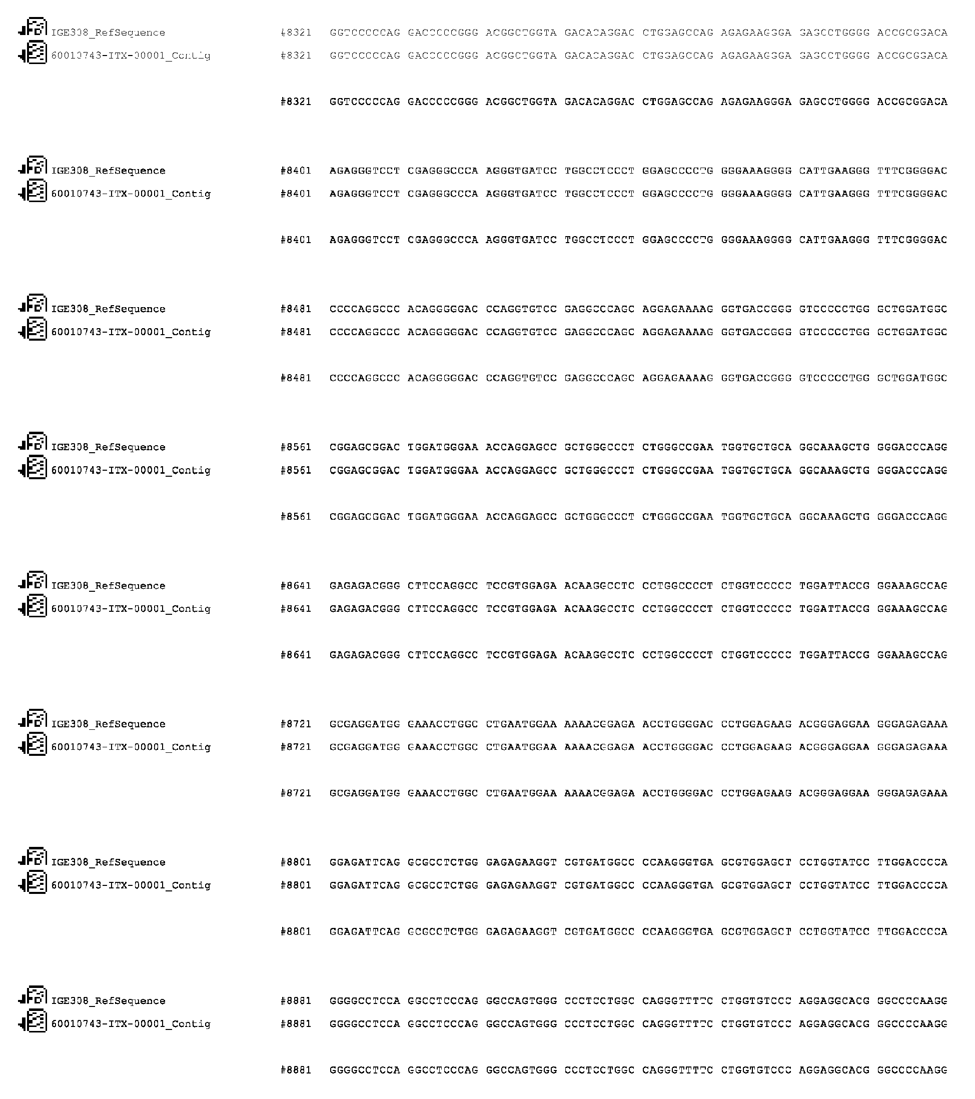

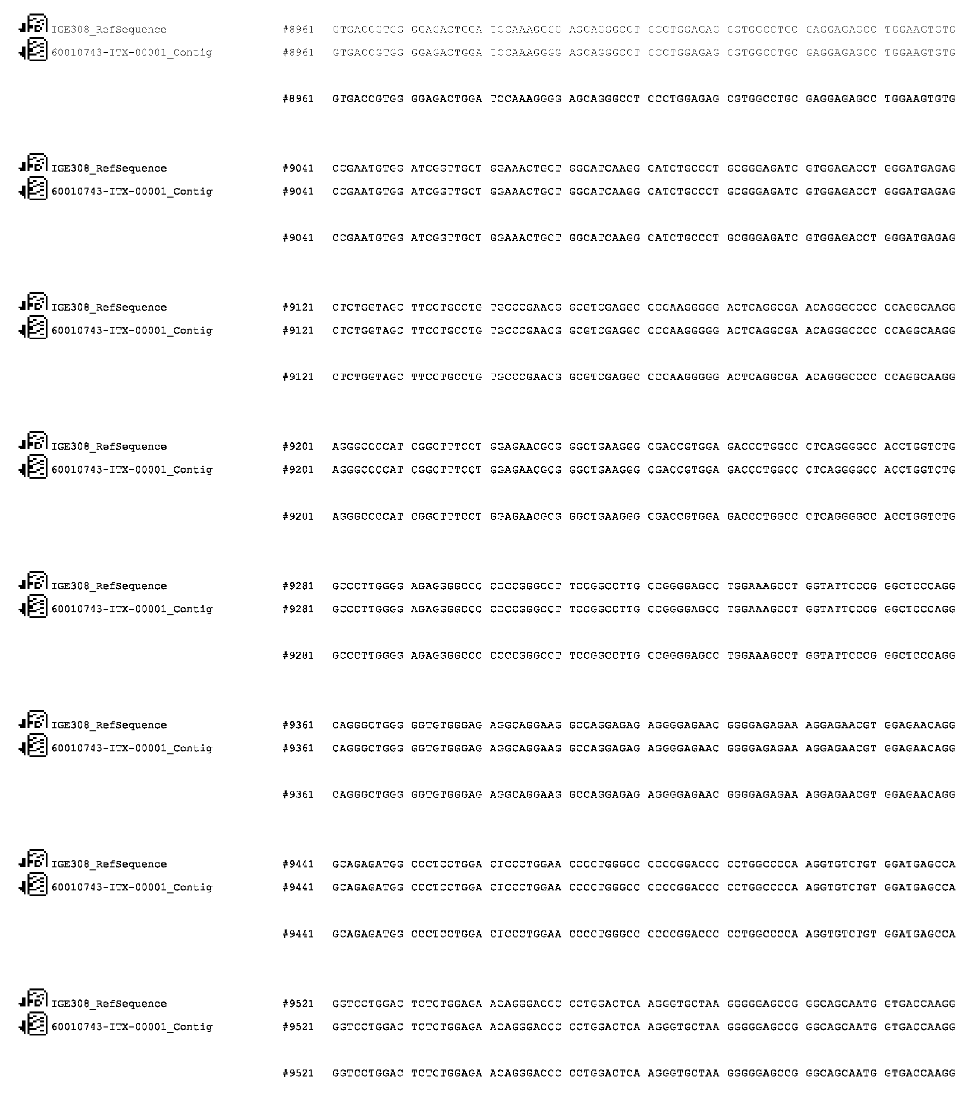

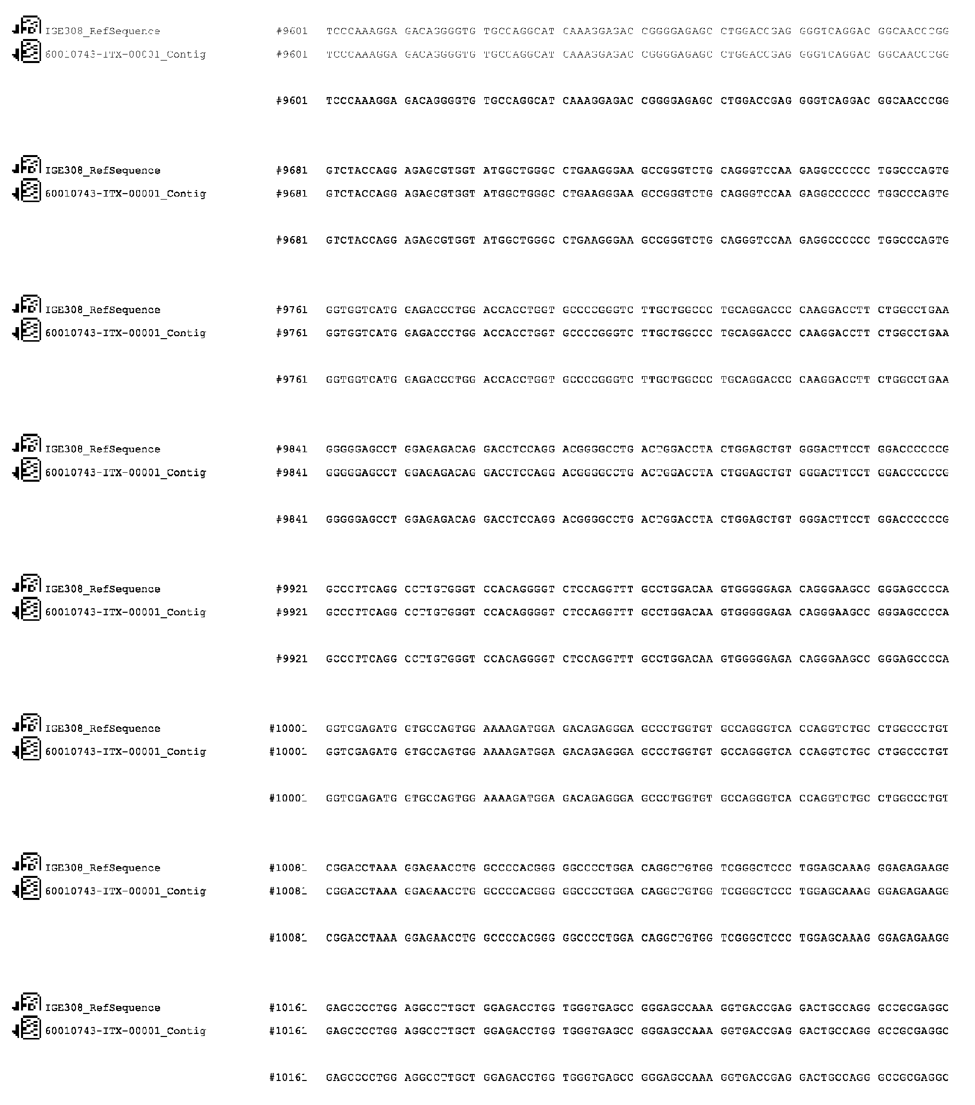

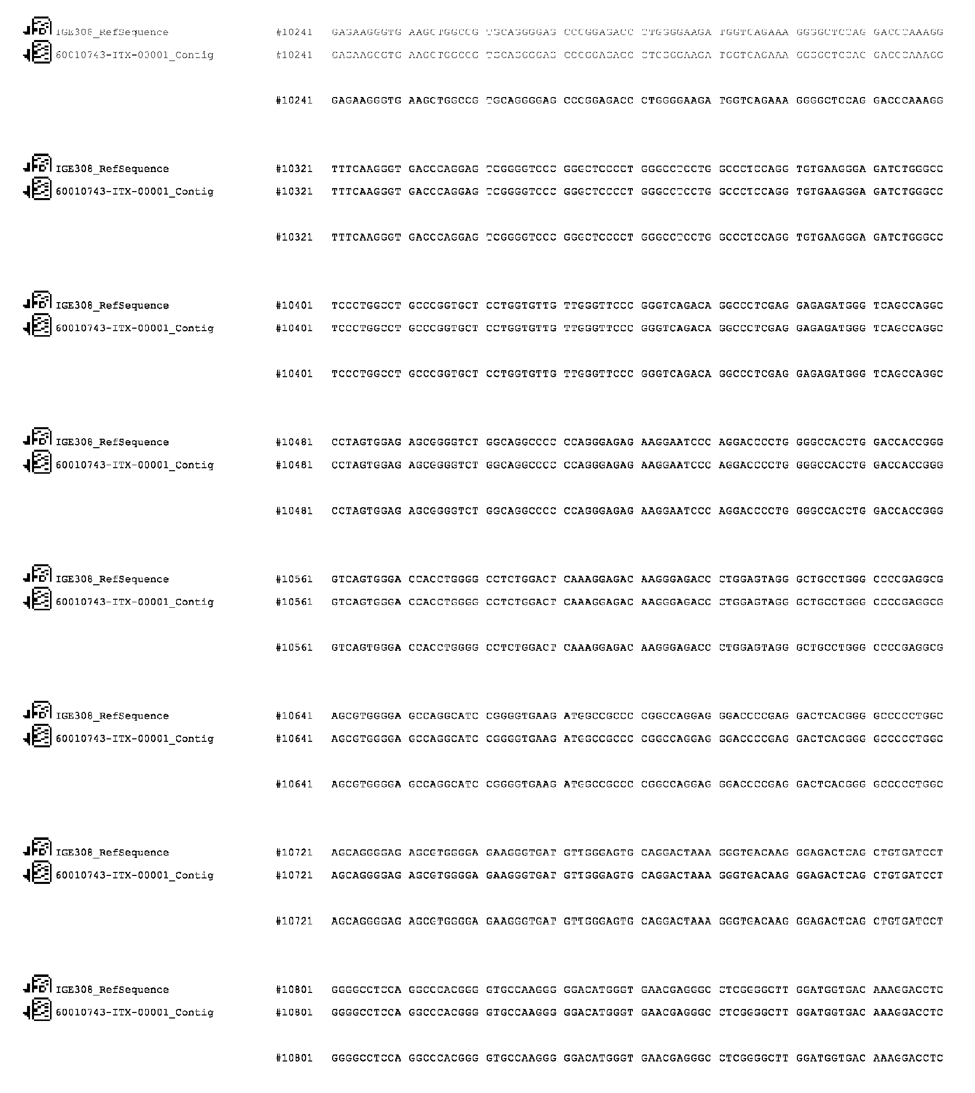

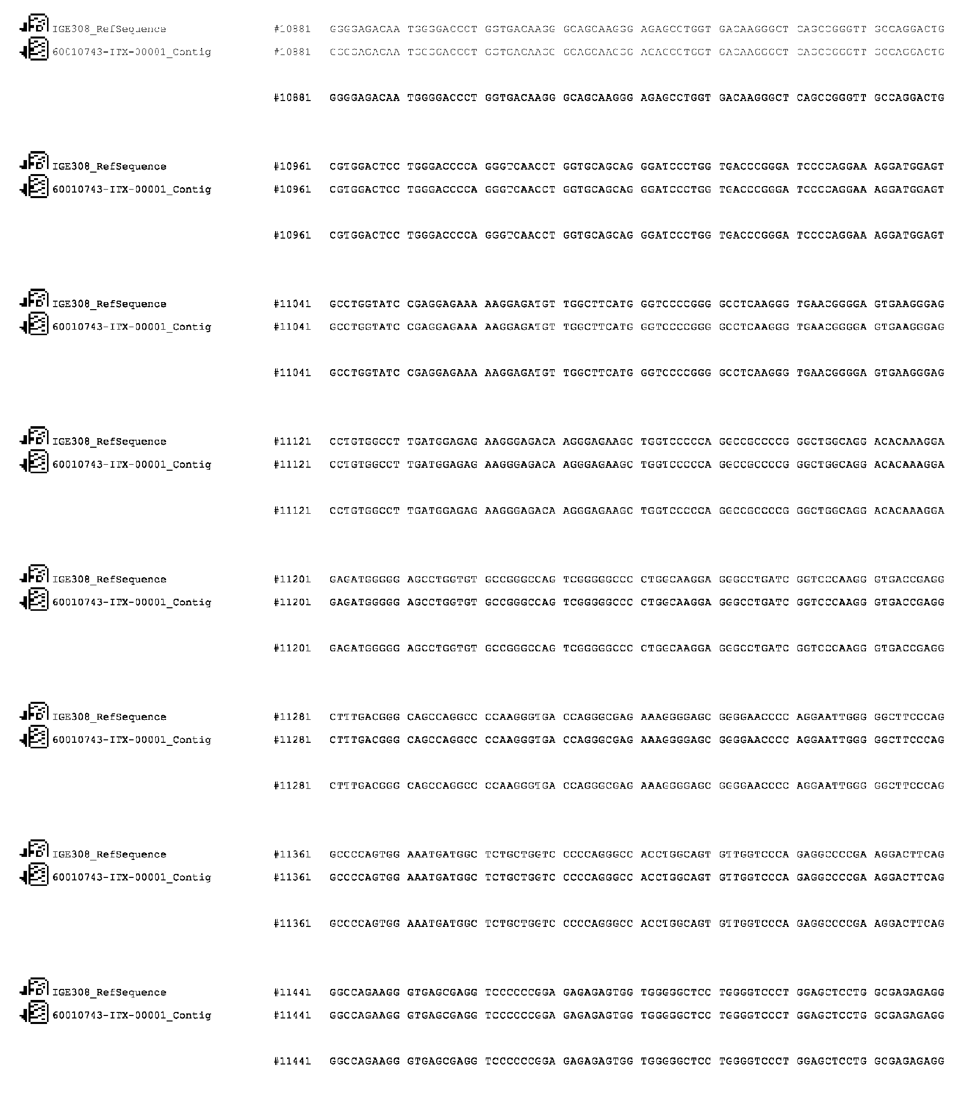

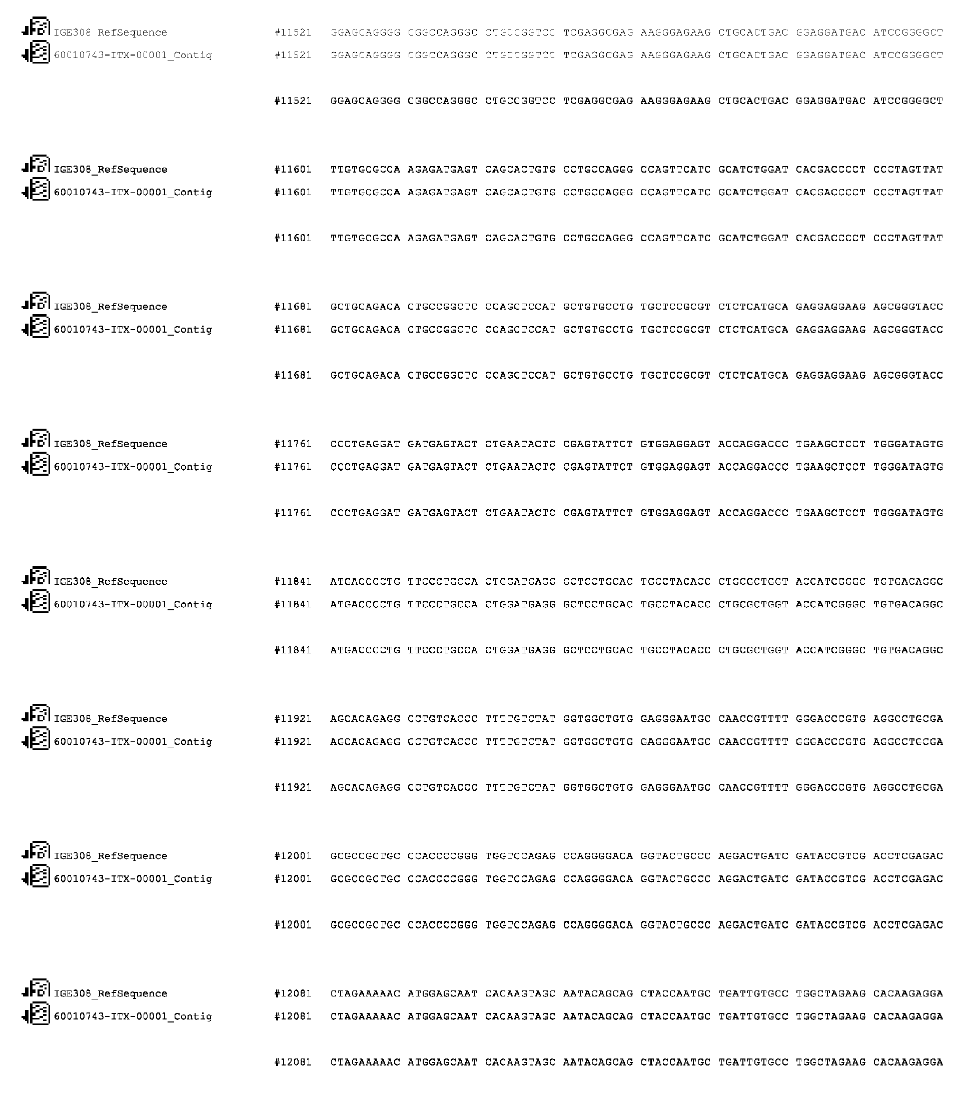

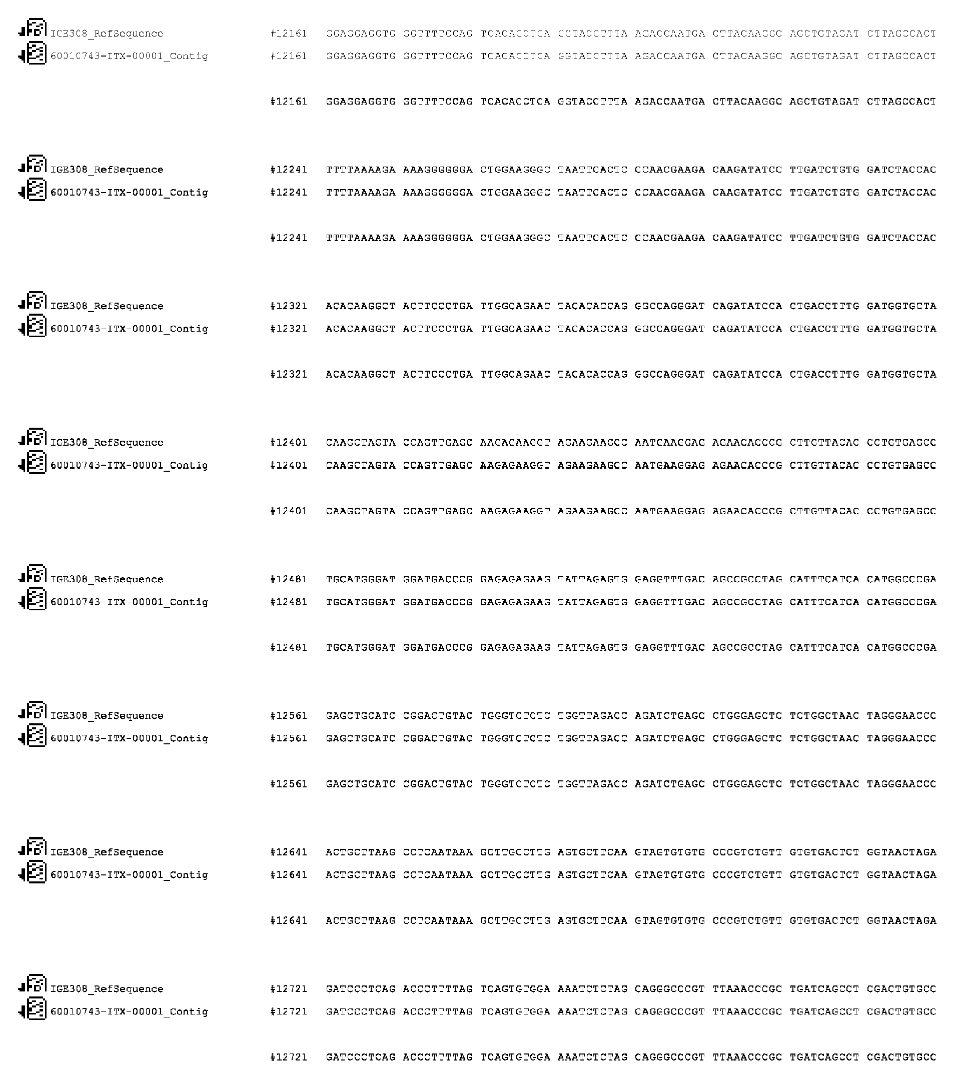

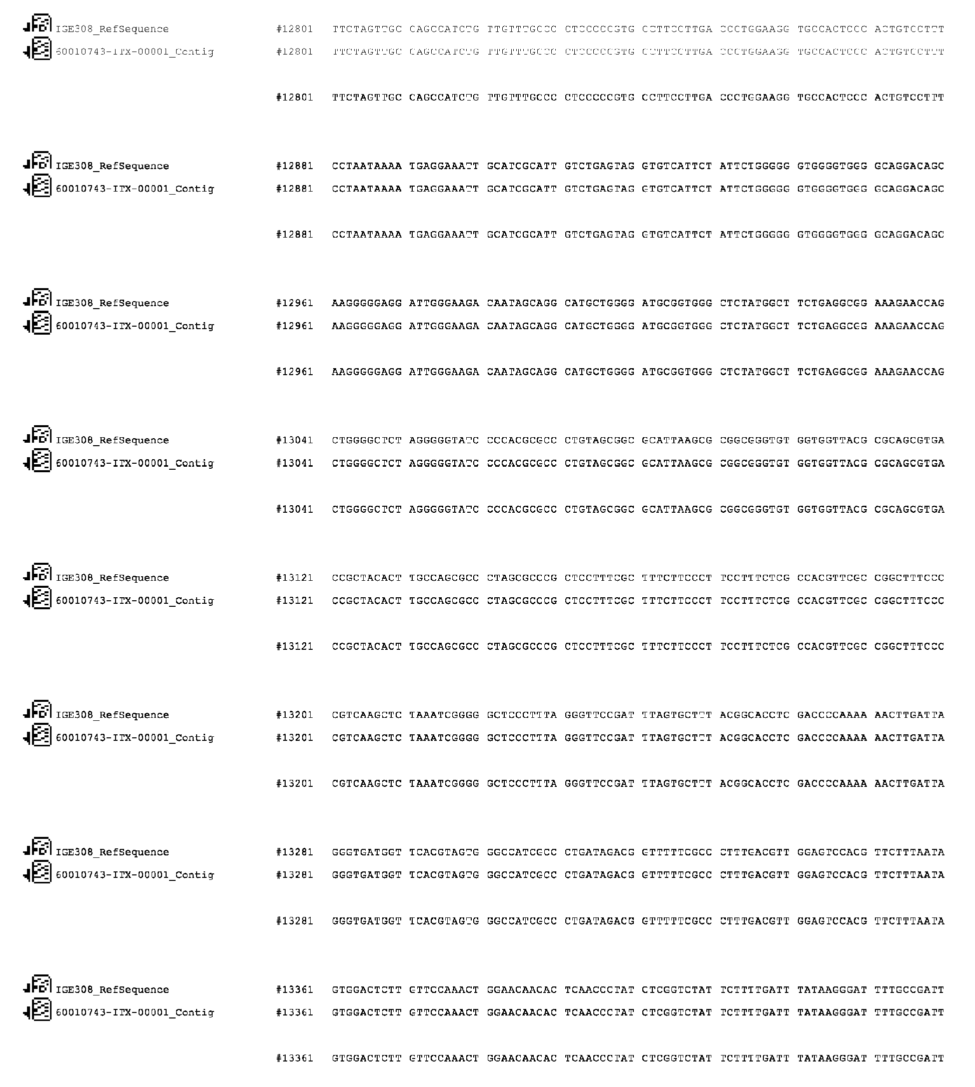

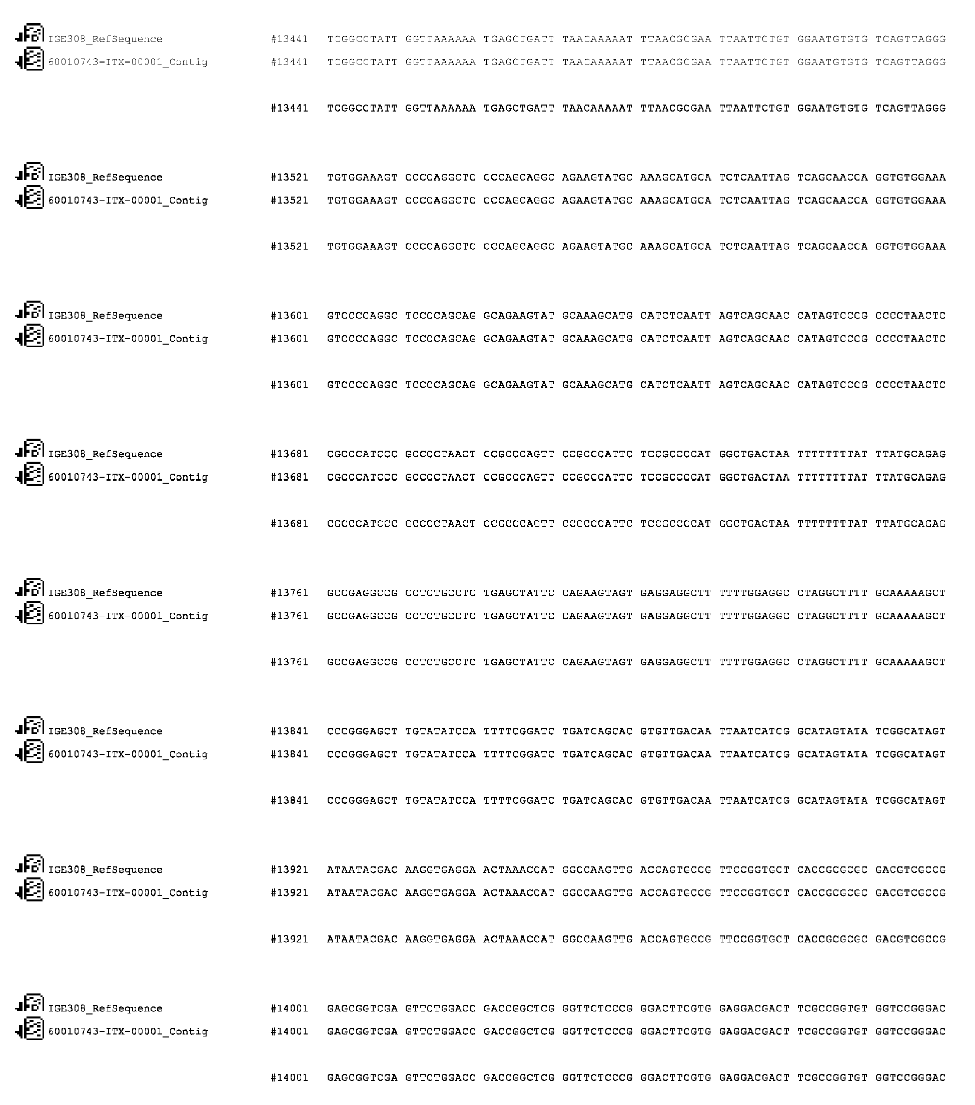

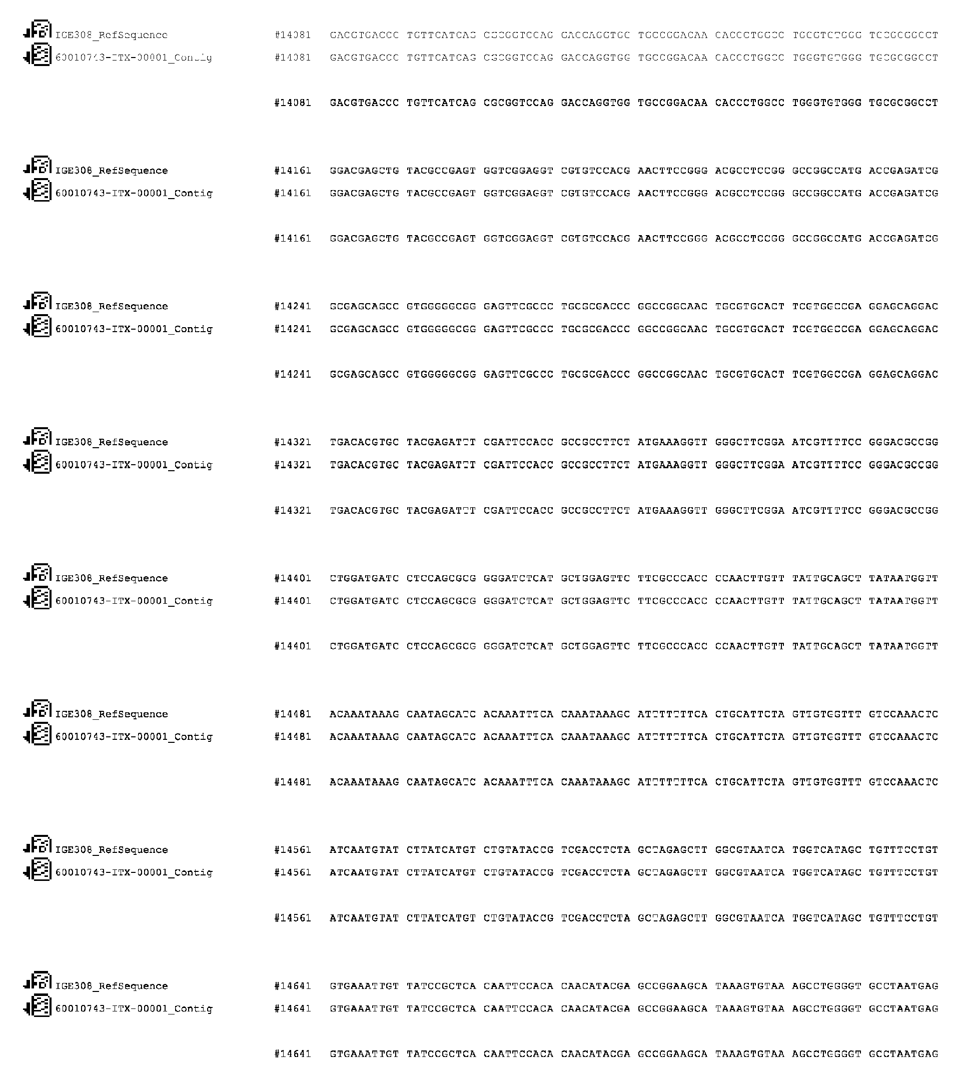

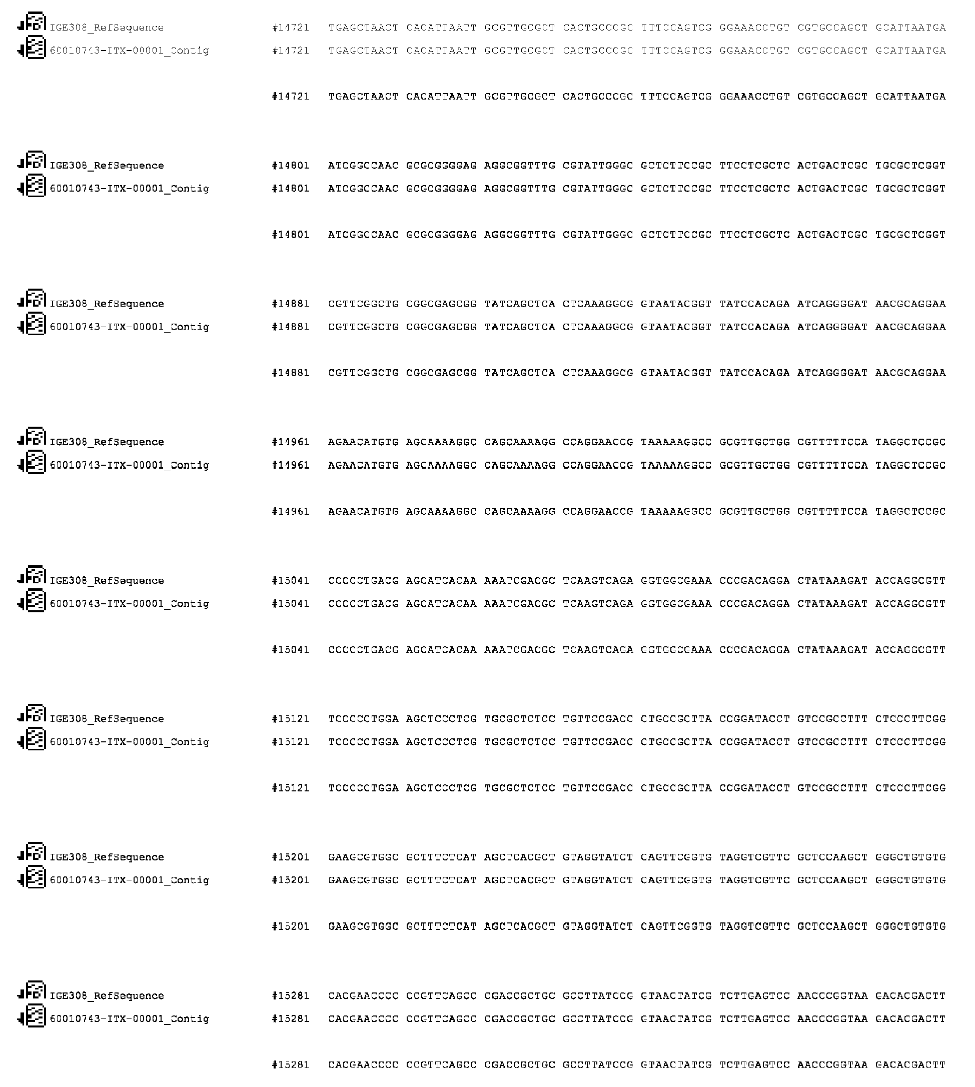

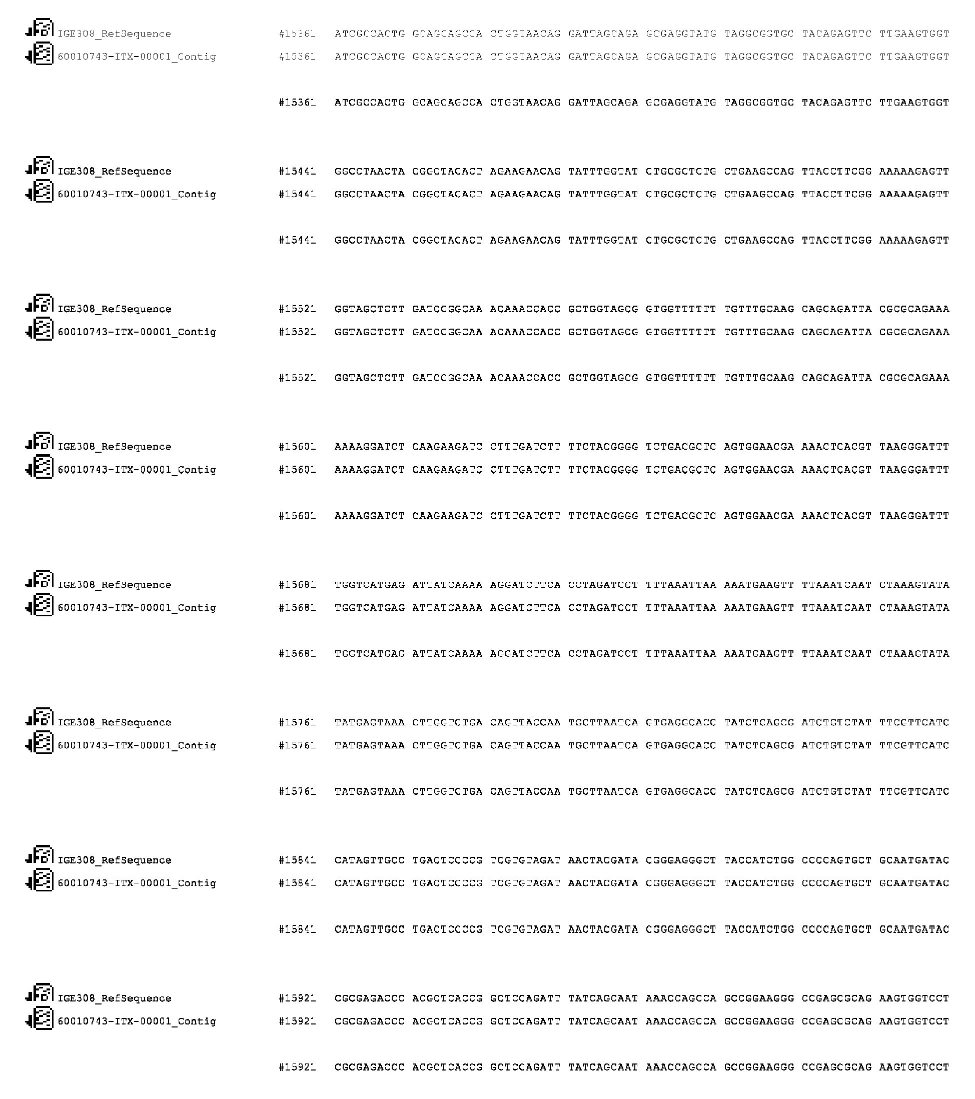

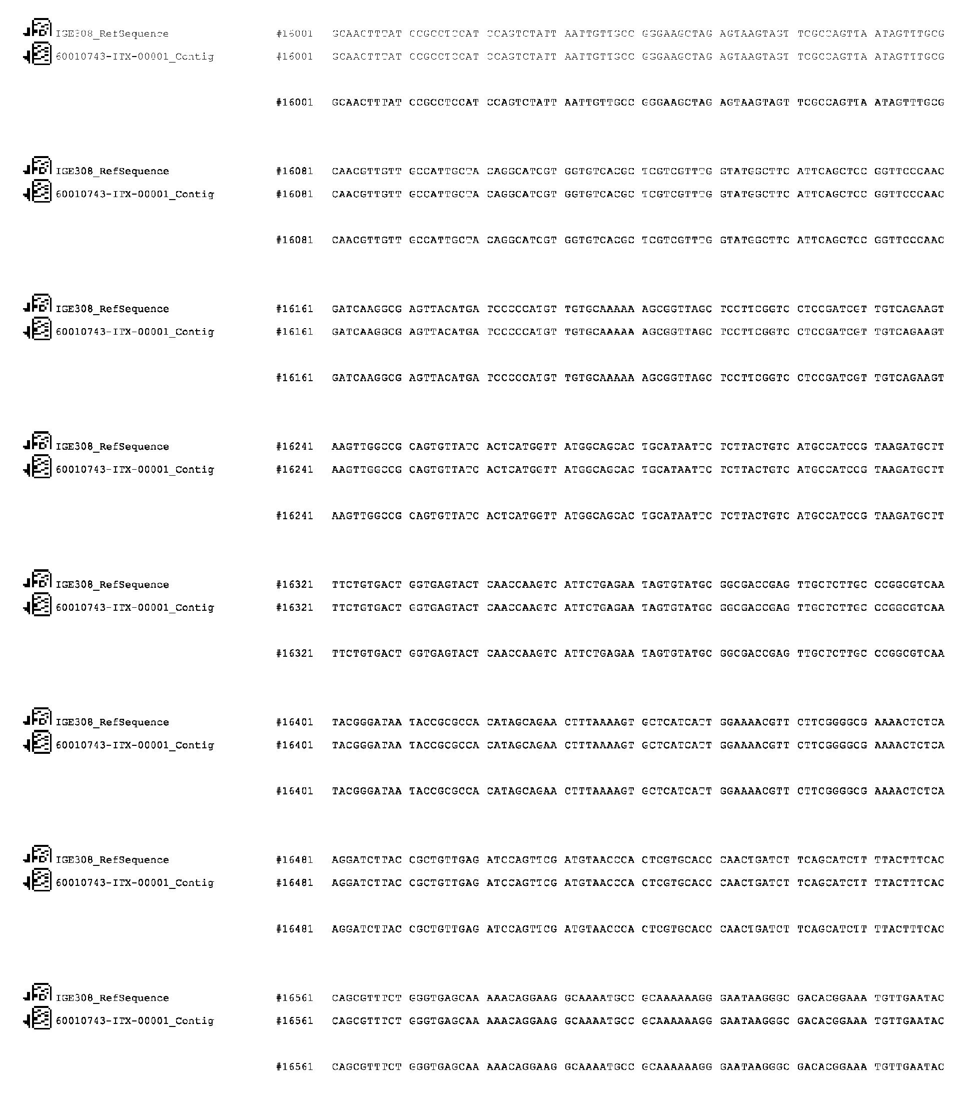

### REFERENCES

A. Aiuti et al. Lentiviral hematopoietic stem cell gene therapy benefits metachromatic leukodystrophy. Science, 2013. August; 341:6148.
Baldeschi C, Gache Y, Rattenholl A, Bouillé P, Danos O, Ortonne JP, Bruckner-Tuderman L, Meneguzzi G. 2003. Genetic correction of canine dystrophic epidermolysis bullosa mediated by retroviral vectors. Human Molecular Genetics 12: 1897-905.
A. Biffi et al. Lentiviral hematopoietic stem cell gene therapy benefits metachromatic leukodystrophy. Science, 2013. August; 341:6148.
Bruckner-Tuderman L, Hopfner B, Hammami-Hauasli N. 1999. Biology of anchoring fibrils: lessons from dystrophic epidermolysis bullosa. Matirx Biology 18: 43-54.
Burgeson R.E. Type VII collagen, anchoring fibrils, and epidermolysis bullosa. J. Invest. Dermatol. 1993;101:252-255.
Chen M, O'Toole EA, Muellenhoff M, Medina E, Kasahara N, Woodley DT. 2000. Development and characterization of a recombinant truncated type VII collagen "minigene". Implication for gene therapy of dystrophic epidermolysis bullosa. J Biol Chem 275: 24429-35.
Chen M, Costa FK, Lindvay CR, Han YP, Woodley DT. 2002a. The recombinant expression of full-length type VII collagen and characterization of molecular mechanisms underlying dystrophic epidermolysis bullosa. J Biol Chem 277: 2118-24.
Chen M, Kasahara N, Keene DR, Chan L, Hoeffler WK, Finlay D, Barcova M, Cannon PM, Mazurek C, Woodley DT. 2002b. Restoration of type VII collagen expression and function in dystrophic epidermolysis bullosa. Nature Genetics 32: 670-5.
Cogan J, Weinstein J, Wang X, Hou Y, Martin S, South AP, Woodley DT, Chen M. 2014. Aminoglycosides restore full-length type VII collagen by overcoming premature termination codons: therapeutic implications for dystrophic epidermolysis bullosa. Molecular Therapy 22: 1741-52.
Deshpande P, Ralston DR, and S McNeil. The use of allodermis prepared from Euro skin bank to prepare autologous tissue engineered skin for clinical use. Burns. 2013. 39(6): 1170-1177.
Goto, M., et al., Fibroblasts show more potential as target cells than keratinocytes in COL7A1 gene therapy of dystrophic epidermolysis bullosa. J Invest Dermatol, 2006. 126(4): p. 766-72.
Greenberg KP, Edwin SL, Schaffer DV, Flannery JG. 2006. Gene Delivery to the Retina Using Lentiviral Vectors. Advances in Experimental Medicine and Biology 572: 255-66
Kim YH, Woodley DT, Wynn KC, Giomi W, Bauer EA. Recessive Dystrophic Epidermylosis Bullosa Phenotype is Preserved in Xenografts Using SCID Mice: Development of an Experimental In Vivo Model. J Invest Dermatol. 1992 Feb;98(2):191-7.
Keene DR, Sakai LY, Lunstrum GP, Morris NP, Burgeson RE. Type VII Collagen Forms an Extended Network of Anchoring Fibrils. J Cell Biol. 1987 Mar;104(3):611-21.
Leigh I.M. Eady R.A. Heagerty A.H. Purkis P.E. Whitehead P.A. Burgeson R.E. Type VII collagen is a normal component of epidermal basement membrane, which shows altered expression in recessive dystrophic epidermolysis bullosa. J. Invest. Dermatol. 1988;90:639-642.
Mavilio F. Pellegrini G. Ferrari S. Di Nunzio F. Di Iorio E. Recchia A. Maruggi G. Ferrari G. Provasi E. Bonini C. Capurro S. Conti A. Magnoni C. Giannetti A. De Luca M. Correction of junctional epidermolysis bullosa by transplantation of genetically modified epidermal stem cells. Nat. Med. 2006;12:1397-1402.
Ortiz-Urda S. Lin Q. Green C.L. Keene D.R. Marinkovich M.P. Khavari P.A. Injection of genetically engineered fibroblasts corrects regenerated human epidermolysis bullosa skin tissue. J. Clin. Invest. 2003;111:251-255.
Remington J. Wang X. Hou Y. Zhou H. Burnett J. Muirhead T. Uitto J. Keene D.R. Woodley D.T. Chen M. Injection of recombinant human type VII collagen corrects the disease phenotype in a murine model of dystrophic epidermolysis bullosa. Mol. Ther. 2009;17:26-33.
Rousselle P, Keene DR, Ruggiero F, Champliaud MF, Rest M, Burgeson RE. 1997. Laminin 5 binds the NC-1 domain of type VII collagen. J Cell Bio, 138: 719-28.
Siprashvili Z, Ngon T. Nguyen, Maria Y. Bezchinsky, M. Peter Marinkovich, Alfred T. Lane, and Paul A. Khavari. Long-term type VII collagen restoration to human epidermylosis bullosa skin tissue. Hum Gene Ther. Oct 2010; 21(10): 1299-1310.
Tareen S, Nicolai C, Campbell D, Flynn P, Slough M, Vin C, Kelly-Clarke B, Odegard J, Robbins S. 2013. A Rev-Independent gag/pol Eliminates Detectable psi-gag Recombination in Lentiviral Vectors. Biores Open Access. 2013 Dec 1; 2(6): 421-430.
Waterman EA, Sakai N, Nguyen NT, Horst BA, Veitch DP, Dey CN, Ortiz-Urda S, Khavari PA, Marinkovich MP. 2007. A laminin-collagen complex drives human epidermal carcinogenesis through phosphoinositol-3-kinase activation. Cancer Research 67: 4264-70.
Woodley D.T. Keene D.R. Atha T. Huang Y. Ram R. Kasahara N. Chen M. Intradermal injection of lentiviral vectors corrects regenerated human dystrophic epidermolysis bullosa skin tissue in vivo. Mol. Ther. 2004;10:318-326.
Woodley D.T. Krueger G.G. Jorgensen C.M. Fairley J.A. Atha T. Huang Y. Clian L. Keene D.R. Chen M. Normal and gene-corrected dystrophic epidermolysis bullosa fibroblasts alone can produce type VII collagen at the basement membrane zone. J. Invest. Dermatol. 2003;121:1021-1028.
Woodley D.T. Keene D.R. Atha T. Huang Y. Lipman K. Li W. Chen M. Injection of recombinant human type VII collagen restores collagen function in dystrophic epidermolysis bullosa. Nat. Med. 2004;10:693-695.

In view of the foregoing, it will be appreciated that the invention described herein inter alia relates to the following items:
1. A method of treating a patient suffering from a type VII collagen (C7) deficiency comprising
   obtaining cells from the dermis or epidermis of the C7-deficient patient,
   contacting said cells with a transducing lentiviral vector comprising a nucleotide sequence encoding COL7A1 or a functional variant thereof to form an autologous genetically modified cell having a vector copy number wherein said lentiviral vector has a transducing vector copy number in the range of 0.1 to 5.0 copies per cell,
   culturing said autologous genetically modified cell,
   and administering an effective amount of the genetically modified cells to the C7-deficient patient.
2. The method of item 1, wherein the cells are selected from fibroblasts and keratinocytes.
3. The method of item 2, wherein the cells are fibroblasts.
4. The method of item 1, wherein the C7-deficiency is dystrophic epidermolysis bullosa.
5. The method of item 4, wherein the dystrophic epidermolysis bullosa is selected from recessive dystrophic epidermolysis bullosa (RDEB) and dominant dystrophic epidermolysis bullosa (DDEB).
6. The method of item 5, wherein the C7-deficiency is RDEB.
7. The method of item 6, wherein the RDEB is a subtype selected from the group consisting of Hallopeau-Siemens, non-Hallopeau-Siemens RDEB, RDEB inversa, pretibial RDEB, acral RDEV, and RDEB centripetalis.
8. The method of item 5, wherein the C7-deficiency is DDEB.
9. The method of item 1, wherein said transducing lentiviral vector is in the form of a lentiviral vector particle.
10. The method of item 9, wherein said transducing lentiviral vector particle is constructed from a transfer lentiviral vector comprising (a) a modified 5' long terminal repeat in LTR, wherein the promoter of the modified 5' LTR is a cytomegalovirus promoter, (b) a functional *COL7A1* gene, (c) at least one lentiviral central polypurine tract, and (d) a modified 3' LTR, wherein the modified 3' LTR comprises a deletion relative to the wild-type 3' LTR, wherein a hepatitis virus post-transcriptional regulatory element (PRE) has been deleted, wherein the *COL7A1* gene or the functional variant thereof is incorporated into the cells to form genetically modified cells having a functional *COL7A1* gene.
11. The method of item 10, wherein there are at least two lentiviral central polypurine tract elements.
12. The method of item 10, wherein the deleted PRE is a woodchuck hepatitis virus post-transcriptional regulatory element (WPRE).
13. The method of item 9, wherein transducing lentiviral vector particle is constructed from a lentiviral expression plasmid vector selected from the group consisting of pSMPUW and pFUGW.
14. The method of item 13, wherein said lentiviral expression plasmid vector is pFUGW.
15. The method of item 9, wherein said transducing lentiviral vector particle is INXN-2004.
16. The method of item 9, wherein said transducing lentiviral vector particle is INXN-2002.
17. The method of item 1, wherein the genetically modified fibroblasts are administered to the patient by injection, topically, orally, or embedded in a biocompatible matrix.
18. The method of item 17, wherein the genetically modified fibroblasts are administered by injection.
19. The method of item 18, wherein the genetically modified cells are intradermally injected into the patient.
20. The method of item 17, wherein the genetically modified fibroblasts are encapsulated in a polymer capsule.
21. The method of item 17, wherein the genetically modified fibroblasts are embedded in a collagen matrix.
22. The method of item 17, wherein the genetically modified fibroblasts are embedded in a hydrogel or mesh.
23. An autologous genetically modified fibroblast from a patient suffering from a Type VII collagen deficiency transduced with a lentiviral vector particle comprising a functional *COL7A1* gene or a functional variant thereof, and expresses type VII collagen, wherein said lentiviral vector particle has a transducing vector copy number in the range of 0.1 to 5.0 copies per cell.
24. An autologous genetically modified fibroblast from a patient suffering from a recessive dystrophic epidermolysis bullosa transduced with a lentiviral vector particle comprising a functional *COL7A1* gene or a functional variant thereof, and expresses type VII collagen, wherein said lentiviral vector particle has a transducing vector copy number in the range of 0.1 to 5.0 copies per cell.
25. An autologous genetically modified fibroblast from a patient suffering from a dominant dystrophic epidermolysis bullosa transduced with a lentiviral vector particle comprising a functional *COL7A1* gene or a functional variant thereof, and expresses type VII collagen, wherein said lentiviral vector particle has a transducing vector copy number in the range of 0.1 to 5.0 copies per cell.
26. A self-inactivating lentiviral vector formed from a transfer vector comprising (a) a modified 5' long terminal in LTR, wherein the promoter of the modified 5' LTR is a cytomegalovirus promoter, (b) the *COL7A1* gene or a functional variant thereof, (c) at least one lentiviral central polypurine tract element, and (d) a modified 3' LTR, wherein the modified 3' LTR comprises a deletion relative to the wild-type 3' LTR, and, wherein a hepatitis virus post-transcriptional regulatory element (PRE) has been deleted.
27. The vector of item 26, wherein the vector comprises at least two lentiviral central polypurine tract elements.
28. The vector of item 26, wherein the deleted PRE is a woodchuck hepatitis virus post-transcriptional regulatory element (WPRE).
29. The vector of item 26, wherein the vector comprises (a) a modified 5' long terminal in LTR, wherein the promoter of the modified 5' LTR is a cytomegalovirus promoter, (b) the *COL7A1* gene, (c) two lentiviral central polypurine tract elements, (d) further comprising a WPRE, and (e) a modified 3' LTR, wherein the modified 3' LTR comprises a deletion relative to the wild-type 3' LTR.
30. The vector of item 26, wherein the vector is designated INXN-2004.
31. The vector of item 26, wherein the vector is designated INXN-2002.
32. A transfer vector designated IGE-308.
33. A lentiviral vector particle designated INXN-2004.
34. A lentiviral vector particle designated INXN-2002.
35. A stable virus packaging cell line producing the INXN-2002 lentiviral vector particle.
36. A stable virus packaging cell line producing the INXN-2004 lentiviral vector particle.
37. A pharmaceutical composition comprising a fibroblast obtained from a patient suffering from RDEB transduced with a lentiviral vector designated INXN-2004 having the sequence of IGE-308.
38. A pharmaceutical composition comprising a fibroblast obtained from a patient suffering from RDEB transduced with a lentiviral vector designated INXN-2002.
39. A pharmaceutical composition comprising a fibroblast obtained from a patient suffering from DDEB transduced with a lentiviral vector designated INXN-2004 having the sequence of IGE-308.
40. A pharmaceutical composition comprising a fibroblast obtained from a patient suffering from DDEB transduced with a lentiviral vector designated INXN-2002.
41. A cell transduced in vitro or ex vivo with the vector of item 33.
42. A cell transduced in vitro or ex vivo with the vector of item 34.
43. A method of treating a patient suffering from pseudosyndactyly comprising administering to said patient an autologous population of cells obtained from said patient transduced with a lentiviral vector particle comprising a functional *COL7A1* gene or a functional variant thereof, and expressing type VII collagen, wherein said lentiviral vector particle has a transducing vector copy number in the range of 0.1 to 5.0 copies per cell.
44. The method of item 43, wherein the autologous population of cells is selected from keratinocytes and fibroblasts.
45. The method of item 43, wherein the autologous population of cells is fibroblasts.
46. The method of item 43, wherein administration of said cells to the patient is by injection, topically, orally, or embedded in a biocompatible matrix.
47. A method of treating, inhibiting or reducing the blistering of a dystrophic epidermolysis bullosa (DEB) patient comprising administering to said patient an autologous population of cells obtained from said patient transduced with a lentiviral vector particle comprising a functional *COL7A1* gene or a functional variant thereof, and expressing type VII collagen, wherein said lentiviral vector particle has a transducing vector copy number in the range of 0.1 to 5.0 copies per cell.
48. The method of item 47, wherein the autologous population of cells is selected from keratinocytes and fibroblasts.
49. The method of item 48, wherein the autologous population of cells is fibroblasts.
50. The method of item 47, wherein administration of said cells to the patient is by injection, topically, orally, or embedded in a biocompatible matrix.
51. The method of item 47, wherein the patient suffers from recessive dystrophic epidermolysis bullosa.
52. The method of item 47, wherein the patient suffers from dominant dystrophic epidermolysis bullosa.
53. A method of treating lesions in patients suffering from recessive dystrophic epidermolysis bullosa comprising administering to said patient an autologous population of cells obtained from said patient transduced with a lentiviral vector particle comprising a functional *COL7A1* gene or a functional variant thereof, and expressing type VII collagen, wherein said lentiviral vector particle has a transducing vector copy number in the range of 0.1 to 5.0 copies per cell.
54. The method of item 53, wherein the autologous population of cells is selected from keratinocytes and fibroblasts.
55. The method of item 53, wherein the autologous population of cells is fibroblasts.
56. The method of item 53, wherein administration of said cells to the patient is by injection, topically, orally, or embedded in a biocompatible matrix.
57. The method of item 53, wherein the lesions are present in oral mucosa.
58. The method of item 53, wherein the lesions are present in the gastrointestinal tract.
59. An isolated population of genetically modified fibroblasts autologous to a patient suffering from a Type VII collagen deficiency transduced with a lentiviral vector particle comprising a functional *COL7A1* gene or a functional variant thereof, and expresses type VII collagen, wherein said lentiviral vector particle has a transducing vector copy number in the range of 0.1 to 5.0 copies per cell.
60. The population of item 59, wherein the lentiviral vector particle is INXN-2002.
61. The population of item 59, wherein the lentiviral vector particle is INXN-2004.
62. An isolated population of genetically modified fibroblast autologous to a patient suffering from a recessive dystrophic epidermolysis bullosa transduced with a lentiviral vector particle comprising a functional *COL7A1* gene or a functional variant thereof, and expresses type VII collagen, wherein said lentiviral vector particle has a transducing vector copy number in the range of 0.1 to 5.0 copies per cell.
63. The population of item 62, wherein the lentiviral vector particle is INXN-2002.
64. The population of item 62, wherein the lentiviral vector particle is INXN-2004.
65. An isolated population of genetically modified fibroblasts autologous to a patient suffering from a dominant dystrophic epidermolysis bullase transduced with a lentiviral vector particle comprising a functional *COL7A1* gene or a functional variant thereof, and expresses type VII collagen, wherein said lentiviral vector particle has a transducing vector copy number in the range of 0.1 to 5.0 copies per cell.
66. The population of item 65, wherein the lentiviral vector particle is INXN-2002.
67. The population of item 65, wherein the lentiviral vector particle is INXN-2004.
68. A method of making an isolated population of Type VII collagen (C7) expressing genetically modified cells autologous to a patient suffering from C7 deficiency comprising harvesting cells from the dermis or epidermis of the C7-deficient patient, contacting said cells with a transducing lentiviral vector particle comprising the *COL7A1* gene or a functional variant thereof to form an autologous genetically modified cell comprising the *COL7A1* gene having a vector copy number of wherein said lentiviral vector particle has a transducing vector copy number in the range of 0.1 to 5.0 copies per cell, and culturing said autologous genetically modified cell to obtain an isolated population of C7 expressing genetically modified cells.
69. The method of item 68, wherein the cells are selected from fibroblasts and keratinocytes.
70. The method of item 69, wherein the cells are fibroblasts.
71. The method of item 68, wherein the C7-deficiency is dystrophic epidermolysis bullosa.
72. The method of item 71, wherein the dystrophic epidermolysis bullosa is selected from recessive dystrophic epidermolysis bullosa (RDEB) and dominant dystrophic epidermolysis bullosa (DDEB).
73. The method of item 71, wherein the C7-deficiency is RDEB.
74. The method of item 73, wherein the RDEB is a subtype selected from the group consisting of Hallopeau-Siemens, non-Hallopeau-Siemens RDEB, RDEB inversa, pretibial RDEB, acral RDEV, and RDEB centripetalis.
75. The method of item 71, wherein the C7-deficiency is DDEB.
76. The method of item 68, wherein the transducing lentiviral vector particle is INXN-2002.
77. The method of item 68, wherein the transducing lentiviral vector particle is INXN-2004.
78. An isolated population of Type VII collagen (C7)-expressing genetically modified cells produced by the method of item 68.
79. An isolated population of Type VII collagen (C7)-expressing genetically modified cells produced by the method of item 76.
80. An isolated population of Type VII collagen (C7)-expressing genetically modified cells produced by the method of item 77.
81. A pharmaceutical formulation comprising the isolated population of item 78.
82. A pharmaceutical formulation comprising the isolated population of item 79.
83. A pharmaceutical formulation comprising the isolated population of item 80.
84. A method of increasing the integrated transgene copy number per cell in genetically modified human dermal fibroblasts or keratinocytes comprising contacting a transducing lentiviral vector comprising a nucleotide sequence encoding a *COL7A1* gene or a functional variant thereof with a human dermal fibroblast or keratinocyte obtained from a C7-deficient patient to form a transduction composition, and subjecting said transduction composition to spinoculation to form transduced human dermal fibroblast or keratinocyte, wherein the integrated transgene copy number of the transduced human dermal fibroblasts or keratinocytes is higher relative to a transduction composition not subjected to spinoculation.
85. The method of item 84, wherein the transduced human dermal fibroblasts are contacted to a second transducing lentiviral vector to form a second tranduscrion composition.
86. The method of item 85, wherein the second transduction composition is subjected to spinoculation.
87. The method of item 84, wherein the transduced human dermal fibroblasts have at least a 1-fold greater integrated transgene copy number per cell relative to a transduction composition not subjected to spinoculation.
88. The method of item 84, wherein the transduced human dermal fibroblasts have at least a 2-fold greater integrated transgene copy number per cell relative to a transduction composition not subjected to spinoculation.
88. The method of item 86, wherein the transduced human dermal fibroblasts have at least a 5-fold greater integrated transgene copy number per cell relative to a transduction composition not subjected to spinoculation or a second transduction.
88. The method of item 86, wherein the transduced human dermal fibroblasts have at least a 10-fold greater integrated transgene copy number per cell relative to a transduction composition not subjected to spinoculation or a second transduction.
88. The method of item 86, wherein the transduced human dermal fibroblasts have at least a 20-fold greater integrated transgene copy number per cell relative to a transduction composition not subjected to spinoculation or a second transduction.
88. The method of item 86, wherein the transduced human dermal fibroblasts have at least a 27-fold greater integrated transgene copy number per cell relative to a transduction composition not subjected to spinoculation or a second transduction.
89. The method of item 84, wherein the transduced human dermal fibroblasts have an integrated transgene copy number per cell of at least 0.05.
90. The method of item 84, wherein the transduced human dermal fibroblasts have an integrated transgene copy number per cell of at least 0.09.
91. The method of item 85, wherein the transduced human dermal fibroblasts have an integrated transgene copy number per cell of at least 0.41.
92. The method of item 86, wherein the transduced human dermal fibroblasts have an integrated transgene copy number per cell of at least 0.74.
93. The method of item 84, wherein the transduced human dermal fibroblasts have an integrated transgene copy number per cell of at between about 0.1 to about 5.
94. The method of item 84, wherein the transduced human dermal fibroblasts have an integrated transgene copy number per cell of between about 0.1 to about 1.
95. The method of item 84, wherein the transduced human dermal fibroblasts have an integrated transgene copy number per cell of between about 0.4 to about 1.
96. The method of item 84, wherein the transduced human dermal fibroblasts have an integrated transgene copy number per cell of between about 0.4 to about 0.75 .
100. The method of item 84, wherein the transducing lentiviral vector is in the form of a lentiviral vector particle.
101. The methd of item 100, wherein the transducing lentiviral vector particle is INXN-2002.
102. The method of item 100, wherein the transducing lentiviral vector particle is INXN-2004.
103. The method of item 86, wherein the transduced human dermal fibroblasts or keratinocytes have an increased expression of C7 relative to transduced human dermal fibroblasts or keratinocytes not subjected to spinoculation or a second transduction.
104. The method of item 86, wherein the transduced human dermal fibroblasts or keratinocytes have at least a 100-fold increased expression of C7 relative to transduced human dermal fibroblasts or keratinocytes not subjected to spinoculation or a second transduction.
105. The method of item 86, wherein the transduced human dermal fibroblasts or keratinocytes have at least a 200-fold increased expression relative to transduced human dermal fibroblasts or keratinocytes not subjected to spinoculation or a second transduction.
106. A method of increasing the integrated transgene copy number per cell in genetically modified human dermal fibroblasts or keratinocytes comprising
   (a) contacting a transducing lentiviral vector comprising a nucleotide sequence encoding a *COL7A1* gene or a functional variant thereof with a human dermal fibroblast
      or keratinocyte obtained from a C7-deficient patient to form a first transduction composition,
   (b) subjecting said first transduction composition to spinoculation to form transduced human dermal fibroblast or keratinocyte,
   (c) contacting the transduced human dermal fibroblasts or keratinocytes of step (b) with a second transducing lentiviral vector to form a second transduction composition;
   (d) subjecting said first transduction composition to spinoculation to form transduced human dermal fibroblast or keratinocyte,
   wherein the integrated transgene copy number of the transduced human dermal fibroblasts or keratinocytes is at least 5, 10, 15, 20, 25, 27, 28, 29, 30, 35, 40, 45, or 50-fold higher relative to transduced human dermal fibroblasts or keratinocytes not subjected to spinoculation or a second transduction step.
107. The method of item 106, wherein the first and second tranducing lentiviral vector are INXN-2004.
108. The method of item 106, wherein the cells are human dermal fibroblasts.
109. The method of item 106, wherein the integrated transgene copy number of the transduced human dermal fibroblasts or keratinocytes is at least 5-fold higher relative to transduced human dermal fibroblasts or keratinocytes not subjected to spinoculation or a second transduction step.
110. The method of item 106, wherein the integrated transgene copy number of the transduced human dermal fibroblasts or keratinocytes is at least 10-fold higher relative to transduced human dermal fibroblasts or keratinocytes not subjected to spinoculation or a second transduction step.
111. The method of item 106, wherein the integrated transgene copy number of the transduced human dermal fibroblasts or keratinocytes is at least 27-fold higher relative to transduced human dermal fibroblasts or keratinocytes not subjected to spinoculation or a second transduction step.
112. The method of item 106, wherein the integrated transgene copy number of the transduced human dermal fibroblasts or keratinocytes is about 5-fold higher relative to transduced human dermal fibroblasts or keratinocytes not subjected to spinoculation or a second transduction step.
113. The method of item 106, wherein the integrated transgene copy number of the transduced human dermal fibroblasts or keratinocytes is about 10-fold higher relative to transduced human dermal fibroblasts or keratinocytes not subjected to spinoculation or a second transduction step.
114. The method of item 106, wherein the integrated transgene copy number of the transduced human dermal fibroblasts or keratinocytes is about 27-fold higher relative to transduced human dermal fibroblasts or keratinocytes not subjected to spinoculation or a second transduction step.

## Claims

1. An autologous genetically modified fibroblast from a patient suffering from a dystrophic epidermolysis bullosa (DEB) or pseudosyndactyly transduced with a lentiviral vector, wherein the lentiviral vector is replication defective and self-inactivating and is derived from the human immunodeficiency virus (HIV-1) which is pseudotyped with a heterologous VSV-G envelope protein instead of the HIV-1 envelope protein, comprising a nucleotide sequence encoding a functional *COL7A1* gene or a functional variant thereof, and expressing type VII collagen, wherein transduction of an autologous population of fibroblast cells with the VSV-G pseudotyped lentiviral vector results in a transducing vector copy number in the range of 0.1 to 5.0 copies per cell, wherein the transducing lentiviral vector comprises (a) a modified 5' long terminal repeat in LTR, wherein the promoter of the modified 5' LTR is a cytomegalovirus promoter, (b) the functional *COL7A1* gene or functional variant thereof, (c) at least one lentiviral central polypurine tract element, and (d) a modified 3' LTR, wherein the modified 3' LTR lacks a post-transcriptional regulatory element (PRE), wherein the functional *COL7A1* gene or functional variant thereof is incorporated into the fibroblast to form a genetically modified fibroblast having the functional *COL7A1* gene or functional variant thereof.

2. The autologous genetically modified fibroblast of claim 1, wherein the DEB is recessive dystrophic epidermolysis bullosa (RDEB).

3. The autologous genetically modified fibroblast of claim 2, wherein the RDEB is Hallopeau-Siemens RDEB.

4. The autologous genetically modified fibroblast of claim 2, wherein the RDEB is non-Hallopeau-Siemens RDEB.

5. The autologous genetically modified fibroblast of claim 2, wherein the RDEB is RDEB inversa.

6. The autologous genetically modified fibroblast of claim 2, wherein the RDEB is pretibial RDEB.

7. The autologous genetically modified fibroblast of claim 2, wherein the RDEB is acral RDEB.

8. The autologous genetically modified fibroblast of claim 2, wherein the RDEB is RDEB centripetalis.

9. The autologous genetically modified fibroblast of claim 1, wherein the DEB is dominant dystrophic epidermolysis bullosa (DDEB).

10. The autologous genetically modified fibroblast of any preceding claim, wherein the genetically modified fibroblast is embedded in a biocompatible matrix.

11. A pharmaceutical formulation comprising the autologous genetically modified fibroblast of any preceding claim.

## Patentansprüche

1. Autologer, genetisch veränderter Fibroblast eines Patienten mit dystrophischer Epidermolysis bullosa (DEB) oder Pseudosyndaktylie, transduziert mit einem lentiviralen Vektor, wobei der lentivirale Vektor replikationsdefekt und selbstinaktivierend ist und vom humanen Immundefizienz-Virus (HIV-1) stammt, das anstelle des HIV-1-Hüllproteins mit einem heterologen VSV-G-Hüllprotein pseudotypisiert ist, umfassend eine Nukleotidsequenz, die für ein funktionelles *COL7A1*-Gen oder eine funktionelle Variante davon kodiert und Kollagen Typ VII exprimiert, wobei die Transduktion einer autologen Fibroblastenpopulation mit dem VSV-G-pseudotypisierten lentiviralen Vektor zu einer Kopienanzahl von transduzierenden Vektoren im Bereich von 0,1 bis 5,0 Kopien pro Zelle führt, wobei der transduzierende lentivirale Vektor (a) ein modifiziertes 5'-LTR (Long Terminal Repeat) umfasst, wobei der Promotor des modifizierten 5'-LTR ein Cytomegalievirus-Promotor ist, (b) das funktionelle *COL7A1*-Gen oder eine funktionelle Variante davon umfasst, (c) mindestens ein lentivirales zentrales Polypurin-Trakt-Element umfasst und (d) ein modifiziertes 3'-LTR umfasst, wobei dem modifizierten 3'-LTR ein posttranskriptionelles regulatorisches Element (PRE) fehlt, wobei das funktionelle *COL7A1*-Gen oder die funktionelle Variante davon in den Fibroblasten eingebaut ist, um einen genetisch veränderten Fibroblasten zu bilden, der das funktionelle *COL7A1*-Gen oder eine funktionelle Variante davon aufweist.

2. Autologer, genetisch veränderter Fibroblast nach Anspruch 1, wobei es sich bei der DEB um eine rezessive dystrophische Epidermolysis bullosa (RDEB) handelt.

3. Autologer, genetisch veränderter Fibroblast nach Anspruch 2, wobei es sich bei der RDEB um Hallopeau-Siemens RDEB handelt.

4. Autologer, genetisch veränderter Fibroblast nach Anspruch 2, wobei es sich bei der RDEB um Nicht-Hallopeau-Siemens-RDEB handelt.

5. Autologer, genetisch veränderter Fibroblast nach Anspruch 2, wobei es sich bei der RDEB um RDEB inversa handelt.

6. Autologer, genetisch veränderter Fibroblast nach Anspruch 2, wobei es sich bei der RDEB um ein prätibiale RDEB handelt.

7. Autologer, genetisch veränderter Fibroblast nach Anspruch 2, wobei es sich bei der RDEB um akrale RDEB handelt.

8. Autologer, genetisch veränderter Fibroblast nach Anspruch 2, wobei es sich bei der RDEB um RDEB centripetalis handelt.

9. Autologer, genetisch veränderter Fibroblast nach Anspruch 1, wobei es sich bei der DEB um eine dominante dystrophische Epidermolysis bullosa (DDEB) handelt.

10. Autologer, genetisch veränderter Fibroblast nach einem der vorherigen Ansprüche, wobei der genetisch veränderte Fibroblast in eine biokompatible Matrix eingebettet ist.

11. Pharmazeutische Formulierung, die den autologen, genetisch veränderten Fibroblasten nach einem der vorherigen Ansprüche umfasst.

## Revendications

1. Fibroblaste autologue génétiquement modifié provenant d'un patient atteint d'épidermolyse bulleuse dystrophique (EBD) ou de pseudo-syndactylie, transduit à l'aide d'un vecteur lentiviral, dans lequel le vecteur lentiviral est défectueux en réplication et s'auto-inactive, et est dérivé du virus de l'immunodéficience humaine (VIH-1) qui est pseudotypé avec une protéine d'enveloppe VSV-G hétérologue à la place de la protéine d'enveloppe du VIH-1, comprenant une séquence nucléotidique codant pour un gène *COL7A1* fonctionnel ou un variant fonctionnel de celui-ci, et exprimant le collagène de type VII, dans lequel la transduction d'une population autologue de cellules fibroblastiques avec le vecteur lentiviral pseudotypé VSV -G aboutit à un nombre de copies du vecteur transducteur dans un intervalle compris entre 0,1 et 5,0 copies par cellule, dans lequel le vecteur lentiviral transducteur comprend (a) une répétition terminale longue 5' modifiée dans le LTR, dans lequel le promoteur du LTR 5' modifié est un promoteur du cytomégalovirus, (b) le gène *COL7A1* fonctionnel ou un variant fonctionnel de celui-ci, (c) au moins un élément de tractus central polypurique lentiviral, et (d) un LTR 3' modifié, dans lequel le LTR 3' modifié est dépourvu d'un élément régulateur post-transcriptionnel (PRE), dans lequel le gène *COL7A1* fonctionnel ou un variant fonctionnel de celui-ci est incorporé dans le fibroblaste pour former un fibroblaste génétiquement modifié possédant le gène *COL7A1* fonctionnel ou un variant fonctionnel de celui-ci.

2. Fibroblaste autologue génétiquement modifié selon la revendication 1, dans lequel la EBD est une épidermolyse bulleuse dystrophique récessive (EBDR).

3. Fibroblaste autologue génétiquement modifié selon la revendication 2, dans lequel la EBDR est une EBDR de type Hallopeau-Siemens.

4. Fibroblaste autologue génétiquement modifié selon la revendication 2, dans lequel la EBDR est une EBDR de type non-Hallopeau-Siemens.

5. Fibroblaste autologue génétiquement modifié selon la revendication 2, dans lequel la EBDR est une EBDR inverse.

6. Fibroblaste autologue génétiquement modifié selon la revendication 2, dans lequel l'épidermolyse bulleuse récessive (EBDR) est une EBDR pré-tibiale.

7. Fibroblaste autologue génétiquement modifié selon la revendication 2, dans lequel l'EBDR est une EBDR acrale.

8. Fibroblaste autologue génétiquement modifié selon la revendication 2, dans lequel l'EBDR est une EBDR centripète.

9. Fibroblaste autologue génétiquement modifié selon la revendication 1, dans lequel l'EBD est une épidermolyse bulleuse dystrophique dominante (EBDD).

10. Fibroblaste autologue génétiquement modifié selon l'une quelconque des revendications précédentes, dans lequel le fibroblaste génétiquement modifié est incorporé dans une matrice biocompatible.

11. Formulation pharmaceutique comprenant le fibroblaste autologue génétiquement modifié selon l'une quelconque des revendications précédentes.
